# EUROPEAN PATENT SPECIFICATION

(11) **EP 4 558 489 B1**
(45) Date of publication and mention of the grant of the patent: **08.07.2026**
(21) Application number: 23742097.1
(22) Date of filing: 18.07.2023
(51) Int. Cl.: C07D 401/04, C07D 401/14, C07D 403/14, C07D 413/14, C07D 487/04, C07D 207/08, A61K 31/4164, A61K 31/42, A61K 31/44, A61K 31/415

(54) **HETEROARYL DERIVATIVES AS DDRS INHIBITORS**
HETEROARYLDERIVATE ALS DDRS-INHIBITOREN
DÉRIVÉS HÉTÉROARYLES EN TANT QU'INHIBITEURS DE DDRS

(30) Priority: 19.07.2022 EP 22185860
(43) Date of publication of application: 28.05.2025
(73) Proprietor: Chiesi Farmaceutici S.p.A., 43122 Parma (IT)
(72) Inventor: MAZZUCATO, Roberta, 43122 Parma (IT); IOTTI, Nicolò, 43122 Parma (IT); CARZANIGA, Laura, 43122 Parma (IT); RIZZI, Andrea, 43122 Parma (IT); GHIDINI, Eleonora, 43122 Parma (IT); LEVANTO, Stefano, 43122 Parma (IT); WHITTAKER, Benjamin Paul, 43122 Parma (IT); CLARK, David Edward, 43122 Parma (IT); RONCHI, Paolo, 43122 Parma (IT)
(74) Representative: Chiesi Farmaceutici S.p.A.
(86) International application number: PCT/EP2023/069885
(87) International publication number: WO 2024/017876

(56) References cited:
- WO-A1-2008/151183
- YOSHIMORI ATSUSHI ET AL: "Design and Synthesis of DDR1 Inhibitors with a Desired Pharmacophore Using Deep Generative Models", vol. 16, no. 6, 1 December 2020 (2020-12-01), DE, pages 955 - 958, XP055867413, ISSN: 1860-7179, Retrieved from the Internet <URL:https://onlinelibrary.wiley.com/doi/full-xml/10.1002/cmdc.202000786> DOI: 10.1002/cmdc.202000786

## Description

### FIELD OF THE INVENTION

The present invention relates to compounds inhibiting Discoidin Domain Receptors (DDR inhibitors), methods of preparing such compounds, intermediate compounds useful in such preparations, pharmaceutical compositions containing them and therapeutic use thereof.

The compounds of the invention may be useful for instance in the treatment of many disorders associated with DDR mechanisms.

### BACKGROUND OF THE INVENTION

Discoidin Domain Receptors (DDRs) are type I transmembrane receptor tyrosine kinase (RTKs). The DDR family comprises two distinct members, DDR1 and DDR2.

DDRs are unique receptors among the other members of the RTK superfamily, in that DDRs are activated by collagen whereas other members of the RTK superfamily are typically activated by soluble peptide-like growth factors (see Vogel, W. (1997) Mol. Cell 1, 13-23; Shrivastava A. Mol Cell. 1997; 1:25-34.). Moreover, DDRs are unusual RTKs also because they form ligand-independent stable dimers that are non-covalently linked (see Noordeen, N. A. (2006) J. Biol. Chem. 281, 22744-22751; Mihai C. J Mol Biol. 2009; 385:432-445).

The DDR1 subfamily is composed of five membrane-anchored isoforms, and the DDR2 subfamily is represented by a single protein. The five DDR1 isoforms all have in common the extracellular and transmembrane domains but differ in the cytoplasmic region (see Valiathan, R. R. (2012) Cancer Metastasis Rev. 31, 295-321, Alves, F. (2001) FASEB J. 15, 1321-1323).

DDR receptor family has been found involved in a series of fibrotic diseases, such as pulmonary fibrosis, and in particular idiopathic pulmonary fibrosis (IDF). The first evidence for a protective role of DDR1 deletion in lung fibrosis was generated in 2006 by the research group of Dr. Vogel (see Avivi-Green C, Am J Respir Crit Care Med 2006;174:420-427). The authors demonstrated that DDR1-null mice were largely protected against bleomycin (BLM)-induced injury. Furthermore, myofibroblast expansion and apoptosis were much lower in these animals compared with their wild-type counterparts. Absence of inflammation in knockout mice was confirmed by lavage cell count and cytokines ELISA. These results indicated that DDR1 expression is a prerequisite for the development of lung inflammation and fibrosis.

DDR2 deficiency or downregulation reduces bleomycin-induced lung fibrosis (see Zhao H, Bian H, Bu X, Zhang S, Zhang P, Yu J, et al Mol Ther 2016; 24:1734-1744). Zhao et al, demonstrated that DDR2 plays a critical role in the induction of fibrosis and angiogenesis in the lung, in particular that DDR2 synergizes with transforming growth factor (TGF)-β to induce myofibroblast differentiation. Furthermore, they showed that treatment of injured mice with specific siRNA against DDR2 exhibited therapeutic efficacy against lung fibrosis. In a second publication, Jia et al showed that mice lacking DDR2 are protected from bleomycin-induced lung fibrosis (see Jia S, Am J Respir Cell Mol Biol 2018;59:295-305). In addition, DDR2-null fibroblasts are significantly more prone to apoptosis than wild-type fibroblasts, supporting a paradigm in which fibroblast resistance to apoptosis is critical for progression of fibrosis.

Some compounds have been described in the literature as DDR1 or DDR2 antagonists.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Of note, antagonizing the DDR receptors may be useful for the treatment of fibrosis and disease, disorder and conditions that result from fibrosis. Even more, antagonizing both receptors DDR1 and DDR2 may be particularly efficacious in the treatment of the above-mentioned disease, disorder and conditions.

Several efforts have been done in the past years to develop novel DDR1 and DDR2 receptor antagonists useful for the treatment of several diseases and some of those compounds have shown efficacy also in humans. However, there remains a potential for developing selective inhibitors of both receptors DDR1 and DDR2 useful for the treatment of diseases, disorders or conditions associated with a dysregulation of DDR receptors, in the respiratory field, in particular idiopathic pulmonary fibrosis (IPF), to be administered by the inhalation route and characterized by a good inhalatory profile, that corresponds to a good activity in the lung, a good lung retention and to a low metabolic stability in order to minimize the systemic exposure and correlated safety issues.

In this direction, a new series of compounds of general formula (I), as herein below reported, has been surprisingly found, which solves the problem of providing inhibitors for receptors DDR1 and DDR2 for administration by inhalation, which act as selective inhibitors of DDR1 and DDR2 receptors with respect to other human protein kinases. Such compounds show high potency, good inhalatory profile, low metabolic stability, low systemic exposure, improved safety and tolerability.

### SUMMARY OF THE INVENTION

In a first aspect the present invention relates to a compound of formula (I) 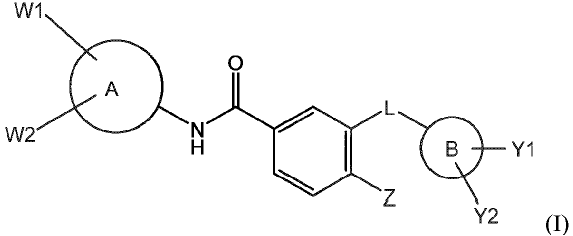 wherein
A is a ring selected from the group consisting of: 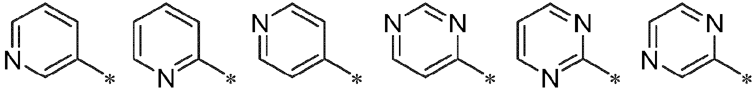 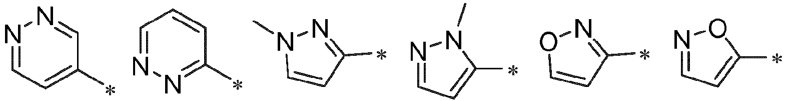 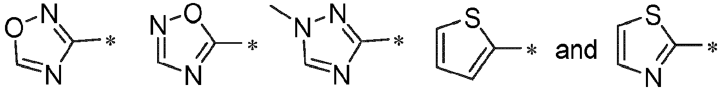 wherein 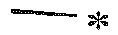 indicates a direct bond to NH;
W1 and W2 are substituents of ring A selected from the group consisting of hydrogen, halogen, (C₁-C₄)alkyl, (C₁-C₄)haloalkyl, (C₁-C₄)alkoxy, (C₁-C₄)haloalkoxy, R1R2N-(C₁-C₄)alkyl and (C₃-C₆)cycloalkyl, preferably selected from H, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ and cyclopropyl;
Z is selected from the group consisting of (C₁-C₄)alkyl, (C₁-C₄)haloalkyl, (C₁-C₄)alkoxy, (C₁-C₄)haloalkoxy, (C₁-C₄)hydroxyalkyl, hydroxy and (C₃-C₆)cycloalkyl, preferably selected from CH₃, CF₂H, OCH₃, CH₂OH and cyclopropyl;
L is selected from the group consisting of 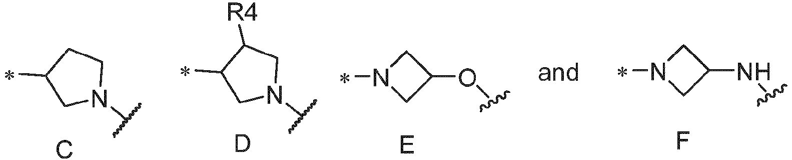 wherein 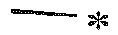 indicates a direct bond to the phenyl ring and 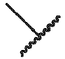 indicates a direct bond to B;
B is mono- or bi-cyclic heteroaryl ring, preferably selected from the group consisting of pyridinyl, pyrimidinyl, pyrazinyl, pyrazolo[1,5-a]pyrazinyl, 1H-pyrazolo[3,4-b]pyridinyl, pyrazolo[1,5-a]pyridinyl, pyrazolo[1,5-a]pyrimidinyl, imidazo[1,2-b]pyridazinyl, imidazo[1,2-a]pyrazinyl, imidazo[1,2-a]pyridinyl, thieno[3,2-d]pyrimidinyl, 1H-pyrrolo[2,3-b]pyridinyl and pyrazolyl;
Y1 and Y2 are substituents of ring B independently selected from the group consisting of hydrogen, (C₁-C₄)alkyl, (C₁-C₄)haloalkyl, (C₁-C₄)alkoxy, (C₁-C₄)alkoxy-(C₁-C₄)alkoxy, hydroxy-(C₁-C₄)alkoxy, hydroxy-(C₁-C₄)alkyl, (C₁-C₄)alkyl-heterocycloalkyl-(C₀-C₄)alkoxy, (Ci-C₄)haloalkoxy, halogen, cyano, cyano-(C₁-C₄)alkyl, cyano-(C₁-C₄)alkyl-heterocycloalkyl, CONR1R2, NHCOR1, NR1R2, R1R2N-(C₁-C₄)alkyl, heterocycloalkyl, (C₁-C₄)alkyl-heterocycloalkyl, heterocycloalkyl-(C₁-C₄)alkyl, (C₁-C₄)alkyl-heterocycloalkyl-carbonyl, (C₀-C₄)alkyl-heterocycloalkyl-carbonyl, (C₁-C₄)alkyl-phenyl and monocyclic (C₁-C₄)alkyl-heteroaryl, wherein Y1 is preferably selected from CONH₂, CF₂H, OCH₃, cyano, Br, NHCOCH₃, NH₂, 4-methylpiperazine-1-carbonyl, 4-methylpiperazin-1-yl and 1-methyl-1H-pyrazol-4-yl, and Y2 is preferably hydrogen;
R1 and R2 are independently selected from the group consisting of hydrogen, (C₁-C₄)alkyl, (C₁-C₄)hydroxyalkyl, (C₁-C₄)alkoxy(C₁-C₄)alkyl, (C₁-C₄)alkylamino-(C₁-C₄)alkyl, di-(Ci-C₄)alkylamino-(C₁-C₄)alkyl, optionally substituted (C₃-C₆)cycloalkyl, optionally substituted heterocycloalkyl and optionally substituted heterocycloalkyl-(C₁-C₄)alkoxy, wherein optional substituents are one or more and are selected from (C₁-C₄)alkyl, (C₁-C₄)alkoxy, (C₁-C₄)haloalkyl, hydroxy and (C₁-C₄)haloalkoxy; and wherein R1 and R2 are preferably independently selected from the group consisting of CH₂CH₂N(CH₃)₂, CH₂CH₂OCH₃, CH₃, CH₂CH₂OH, oxetan-3-yl and 3-hydroxycyclobutyl;
R4 is selected from hydroxy and hydroxymethyl;
or a stereoisomer, tautomer, solvate and pharmaceutically acceptable salt thereof.

In a second aspect, the invention refers to a pharmaceutical composition comprising a compound of formula (I), or pharmaceutically acceptable salts thereof, in admixture with at least one or more pharmaceutically acceptable carrier and/or excipient.

In a third aspect, the invention refers to a compound of formula (I), or pharmaceutically acceptable salts thereof, or to a pharmaceutical composition comprising a compound of formula (I), or pharmaceutically acceptable salts thereof, for use as a medicament.

In a further aspect, the invention refers to a compound of formula (I), or pharmaceutically acceptable salts thereof, or to a pharmaceutical composition comprising a compound of formula (I), or pharmaceutically acceptable salts thereof, for use in preventing and/or treating a disease, disorder or condition associated with dysregulation of DDR.

In another aspect, the invention refers to a compound of formula (I), or pharmaceutically acceptable salts thereof, or to a pharmaceutical composition comprising a compound of formula (I), or pharmaceutically acceptable salts thereof, for use in preventing and/or treating fibrosis and/or diseases, disorders or conditions that involve fibrosis.

In yet another aspect, the invention refers to a compound of formula (I), or pharmaceutically acceptable salts thereof, or to a pharmaceutical composition comprising a compound of formula (I), or pharmaceutically acceptable salts thereof, for use in preventing and/or treating idiopathic pulmonary fibrosis (IPF).

In a further aspect, the invention refers to processes for the preparation of compounds of formula (I) and to intermediate compounds that are useful in their preparation.

### DETAILED DESCRIPTION OF THE INVENTION

### Definitions

Unless otherwise specified, the compounds of formula (I) of the present invention are intended to include stereoisomers, tautomers, solvates and pharmaceutically acceptable salts thereof.

Unless otherwise specified, the compounds of formula (I) of the present invention are intended to include the compounds of formula (Ia), (Ia'), (Ia"), (Ib) and (Ib1).

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable. Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals, such as potassium, sodium, calcium or magnesium.

The salts obtained by reacting the main compound, functioning as a base, with an inorganic or organic acid comprise, for example, salts of hydrochloric acid, hydrobromic acid, sulfuric acid, phosphoric acid, methanesulfonic acid, camphorsulfonic acid, acetic acid, oxalic acid, maleic acid, fumaric acid, succinic acid and citric acid.

The term "stereoisomer" refers to isomers of identical constitution that differ in the arrangement of their atoms in space. Enantiomers and diastereomers are examples of stereoisomers.

The term "enantiomer" refers to one of a pair of molecular species that are mirror images of each other and are not superimposable.

The term "racemate" or "racemic mixture" refers to a composition composed of equimolar quantities of two enantiomeric species, wherein the composition is devoid of optical activity.

The term "halogen" or "halogen atoms" or "halo" as used herein includes fluorine, chlorine, bromine and iodine atom.

The term "(Cₓ-C_{y})alkyl", wherein x and y are integers, refers to a straight or branched chain alkyl group having from x to y carbon atoms. Thus, when x is 1 and y is 4, for example, the term "(C₁-C₄)alkyl" comprises methyl, ethyl, n-propyl, isopropyl, n-butyl, isobutyl, sec-butyl, and t-butyl. Such groups are herein indicated also by their atoms arrangement, i.e. as CH₃, CH₂CH₃, etc.

The term "(Cₓ-C_{y})haloalkyl", wherein x and y are integers, refers to a straight or branched chain alkyl group having from x to y carbon atoms, comprising at least one halogen substituent. Examples include CF₃, C(CH₃)₂CF₃ and OCF₂H.

The term "(Cₓ-C_{y})alkoxy", wherein x and y are integers, refers to a straight or branched chain alkyl group having from x to y carbon atoms, comprising at least one oxygen atom, in particular, but not only, an oxygen atom directly linked to ring A or ring B or the phenyl ring, i.e. when W, Y or Z, respectively, are (C₁-C₄)alkoxy. Examples include OCH₃ and OCH₂CH₃.

The term "(Cₓ-C_{y})haloalkoxy", wherein x and y are integers, refers to a "(Cₓ-C_{y})alkoxy comprising at least one halogen substituent. Examples include OCF₃ and OCF₂H.

The term "(Cₓ-C_{y})hydroxyalkyl", wherein x and y are integers, refers to a (Cₓ-C_{y})alkyl comprising at least one hydroxy substituent. Examples include CH₂OH, CH(OH)CH₃ and CH₂CH₂OH.

The term "(Cₓ-C_{y})cycloalkyl" refers to a saturated hydrocarbon ring comprising a number of ring carbon atoms from x to y. Examples include cyclopropyl, cyclobutyl, cyclopentyl, cyclohexyl, and cycloheptyl.

The term "mono- or bi-cyclic heteroaryl ring" refers to a mono- or bi-cyclic aromatic group comprising 1 to 3 heteroatoms independently selected from N, S and O, and includes groups having two such monocyclic rings, or one such monocyclic ring and one monocyclic aryl ring, such as a phenyl ring, which are fused through a common bond or linked by a single bond. The mono- or bi-cyclic heteroaryl rings comprise pyrazolyl, furanyl, tiophenyl, oxazolyl, isoxazolyl, isothiazolyl, thiazolyl, pyridazinyl, imidazoyl, benzofuranyl, 1H-benzo[d]imidazolyl, 1H-indazolyl, benzothiophenyl, benzo[c]thiophenyl, quinazolinyl, pteridinyl, 1H-pyrazolo[5,1-c][1,2,4]triazolyl, pyrrolizinyl, indolizinyl, benzothiazolyl, pyrazolo[5,1-b]thiazolyl, 1H-imidazo[1,2-b]pyrazolyl, 1H-pyrazolo[3,4-b]pyridinyl, 1,6-dihydropyrrolo[2,3-b]pyrrolyl, 1,4-dihydropyrrolo[3,2-b]pyrrolyl, 4H-thieno[3,2-b]pyrrolyl, isobenzofuranyl, 1,2,4-triazolyl, 1,2,5 oxadiazolyl, 1,2,3 oxadiazolyl, 1,2,5 thiadiazolyl, 1,2,3 thiadiazolyl, tetrazolyl, 6H-furo[2,3-b]pyrrolyl, 6H-thieno[2,3-b]pyrrolyl, 4H-furo[3,2-b]pyrrolyl, benzo[d]isothiazolyl, thiazolo[4,5-b]pyridinyl, 1,3,5-triazinyl, 1,2,3,4-thiatriazolyl, 1,2,3,4-oxatriazolyl, 1,2,3,4-tetrazinyl, 1,2,4,5-tetrazinyl, 1,2,3,5-tetrazinyl, 1H-imidazo[4,5-b]pyridinyl, 7H-purinyl, 1H-pyrrolyl, 1-methyl-1H-pyrrolyl, 1-methyl-1H-1,2,4-triazolyl, 1-methyl-1H-tetrazolyl, pyridinyl, pyrimidinyl, pyridazinyl, imidazolyl, imidazo[1,2-b]pyridazinyl, pyrazolo[1,5-a]pyrazinyl, imidazo[1,2-a]pyrazinyl, imidazo[1,2-a]pyrazinyl, imidazo[1,2-a]pyridinyl, pyrazolo[1,5-a]pyridinyl, thieno[3,2-d]pyrimidinyl, SH-pyrrolo[3,2-d]pyrimidinyl, 1H-pyrrolo[2,3-b]pyridinyl, 5,6-dihydro-8H-imidazo[2,1-c][1,4]oxazinyl, furo[3,2-d]pyrimidin-4-yl and pyrazolo[1,5-a]pyrimidinyl.

The term "heterocycloalkyl" refers to a saturated or partly unsaturated mono-, bi- or spiro-cyclic ring system of 3 to 12 ring atoms comprising one or more heteroatoms independently selected from N, S and O. Examples of heterocycloalkyl include piperazinyl, pyrrolidinyl, azetidinyl, morpholinyl and piperidinyl.

The term "spiro-cyclic ring system" refers to a saturated or partly unsaturated bi-cyclic ring system of 5 to 12 ring atoms, comprising one or more heteroatoms selected from N, S and O, wherein the two rings have only one common atom.

Any composite term, like "(C₁-C₄)alkyl-heterocycloalkyl-carbonyl", should be intended as conventionally construed by the parts from which it derives, e.g. by a (C₁-C₄)alkyl, a heterocycloalkyl and a carbonyl group which are linked together in the indicated sequence, and wherein the carbonyl group carbon atom is the point of attachment to the residual part of the compound of formula (I).

When referring to substituents, a dash ("-") that is not between two letters, words, or symbols is meant to represent the point of attachment for such substituents.

The carbonyl group is herein preferably represented as CO, as an alternative to the other common representations such as -C(O)-, -CO-, -(CO)- or -C(=O)-.

Whenever basic amino groups are present in the compounds of formula (I), physiologically acceptable anions may be present, selected among chloride, bromide, iodide, trifluoroacetate, formate, sulfate, phosphate, methane sulfonate, nitrate, maleate, acetate, citrate, fumarate, tartrate, oxalate, succinate, benzoate, p-toluenesulfonate, pamoate and naphthalene disulfonate. Likewise, in the presence of acidic groups, corresponding physiological cations may be present as well, for instance including alkaline or alkaline earth metal ions.

The term "Ki" indicates the dissociation constant for the enzyme-inhibitor complex, expressed in molar units. It is an indicator of the binding affinity between inhibitor and DDR1 or DDR2 receptors.

As above indicated, the present invention refers to a series of compounds represented by the general formula (I) as herein below described in detail, which are endowed with an inhibitory activity on receptors DDR1 and DDR2. Antagonizing receptors DDR1 and DDR2 can be particularly effective in the treatment of those diseases where the DDR receptors play a role, such as fibrosis and any other disease, disorder and condition related to fibrosis.

Indeed, as detailed in the experimental part below, the compounds of formula (I) of the present invention are able to act as inhibitors of both DDR1 and DDR2 receptors in a substantive and effective way. In particular, Table 3 further below shows that for representative compounds of the present invention the inhibitory activity against either DDR1 and DDR2 receptors is lower than 100 nM in the binding assay (expressed as Ki). This confirms that the compounds of formula (I) are able to inhibit the two isoforms of DDR receptor mainly involved in fibrosis and diseases resulting from fibrosis. Accordingly, the compounds of formula (I) can be used in the treatment of fibrosis, in particular pulmonary fibrosis, when DDR1 and DDR2 are involved.

As indicated in the experimental part, comparative examples section, in particular in Table 4, conversely to comparative Example C1, characterized by having a -CH₂- linker between L and heteroaryl ring B, the presence of a direct link between L and heteroaryl ring B in the compounds of the present invention unexpectedly and remarkably determines a relevant increase in the inhibitory activity on the DDR1 and DDR2 receptors.

Furthermore, as indicated in the same experimental part section, data demonstrate that, conversely to comparative Example C2, characterized by the replacement of heteroaryl ring B with an alkyl group, the presence of such heteroaryl ring B in the compounds of the present invention unexpectedly and noteworthy determines a relevant increase in the inhibitory activity on the DDR1 and DDR2 receptors.

Advantageously, the compounds of the present invention are endowed by a very high potency and could be administered in human at a lower dosage respect to the compounds of the prior art, thus reducing the adverse events that typically occur administering higher dosages of drug.

In addition to being notably potent with respect to their inhibitory activity on both receptors DDR1 and DDR2, the compounds of the present invention are also characterized by being selective inhibitors of DDR1 and DDR2 receptors with respect to other human protein kinases, by a good inhalatory profile, that allows to act effectively on the lung compartment and have, at the same time, a low metabolic stability, that allows to minimize the drawbacks associated with the systemic exposure, such as safety and tolerability issues.

Therefore, the compounds of the present invention may be particularly appreciated when looking at suitable and efficacious compounds useful for the treatment of fibrosis, in particular idiopathic pulmonary fibrosis, administered by the inhalation route and characterized by a good inhalatory profile, that corresponds to a good activity on the lung, a good lung retention and a low metabolic stability, that minimizes the systemic exposure and correlated safety issues.

Accordingly, the present invention relates to a compound of formula (I) 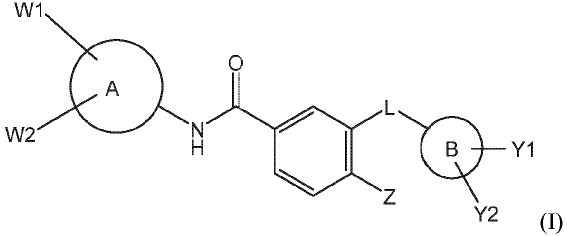 wherein
A is a ring selected from the group consisting of : 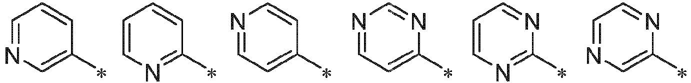 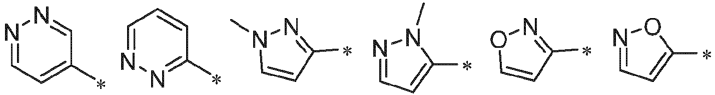 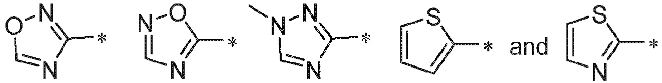
wherein 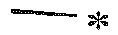 indicates a direct bond to NH;
W1 and W2 are substituents of ring A selected from the group consisting of hydrogen, (Ci-C₄)alkyl, (C₁-C₄)haloalkyl, (C₁-C₄)alkoxy, (C₁-C₄)haloalkoxy, R1R2N-(C₁-C₄)alkyl and (C₃-C₆)cycloalkyl;
Z is selected from the group consisting of (C₁-C₄)alkyl, (C₁-C₄)haloalkyl, (C₁-C₄)alkoxy, (C₁-C₄)haloalkoxy, (C₁-C₄)hydroxyalkyl, hydroxy and (C₃-C₆)cycloalkyl;
L is selected from the group consisting of 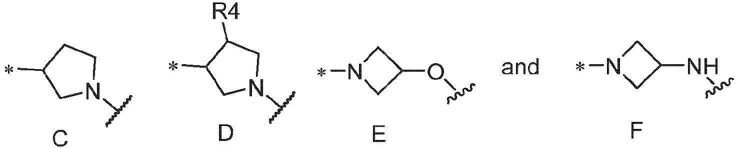 wherein 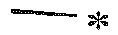 indicates a direct bond to the phenyl ring and 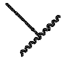 indicates a direct bond to B;
B is mono- or bi-cyclic heteroaryl ring;
Y1 and Y2 are substituents of ring B independently selected from the group consisting of hydrogen, (C₁-C₄)alkyl, (C₁-C₄)haloalkyl, (C₁-C₄)alkoxy, (C₁-C₄)alkoxy-(C₁-C₄)alkoxy, hydroxy-(C₁-C₄)alkoxy, hydroxy-(C₁-C₄)alkyl, (C₁-C₄)alkyl-heterocycloalkyl-(C₀-C₄)alkoxy, (Ci-C₄)haloalkoxy, halogen, cyano, cyano-(C₁-C₄)alkyl, cyano-(C₁-C₄)alkyl-heterocycloalkyl, CONR1R2, NHCOR1, NR1R2, R1R2N-(C₁-C₄)alkyl, heterocycloalkyl, (C₁-C₄)alkyl-heterocycloalkyl, heterocycloalkyl-(C₁-C₄)alkyl, (C₁-C₄)alkyl-heterocycloalkyl-carbonyl, (C₀-C₄)alkyl-heterocycloalkyl-carbonyl (C₁-C₄)alkyl-phenyl and monocyclic (C₁-C₄)alkyl-heteroaryl;
R1 and R2 are independently selected from the group consisting of hydrogen, (C₁-C₄)alkyl, (C₁-C₄)hydroxyalkyl, (C₁-C₄)alkoxy(C₁-C₄)alkyl, (C₁-C₄)alkylamino-(C₁-C₄)alkyl, di-(C₁-C₄)alkylamino-(C₁-C₄)alkyl, optionally substituted (C₃-C₆)cycloalkyl, optionally substituted heterocycloalkyl and optionally substituted heterocycloalkyl-(C₁-C₄)alkoxy, wherein optional substituents are one or more and are selected from (C₁-C₄)alkyl, (C₁-C₄)alkoxy, (C₁-C₄)haloalkyl and (C₁-C₄)haloalkoxy;
R4 is selected from hydroxy and hydroxymethyl;
or a stereoisomer, tautomer, solvate and pharmaceutically acceptable salt thereof.

All the listed meanings of each of the variable moieties A, B, L, Z, W1, W2, Y1, Y2, R1, R2 and R4 of the compound of formula (I) of the invention have to be intended as alternatives and may be combined with each other in embodiments which are included in the scope of the invention.

Preferred halogens in (C₁-C₄)haloalkyl and (C₁-C₄)haloalkoxy substituents are fluorine and chlorine, wherein fluorine is more preferred.

W1 and W2 are substituents of ring A which can be attached to A at any available position. W1 and W2 are preferably selected from the group consisting of H, F, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ and cyclopropyl.

Z is preferably selected from the group consisting of CH₃, CF₂H, OCH₃, CH₂OH and cyclopropyl.

L is selected from the group consisting of

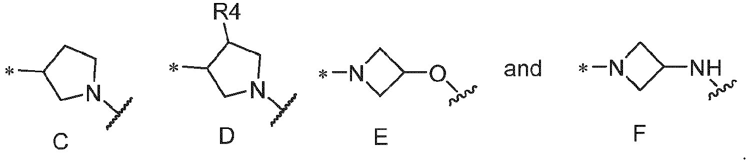

In a preferred embodiment, L is selected from the group consisting of

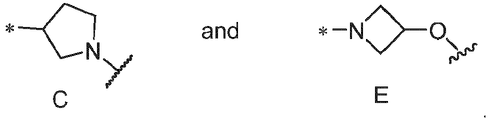

B is mono- or bi-cyclic heteroaryl ring, preferably selected from the group consisting of pyridinyl, pyrimidinyl, pyrazinyl, pyrazolo[1,5-a]pyrazinyl, 1H-pyrazolo[3,4-b]pyridinyl, pyrazolo[1,5-a]pyridinyl, pyrazolo[1,5-a]pyrimidinyl, imidazo[1,2-b]pyridazinyl, imidazo[1,2-a]pyrazinyl, imidazo[1,2-a]pyridinyl, thieno[3,2-d]pyrimidinyl, 1H-pyrrolo[2,3-b]pyridinyl and pyrazolyl. Particularly preferred B rings are pyridin-3-yl, pyrimidin-5-yl, pyrazinyl, pyrazolo[1,5-a]pyridin-3-yl, pyrazolo[1,5-a]pyrazin-3-yl, pyrazolo[1,5-a]pyrimidin-3-yl, imidazo[1,2-a]pyrazin-3-yl, 1H-pyrazolo[3,4-b]pyridin-5-yl and imidazo[1,2-b]pyridazin-3-yl.

Y1 is a substituent of ring B which can be attached to B at any available position.

Y1 is preferably selected from the group consisting of hydrogen, (C₁-C₄)haloalkyl, (Ci-C₄)alkoxy, cyano, CONR1R2, NHCOR1, NR1R2, (C₁-C₄)alkyl-heterocycloalkyl-carbonyl and monocyclic (C₁-C₄)alkyl-heteroaryl, wherein the preferred monocyclic heteroaryl is pyrazolyl. Y1 is more preferably selected from the group consi sting of CONH₂, CF₂H, OCH₃, cyano, NHCOCH₃, NH₂, 4-methylpiperazine-1-carbonyl, 4-methylpiperazin-1-yl and 1-methyl-1H-pyrazol-4-yl.

Y2 is a substituent of ring B which can be attached to B at any available position. In preferred embodiments Y2 has independently the same preferred meanings of Y1, wherein more preferably Y2 is hydrogen.

R1 and R2 are independently preferably selected from the group consisting of CH₂CH₂N(CH₃)₂, CH₂CH₂OCH₃, CH₃, CH₂CH₂OH, oxetan-3-yl and 3-hydroxycyclobutyl.

Optional substituents of R1 and/or R2 are one or more, preferably 1 to 3, and are selected from the group consisting of (C₁-C₄)alkyl, (C₁-C₄)alkoxy, (C₁-C₄)haloalkyl and (Ci-C₄)haloalkoxy, preferably selected from CH₃, OCH₃, OCF₃, CF₃, CF₂H, C(CH₃)₃, C(CH₃)₂CF₃ and OCF₂H.

Accordingly, in a preferred embodiment, the invention relates to a compound of formula (I) wherein
A is a ring selected from the group consisting of: 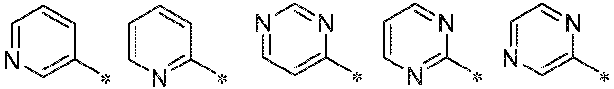 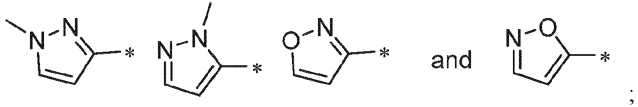
W1 and W2 are independently selected from the group consisting of H, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ and cyclopropyl;
Z is selected from the group consisting of CH₃, CF₂H, OCH₃, CH₂OH and cyclopropyl;
L is selected from the group consisting of 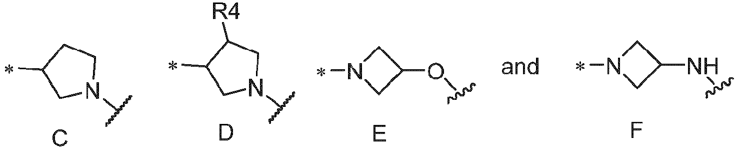
or from the group consisting of 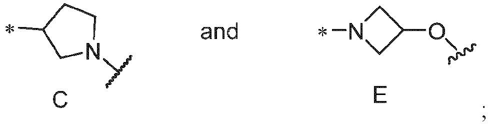
B is selected from the group consisting of pyridinyl, pyrimidinyl, pyrazinyl, pyrazolo[1,5-a]pyrazinyl, 1H-pyrazolo[3,4-b]pyridinyl, pyrazolo[1,5-a]pyridinyl, pyrazolo[1,5-a]pyrimidinyl, imidazo[1,2-b]pyridazinyl, imidazo[1,2-a]pyrazinyl, imidazo[1,2-a]pyridinyl, thieno[3,2-d]pyrimidinyl, 1H-pyrrolo[2,3-b]pyridinyl and pyrazolyl;
Y1 is selected from the group consisting of hydrogen, (C₁-C₄)haloalkyl, (C₁-C₄)alkoxy, cyano, CONR1R2, NHCOR1, NR1R2, (C₁-C₄)alkyl-heterocycloalkyl-carbonyl and monocyclic (C₁-C₄)alkyl-heteroaryl; more preferably selected from the group consisting of CONH₂, CF₂H, OCH₃, cyano, NHCOCH₃, NH₂, 4-methylpiperazine-1-carbonyl and 4-methylpiperazin-1-yl; and Y2 is hydrogen;
R1 and R2 are independently selected from the group consisting of CH₂CH₂N(CH₃)₂, CH₂CH₂OCH₃, CH₃, CH₂CH₂OH, oxetan-3-yl and 3-hydroxycyclobutyl;
R4 is hydroxy or hydroxymethyl;
or a stereoisomer, tautomer, solvate and pharmaceutically acceptable salt thereof.

In another preferred embodiment, the present invention relates to a compound of formula (I) wherein L is C 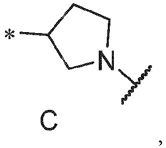 represented by formula (Ia) 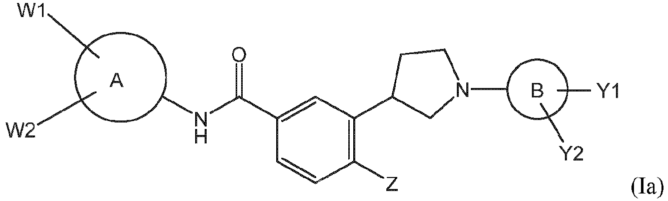 wherein W1, W2, A, Z, B, Y1 and Y2 are as defined above.

A compound of formula (Ia) comprises a stereocenter, which is the carbon atom of the pyrrolidine ring linked to the phenyl moiety. In a preferred embodiment of a compound of formula (Ia), said stereocenter has S configuration, thus a preferred C moiety is C*: 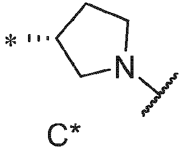 and W1, W2, A, Z, Y1 and Y2 are as defined above.

In yet another preferred embodiment, the present invention relates to a compound of formula (I), which is referred to as a compound of formula (Ib), wherein L is selected from 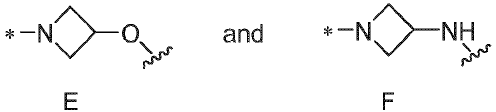 and wherein W1, W2, A, Z, B,Y1 and Y2 are as defined above.

Particularly preferred embodiments of the invention are the compounds of Formula (Ia) listed in Table 1 below, and pharmaceutically acceptable salts thereof. These compounds are particularly active on receptors DDR1 and DDR2, as shown in Table 3 further below.

**Table 1: List of representative compounds of Formula (Ia)**

| **Example N°** | **Structure** | **Chemical Name** |
|---|---|---|
| **1** | 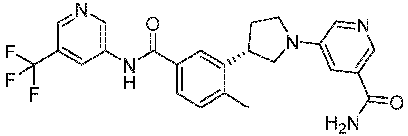 | (S)-5-(3-(2-methyl-5-((5-(trifluoromethyl)pyridin-3-yl)carbamoyl)phenyl)pyrroli din-1-yl)nicotinamide |
| **2** | 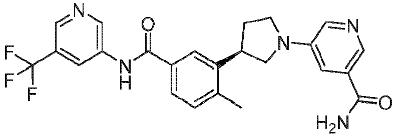 | (R)-5-(3-(2-methyl-5-((5-(trifluoromethyl)pyridin-3-yl)carbamoyl)phenyl)pyrroli din-1-yl)nicotinamide |
| **3** | 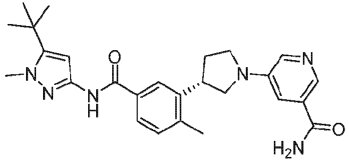 | (S)-5-(3-(5-((5-(tert-butyl)-1-methyl-1H-pyrazol-3-yl)carbamoyl)-2-methylphenyl)pyrrolidin-1-yl)nicotinamide |
| **4** | 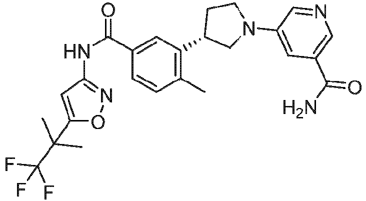 | (S)-5-(3-(2-methyl-5-((5-(1,1,1-trifluoro-2-methylpropan-2-yl)isoxazol-3-yl)carbamoyl)phenyl)pyrroli din-1-yl)nicotinamide |
| **5** | 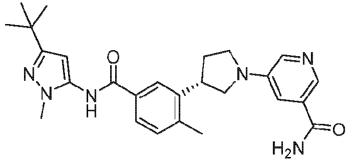 | (S)-5-(3-(5-((3-(tert-butyl)-1-methyl-1H-pyrazol-5-yl)carbamoyl)-2-methylphenyl)pyrrolidin-1-yl)nicotinamide |
| **6** | 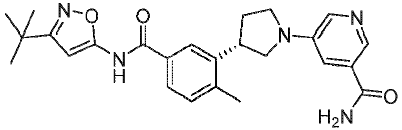 | (S)-5-(3-(5-((3-(tert-butyl)isoxazol-5-yl)carbamoyl)-2-methylphenyl)pyrrolidin-1-yl)nicotinamide |
| **7** | 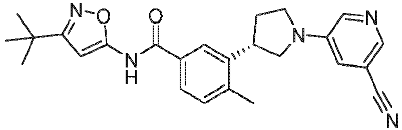 | (S)-N-(3-(tert-butyl)isoxazol-5-yl)-3-(1-(5-cyanopyridin-3-yl)pyrrolidin-3-yl)-4-methylbenzamide |
| **8** | 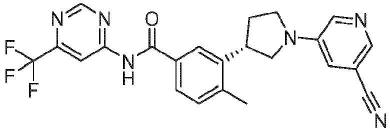 | (S)-3-(1-(5-cyanopyridin-3-yl)pyrrolidin-3-yl)-4-methyl-N-(6-(trifluoromethyl)pyrimidin-4-yl)benzamide |
| **9** | 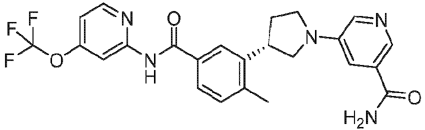 | (S)-5-(3-(2-methyl-5-((4-(trifluoromethoxy)pyridin-2-yl)carbamoyl)phenyl)pyrroli din-1-yl)nicotinamide |
| **10** | 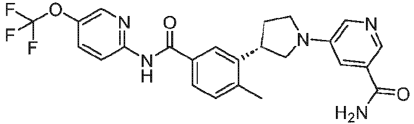 | (S)-5-(3-(2-methyl-5-((5-(trifluoromethoxy)pyridin-2-yl)carbamoyl)phenyl)pyrroli din-1-yl)nicotinamide |
| **11** | 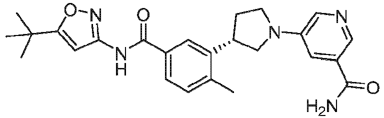 | (S)-5-(3-(5-((5-(tert-butyl)isoxazol-3-yl)carbamoyl)-2-methylphenyl)pyrrolidin-1-yl)nicotinamide |
| **12** | 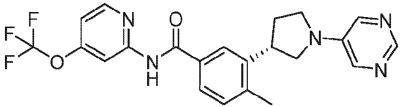 | (S)-4-methyl-3-(1-(pyrimidin-5-yl)pyrrolidin-3-yl)-N-(4-(trifluoromethoxy)pyridin-2-yl)benzamide |
| **13** | 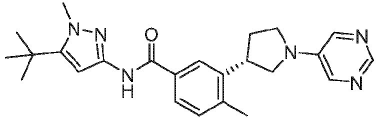 | (S)-N-(5-(tert-butyl)-1-methyl-1H-pyrazol-3-yl)-4-methyl-3-(1-(pyrimidin-5-yl)pyrrolidin-3-yl)benzamide |
| **14** | 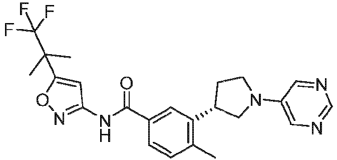 | (S)-4-methyl-3-(1-(pyrimidin-5-yl)pyrrolidin-3-yl)-N-(5-(1,1,1-trifluoro-2-methylpropan-2-yl)isoxazol-3-yl)benzamide |
| **15** | 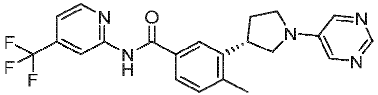 | (S)-4-methyl-3-(1-(pyrimidin-5-yl)pyrrolidin-3-yl)-N-(4-(trifluoromethyl)pyridin-2-yl)benzamide |
| **16** | 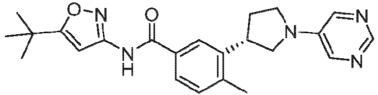 | (S)-N-(5-(tert-butyl)isoxazol-3-yl)-4-methyl-3-(1-(pyrimidin-5-yl)pyrrolidin-3-yl)benzamide |
| **17** | 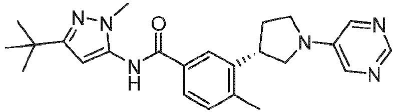 | (S)-N-(3-(tert-butyl)-1-methyl-1H-pyrazol-5-yl)-4-methyl-3-(1-(pyrimidin-5-yl)pyrrolidin-3-yl)benzamide |
| **18** | 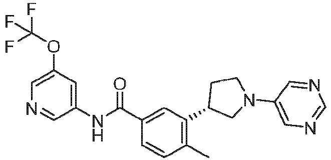 | (S)-4-methyl-3-(1-(pyrimidin-5-yl)pyrrolidin-3-yl)-N-(5-(trifluoromethoxy)pyridin-3-yl)benzamide |
| **19** | 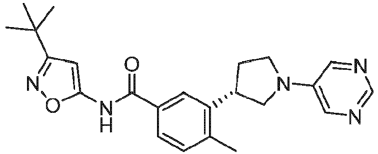 | (S)-N-(3-(tert-butyl)isoxazol-5-yl)-4-methyl-3-(1-(pyrimidin-5-yl)pyrrolidin-3-yl)benzamide |
| **20** | 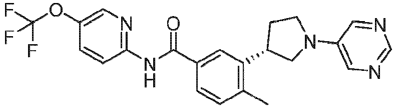 | (S)-4-methyl-3-(1-(pyrimidin-5-yl)pyrrolidin-3-yl)-N-(5-(trifluoromethoxy)pyridin-2-yl)benzamide |
| **21** | 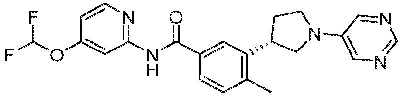 | (S)-N-(4-(difluoromethoxy)pyridin-2-yl)-4-methyl-3-(1-(pyrimidin-5-yl)pyrrolidin-3-yl)benzamide |
| **22** | 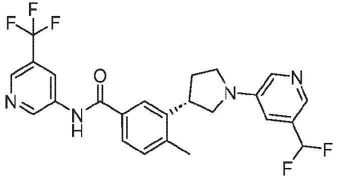 | (S)-3-(1-(5-(difluoromethyl)pyridin-3-yl)pyrrolidin-3-yl)-4-methyl-N-(5-(trifluoromethyl)pyridin-3-yl)benzamide |
| **23** | 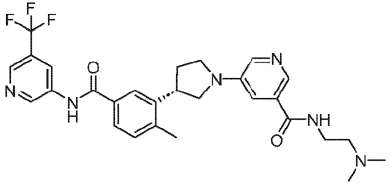 | (S)-N-(2-(dimethylamino)ethyl)-5-(3-(2-methyl-5-((5-(trifluoromethyl)pyridin-3-yl)carbamoyl)phenyl)pyrroli din-1-yl)nicotinamide |
| **24** | 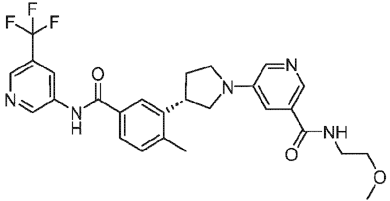 | (S)-N-(2-methoxyethyl)-5-(3-(2-methyl-5-((5-(trifluoromethyl)pyridin-3-yl)carbamoyl)phenyl)pyrroli din-1-yl)nicotinamide |
| **25** | 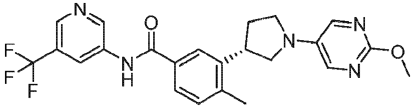 | (S)-3-(1-(2-methoxypyrimidin-5-yl)pyrrolidin-3-yl)-4-methyl-N-(5-(trifluoromethyl)pyridin-3-yl)benzamide |
| **26** | 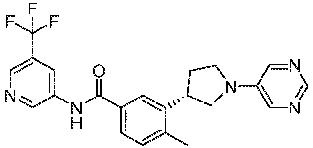 | (S)-4-methyl-3-(1-(pyrimidin-5-yl)pyrrolidin-3-yl)-N-(5-(trifluoromethyl)pyridin-3-yl)benzamide |
| **27** | 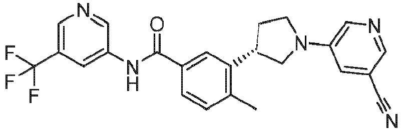 | (S)-3-(1-(5-cyanopyridin-3-yl)pyrrolidin-3-yl)-4-methyl-N-(5-(trifluoromethyl)pyridin-3-yl)benzamide |
| **28** | 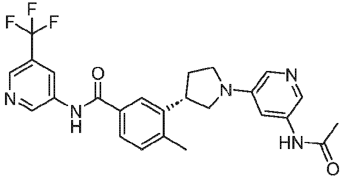 | (S)-3-(1-(5-acetamidopyridin-3-yl)pyrrolidin-3-yl)-4-methyl-N-(5-(trifluoromethyl)pyridin-3-yl)benzamide |
| **29** | 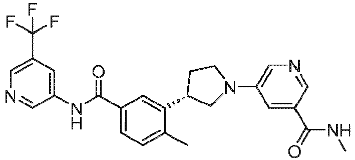 | (S)-N-methyl-5-(3-(2-methyl-5-((5-(trifluoromethyl)pyridin-3-yl)carbamoyl)phenyl)pyrroli din-1-yl)nicotinamide |
| **30** | 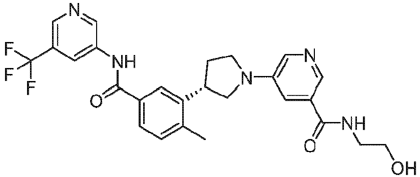 | (S)-N-(2-hydroxyethyl)-5-(3-(2-methyl-5-((5-(trifluoromethyl)pyridin-3-yl)carbamoyl)phenyl)pyrroli din-1-yl)nicotinamide |
| **31** | 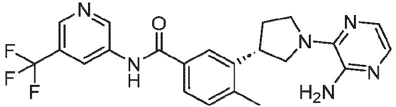 | (S)-3-(1-(3-aminopyrazin-2-yl)pyrrolidin-3-yl)-4-methyl-N-(5-(trifluoromethyl)pyridin-3-yl)benzamide |
| **32** | 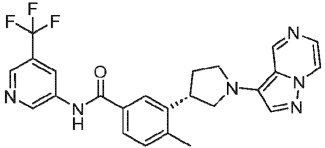 | (S)-4-methyl-3-(1-(pyrazolo[1,5-a]pyrazin-3-yl)pyrrolidin-3-yl)-N-(5-(trifluoromethyl)pyridin-3-yl)benzamide |
| **33** | 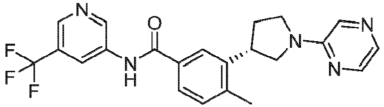 | (S)-4-methyl-3-(1-(pyrazin-2-yl)pyrrolidin-3-yl)-N-(5-(trifluoromethyl)pyridin-3-yl)benzamide |
| **34** | 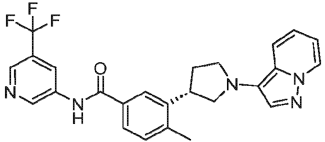 | (S)-4-methyl-3-(1-(pyrazolo[1,5-a]pyridin-3-yl)pyrrolidin-3-yl)-N-(5-(trifluoromethyl)pyridin-3-yl)benzamide |
| **35** | 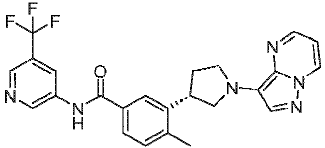 | (S)-4-methyl-3-(1-(pyrazolo[1,5-a]pyrimidin-3-yl)pyrrolidin-3-yl)-N-(5-(trifluoromethyl) pyridin-3-yl)benzamide |
| **36** | 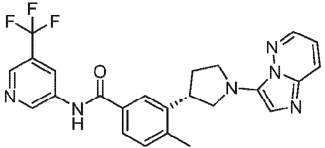 | (S)-3-(1-(imidazo[1,2-b]pyridazin-3-yl)pyrrolidin-3-yl)-4-methyl-N-(5-(trifluoromethyl)pyridin-3-yl)benzamide |
| **37** | 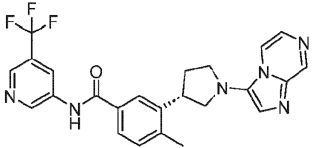 | (S)-3-(1-(imidazo[1,2-a]pyrazin-3-yl)pyrrolidin-3-yl)-4-methyl-N-(5-(trifluoromethyl)pyridin-3-yl)benzamide |
| **38** | 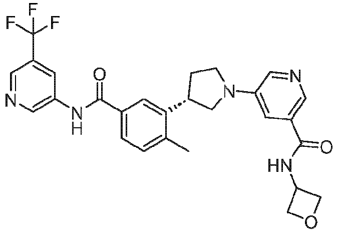 | (S)-5-(3-(2-methyl-5-((5-(trifluoromethyl)pyridin-3-yl)carbamoyl)phenyl)pyrroli din-1-yl)-N-(oxetan-3-yl)nicotinamide |
| **39** | 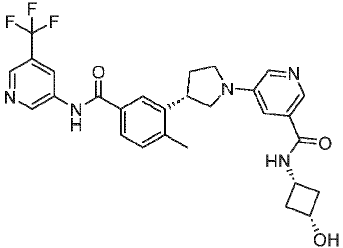 | N-((1s,3R)-3-hydroxycyclobutyl)-5-((S)-3-(2-methyl-5-((5-(trifluoromethyl)pyridin-3-yl)carbamoyl)phenyl)pyrroli din-1-yl)nicotinamide |
| **40** | 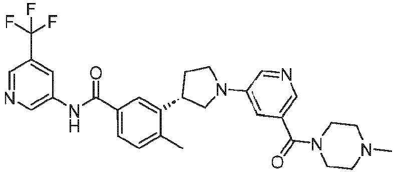 | (S)-4-methyl-3-(1-(5-(4-methylpiperazine-1-carbonyl)pyridin-3-yl)pyrrolidin-3-yl)-N-(5-(trifluoromethyl)pyridin-3-yl)benzamide |
| **41** | 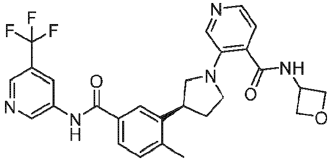 | (S)-3-(3-(2-methyl-5-((5-(trifluoromethyl)pyridin-3-yl)carbamoyl)phenyl)pyrroli din-1-yl)-N-(oxetan-3-yl)isonicotinamide |
| **42** | 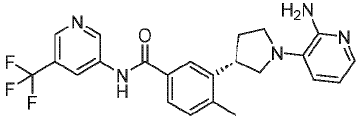 | (S)-3-(1-(2-aminopyridin-3-yl)pyrrolidin-3-yl)-4-methyl-N-(5-(trifluoromethyl)pyridin-3-yl)benzamide |
| **43** | 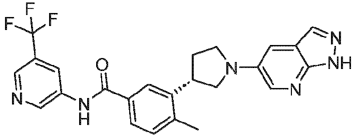 | (S)-3-(1-(1H-pyrazolo[3,4-b]pyridin-5-yl)pyrrolidin-3-yl)-4-methyl-N-(5-(trifluoromethyl)pyridin-3-yl)benzamide |
| **51** | 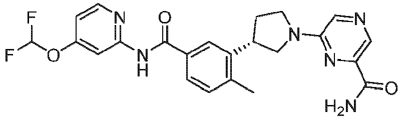 | (*S*)-6-(3-(5-((4-(difluoromethoxy)pyridin-2-yl)carbamoyl)-2-methylphenyl)pyrrolidin-1-yl) pyrazine-2-carboxamide |
| **52** | 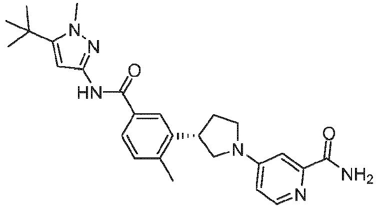 | (S)-4-(3-(5-((5-(tert-butyl)-1-methyl-1H-pyrazol-3-yl)carbamoyl)-2-methylphenyl)pyrrolidin-1-yl)picolinamide |
| **53** | 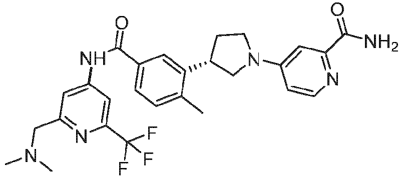 | (S)-4-(3-(5-((2-((dimethylamino)methyl)-6-(trifluoromethyl) pyridin-4-yl)carbamoyl)-2-methylphenyl)pyrrolidin-1-yl)picolinamide |
| **54** | 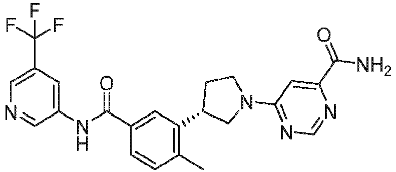 | (S)-6-(3-(2-methyl-5-((5-(trifluoromethyl) pyridin-3-yl)carbamoyl)phenyl) pyrrolidin-1-yl)pyrimidine-4-carboxamide |
| **55** | 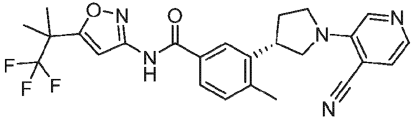 | (S)-3-(1-(4-cyanopyridin-3-yl)pyrrolidin-3-yl)-4-methyl-N-(5-(1,1,1-trifluoro-2-methylpropan-2-yl)isoxazol-3-yl)benzamide |
| **56** | 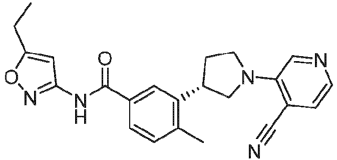 | (S)-3-(1-(4-cyanopyridin-3-yl)pyrrolidin-3-yl)-N-(5-ethylisoxazol-3 -yl)-4-methylbenzamide |
| **57** | 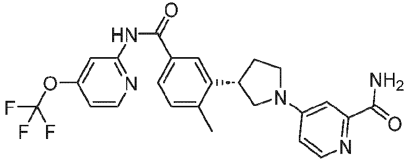 | (S)-4-(3-(2-methyl-5-((4-(trifluoromethoxy)pyridin-2-yl)carbamoyl)phenyl)pyrroli din-1-yl)picolinamide |
| **58** | 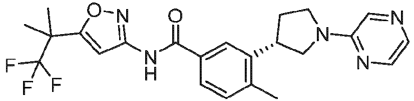 | (*S*)-4-methyl-3-(1-(pyrazin-2-yl)pyrrolidin-3-yl)-*N*-(5-(1,1,1-trifluoro-2-methylpropan-2-yl)isoxazol-3-yl)benzamide |
| **59** | 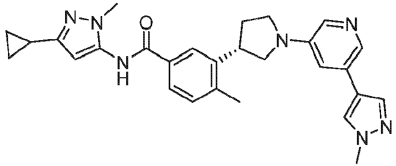 | (*S*)-*N*-(3-cyclopropyl-1-methyl-1*H*-pyrazol-5-yl)-4-methyl-3-(1-(5-(1-methyl-1*H*-pyrazol-4-yl)pyridin-3-yl)pyrrolidin-3-yl)benzamide |
| **60** | 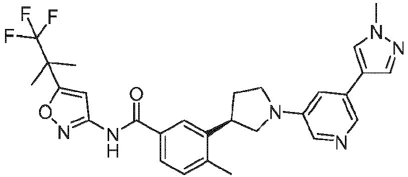 | (R)-4-methyl-3-(1-(5-(1-methyl-1H-pyrazol-4-yl)pyridin-3-yl)pyrrolidin-3-yl)-N-(5-(1,1,1-trifluoro-2-methylpropan-2-yl)isoxazol-3-yl)benzamide |
| **61** | 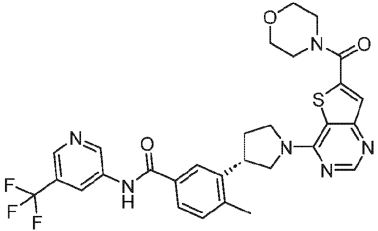 | (S)-4-methyl-3-(1-(6-(morpholine-4-carbonyl)thieno[3,2-d]pyrimidin-4-yl)pyrrolidin-3-yl)-N-(5-(trifluoromethyl)pyridin-3-yl)benzamide |
| **62** | 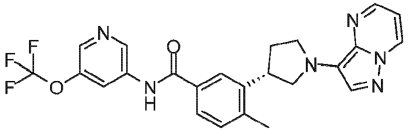 | (*S*)-4-methyl-3-(1-(pyrazolo[1,5-*a*]pyrimidin-3-yl)pyrrolidin-3-yl)-*N*-(5-(trifluoromethoxy)pyridin-3-yl)benzamide |
| **63** | 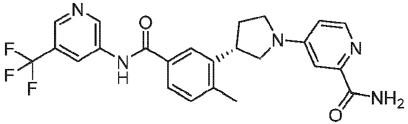 | (S)-4-(3-(2-methyl-5-((5-(trifluoromethyl) pyridin-3-yl)carbamoyl)phenyl)pyrroli din-1-yl)picolinamide |
| **64** | 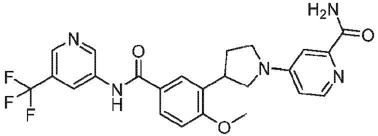 | 4-(3-(2-methoxy-5-((5-(trifluoromethyl)pyridin-3-yl)carbamoyl)phenyl)pyrroli din-1-yl)picolinamide |
| **65** | 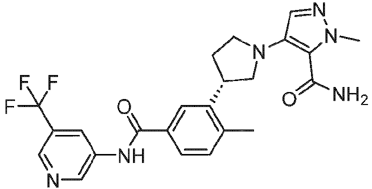 | (S)-1-methyl-4-(3-(2-methyl-5-((5-(trifluoromethyl)pyridin-3-yl)carbamoyl) phenyl)pyrrolidin-1-yl)-1H-pyrazole-5-carboxamide |
| **66** | 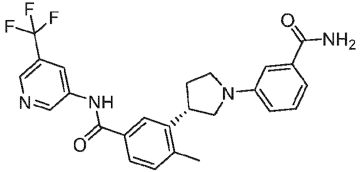 | (S)-3-(1-(3-carbamoylphenyl)pyrrolidin -3-yl)-4-methyl-N-(5-(trifluoromethyl) pyridin-3-yl)benzamide |
| **67** | 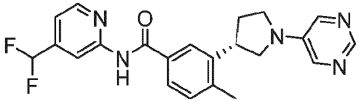 | (S)-N-(4-(difluoromethyl)pyridin-2-yl)-4-methyl-3-(1-(pyrimidin-5-yl)pyrrolidin-3-yl)benzamide |
| **68** | 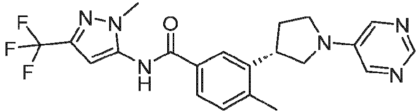 | (S)-4-methyl-N-(1-methyl-3-(trifluoromethyl)-1H-pyrazol-5-yl)-3-(1-(pyrimidin-5-yl)pyrrolidin-3-yl)benzamide |
| **69** | 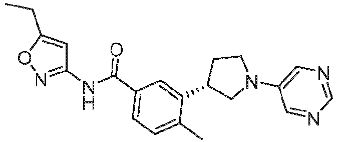 | (S)-N-(5-ethylisoxazol-3-yl)-4-methyl-3-(1-(pyrimidin-5-yl)pyrrolidin-3-yl)benzamide |
| **70** | 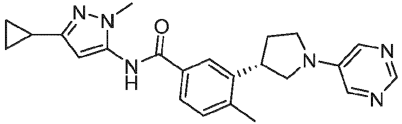 | (S)-N-(3-cyclopropyl-1-methyl-1H-pyrazol-5-yl)-4-methyl-3-(1-(pyrimidin-5-yl)pyrrolidin-3-yl)benzamide |
| **71** | 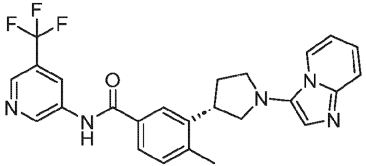 | (S)-3-(1-(imidazo[1,2-a]pyridin-3-yl)pyrrolidin-3-yl)-4-methyl-N-(5-(trifluoromethyl)pyridin-3-yl)benzamide |
| **72** | 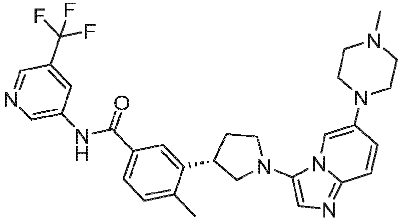 | (S)-4-methyl-3-(1-(6-(4-methylpiperazin-1-yl)imidazo[1,2-a]pyridin-3-yl)pyrrolidin-3-yl)-N-(5-(trifluoromethyl)pyridin-3-yl)benzamide |
| **73** | 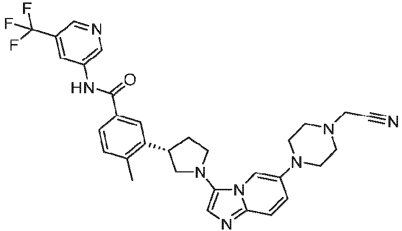 | (S)-3-(1-(6-(4-(cyanomethyl)piperazin-1-yl)imidazo[1,2-a]pyridin-3-yl)pyrrolidin-3-yl)-4-methyl-N-(5-(trifluoromethyl)pyridin-3-yl)benzamide |
| **74** | 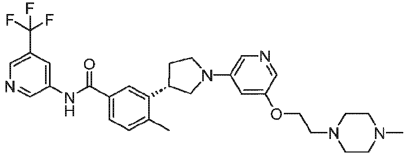 | (S)-4-methyl-3-(1-(5-(2-(4-methylpiperazin-1-yl)ethoxy)pyridin-3-yl)pyrrolidin-3-yl)-N-(5-(trifluoromethyl)pyridin-3-yl)benzamide |
| **75** | 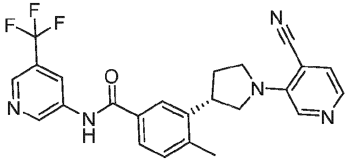 | (S)-3-(1-(4-cyanopyridin-3-yl)pyrrolidin-3-yl)-4-methyl-N-(5-(trifluoromethyl)pyridin-3-yl)benzamide |
| **76** | 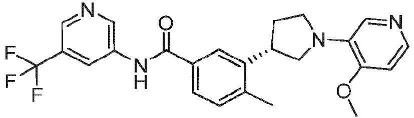 | (S)-3-(1-(4-methoxypyridin-3-yl)pyrrolidin-3-yl)-4-methyl-N-(5-(trifluoromethyl)pyridin-3-yl)benzamide |
| **77** | 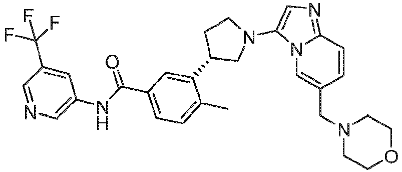 | (S)-4-methyl-3-(1-(6-(morpholinomethyl)imidazo [1,2-a]pyridin-3-yl)pyrrolidin-3-yl)-N-(5-(trifluoromethyl)pyridin-3-yl)benzamide |
| **78** | 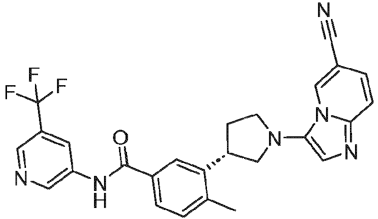 | (S)-3-(1-(6-cyanoimidazo[1,2-a]pyridin-3-yl)pyrrolidin-3-yl)-4-methyl-N-(5-(trifluoromethyl)pyridin-3-yl)benzamide |
| **79** | 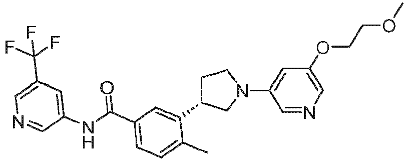 | (S)-3-(1-(5-(2-methoxyethoxy)pyridin-3-yl)pyrrolidin-3-yl)-4-methyl-N-(5-(trifluoromethyl)pyridin-3-yl)benzamide |
| **80** | 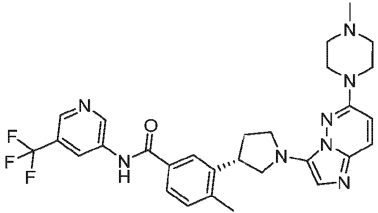 | (S)-4-methyl-3-(1-(6-(4-methylpiperazin-1-yl)imidazo[1,2-b]pyridazin-3-yl)pyrrolidin-3-yl)-N-(5-(trifluoromethyl)pyridin-3-yl)benzamide |
| **81** | 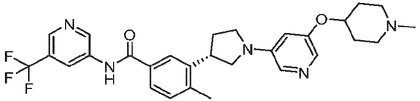 | (S)-4-methyl-3-(1-(5-((1-methylpiperidin-4-yl)oxy)pyridin-3-yl)pyrrolidin-3-yl)-N-(5-(trifluoromethyl)pyridin-3-yl)benzamide |
| **82** | 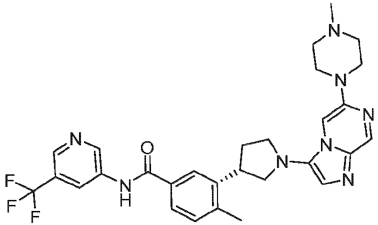 | (S)-4-methyl-3-(1-(6-(4-methylpiperazin-1-yl)imidazo[1,2-a]pyrazin-3-yl)pyrrolidin-3-yl)-N-(5-(trifluoromethyl)pyridin-3-yl)benzamide |
| **83** | 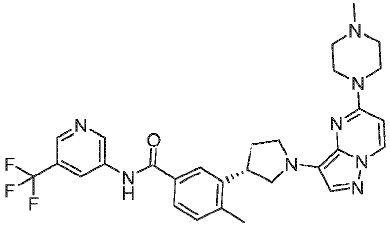 | (S)-4-methyl-3-(1-(5-(4-methylpiperazin-1-yl)pyrazolo[1,5-a]pyrimidin-3-yl)pyrrolidin-3-yl)-N-(5-(trifluoromethyl)pyridin-3-yl)benzamide |
| **84** | 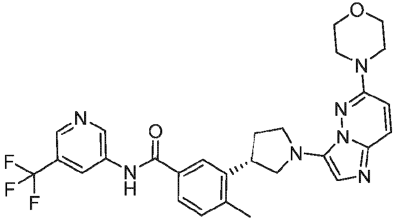 | (S)-4-methyl-3-(1-(6-morpholinoimidazo[1,2-b]pyridazin-3-yl)pyrrolidin-3-yl)-N-(5-(trifluoromethyl)pyridin-3-yl)benzamide |
| **85** | 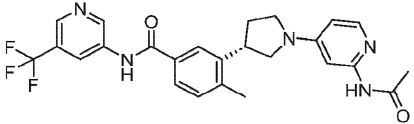 | (S)-3-(1-(2-acetamidopyridin-4-yl)pyrrolidin-3-yl)-4-methyl-N-(5-(trifluoromethyl)pyridin-3-yl)benzamide |
| **86** | 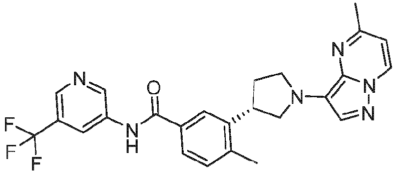 | (S)-4-methyl-3-(1-(5-methylpyrazolo[1,5-a]pyrimidin-3-yl)pyrrolidin-3-yl)-N-(5-(trifluoromethyl)pyridin-3-yl)benzamide |
| **87** | 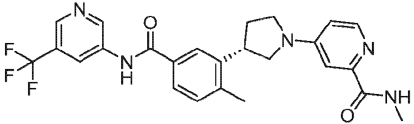 | (S)-N-methyl-4-(3-(2-methyl-5-((5-(trifluoromethyl)pyridin-3-yl)carbamoyl)phenyl)pyrroli din-1-yl)picolinamide |
| **88** | 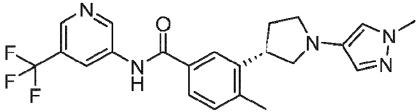 | (S)-4-methyl-3-(1-(1-methyl-1H-pyrazol-4-yl)pyrrolidin-3-yl)-N-(5-(trifluoromethyl)pyridin-3-yl)benzamide |
| **89** | 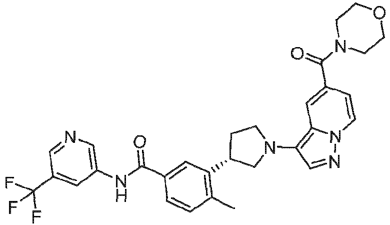 | (S)-4-methyl-3-(1-(5-(morpholine-4-carbonyl)pyrazolo[1,5-a]pyridin-3-yl)pyrrolidin-3-yl)-N-(5-(trifluoromethyl)pyridin-3-yl)benzamide |
| **90** | 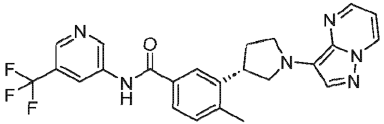 | 4-methyl-3-[(3*R*)-1-pyrazolo[1,5-*a*]pyrimidin-3-ylpyrrolidin-3-yl]-*N*-[5-(trifluoromethyl)-3-pyridyl]benzamide |
| **91** | 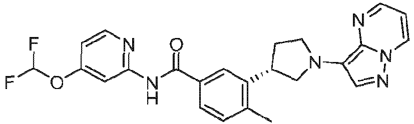 | *N*-[4-(difluoromethoxy)-2-pyridyl]-4-methyl-3-[(3*S*)-1-pyrazolo[1,5-*a*]pyrimidin-3-ylpyrrolidin-3-yl]benzamide |
| **92** | 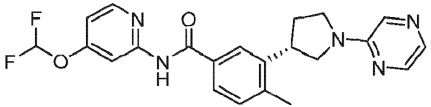 | *N*-[4-(difluoromethoxy)-2-pyridyl]-4-methyl-3-[(3*S*)-1-pyrazin-2-ylpyrrolidin-3-yl]benzamide |
| **93** | 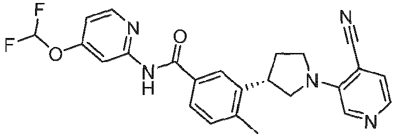 | 3-[(3*S*)-1-(4-cyano-3-pyridyl)pyrrolidin-3-yl]-*N-*[4-(difluoromethoxy)-2-pyridyl]-4-methylbenzamide |
| **94** | 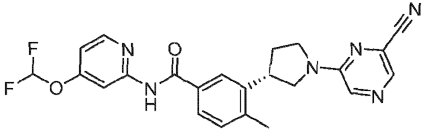 | (*S*)-3-(1-(6-cyanopyrazin-2-yl)pyrrolidin-3-yl)-*N*-(4-(difluoromethoxy) pyridin-2-yl)-4-methylbenzamide |
| **95** | 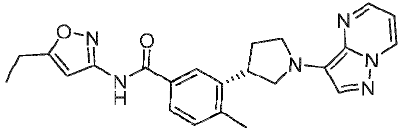 | (*S*)-*N*-(5-ethylisoxazol-3-yl)-4-methyl-3-(1-(pyrazolo[1,5-*a*]pyrimidin-3-yl)pyrrolidin-3-yl)benzamide |
| **96** | 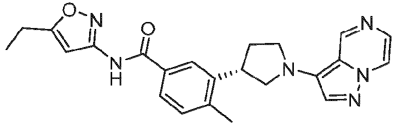 | (S)-N-(5-ethylisoxazol-3-yl)-4-methyl-3-(1-(pyrazolo[1,5-a]pyrazin-3-yl)pyrrolidin-3-yl)benzamide |
| **97** | 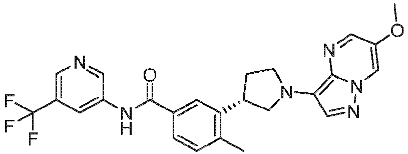 | (S)-3-(1-(6-methoxypyrazolo[1,5-a]pyrimidin-3-yl)pyrrolidin-3-yl)-4-methyl-N-(5-(trifluoromethyl)pyridin-3-yl)benzamide |
| **98** | 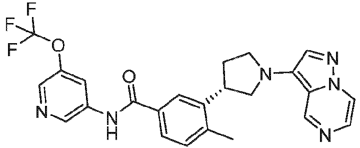 | (S)-4-methyl-3-(1-(pyrazolo[1,5-a]pyrazin-3-yl)pyrrolidin-3-yl)-N-(5-(trifluoromethoxy)pyridin-3-yl)benzamide |
| **99** | 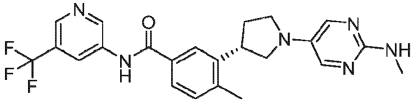 | (S)-4-methyl-3-(1-(2-(methylamino)pyrimidin-5-yl)pyrrolidin-3-yl)-N-(5-(trifluoromethyl)pyridin-3-yl)benzamide |
| **100** | 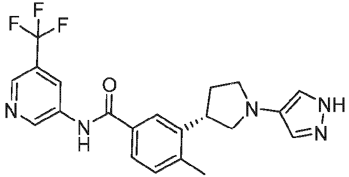 | (S)-3-(1-(1H-pyrazol-4-yl)pyrrolidin-3-yl)-4-methyl-N-(5-(trifluoromethyl)pyridin-3-yl)benzamide |
| **101** | 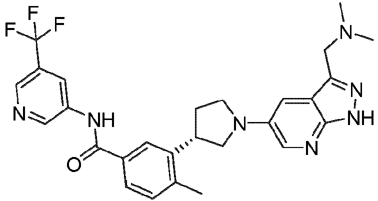 | (S)-3-(1-(3-((dimethylamino)methyl)-1H-pyrazolo[3,4-b]pyridin-5-yl)pyrrolidin-3-yl)-4-methyl-N-(5-(trifluoromethyl)pyridin-3-yl)benzamide |
| **102** | 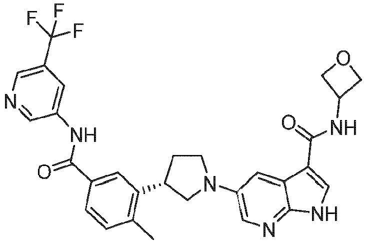 | (S)-5-(3-(2-methyl-5-((5-(trifluoromethyl)pyridin-3-yl)carbamoyl)phenyl)pyrroli din-1-yl)-N-(oxetan-3-yl)-1H-pyrrolo[2,3-b]pyridine-3-carboxamide |
| **103** | 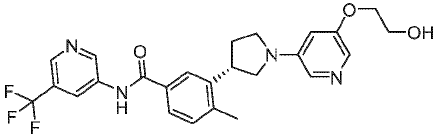 | (S)-3-(1-(5-(2-hydroxyethoxy)pyridin-3-yl)pyrrolidin-3-yl)-4-methyl-N-(5-(trifluoromethyl)pyridin-3-yl)benzamide |
| **104** | 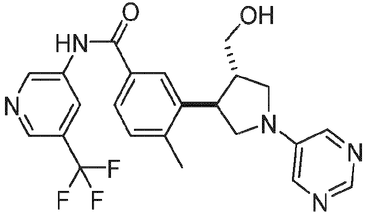 | 3-((3R,4S)-3-(4-(hydroxymethyl)-1-(pyrimidin-5-yl)pyrrolidin-3-yl)-4-methyl-N-(5-(trifluoromethyl)pyridin-3-yl)benzamide and 3-((3S,4R)-3-(4-(hydroxymethyl)-1-(pyrimidin-5-yl)pyrrolidin-3-yl)-4-methyl-N-(5-(trifluoromethyl)pyridin-3-yl)benzamide |
| **105** | 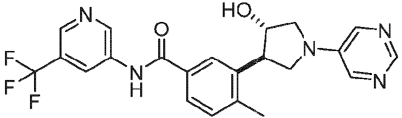 | 3-((3S,4R)-3-(4-hydroxy-1-(pyrimidin-5-yl)pyrrolidin-3-yl)-4-methyl-N-(5-(trifluoromethyl)pyridin-3-yl)benzamide and 3-((3R,4S)-4-hydroxy-1-(pyrimidin-5-yl)pyrrolidin-3-yl)-4-methyl-N-(5-(trifluoromethyl)pyridin-3-yl)benzamide |
| **106** | 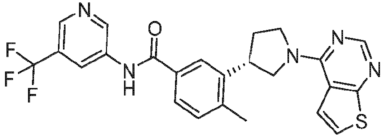 | (S)-4-methyl-3-(1-(thieno[2,3-d]pyrimidin-4-yl)pyrrolidin-3-yl)-N-(5-(trifluoromethyl)pyridin-3-yl)benzamide |
| **107** | 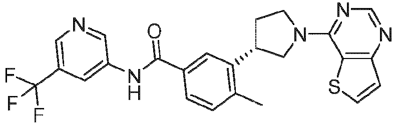 | (S)-4-methyl-3-(1-(thieno[3,2-d]pyrimidin-4-yl)pyrrolidin-3-yl)-N-(5-(trifluoromethyl)pyridin-3-yl)benzamide |
| **108** | 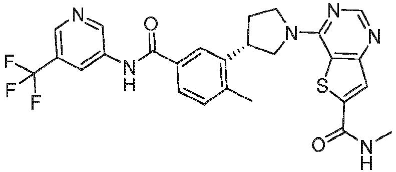 | (S)-N-methyl-4-(3-(2-methyl-5-((5-(trifluoromethyl)pyridin-3-yl)carbamoyl)phenyl)pyrroli din-1-yl)thieno[3,2-d]pyrimidine-6-carboxamide |
| **109** | 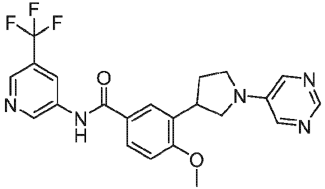 | 4-methoxy-3-(1-(pyrimidin-5-yl)pyrrolidin-3-yl)-N-(5-(trifluoromethyl)pyridin-3-yl)benzamide 1^{st} eluted enantiomer |
| **110** | 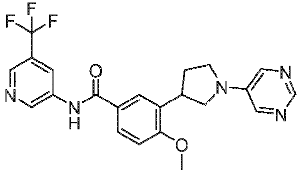 | 4-methoxy-3-(1-(pyrimidin-5-yl)pyrrolidin-3-yl)-N-(5-(trifluoromethyl)pyridin-3-yl)benzamide 2^{nd} eluted enantiomer |
| **111** | 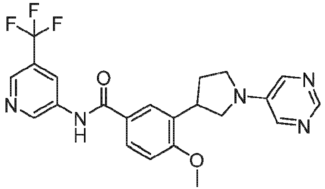 | 4-methoxy-3-(1-(pyrimidin-5-yl)pyrrolidin-3-yl)-N-(5-(trifluoromethyl) pyridin-3-yl)benzamide racemic mixture |
| **112** | 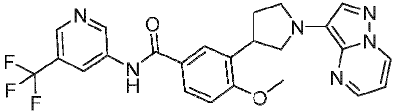 | 4-methoxy-3-(1-(pyrazolo[1,5-a]pyrimidin-3-yl)pyrrolidin-3-yl)-N-(5-(trifluoromethyl)pyridin-3-yl)benzamide |
| **113** | 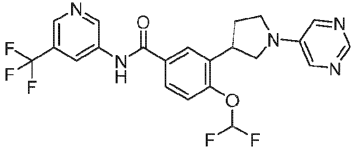 | 4-(difluoromethoxy)-3-(1-(pyrimidin-5-yl)pyrrolidin-3-yl)-N-(5-(trifluoromethyl)pyridin-3-yl)benzamide |
| **114** | 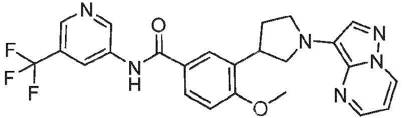 | 4-methoxy-3-(1-(pyrazolo[1,5-a]pyrimidin-3-yl)pyrrolidin-3-yl)-N-(5-(trifluoromethyl)pyridin-3-yl)benzamide 1^{st} eluted enantiomer |
| **115** | 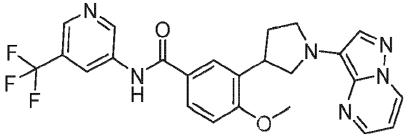 | 4-methoxy-3-(1-(pyrazolo[1,5-a]pyrimidin-3-yl)pyrrolidin-3-yl)-N-(5-(trifluoromethyl)pyridin-3-yl)benzamide 2^{nd} eluted enantiomer |
| **116** | 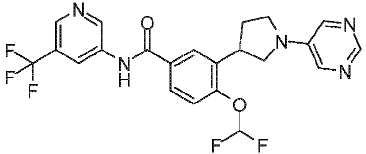 | 4-(difluoromethoxy)-3-(1-(pyrimidin-5-yl)pyrrolidin-3-yl)-N-(5-(trifluoromethyl)pyridin-3-yl)benzamide 1^{st} eluted enantiomer |
| **117** | 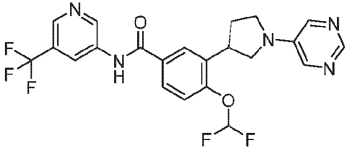 | 4-(difluoromethoxy)-3-(1-(pyrimidin-5-yl)pyrrolidin-3-yl)-N-(5-(trifluoromethyl)pyridin-3-yl)benzamide 2^{nd} eluted enantiomer |
| **118** | 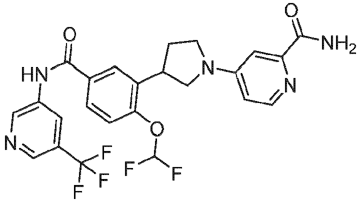 | 4-(3-(2-(difluoromethoxy)-5-((5-(trifluoromethyl)pyridin-3-yl)carbamoyl)phenyl)pyrroli din-1-yl)picolinamide 1^{st} eluted enantiomer |
| **119** | 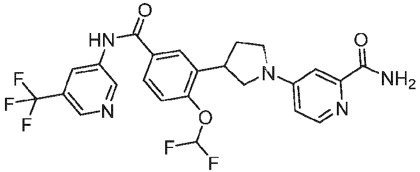 | 4-(3-(2-(difluoromethoxy)-5-((5-(trifluoromethyl)pyridin-3-yl)carbamoyl)phenyl)pyrroli din-1-yl)picolinamide 2^{nd} eluted enantiomer |
| **120** | 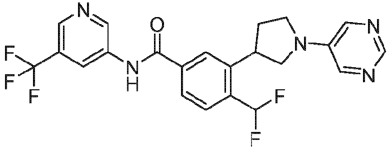 | 4-(difluoromethyl)-3-(1-(pyrimidin-5-yl)pyrrolidin-3-yl)-N-(5-(trifluoromethyl)pyridin-3-yl)benzamide |
| **121** | 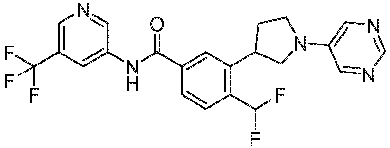 | (1-(pyrimidin-5-yl)pyrrolidin-3-yl)-N-(5-(trifluoromethyl)pyridin-3-yl)benzamide 2^{nd} eluted enantiomer |
| **122** | 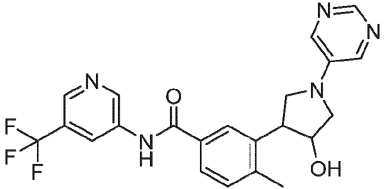 | 3-(4-hydroxy-1-(pyrimidin-5-yl)pyrrolidin-3-yl)-4-methyl-N-(5-(trifluoromethyl)pyridin-3-yl)benzamide 2^{nd} eluted enantiomer |
| **123** | 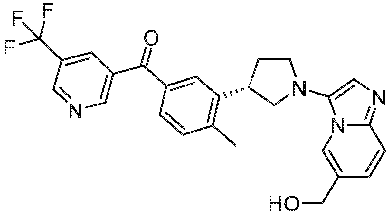 | (S)-(3-(1-(6-(hydroxymethyl)imidazo[1, 2-a]pyridin-3-yl)pyrrolidin-3-yl)-4-methylphenyl)(5-(trifluoromethyl)pyridin-3-yl)methanone |
| **124** | 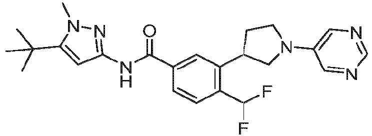 | N-(5-(tert-butyl)-1-methyl-1H-pyrazol-3-yl)-4-(difluoromethyl)-3-(1-(pyrimidin-5-yl)pyrrolidin-3-yl)benzamide |
| **125** | 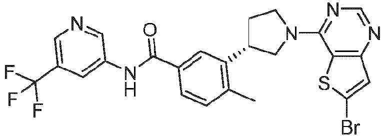 | (S)-3-(1-(6-bromothieno[3,2-d]pyrimidin-4-yl)pyrrolidin-3-yl)-4-methyl-N-(5-(trifluoromethyl)pyridin-3-yl)benzamide |
| **126** | 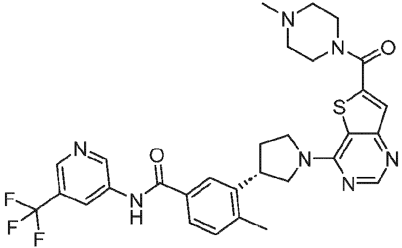 | (S)-4-methyl-3-(1-(6-(4-methylpiperazine-1-carbonyl)thieno[3,2-d]pyrimidin-4-yl)pyrrolidin-3-yl)-N-(5-(trifluoromethyl)pyridin-3-yl)benzamide |
| **127** | 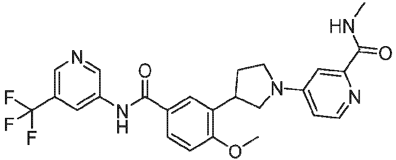 | 4-(3-(2-methoxy-5-((5-(trifluoromethyl)pyridin-3-yl)carbamoyl)phenyl)pyrroli din-1-yl)-N-methylpicolinamide |
| **128** | 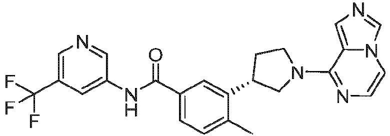 | (S)-3-(1-(imidazo[1,5-a]pyrazin-8-yl)pyrrolidin-3-yl)-4-methyl-N-(5-(trifluoromethyl)pyridin-3-yl)benzamide |
| **129** | 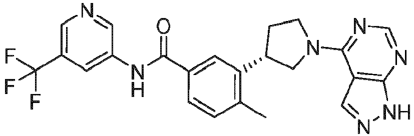 | (S)-3-(1-(1H-pyrazolo[3,4-d]pyrimidin-4-yl)pyrrolidin-3-yl)-4-methyl-N-(5-(trifluoromethyl)pyridin-3-yl)benzamide |
| **130** | 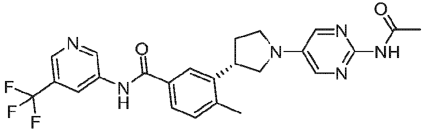 | (S)-3-(1-(2-acetamidopyrimidin-5-yl)pyrrolidin-3-yl)-4-methyl-N-(5-(trifluoromethyl)pyridin-3-yl)benzamide |
| **131** | 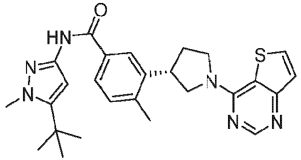 | (S)-N-(5-(tert-butyl)-1-methyl-1H-pyrazol-3-yl)-4-methyl-3-(1-(thieno[3,2-d]pyrimidin-4-yl)pyrrolidin-3-yl)benzamide |

In a particularly preferred embodiment, the present invention relates to a compound of formula (Ib1), wherein L is E:

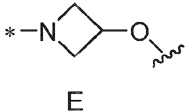

Further particularly preferred embodiments of the invention are the compounds of Formula (Ib 1) listed in Table 2 below, and pharmaceutically acceptable salts thereof. These compounds are particularly active on receptors DDR1 and DDR2, as shown in Table 3 further below.

**Table 2: List of representative compounds of Formula (Ib1)**

| **Example N°** | **Structure** | **Chemical Name** |
|---|---|---|
| **44** | 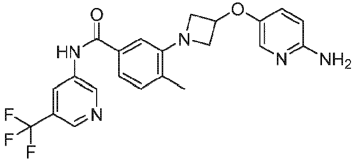 | 3-(3-((6-aminopyridin-3-yl)oxy)azetidin-1-yl)-4-methyl-N-(5-(trifluoromethyl)pyridin -3-yl)benzamide |
| **45** | 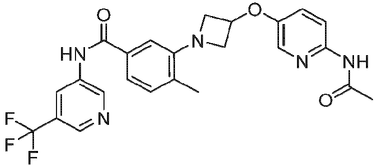 | 3-(3-((6-acetamidopyridin-3-yl)oxy)azetidin-1-yl)-4-methyl-N-(5-(trifluoromethyl)pyridin -3-yl)benzamide |
| **46** | 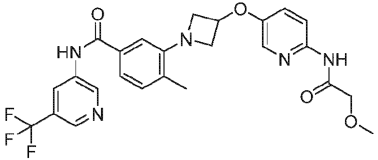 | 3-(3-((6-(2-methoxyacetamido)pyri din-3-yl)oxy)azetidin-1-yl)-4-methyl-N-(5-(trifluoromethyl)pyridin -3-yl)benzamide |
| **47** | 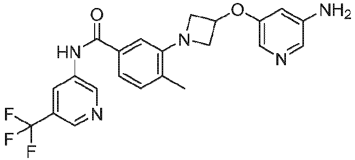 | 3-(3-((5-aminopyridin-3-yl)oxy)azetidin-1-yl)-4-methyl-N-(5-(trifluoromethyl)pyridin -3-yl)benzamide |
| **132** | 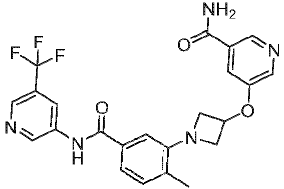 | 5-((1-(2-methyl-5-((5-(trifluoromethyl)pyridin -3-yl)carbamoyl) phenyl)azetidin-3-yl)oxy)nicotinamide |
| **133** | 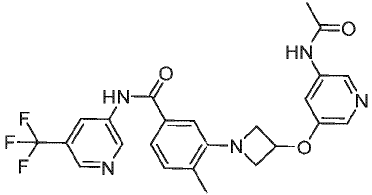 | 3-(3-((5-acetamidopyridin-3-yl)oxy)azetidin-1-yl)-4-methyl-N-(5-(trifluoromethyl)pyridin -3-yl)benzamide |

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimal reaction conditions may vary depending on the particular reactants or solvent used, such conditions can be readily determined by those skilled in the art by routine optimization procedures. Thus, processes described below should not be viewed as limiting the scope of the synthetic methods available for the preparation of the compounds of the invention.

In some cases, generally known protective groups (PG) may be employed when needed to mask or protect sensitive or reactive moieties, in accordance to general principles of chemistry (Protective group in organic syntheses, 3rd ed. T. W. Greene, P. G. M. Wuts).

Compounds of formula (I) may be prepared as described hereinafter, wherein at least one non-limiting synthetic route is provided for the preparation of the exemplified compounds (i.e. Examples).

The compounds of formula (Ia) as defined above can be generally prepared according to the procedures outlined in Schemes 1 to 16 shown below.

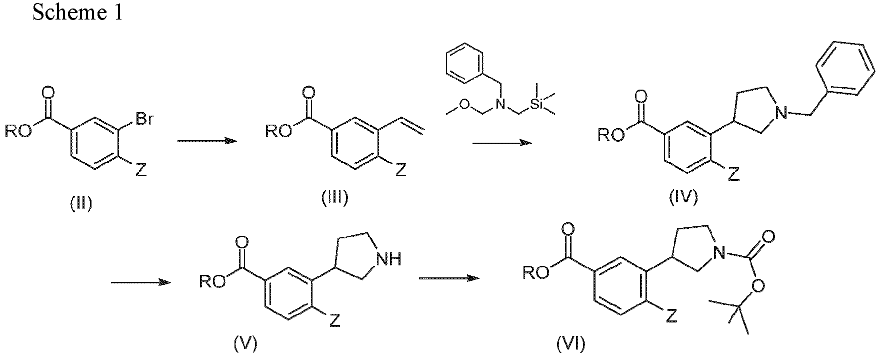

Compounds of formula (Ia) may be obtained for instance starting from commercially available compound (II) as depicted in Scheme 1, wherein Z is as defined above but hydroxy, and R is (Ci-C₄)alkyl, preferably ethyl. A compound of formula (III) can be prepared from a compound of formula (II) through a palladium-catalyzed cross-coupling. The reaction may be carried out by reacting a compound of formula (II) with potassium alkyltrifluoroborate by following the usual Suzuki protocol, in a suitable organic solvent such as dioxane, in the presence of an inorganic base such as cesium carbonate, with an appropriate palladium catalytic system such as Pd₂(dppf)Cl₂ at high temperature (around 100°C) for few hours. 1,3-dipolar cycloaddition may be carried out reacting the α,β-unsaturated compound of formula (III) with a 1,3-dipole or a suitable precursor such as an azomethine ylide, for example with N-benzyl-1-methoxy-N-((trimethylsilyl) methyl)methanamine as shown in Scheme 1, under acid catalysis, for instance by TFA, in a solvent, such as dioxane. The compound of formula (IV) can be converted into the debenzylated compound of formula (V) by reduction under hydrogen atmosphere, typically at 4 bar pressure, in the presence of a suitable catalyst such as Pd/C in a suitable solvent such as, but not limited to, EtOH at a temperature ranging from rt to 60 °C, for few hours.

A Boc-protected compound of formula (VI) can be prepared by reacting a compound of formula (V) with di-*tert*-butyl-dicarbonate, under basic conditions using an organic base such as TEA, in an appropriate solvent such as dichloromethane. Compounds of formula (IV), (V) and (VI) comprise a stereocenter, which is the carbon atom of the pyrrolidine ring linked to the phenyl moiety. These compounds are obtained as racemic mixtures. Separation of the racemic mixture of compounds of formula (VI) is achieved by chiral resolution methods such as chiral purification. Both enantiomers of compounds of formula (Ia) are included in the scope of the present invention.

Accordingly, the present invention provides a process for the preparation of a compound of formula (Ia), or a pharmaceutically acceptable salt thereof, comprising the step of:
a) reacting a compound of formula (III) 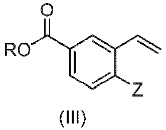 with N-benzyl-1-methoxy-N-((trimethylsilyl)methyl)methanamine 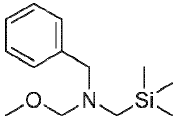 under acid catalysis in the presence of a solvent such as to obtain an intermediate compound of formula (IV) 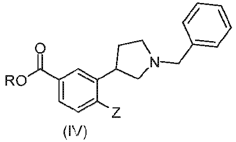 wherein R and Z are as defined above.

In a preferred embodiment, the process further comprises the steps of:
b) cleaving the benzyl group of the intermediate compound of formula (IV) such as to obtain an intermediate compound of formula (V): 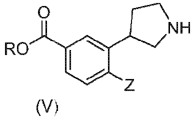 by reduction under hydrogen atmosphere in the presence of a Pd catalyst; and
c) Boc-protecting the free nitrogen of the compound of formula (V) such as to obtain an intermediate compound of formula (VI) 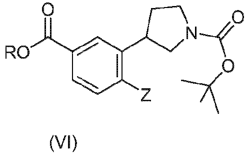 by reaction with di-*tert*-butyl-dicarbonate.

The process according to the invention further comprises converting the intermediate compound of formula (VI) into a compound of formula (Ia).

Accordingly, the present invention provides an intermediate compound of formula (IV) 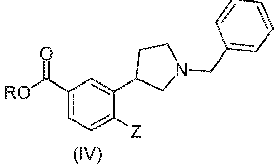 wherein R is (C₁-C₄)alkyl, preferably ethyl, and Z is selected from the group consisting of (Ci-C₄)alkyl, (C₁-C₄)haloalkyl, (C₁-C₄)alkoxy, (C₁-C₄)haloalkoxy, (C₁-C₄)hydroxyalkyl and (C₃-C₆)cycloalkyl.

The invention further provides the use of the intermediate compound of formula (IV) as defined above in the preparation of a compound of formula (Ia).

The present invention also provides an intermediate compound of formula (VI) 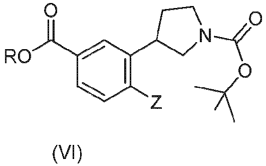 wherein R is (C₁-C₄)alkyl, preferably ethyl, and Z is selected from the group consisting of (Ci-C₄)alkyl, (C₁-C₄)haloalkyl, (C₁-C₄)alkoxy, (C₁-C₄)haloalkoxy, (C₁-C₄)hydroxyalkyl and (C₃-C₆)cycloalkyl; in its racemic or enantiomeric form, i.e. (R) or (S).

The invention further provides the use of the intermediate compound of formula (VI) as defined above in the preparation of a compound of formula (Ia).

Thus, the invention provides the use of the intermediate compound of formula (IV) and/or the intermediate compound of formula (VI) as defined above in the preparation of a compound of formula (Ia).

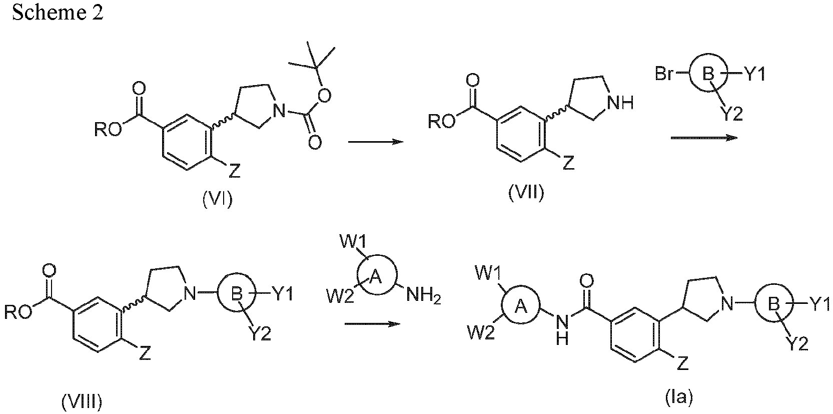

Compounds of formula (Ia) with a defined stereochemistry can be prepared as depicted in Scheme 2 starting from the compounds of formula (VI), wherein R is (C₁-C₄)alkyl, preferably ethyl, with the suitable configuration (R) or (S) at the stereocenter. Compounds of formula (VII) can be obtained from compounds of formula (VI) by removing the Boc protecting group in acidic conditions, using for instance an organic acid such as TFA. Compounds of formula (VII) may be converted in compounds of formula (VIII) by cross-coupling reactions like Buchwald coupling in the presence of a palladium catalyst such as Pd₂(dppf)Cl₂ or another palladium source/phosphine-based ligand at high temperature (around 100 °C) for a few hours with a suitable heteroaryl halide, in an organic solvent such as dimethylacetamide with an appropriate base, such as Cs₂CO₃. Compounds of formula (Ia) can be prepared by reacting compounds of formula (VIII) with an heteroaryl amine by means of a transamidation reaction in the presence of a strong base such as BuLi, LiHMDS or LDA in an appropriate solvent, such as THF.

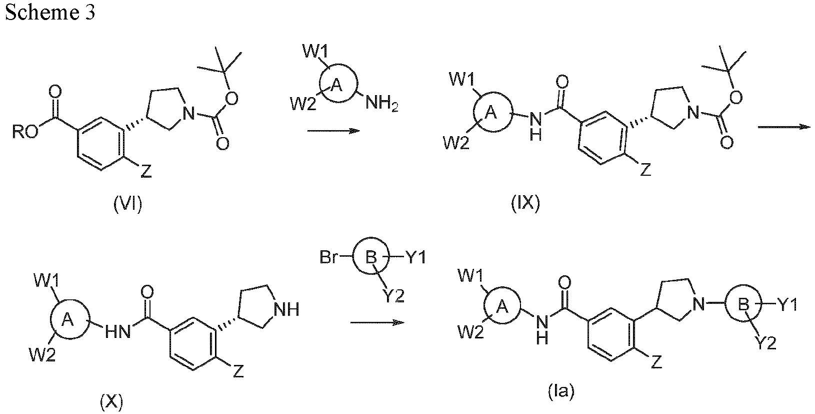

In a different approach, compounds of formula (Ia), for instance with S configuration at the stereocenter, can be obtained as described in Scheme 3, by performing as first step a transamidation reaction between an heteroaryl amine and a compound of formula (VI), wherein R is (C₁-C₄)alkyl, preferably ethyl, in the presence of a strong base such as BuLi, LiHMDS or LDA in an appropriate solvent, like THF, followed by the removal of the protecting group in acidic conditions, using TFA or HCl in 4-dioxane to give compounds of formula (X). Compounds of formula (X) can be converted in compounds of formula (Ia) by means of a Buchwald coupling reaction, using an appropriate palladium catalyst such as Pd₂(dppf)Cl₂, Buchwald 3rd generation catalysts, such as for instance BPC-304 or BPC-305 or Pd 175 (all by Johnson Matthey, UK), or any other palladium source/phosphine-based ligand at high temperature (around 100 °C) for few hours, in the presence of a base, such as Cs₂CO₃, NaOtBu or LiHMDS, in the appropriate solvent, such as 4-dioxane or DMA.

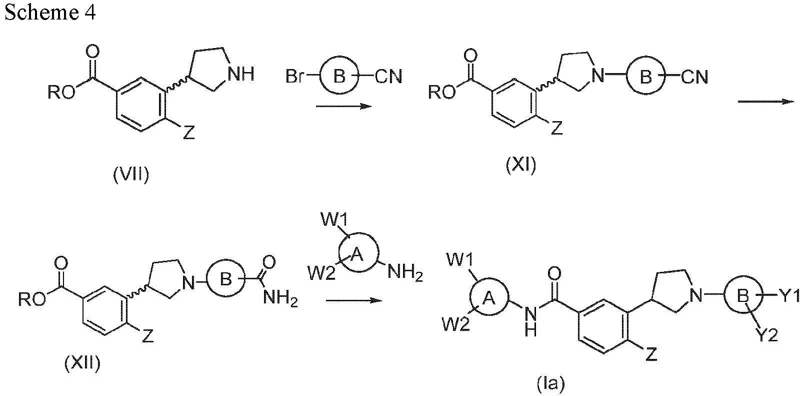

In a different approach, compounds of formula (Ia) with a defined stereochemistry, and wherein Y1 is CONH₂ and Y2 is hydrogen, can be prepared as depicted in Scheme 4 from compounds of formula (VII), wherein R is (C₁-C₄)alkyl, preferably ethyl, with the suitable configuration at the stereocenter. Such compounds of formula (VII) can undergo a Buchwald coupling reaction with the appropriate cyano-heteroaryl bromide, in the presence of a suitable palladium source/phosphine-based ligand at high temperature, in the presence of DMA and an inorganic base such as Cs₂CO₃ to give compounds of formula (XI). Compounds of formula (XII) may be obtained after conversion of cyano group (CN) to primary amide group in the presence of a peroxide, such as hydrogen peroxide, an inorganic base such as K₂CO₃ and the suitable solvent, such as DMSO; followed by a transamidation reaction with an heteroaryl amine in the presence of a strong base, such as BuLi, LiHMDS or LDA, in an appropriate solvent, like THF, to give compounds of formula (Ia) (wherein Y1 is CONH₂ and Y2 is hydrogen).

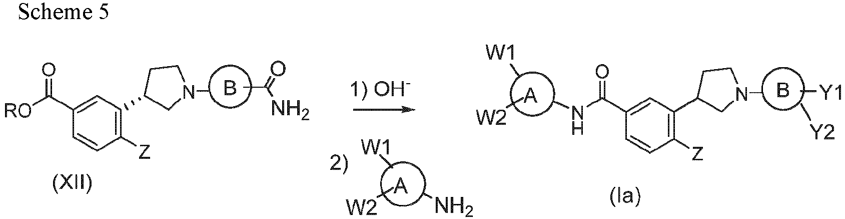

Alternatively, compounds of formula (Ia) with a defined stereochemistry, and wherein Y1 is CONH₂ and Y2 is hydrogen, can be obtained as depicted in Scheme 5 from compounds of formula (XII), wherein R is (C₁-C₄)alkyl, preferably ethyl, by means of basic hydrolysis of the ester in the presence of LiOH in a mixture of H₂O/THF, followed by amidation using common coupling reagents such as HATU, in presence of an organic base as DIPEA in the appropriate solvent as DMF to give compounds of formula (Ia) (wherein Y1 is CONH₂ and Y2 is hydrogen).

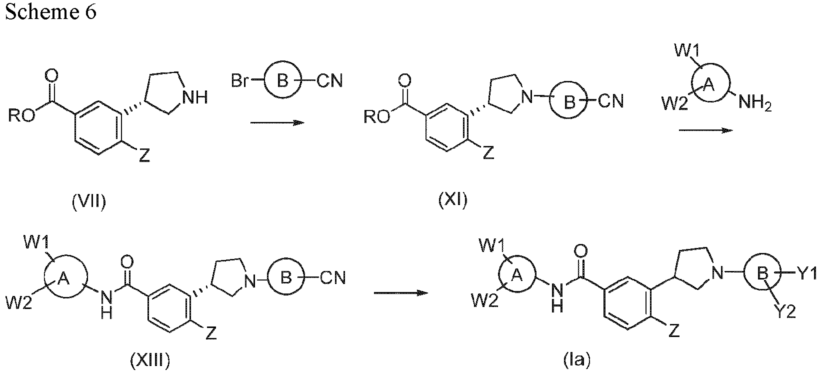

Alternatively, compounds of formula (Ia) with a defined stereochemistry, and wherein Y1 is CONH₂ and Y2 is hydrogen, can be prepared as depicted in Scheme 6 by starting from compounds of formula (VII), wherein R is (C₁-C₄)alkyl, preferably ethyl, to give compounds of formula (XI) through a Buchwald coupling reacting the appropriate cyano-heteroaryl bromide in the presence of a suitable palladium source/phosphine-based ligand at high temperature, in the presence of DMA and an inorganic base such as Cs₂CO₃. Compounds of formula (XI) can be converted in compounds of formula (XIII) through a transamidation with the suitable amine, followed by conversion from cyano group to primary amide group in oxidizing conditions in the presence of a peroxide, such as such as H₂O₂, an inorganic base, such as K₂CO₃, and a suitable solvent, such as DMSO, to give compound of formula (Ia).

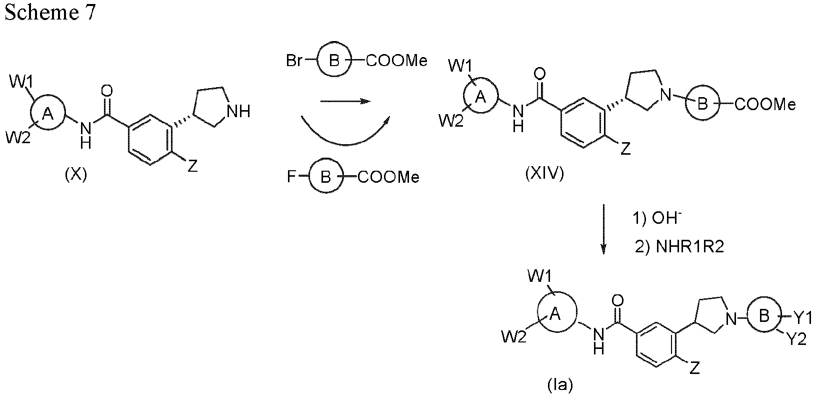

In another approach, compounds of formula (Ia) with a defined stereochemistry, and wherein Y1 is CONR1R2 and Y2 is hydrogen, can be prepared as depicted in Scheme 7 by starting from compounds of formula (X) by performing a Buchwald coupling using the appropriate commercially available bromo-heteroaryl ester. After basic hydrolysis of the ester, for instance by using LiOH in a mixture of H₂O/THF, compounds of formula (Ia) can be prepared by amidation with an amine NHR1R2 under suitable amide coupling reaction conditions in the presence of an activating agent, such as HATU or TBTU, with an organic base, such as DIPEA or TEA, in a suitable organic solvent, such as DCM or DMF, and at a temperature generally around RT for a time ranging from a few hours to overnight. Alternatively, such compounds of formula (Ia) can be obtained by converting compounds of formula (X) into compounds of formula (XIV) through a nucleophilic aromatic substitution (S_{N}Ar) using the suitable commercially available fluoro-heteroaryl ester in the presence of a suitable base, such as DIPEA, and the appropriate solvent, such as DMSO, followed by basic ester hydrolysis and amidation to give said compounds of formula (Ia).

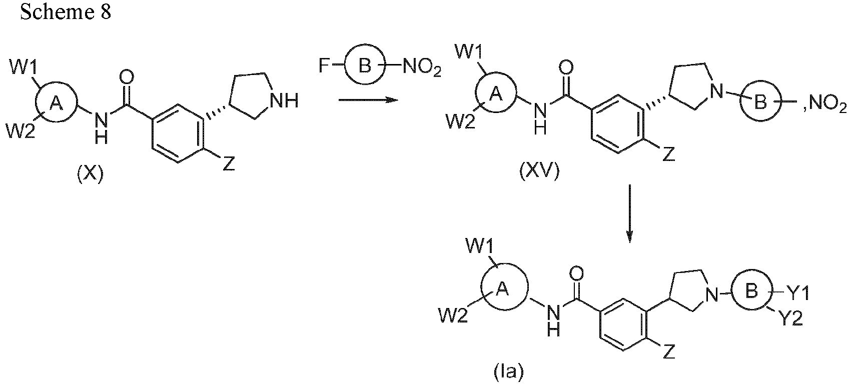

In a different approach, compounds of formula (Ia) with a defined stereochemistry, and wherein Y1 is NH₂ and Y2 is hydrogen, can be obtained as depicted in Scheme 8, from compounds of formula (X) by reacting with the appropriate commercially available NO₂-heteroaryl fluoride under nucleophilic aromatic substitution conditions using an appropriate inorganic base, such as cesium carbonate, followed by reduction of the nitro group to amine group under hydrogen atmosphere and in the presence of a suitable catalyst, such as Pd/C, in a suitable solvent, such as EtOH or EtOAc.

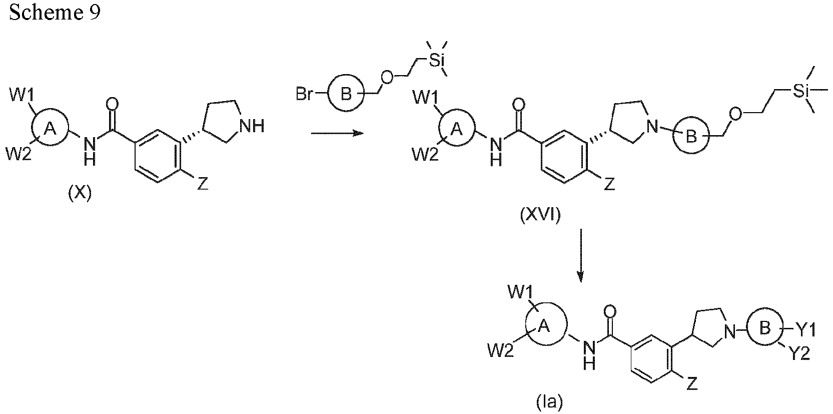

Alternatively, compounds of formula (Ia) can be obtained as depicted in Scheme 9 starting from compounds of formula (X) by reaction with a SEM-protected heteroaryl bromide under Buchwald coupling conditions using a suitable palladium source/phosphine-based ligand at high temperature, in a suitable solvent, such as dioxane, in the presence of the appropriate base, such as NaOtBu. Compounds of formula (Ia) may be obtained after SEM removal under acidic conditions in the presence of HCl in dioxane.

Accordingly, the present invention provides intermediate compounds of formula (IV), (V), (VI), (VII), (VIII), (IX), (X), (XI), (XII), (XIII), (XIV), (XV), and (XVI), as defined above, and their use in the preparation of compounds of formula (Ia).

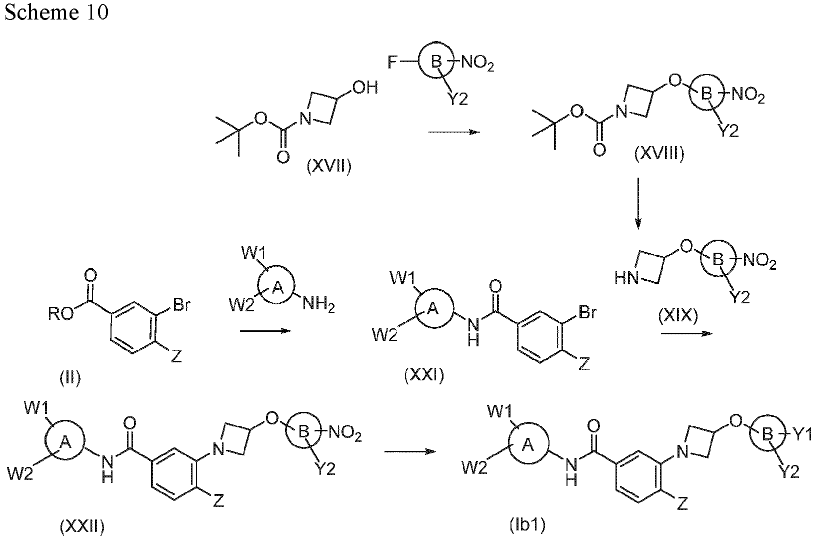

Compounds of formula (Ib1), which are compounds of formula (Ib) wherein L is 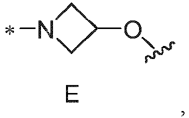 can be prepared as depicted in Scheme 10 by starting from compounds of formula (XVII) by reacting with the appropriate commercially available nitro-heteroaryl fluoride under nucleophilic aromatic substitution conditions using an appropriate inorganic base, such as cesium carbonate, and the appropriate solvent, such as DMSO, followed by the removal of the protecting group in acidic conditions, for instance by using HCl in 4-dioxane to give compounds of formula (XIX). Such compounds of formula (XIX) can undergo a Buchwald coupling reaction with compounds of formula (XXI), obtained after a transamidation between compounds of formula (II), as defined above, wherein R is (C₁-C₄)alkyl, preferably methyl, and appropriate eteroarylamines, in the presence of a suitable palladium source/phosphine-based ligand at high temperature, in the presence of DMA and an inorganic base, such as Cs₂CO₃, to give compounds of formula (XXII). Compounds of formula (Ib1) wherein Y1 is NH₂ were obtained from compounds of formula (XXII) by reduction of the nitro group to amine group under hydrogen atmosphere and in the presence of a suitable catalyst, such as Pd/C, in a suitable solvent, such as EtOH or EtOAc.

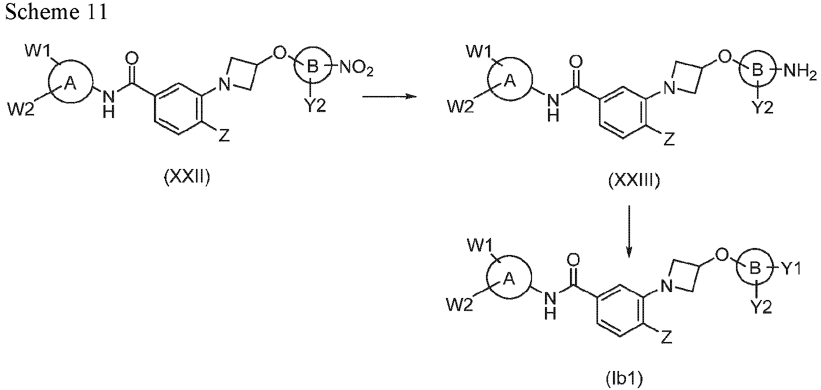

In a different approach, compounds of formula (Ib1) wherein Y1 is NHCOR1 can be obtained as depicted in Scheme 11 by starting from compounds of formula (XXII) by reduction of the nitro group to amine group under hydrogen atmosphere and in the presence of a suitable catalyst, such as Pd/C, in a suitable solvent, such as EtOH or EtOAc to give compounds of formula (XXIII). Compounds of formula (Ib 1) can be prepared by acetylation of compounds of formula (XXIII) with the suitable commercially available acyl chloride, i.e. R1COC1, or anhydride, in the presence of an appropriate base, such as pyridine or TEA.

Accordingly, the present invention provides intermediate compounds of formula (XVII), (XIX), (XXI), (XXII), and (XXIII), as defined above, and their use in the preparation of compounds of formula (Ib) or (Ib 1).

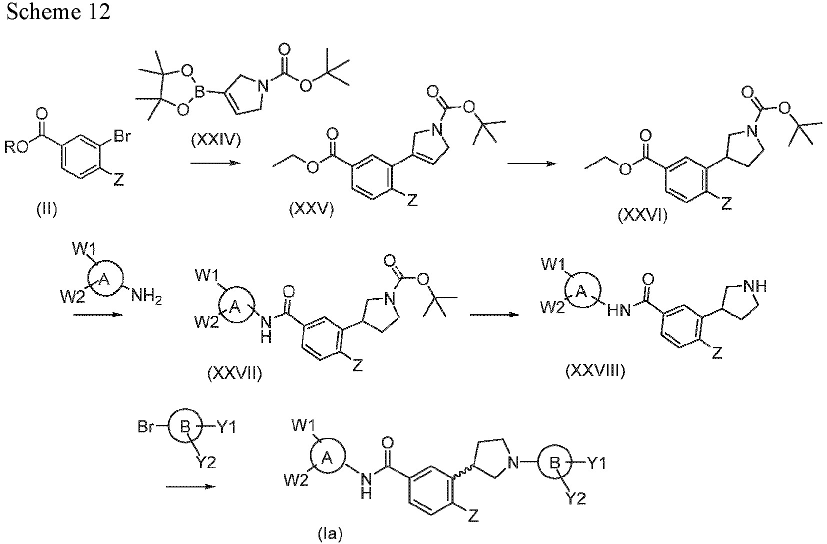

Compounds of formula (Ia) with a defined stereochemistry can be prepared as depicted in Scheme 12 starting from the compounds of formula (II) as defined above. The reaction may be carried out by reacting a compound of formula (II) with tert-butyl 3-(4,4,5,5-tetramethyl-1,3,2-dioxaborolan-2-yl)-2,5-dihydro-1H-pyrrole-1-carboxylate by following the usual Suzuki protocol, in a suitable organic solvent, such as dioxane, in the presence of an inorganic base, such as cesium carbonate, with an appropriate palladium catalytic system, such as Pd₂(dppf)Cl₂, at high temperature (around 100°C) for few hours. The compound of formula (XXV) can be converted into a compound of formula (XXVI) by reduction under hydrogen atmosphere, typically at 5 bar pressure, in the presence of a suitable catalyst, such as Pd/C, in a suitable solvent, such as, but not limited to, EtOH, at a temperature ranging from rt to 60 °C, for few hours.

Compounds of formula (XXVIII) can be prepared by reacting compounds of formula ((XXVI) with an heteroaryl amine by means of a transamidation reaction in the presence of a strong base, such as BuLi, LiHMDS or LDA, in an appropriate solvent, such as THF, followed by removing the Boc protecting group in acidic conditions, using for instance an organic acid, such as TFA.

Compounds of formula (Ia) can be obtained by cross-coupling reactions like Buchwald coupling in the presence of a palladium catalyst, such as Pd-170 or another palladium source/phosphine-based ligand, at high temperature (around 120 °C) for a few hours with a suitable heteroaryl halide, in an organic solvent, such as dimethylacetamide, with an appropriate base, such as Cs₂CO₃.

Accordingly, the present invention provides intermediate compounds of formula (XXV), (XXVI), (XXVII) and (XXVIII), as defined above, and their use in the preparation of compounds of formula (Ia).

Compounds of formula (Ia), comprising a stereocenter at the pyrrolidine ring, are obtained as racemic mixtures, if not specified otherwise. Separation of racemic mixtures may be achieved by chiral resolution methods, such as chiral chromatographic purification. Both enantiomers of compounds of formula (Ia) are included in the scope of the present invention. When the absolute configuration at the stereocenter is not determined, the stereoisomers, or enantiomers, are identified as "1^{st} eluted" and "2^{nd} eluted" in the chiral chromatographic purification, according to the method used.

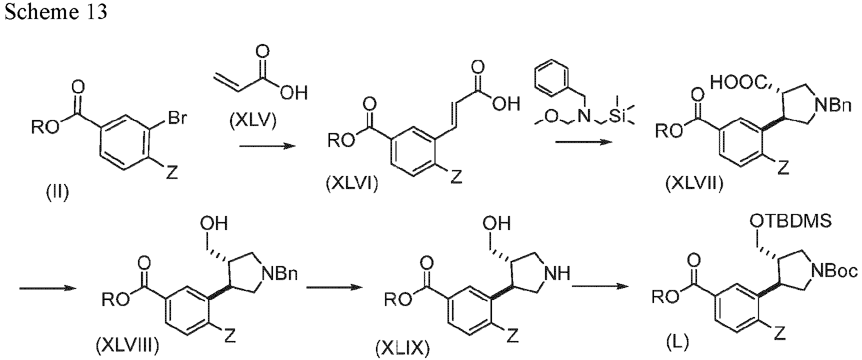

As described in Scheme 13, Intermediate compounds of formula (L) can be prepared reacting a compound of formula (II), as defined above, and compound (XLV), by performing a palladium-catalyzed cross-coupling Heck type. The reaction may be carried out by reacting a compound of formula (II) and a general enone, such as propenoic acid (XLV), in the presence of a base, such as TEA, of a ligand, such as triphenylphosphine, of a palladium source, such as palladium acetate, in a suitable organic solvent, such as DMF, at high temperature, around 110°C, for few hours.

1,3-dipolar cycloaddition may be carried out reacting the α,β-unsaturated compound of formula (XLVI) with a 1,3-dipole or a suitable precursor, such as an azomethine ylide, for example with N-benzyl-1-methoxy-N-((trimethylsilyl)methyl)methanamine as shown in Scheme 13, under acid catalysis, for instance by TFA, in a solvent, such as dioxane, to obtain a compound of formula (XLVII). Such compounds can be converted into compounds of formula (XLVIII) via acyl chloride formation, using an appropriate reagent, such as oxalyl chloride, in a suitable solvent, such as THF, at rt, followed by reduction with a reducing agent, such as lithium borohydride. Compounds of formula (XLIX) can then be prepared by hydrogenation using Pd/C in acidic pH in a suitable solvent, such as EtOH at rt, for the removal of the benzyl (Bn) protecting group. Compounds of formula (L) can be double-protected using di-tert-butyl dicarbonate, in the presence of a base, such as TEA and 4-dimethylamino pyridine, in a suitable solvent, such as DCM at rt, followed by addition of tert-butyldimethylchlorosilane, in the presence of TEA at rt.

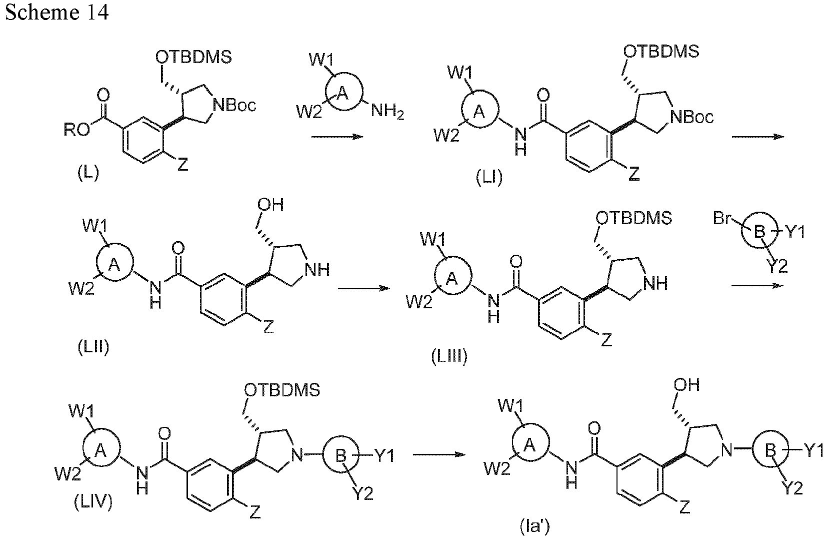

Compounds of formula (Ia'), which are compounds of formula (I) wherein L is 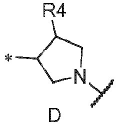 wherein R4 is hydroxymethyl, can be prepared as depicted in Scheme 14 by starting from corresponding compounds of formula (L).

Compounds of formula (LII) can be prepared by reacting compounds of formula (L), wherein Z is as defined above and R is (C₁-C₄)alkyl, preferably ethyl, with an heteroaryl amine by means of a transamidation reaction in the presence of a strong base such as BuLi, LiHMDS or LDA, in an appropriate solvent, such as THF, followed by the removal of the Boc protecting group in acidic conditions, using TFA, in a solvent, such as DCM, at rt. Compounds of formula (LIII) can be obtained by reacting compounds of formula (LII) with tert-butyldimethylchlorosilane in the presence of a base, such as TEA, in a suitable solvent, such as DCM at rt. Compounds of formula (Ia') can then be obtained by means of a Buchwald coupling reaction, using an appropriate palladium catalyst, such as Pd₂(dppf)Cl₂, Buchwald 3rd generation catalysts or any other palladium source/phosphine-based ligand, at high temperature (around 100 °C) for few hours, in the presence of a base, such as Cs₂CO₃, NaOtBu or LiHMDS, in the appropriate solvent, such as 4-dioxane or DMA, followed by the removal of the TBDMS protecting group in acidic conditions, using HCl 37%, in a solvent, such as water and ethanol, at rt.

Accordingly, the present invention provides intermediate compounds of formula (XLVI), (XLVII), (XLVIII), (XLIX), (L), (LI), (LII), (LIII) and (LIV), as defined above, and their use in the preparation of compounds of formula (Ia').

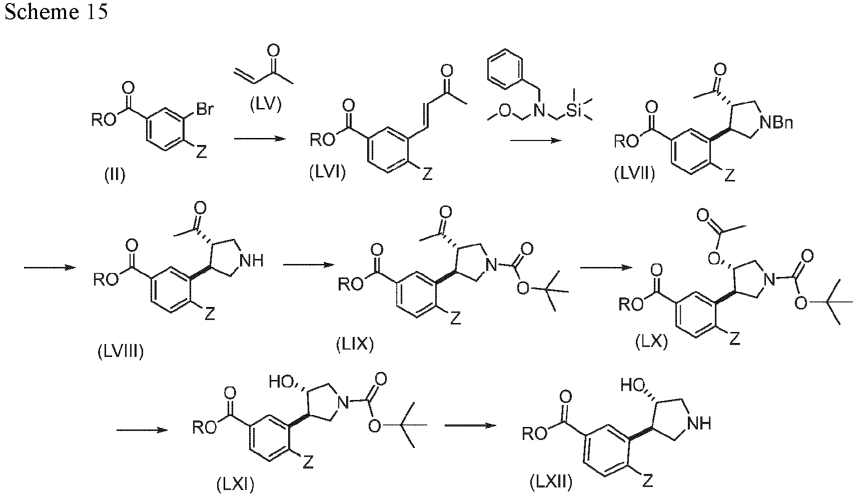

As described in Scheme 15, Intermediate compounds of formula (LXII) can be prepared by reacting a compound of formula (II), as defined above, and compound (LV) in a Heck type palladium-catalyzed cross-coupling. The reaction may be carried out by reacting a compound of formula (II) and a general enone, such as but-3-en-2-one, in the presence of a base, such as TEA, in the presence of a ligand, such as triphenylphosphine, in the presence of a palladium source, such as palladium acetate, in a suitable organic solvent, such as DMF, at high temperature, around 110°C, for few hours.

1,3-dipolar cycloaddition may be then carried out reacting the α,β-unsaturated compound of formula (LVI) with a 1,3-dipole, or a suitable precursor such as an azomethine ylide, for example with N-benzyl-1-methoxy-N-((trimethylsilyl) methyl)methanamine as shown in Scheme 15, under acid catalysis, for instance by TFA, in a solvent, such as dioxane. Compounds of formula (LVIII) can be prepared by hydrogenation using Pd/C at acidic pH in a suitable solvent, such as EtOH at rt. Compounds of formula (LIX) can be prepared using di-tert-butyl dicarbonate, in the presence of a base, such as TEA and 4-dimethylamino pyridine, in a suitable solvent, such as DCM at rt.

Compounds of formula (LX) can be prepared via Baeyer-Villiger reaction, in the presence of a peracid, as an oxidizing agent, for example trifluoroperacetic acid, formed in situ combining urea-hydrogen peroxide (1:1) complex and trifluoroacetic anhydride in a suitable solvent, such as DCM at rt and eventually in the presence of a suitable base, such as potassium carbonate, or a buffer agent, such as sodium phosphate dibasic.

Compounds of formula (LXII) can be obtained by deprotection in the presence of a base, such as potassium carbonate, in a suitable solvent, such as EtOH, at 40 °C, followed by the removal of the protecting group in acidic conditions, using e.g. HCl in 1,4-dioxane, at rt.

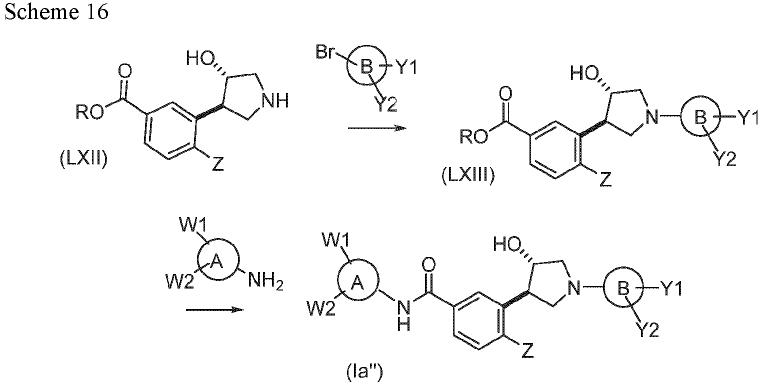

Compounds of formula (Ia"), which are compounds of formula (I) wherein L is 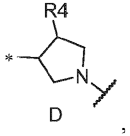 wherein R4 is hydroxy, can be prepared as depicted in Scheme 16 by starting from corresponding compounds of formula (LXII).

Compounds of formula (LXIII) can be obtained by means of a Buchwald coupling reaction, using an appropriate palladium catalyst, such as Pd₂(dppf)Cl₂, Buchwald 3rd generation catalysts or any other palladium source/phosphine-based ligand, at high temperature (around 100 °C) for few hours, in the presence of a base, such as Cs₂CO₃, NaOtBu or LiHMDS, in the appropriate solvent, such as 4-dioxane or DMA. Compounds of formula (Ia") can be prepared by reacting compounds of formula (LXIII) with an heteroaryl amine by means of a transamidation reaction in the presence of a strong base, such as BuLi, LiHMDS or LDA, in an appropriate solvent, such as THF.

Accordingly, the present invention provides intermediate compounds of formula (LVI), (LVII), (LVIII), (LIX), (LX), (LXI), (XLII) and (LXIII), as defined above, and their use in the preparation of compounds of formula (Ia").

The compounds of formula (I) of the present invention have surprisingly been found to effectively inhibit both receptor DDR1 and DDR2. Advantageously, the inhibition of receptors DDR1 and DDR2 may result in efficacious treatment of the diseases, disorders or conditions wherein the DDR receptors are involved.

In this respect, it has been found that the compounds of formula (I) of the present invention have a very high antagonist drug potency on DDR1 and DDR2. Table 3 in the present experimental part reports such potency expressed as inhibition constant Ki for representative compounds of formula (I) of the invention. In particular, potencies wherein the Ki is below 100 nM are reported. Preferred compounds of the present invention have a Ki on DDR1 and DDR2 between 25 and 5 nM. Even more preferred compounds of the present invention have a Ki on DDR1 and DDR2 lower than 5 nM.

In one aspect, the present invention refers to a compound of formula (I) according to any of the embodiments disclosed above for use as a medicament.

In a preferred embodiment, the invention refers to a compound of formula (I), and pharmaceutically acceptable salts thereof, for use in treating diseases, disorders, or conditions associated with dysregulation of DDR.

In another aspect, the invention refers to the use of a compound of formula (I) as above described, and pharmaceutically acceptable salts thereof, in the preparation of a medicament for the treatment of disorders associated with dysregulation of DDR.

In another preferred embodiment, the invention refers to a compound of formula (I), and pharmaceutically acceptable salts thereof, for use in the prevention and/or treatment of a disease, disorder or condition associated with DDR receptor mechanism. In a more preferred embodiment, the present invention refers to a compound of formula (I) for use in the prevention and/or treatment of fibrosis and/or diseases, disorders or conditions that involve fibrosis.

The terms "fibrosis" or "fibrosing disorder," as used herein, refer to conditions that are associated with the abnormal accumulation of cells and/or fibronectin and/or collagen and/or increased fibroblast recruitment and include, but are not limited to, fibrosis of individual organs or tissues such as the heart, kidney, liver, joints, lung, pleural tissue, peritoneal tissue, skin, cornea, retina, musculoskeletal and digestive tract.

Preferably, the compounds of formula (I) as above described are useful for the treatment and/or prevention of fibrosis, such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) as above described are useful for the treatment of idiopathic pulmonary fibrosis (IPF).

In one aspect, the invention also refers to a method for the prevention and/or treatment of disorders associated with DDR receptors mechanisms, said method comprising the step of administering to a patient in need thereof a therapeutically effective amount of a compound of formula (I) as above described.

In a further aspect, the invention refers to the use of a compound of formula (I) as above described for the treatment of disorders associated with DDR receptors mechanism.

In another aspect, the invention refers to the use of a compound of formula (I) as above described in the preparation of a medicament for the treatment of disorders associated with DDR receptors mechanism.

In a further aspect, the invention refers to a method for the prevention and/or treatment of a disorder or condition associated with the dysregulation of DDR receptors 1 and 2, said method comprising administering to a patient in need of such treatment a therapeutically effective amount of a compound of formula (I) as above described.

In a further aspect, the present invention refers to the use of a compound of formula (I) as above described for the treatment of a disease, disorder or condition associated with dysregulation of DDR receptors 1 and 2.

As used herein, "safe and effective amount" in reference to a compound of formula (I) or a pharmaceutically acceptable salt thereof or other pharmaceutically active agent means an amount of the compound sufficient to treat the patient's condition but low enough to avoid serious side effects and that can nevertheless be routinely determined by the skilled artisan.

The compounds of formula (I) may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) according to any of its embodiment in admixture with at least one or more pharmaceutically acceptable carrier and/or excipient.

In one embodiment, the invention refers to a pharmaceutical composition of compounds of formula (I) in admixture with at least one or more pharmaceutically acceptable carrier and/or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage form such as aqueous and non-aqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of Formula (I) according to the invention, in form of a single- or multidose dry powder inhaler or a metered dose inhaler.

All preferred groups or embodiments described above for compounds of formula (I) may be combined with each other and apply as well *mutatis mutandis.*

The various aspects of the invention described in this application are illustrated by the following examples which are not meant to limit the invention in any way.

### PREPARATIONS OF INTERMEDIATES AND EXAMPLES

Chemical Names of the compounds were generated with Structure-To-Name tool of PerkinElmer ChemDraw Professional application (v. 20.0.0.41.). All reagents, for which the synthesis is not described in the experimental part, are either commercially available, or are known compounds or may be formed from known compounds by known methods by a person skilled in the art.

In the procedures that follow, some of the starting materials are identified through an "Intermediate" or "Example" number with indications on step number. This is provided merely for assistance to the skilled chemist.

When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions. All compounds were obtained as a free base, unless stated otherwise.

### Abbreviations

AcOH = Acetic acid; Acetone-d₆= deuterated acetone; ACN = acetonitrile; ACN-d₃ = deuterated acetonitrile; CDCl₃ = deuterated chloroform; CV = Column Volumes; DCM = dichloromethane; DIPEA = N,N-Diisopropylethylamine; DMF = dimethylformamide; DMSO = dimethyl sulfoxide; DMSO-d₆ = deuterated dimethyl sulfoxide; ee = enantiomeric excess; Et₂O = diethyl ether; EtOAc =Ethyl acetate; eq.= equivalents; FCC= flash column chromatography; h = hour/s; HATU = 1-[Bis(dimethylamino)methylene]-1H-1,2,3-triazolo[4,5-b]pyridinium 3-oxid hexafluorophosphate; HCOOH = formic acid; HPLC = high pressure liquid chromatography; LC-MS = Liquid Chromatography/Mass Spectrometry; LDA = Lithium diisopropylamide; Na/LiHMDS = Sodium/Lithium bis(trimethylsilyl)amide; MeOH = methyl alcohol; min = minute/s; TBAF = Tetrabutylammonium fluoride; 2-MeTHF= 2-Methyltetrahydrofuran; NaBH₃CN = sodium cyanoborohydride; NMR = nuclear magnetic resonance; MeOH-d₄ = deuterated methanol; Pd(dba)₂ = Bis(dibenzylideneacetone)palladium(O); Pd(dppf)Cl₂ = [1,1'-Bis(diphenylphosphino)ferrocene]dichloropalladium(II); RT/rt = room temperature; RuPhos = 2-Dicyclohexylphosphino-2',6'-diisopropoxybiphenyl; SCX = strong cation exchange; SFC = supercritical fluid chromatography; STAB = sodium triacetoxyborohydride; SM = starting material; tBu = tert-Butyl; tBuBrettPhos Pd G3= [(2-Di-tert-butylphosphino-3,6-dimethoxy-2',4',6'-triisopropyl-1,1'-biphenyl)-2-(2'-amino-1,1'-biphenyl)]palladium(II) methanesulfonate generation 3; tBuOK= Potassium tert-butoxide; TBDMS = tert-butyldimethylsilyl; TBTU= 2-(1H-Benzotriazole-1-yl)-1,1,3,3-tetramethylaminium tetrafluoroborate; TEA = triethylamine; TFA= trifluoroacetic acid; THF = tetrahydrofuran; TLC = Thin Layer Chromatography; t*_{R}* = retention time; UPLC = Ultra Performance Liquid Chromatography; VCD= Vibrational circular dichroism; XantPhos = 4,5-Bis(diphenylphosphino)-9,9-dimethylxanthene; IPA = Isopropanol; WE = working electrode; CE = Counter electrode.

### General Experimental details

### NMR characterization:

¹H NMR spectra were recorded on Varian MR-400 spectrometer operating at 400 MHZ (proton frequency), equipped with: a self-shielded Z-gradient coil 5 mm 1H/nX broadband probe head for reverse detection, deuterium digital lock channel unit, quadrature digital detection unit with transmitter offset frequency shift. Chemical shifts are reported as δ values in ppm relative to tetramethyl silane (TMS) as an internal standard. Coupling constants (J values) are given in hertz (Hz) and multiplicities are reported using the following abbreviation (s= singlet, d=doublet, t=triplet, q=quartet, dd= doublet of doublets, dt=doublet of triplets, m=multiplet, br=broad, nd=not determined).

In some cases, signals NH from amide bond or amine bond (Exchangeable protons) are not visible. In a few cases, some signals could be hidden under the signal of water or under the signal of DMSO or other residual solvents.

### LC/UV/MS Analytical Methods

LC/MS retention times are estimated to be affected by an experimental error of ± 0.5 min.

**Method 1:** Acquity CSH C18 column 50mm x 2.1mm 1.7µm, maintained at 40°C; Mobile Phase: Eluent B (ACN/water 95:5 +0.05% HCOOH) in Eluent A (water/ACN 95:5 +0.05% HCOOH) from 1% to 99.9% within 1.5 min. Flow rate: 1 mL/min. Wavelength: 210-400 nm DAD. UPLC + Waters PDA + Waters QDA.

**Method 2:** Waters Sunfire C18 column, 4.6x50mm, 3.5 µm, maintained at 40°C. Mobile phase ACN in water + 10mM ammonium bicarbonate, from 5 to 95% within 2.5 min. Flow rate: 2.0 mL/min. Wavelength: 210-400 nm DAD. Waters 2795 separations module + Waters DAD + Micromass ZQ, single quadrapole LC-MS.

**Method 3:** Acquity CSH C18 column 50mm x 2.1mm 1.7µm, maintained at 40°C; Mobile Phase: Eluent B (ACN/water 95:5 +0.05% HCOOH) in Eluent A (water/ACN 95:5 +0.05% HCOOH) from 1% to 99.9% within 3.5 min. Flow rate: 1 mL/min. Wavelength: 210-400 nm DAD. UPLC + Waters PDA + Waters QDA.

**Method 4:** Waters Acquity QSM, Kinetex C8 column 100mm x 2.1mm 1.7µm, maintained at 55°C; Mobile Phase: Eluent A (HCOONH4 0.025M pH 3), Eluent B (ACN+0.1% FA). Gradient mode: from 0 to 3 min. eluent B is increased from 1% to 30%, from 3 to 6.50 min. is increased from 30% to 50%, from 6.50 to 7.50 min. is increased from 50% to 80%, from 7.50 to 8 min is kept at 80%, from 8 to 8.10 min. is decreased from 80% to 1% and from 8.10 it is kept at 1% till the end at 10 min. Flow rate: 0.5 mL/min. Wavelength: 210-400 nm PAD. UPLC + Waters PDA + Xevo TQS MS instrument.

**Method 5:** Acquity UPLC HSS C18 column, 100 × 2.1mm, 1.8 µm (Plus guard cartridge), maintained at 40°C. Mobile phase: ACN (0.1% formic acid) in water (0.1% formic acid) from 5% to 95% within 5.6 min. Flow rate: 0.4 ml/min. Wavelength: 210-400 nm DAD. UPLC + Waters DAD + Waters SQD2, single quadrapole UPLC-MS.

**Method 6:** Agilent Zorbax column 4.6x50mm, 3.5 µm, maintained at 40°C. Mobile phase: ACN (0.1% formic acid) in water (0.1% formic acid), from 5% to 95% within 2 min. Flow rate: 3.0 mL/min. Wavelength: 210-400 nm DAD. Waters 2795/2695 separations module + Waters DAD + Micromass ZQ, single quadrapole LC-MS.

**Method 7:** Acquity BEH C18 (2.1mm x 50mm, 1.7um) maintained at 60°C; Flow Rate 1.0 mL/min; Detector Wavelength 220-300nm; Injection Volume 1.0 uL; Mobile Phase Eluent B ACN Eluent A Water (0.1% v/v TFA) from 2% to 98% within 2 min. Flow rate: 1 mL/min. Wavelength: 220-300 nm.

**Method 8:** Acquity UPLC BEH Shield RP18 column, 100 × 2.1mm, 1.72µm (Plus guard cartridge), maintained at 40°C. Mobile phase: ACN in water + 10 nM ammonium bicarbonate from 5% to 95% within 5.6 min. Flow rate: 0.4 ml/min. Wavelength: 210-400 nm DAD. UPLC + Waters DAD + Waters SQD2, single quadrapole UPLC-MS.

**Method 9:** Kinetex^{®} XB-C18 column, 4.6x50 mm, 2.6 µm maintained at 25 °C. Mobile phase: water (0.1% formic acid) in MeCN (0.1% formic acid), from 80% to 5% within 3.90 min; Flow rate: 1.0 ml/min; wavelength: 190-340 nm DAD. Dionex UHPLC Ultimate 3000 with DAD detector/Thermo Scientific ISQ EC mass spectrometer.

**Method 10:** Kinetex^{®} XB-C18 column, 4.6x50 mm, 2.6 µm maintained at 25 °C. Mobile phase: water (0.1% formic acid) in MeCN (0.1% formic acid), from 90% to 5% within 3.90 min; Flow rate: 1.0 ml/min; wavelength: 190-340 nm DAD. Dionex UHPLC Ultimate 3000 with DAD detector/Thermo Scientific ISQ EC mass spectrometer.

**Method 11:** Kinetex^{®} XB-C18 column, 4.6x50 mm, 2.6 µm maintained at 25 °C. Mobile phase: water (0.1% formic acid) in MeCN (0.1% formic acid), from 90% to 5% within 3.90 min; Flow rate: 1.0 ml/min; wavelength: 190-340 nm DAD. Dionex UHPLC Ultimate 3000 with DAD detector/Thermo Scientific ISQ EC mass spectrometer.

**Method 12:** Kinetex^{®} XB-C18 column, 4.6x50 mm, 2.6 µm maintained at 25 °C. Mobile phase: water (0.1% formic acid) in MeCN (0.1% formic acid), from 60% to 5% within 3.90 min; Flow rate: 1.0 ml/min; wavelength: 190-340 nm DAD. Dionex UHPLC Ultimate 3000 with DAD detector/Thermo Scientific ISQ EC mass spectrometer.

**Method 13:** Acquity CSH C18 column 50mm x 2.1mm 1.7µm, maintained at 40°C; Mobile Phase: Eluent B (ACN) in Eluent A (water +0.1% HCOOH) from 1% to 99.9% within 1.5 min. Flow rate: 1 mL/min. Wavelength: 210-400 nm DAD. UPLC + Waters PDA + Waters QDA.

**Method 14:** Acquity CSH C18 column 50mm x 2.1mm 1.7µm, maintained at 40°C; Mobile Phase: Eluent B (ACN) in Eluent A (water +0.1% HCOOH) from 1% to 99.9% within 3.5 min. Flow rate: 1 mL/min. Wavelength: 210-400 nm DAD. UPLC + Waters PDA + Waters QDA.

**Method 15:** Agilent Zorbax column 4.6x50 mm, 3.5 µm, maintained at 40°C. Mobile phase: MeCN (0.1% formic acid) in water (0.1% formic acid), from 5% to 95% within 2 minutes. Flow rate: 3.0 mL/minutes. Wavelength: 210-400 nm DAD. Waters 2795/2695 separations module + Waters DAD + Micromass ZQ, single quadrapole LCMS.

**Method 16:** Acquity UPLC BEH - Waters, 1.7 µm C18 (2.1 x 100 mm), 130 Å, maintained at 25 °C. Mobile phase: water (0.1% formic acid) in MeCN (0.1% formic acid), from 80% to 5% within 2.70 min; Flow rate: 0.5 ml/min; wavelength: 254 nm. Shimadzu LCMS-2020 Single Quadrupole Liquid Chromatograph Mass Spectrometer.

### Chiral Supercritical Fluid Chromatography (SFC) separation protocol

The diastereomeric separation of compounds was achieved by Supercritical Fluid Chromatography (SFC) using a Gilson Preparative LC system. Analysis of two enantiomers was performed from reconstituted final samples.

Otherwise, the diastereomeric separation of compounds was achieved by Supercritical Fluid Chromatography (SFC) using a Waters Thar Prep100 preparative SFC system (P200 CO2 pump, 2545 modifier pump, 2998 UV/VIS detector, 2767 liquid handler with Stacked Injection Module). The Waters 2767 liquid handler acted as both auto-sampler and fraction collector. Appropriate isocratic methods were selected based on methanol, ethanol or isopropanol solvent systems under un-modified or basic conditions. The standard SFC method used was modifier, CO2, 100 mL/min, 120 Bar backpressure, 40 °C column temperature. The modifier used under basic conditions was diethylamine (0.1% V/V). The modifier used under acidic conditions was either formic acid (0.1% V/V) or trifluoroacetic acid (0.1% V/V). The SFC purification was controlled by Waters Fractionlynx software through monitoring at 210-400 nm and triggered at a threshold collection value, typically 260 nm. Collected fractions were analysed by SFC (Waters/Thar SFC systems with Waters SQD). The fractions that contained the desired product were concentrated by vacuum centrifugation.

### Supercritical Fluid Chromatography - Mass Spectrometry analytical conditions

**Method 1:** SFC-MS was performed on a Gilson Preparative LC system (Gilson Pump - 333; Gilson 151; Gilson Valvemate 6 position) using a Chiralcel OD-H (30mm x 250mm, 5um) column with an isocratic run (10:90 MeOH:CO₂), Flow Rate 180 mL/min, BPR 120 BarG, Detector Wavelength 237 nm, Injection Volume 1500 µL (135 mg), 40°C column temperature.

**Method 2:** SFC-MS was performed on a Gilson Preparative LC system using a Lux C1 (4.6mm x 250mm, 5um) column with a isocratic run (20:80 MeOH: CO₂ (0.2% v/v NH3), Detector Wavelength 210-400nm, Injection Volume 1.0 µL, BPR 125 BarG, at 4 mL/min, 40°C column temperature.

**Method 3:** SFC-MS was performed on a Gilson Preparative LC system using a AMS (4.6mm x 250mm, 5um) column with a isocratic run (20:80 MeOH:CO₂ (0.2% v/v NH₃), Detector Wavelength 210-400nm, Injection Volume 1.0 µL, BPR 125 BarG, at 4mL/min, 40°C column temperature.

**Method 4:** SFC-MS was performed on a Waters^{™}/Thar SFC systems with Waters^{™} SQD using a LUX Cellulose-1 (20x250 mm, 5 µm) column with an isocratic run (35% MeOH (NH₄OH 0.1%):CO₂), flow rate 100 mL/min, 120 bar, 40 °C column temperature, DAD wavelength 265 nm.

**Method 5:** SFC-MS was performed on a Waters^{™}/Thar SFC systems with Waters^{™} SQD using a LUX Cellulose-1 (20x250 mm, 5 µm) column with an isocratic run (50% IPA (NH₄OH 0.1%):CO₂), flow rate 100 mL/min, 120 bar, 40 °C column temperature, DAD wavelength 265 nm.

**Method 6:** SFC-MS was performed on a Waters^{™}/Thar SFC systems with Waters^{™} SQD using a LUX Cellulose-iC5 (20x250 mm, 5 µm) column with an isocratic run (50% EtOH (NH₄OH 0.1%):CO₂), flow rate 100 mL/min, 120 bar, 40 °C column temperature, DAD wavelength 265 nm.

**Method 7:** SFC-MS was performed on a Waters^{™}/Thar SFC systems with Waters^{™} SQD using a LUX Cellulose-iC5 (20x250 mm, 5 µm) column with an isocratic run (30% IPA (NH₄OH 0.1%):CO₂), flow rate 100 mL/min, 120 bar, 40 °C column temperature, DAD wavelength 265 nm.

**Method 8:** SFC-MS was performed on a Waters^{™}/Thar SFC systems with Waters^{™} SQD using a LUX Cellulose-iC5 (20x250 mm, 5 µm) column with an isocratic run (40% MeOH (NH₄OH 0.1%):CO₂), flow rate 100 mL/min, 120 bar, 40 °C column temperature, DAD wavelength 265 nm.

### IR and VCD Spectroscopy

The stereochemistry of two enantiomers of tert-butyl 3-(2-methyl-5-((3-(trifluoromethyl)phenyl)carbamoyl)phenyl)pyrrolidine-1-carboxylate was determined using vibrational circular dichroism (VCD) spectroscopy. By comparing experimentally obtained VCD spectra to computationally simulated ones, the absolute configuration of the enantiomeric pair can be confidently assigned without prior knowledge of their relative stereochemistry. IR spectra are used to aid the assignment of the relative stereochemistry. The IR and VCD difference spectra further confirm the assignment of two enantiomers.

IR and VCD were recorded at BioTools, Inc. on a ChiralIR w/ DualPEM spectrometer at rt. The PEMs were optimized for 1400 cm⁻¹, and a resolution of 4 cm-1 was used throughout. For all experiments, solutions with concentration of 7.1mg in 100uL of CDCl₃ were investigated using a 100 µm path-length cell equipped with BaF₂ windows. The solution spectra were recorded for 24 h per enantiomer. Because both enantiomers are available, baseline corrections were introduced using the spectrum of a virtual racemate.

All solvents were purchased from commercial sources and were used without additional purification. Flash chromatography (FCC) was performed on Biotage Isolera, then SCX (NH) was utilized to obtain free base of the product, unless differently stated.

For reverse phase FCC the following gradient/eluent were used: gradient A:B from 100:0 to 0:10 in 12 CV eluent A: H₂O/ACN/HCOOH 95:5:0.1 Eluent B: H₂O/ACN/HCOOH 5:95:0.1.

### General Synthetic procedures

### Intermediate 1: 5-bromo-N-(2-(dimethylamino)ethyl)nicotinamide

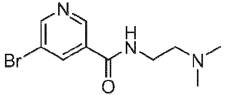

In a 20 mL vial, 5-bromonicotinic acid (200 mg, 0.990 mmol), N1,N1-dimethylethane-1,2-diamine (131 mg, 1.485 mmol) and TBTU (477 mg, 1.485 mmol) were dissolved in DCM (2 ml), then DIPEA (0.345 ml, 1.980 mmol) was added in one portion. The solution was stirred at rt overnight. The crude was washed with sat. sol. NH₄Cl (1x10 ml), sat. sol. NaHCO₃ (1x10 ml) and brine (1x15 mL). The organic layer was dried over MgSO₄ and under reduced pressure. The crude was purified via reverse phase chromatography: gradient A:B from 100:0 to 0:100 in 10 CV; eluent A: water /ACN/HCOOH 95:5:0.1; Eluent B = water/ACN/HCOOH 5:95:0.1. The relevant fractions were combined and loaded onto an Isolute SCX-2 cartridge, washed with MeOH and the product was eluted with 2N methanolic ammonia. The residue was concentrated in vacuo to afford the title compound (60 mg, 0.220 mmol, 22.27 % yield.
LC-MS (ESI, m/z): method 1, t*_{R}* = 0.2 min, m/z (M+1) = 271.83/273.78

Analogously, the following Intermediates were prepared by reacting the bromo derivative with the suitable amine.

### Intermediate 2: 5-bromo-N-(2-methoxyethyl)nicotinamide

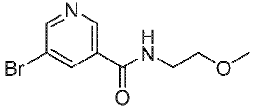

SM: 5-bromonicotinic acid: 0.5 mg (1 eq.)
2-methoxyethan-1-amine: 1.5 g (1.5 eq.)
Amount/yield (275 mg, 1.061 mmol, 43 %)
LC-MS (ESI, m/z): method 1, t*_{R}* = 0.56 min, m/z (M+1) = 258.8/260.7

### Intermediate 3: 5-bromo-N-(2-hydroxyethyl)nicotinamide

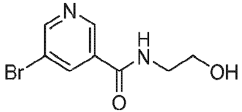

SM: 5-bromonicotinic acid 2.5 g (1 eq.); 2-aminoethan-1-ol: 1.134 g (1.5 eq.)
Amount/yield: 660 mg/ 22%
LC-MS (ESI, m/z): method 1, t*_{R}* = 0.44 min, m/z (M+1) = 245.0/247.0
m/z (M+1) = 297.85

### Intermediate 35: (4-chlorothieno[3,2-d]pyrimidin-6-yl)(morpholino)methanone

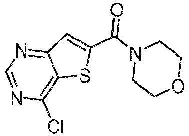

SM: 4-chlorothieno[3,2-d]pyrimidine-6-carboxylic acid 100 mg (1 eq.)
Morpholine: 41 mg (1 eq.); HATU: 213 mg (1.2 eq.)
Amount/yield: 132 mg/ 100%
LC-MS (ESI, m/z): method 17, t*_{R}* = 1.09 min, m/z (M+1) = 284.07

### Intermediate 4: N-(5-bromopyridin-3-yl)acetamide

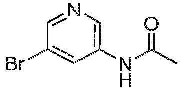

To a solution of 5-bromopyridin-3-amine (1 g, 5.78 mmol) in DCM (30 ml) acetic anhydride (1.745 ml, 18.50 mmol) was added followed by DIPEA (2.73 ml, 15.61 mmol). Solution was stirred at rt. On the following day, UPLC-MS analysis showed complete conversion of SM. Solution was diluted with NaHCO₃ sat solution (20 mL), extracted with DCM (20 mL x 2) and then with EtOAc (20 mL). The organic layer was dried over MgSO₄ and under reduced pressure. The crude was purified by FCC on a silica gel column eluting with a gradient of 0 - 80% EtOAc in n-Heptane to provide the title compound (1.1 g, 5.17 mmol, yield 88%).
LC-MS (ESI, m/z): method 1, t*_{R}* = 0.56 min, m/z (M+1) = 214.8/216.8

### Intermediate 5: 2-[(5-bromopyrazolo[3,4-b]pyridin-1-yl)methoxy]ethyl-trimethylsilane

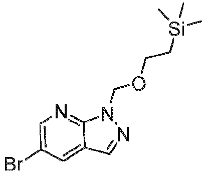

To a solution of 5-bromo-1H-pyrazolo[3,4-b]pyridine (1000 mg, 5.05 mmol, 1.00 eq) and tBuOK (850 mg, 7.57 mmol, 1.50 eq) in DMF (15.00 mL) at 0°C 2-(trimethylsilyl)ethoxymethyl chloride (1.1 mL, 6.06 mmol, 1.20 eq) was added slowly, the mixture was allowed to warm to rt and stirred overnight. The reaction mixture was quenched with NH₄Cl (sat aq., 20 mL) and extracted with EtOAc (50 mL). The organic phase was washed with H₂O (50 mL), brine (sat. aq., 50 mL), dried over MgSO4, filtered and concentrated in vacuo. The residue was purified by FCC on silica gel, eluting with a gradient of 0-60% EtOAc in cyclohexane to give the title compound (1.2 g, 3.65 mmol, 72%).
LC-MS (ESI, m/z): (Method 2), t*_{R}* = 1.95 min, m/z (M+1)= 328.0/ 330.0
¹H NMR (400 MHz, DMSO-d₆) δ 8.80 (d, J=2.3 Hz, 1H), 8.71 (d, J=2.1 Hz, 1H), 8.36 (s, 1H), 5.88 (s, 2H), 3.70 (t, J=8.0 Hz, 2H), 0.92 (t, J= 8.0 Hz, 2H), 0.01(s, 9H).

### Intermediate 6: 5-(tert-butyl)-1-methyl-1H-pyrazol-3-amine

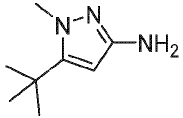

To a solution of 3-chloro-4,4-dimethylpent-2-enenitrile (5.00 g, 34.8 mmol, 1.00 eq) and K₂CO₃ (4812 mg, 34.8 mmol, 1.00 eq) in EtOH (25 mL) methylhydrazine (2.0 mL, 38.3 mmol, 1.10 eq) was added and the reaction mixture was heated at 80 °C for 4 h. The reaction mixture was cooled to rt, filtered through a Celite^{®} pad and the filtrate was concentrated *in vacuo.* The residue was purified by column chromatography on silica gel, eluting with 0-100 % EtOAc in cyclohexane followed by 0-100% MeOH in EtOAc to give a 5:1 mixture of the desired product:its regioisomer. The mixture was washed with cyclohexane and filtered. The obtained residue was separated by achiral SFC (YMC Cellulose-SC 20x250 mm, 5 µm 10/90 IPA (0.1% DEA)/CO₂, 100 ml/min, 120 bar, 40 °C, DAD 230 nm), and dried *in vacuo* at 40 °C to give the title compound (2.75 g, 17.2 mmol, 49%).
LC-MS (ESI, m/z): (Method 6), t*_{R}* = 2.09 min, m/z (M+1)= 154.2
¹H NMR (400 MHz, CDCl₃) δ 5.40 (s, 1H), 3.76 (s, 3H), 3.32 - 3.31 (m, 2H), 1.32 (s, 9H).

### Intermediate 7: 5-(trifluoromethoxy)pyridin-3-amine hydrochloride

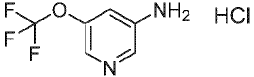

### Step 1 - tert-butyl N-[5-(trifluoromethoxy)-3-pyridyl]carbamate (Intermediate 8)

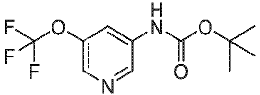

A mixture of tert-butyl carbamate (102 mg, 0.868 mmol, 1.20 eq), Xantphos (63 mg, 0.108 mmol, 0.150 eq), tris(dibenzylideneacetone)dipalladium(0)-chloroform adduct (37 mg, 0.0362 mmol, 0.0500 eq) and Cs₂CO₃ (283 mg, 0.868 mmol, 1.20 eq) in 1,4-dioxane (5 mL) was degassed with nitrogen and treated with 3-bromo-5-(trifluoromethoxy)pyridine (175 mg, 0.723 mmol, 1.00 eq). The reaction was stirred at 100 °C for 1 h. The reaction mixture was allowed to cool to rt, filtered through a pad of Celite^{®}, which was then washed with dioxane, and the combined organic phases concentrated *in vacuo.* The residue was purified by column chromatography on silica gel, eluting with 0-100%, EtOAc in cyclohexane, then dried *in vacuo* overnight to afford the title compound (115 mg, 0.413 mmol, 57%).
LC-MS (ESI, m/z): (Method 2), t*_{R}* = 1.24 min, m/z (M+1)= 279.1
¹H NMR (400 MHz, CDCl3) δ 8.33 (d, J=2.3 Hz, 1H), 8.23 - 8.21 (m, 1H), 8.07 (s, 1H), 7.04 (s, 1H), 1.54 (s, 9H).

### Step 2 - 5-(trifluoromethoxy)pyridin-3-amine hydrochloride (Intermediate 7)

HCl 4N in dioxane (3.0 mL, 0.413 mmol, 1.00 eq) was added to a solution of Intermediate 8 (115 mg, 0.413 mmol, 1.00 eq) in 1,4-dioxane (3 mL). The reaction mixture was stirred at rt overnight. The reaction mixture was diluted with diethyl ether (20 mL) and filtered to yield a white solid, washed with ether and dried in vacuo to afford the title compound (60 mg, 0.280 mmol, 68%).
LC-MS (Method 2): t*_{R}* = 0.88 min; m/z (M+1)= 179.0.
¹H NMR (400 MHz, DMSO-d₆) δ 8.07 (d, J=2.0 Hz, 1H), 8.03 (d, J=1.5 Hz, 1H), 7.30 (d, J=1.0 Hz, 1H).

### Intermediate 36: 2-[4-(3-bromoimidazo[1,2-a]pyridin-6-yl)piperazin-1-yl]acetonitrile

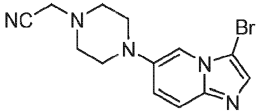

To a solution of 3-bromo-6-piperazin-1-yl-imidazo[1,2-a]pyridine (362 mg, 1.29 mmol) and formaldehyde solution (37% aq., 0.12 mL, 1.67 mmol) in MeOH (6.00 mL), NaBH₃CN (105 mg, 1.67 mmol) was added and the mixture was stirred at rt for 5 h. The reaction mixture was diluted in EtOAc and washed with NaHCO₃, water and brine. The organic phase was dried over MgSO₄, filtered and concentrated *in vacuo.* The residue was loaded onto an Isolute SCX-II cartridge, washed with MeOH/water (50 mL) and released with 1 N NH₃ in MeOH (25 mL). The ammonia eluent was concentrated *in vacuo* to afford a crude which was purified by FCC (cyclohexane: 10% 0.7 N NH₃ in MeOH in DCM, 3:1) to give the title compound (54 mg, 0.169 mmol, 13%).
LC-MS (ESI): method 2, t*_{R}* = 1.32 min; m/z (M+1) = 320.0; 322.0

### Intermediate 37 - 5-bromo-N-(oxetan-3-yl)-1-((2-(trimethylsilyl)ethoxy)methyl)-1H-pyrrolo[2,3-b]pyridine-3-carboxamide

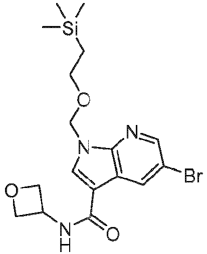

A 5-bromo-N-(oxetan-3-yl)-1H-pyrrolo[2,3-b]pyridine-3-carboxamide (0.75 g, 2.53 mmol) was dissolved in anhydrous THF (5.3 ml) and cooled down to 0°C. Next NaH (60% in mineral oil) (0.203 g, 5.07 mmol) was slowly added and solution was stirred at the same temperature for 30 min. The (2-(chloromethoxy)ethyl)trimethylsilane (0.897 ml, 5.07 mmol) was added and the reaction mixture was stirred at RT overnight.

The mixture was diluted with water and extracted with AcOEt. The organic layer was dried over Na₂SO₄. The crude material was purified via FCC (cyclohexane -> 100% AcOEt) to give desired product (400 mg, 940 mmol, 37%).

LC-MS (ESI): Method 16, t*_{R}* = 2.55 min; m/z (M+1) = 427.85

### Intermediate 38 - 1-(5-bromo-1-((2-(trimethylsilyl)ethoxy)methyl)-1H-pyrazolo[3,4-b]pyridin-3-yl)-N,N-dimethylmethanamine

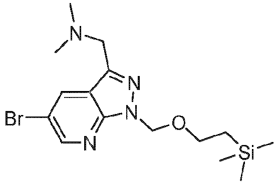

The intermediate was prepared following the procedure of Intermediate 37.
SM: 1-(5-bromo-1H-pyrazolo[3,4-b]pyridin-3-yl)-N,N-dimethylmethanamine 374 mg (1 eq.); (2-(chloromethoxy)ethyl)trimethylsilane 367 mg (1.5 eq.)
Amount/yield: 516 mg/ 78%
LC-MS (ESI): Method 16, t*_{R}* = 1.83 min; m/z (M+1) = 387.05

### Intermediate 39- 4-[(3-bromoimidazo[1,2-a]pyridin-6-yl)methyl]morpholine

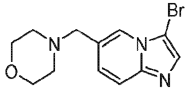

Intermediate 39 was prepared by following the same procedure reported for the synthesis of Example C1, using DCM as solvent and NaBH(OAc)₃ as reducing agent.
SM: 3-bromoimidazo[1,2-a]pyridine-6-carbaldehyde: 500 mg (1 eq.); morpholine (0.24 mL, 1.25 eq)
Amount/yield: 320 mg/ 57%
LC-MS (ESI): method 2, t*_{R}* = 1.26 min; m/z (M+1) = 296.0; 298.0

### Intermediate 40 - 3-bromo-6-(4-methylpiperazin-1-yl)imidazo[1,2-b]pyridazine

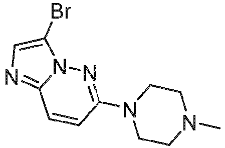

A suspension of 3-bromo-6-chloroimidazo[1,2-b]pyridazine (500 mg, 2.15 mmol), 1-methylpiperazine (0.72 mL, 6.45 mmol) and DIPEA (0.37 mL, 2.15 mmol) in DME (1.5 mL) was heated to 100 °C on. The reaction mixture was diluted in EtOAc and washed with NaHCO₃, the organic phase was dried over MgSO₄, filtered and concentrated *in vacuo.* The residue was purified by FCC (0-100% EtOAc in cyclohexane, followed by 10% 0.7 N NH₃ in MeOH in DCM) to give the title compound (610 mg, 2.06 mmol, 96%).
LC-MS (ESI): method 2, t*_{R}* = 1.32 min; m/z (M+1) =296.0; 298.1

The following Intermediates were prepared using the procedure described for the synthesis of Intermediate 40.

| **Interme diate N°** | **Structure** | **Product Amount (Yield)** |
|---|---|---|
| | **Analytical Data** | |
| **41** | 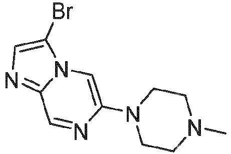 | 261 mg (41%) |
| | LC-MS (ESI): method 2, t*_{R}* = 1.22 min; m/z (M+1)= 296.1; 298.0 | |
| **42** | 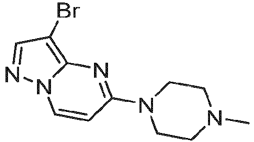 | 499 mg (78%) |
| | LC-MS (ESI): method 2, t*_{R}* = 1.33 minutes, m/z (M+1)= 296.1; 298.0 | |

### Intermediate 43: N-(3-bromopyrazolo[1,5-a]pyrimidin-5-yl)acetamide

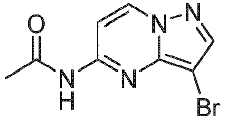

One chamber of a COware gas reactor was charged with a solution of 3-bromo-5-chloropyrazolo[1,5-a]pyrimidine (500 mg, 2.151 mmol) in THF (10 mL), then TEA (0.300 mL, 2.15 mmol) was added. The other one was charged with NH₃ water solution (5 mL, 73.4 mmol). the tubes were closed with sealed cap and the system was purged with nitrogen and placed under vacuum. The tubes were heated at 80° C and stirred until complete conversion. The volatiles were removed under reduced pressure, then the residue was dissolved in pyridine (10 mL) and acetyl chloride (253 mg, 3.2 mmol) was added in one portion. The reaction mixture was diluted with DCM and the crude was washed with NaHCO₃, NH₄Cl and brine. The organic layer was dried over Na₂SO₄ and evaporated under vacuum. Title compound was obtained (336 mg, 1.32 mmol, 61 % yield) and used as it is in the next step.
LC-MS (ESI): Method 14 t*_{R}* = 0.97 min; m/z (M+1) = 254.9.0-226.8

### Intermediate 44: 5-bromo-N,N-bis(4-methoxybenzyl)pyrimidin-2-amine

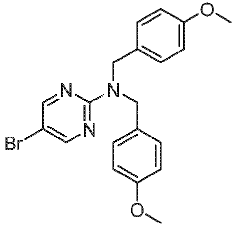

**Reference:** "Modification of a dihydropyrrolopyrimidine phosphoinositide 3-kinase (PI3K) inhibitor to improve oral bioavailability" Kawada, Hatsuo; et al, Bioorganic & Medicinal Chemistry (2015), 23(24), 7650-7660

### Example 1 - (S)-5-(3-(2-methyl-5-((5-(trifluoromethyl)pyridin-3-yl)carbamoyl)phenyl)pyrrolidin-1-yl)nicotinamide

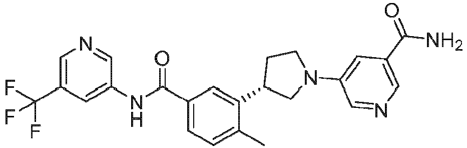

### Step 1 - Ethyl 4-methyl-3-vinylbenzoate (Intermediate 10)

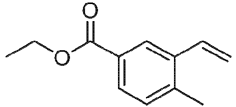

In an oven dried 2L reactor, ethyl 3-bromo-4-methylbenzoate (100 g, 411 mmol), potassium trifluoro(vinyl)borate (83 g, 617 mmol) and K₂CO₃ (114 g, 823 mmol) were placed and dioxane (1000 ml) was added under argon atmosphere. The solution was filled with argon (10 min), next Pd(dppf)Cl₂ (30.1 g, 41.1) was added. The solution was stirred at reflux for 6 h. The reaction mixture was filtered over a Celite^{®} pad and dried under reduced pressure. The residue was purified via FCC on silica gel, eluting with 0-20% of AcOEt in n-Heptane. Appropriate fractions were collected and dried to afford the title compound (61.5 g, 323 mmol, 79 % yield)
LC-MS (ESI): (Method 3) t*_{R}* = 2.23 min; m/z (M+1) = 190.9
¹H NMR ( CDCl₃, 400 MHz) δ 8.15 (s, 1H), 7.84 (dd, 1H, *J*=1.5*,* 7.9 Hz), 7.22 (d, 1H, *J*=7.9 Hz), 6.94 (dd, 1H, *J*=11.0*,* 17.5 Hz), 5.7-5.8 (m, 1H), 5.38 (dd, 1H, *J*=0.9, 11.0 Hz), 4.39 (q, 2H, *J*=7.0 Hz), 2.40 (s, 3H), 1.41 (t, 3H, *J*=7.1 Hz)

### Step 2 - Ethyl 3-(1-benzylpyrrolidin-3-yl)-4-methylbenzoate (Intermediate 11)

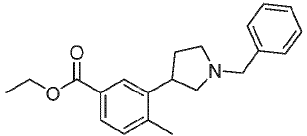

A mixture of Intermediate 10 (78.4 g, 412 mmol), N-benzyl-1-methoxy-N-((trimethylsilyl)methyl)methanamine (73.4 g, 309 mmol) and then dioxane (1L) containing 0.01% of TFA (0.317 ml, 4.12 mmol) was placed in a round-bottomed vessel and heated to reflux for 2 h. The reaction was cooled down at RT and another equivalent of N-benzyl-1-methoxy-N-((trimethylsilyl)methyl)methanamine (73.4 g, 309 mmol) was added, then the solution was stirred at reflux. This step was repeated twice, then the mixture was kept under stirring at reflux until completion. The solvent was removed under vacuum and the crude was used in the next step without further purification.
LC-MS (ESI): (Method 3) t*_{R}* = 1.07 min; m/z (M+1) = 324.0

### Step 3 - Ethyl 4-methyl-3-(pyrrolidin-3-yl)benzoate (Intermediate 12)

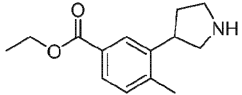

A 1L reactor was purged with nitrogen and charged with palladium/C 10 (50% wet) (21.88 g, 10.28 mmol). The system was purged once more with nitrogen and placed under vacuum. A solution of Intermediate 11 (133 g, 411 mmol) in ethanol (500 ml) was then charged and the system was purged with nitrogen once more. The solution was then placed under an atmosphere of hydrogen (4 bar), warmed to 60± °C and stirred for 8 h. The reactor was emptied and rinsed with 200 mL EtOH. The reaction mixture was filtered through a Celite^{®} pad and concentrated, then the crude was dissolved in Et₂O (400 mL) and extracted with HCL 1N (3x 150 mL).
LC-MS (ESI): (Method 3) t*_{R}* = 0.69 min; m/z (M+1) =233.9
¹H NMR (CDCl₃, 400 MHz) δ 7.91 (s, 1H), 7.79 (dd, 1H, *J*=1.5, 7.9 Hz), 7.22 (d, 1H, *J*=7.6 Hz), 4.37 (q, 2H, *J*=7.0 Hz), 3.4-3.5 (m, 1H), 3.4-3.4 (m, 1H), 3.31 (ddd, 2H, *J*=4.6, 8.6, 11.0 Hz), 3.17 (td, 1H, *J*=7.6, 11.0 Hz), 2.93 (dd, 1H, *J*=8.1, 10.7 Hz), 2.42 (s, 3H), 2.2-2.3 (m, 1H), 1.9-2.0 (m, 1H), 1.40 (t, 3H, *J*=7.1 Hz)

### Step 4 - tert-butyl 3-(5-(ethoxycarbonyl)-2-methylphenyl)pyrrolidine-1-carboxylate (Intermediate 13)

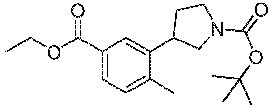

Intermediate 12 (20.35 g, 87 mmol) and di-tert-butyl dicarbonate were placed in a 500 mL flask and dissolved in DCM (300 ml) then TEA (18.24 ml, 131 mmol) was added in one portion and the reaction was stirred at rt on. The crude was washed with NH₄Cl solution (2x 100 mL) and brine (1x 100mL). The crude was purified with FCC silica gel, eluting with a gradient of 0-20% EtOAc in n-heptane. Relevant fractions were collected to afford the title compound (24.5 g, 73.5 mmol, 84 % yield).
LC-MS (ESI): (Method 1) t*_{R}* = 1.35 min; m/z (M+1-tBu): 277.9

### Intermediate 9: 3-bromo-4-methyl-N-(5-(trifluoromethyl)pyridin-3-yl)benzamide

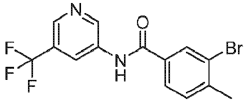

In a 400 mL reactor, 5-(trifluoromethyl)pyridin-3-amine (10.40 g, 64.2 mmol) was dissolved in THF (100 ml) under nitrogen; the mixture was stirred at -5°C and NaHMDS 1M in THF (92 ml, 92 mmol) was added dropwise. The reaction was stirred for 30 min at -5°C. Methyl 3-bromo-4-methylbenzoate (4.76 ml, 30.6 mmol) was added, the temperature was increased until 25°C in 1h. The reaction was quenched with water (13.7 ml, 760 mmol) then the solution was transferred in a 1L round-bottomed flask and solvents were evaporated under reduced pressure. The crude was dissolved in DCM (200 mL) and washed with HCl 1N (2x200 mL) and brine (1x100 mL). The organic layer was dried under reduced pressure and used in the next step without further purification.
¹H NMR (Acetone-d₆, 400 MHz) δ 10.07 (br s, 1H), 9.18 (d, 1H, *J*=1.3 Hz), 8.74 (s, 1H), 8.68 (s, 1H), 8.22 (d, 1H, *J*=1.1 Hz), 7.9-8.0 (m, 1H), 7.52 (d, 1H, *J*=7.9 Hz), 2.47 (s, 3H)

### Step 5 - tert-butyl (R)-3-(5-(ethoxycarbonyl)-2-methylphenyl)pyrrolidine-1-carboxylate (Intermediate 14a) and tert-butyl (S)-3-(5-(ethoxycarbonyl)-2-methylphenyl)pyrrolidine-1-carboxylate (Intermediate 14b)

Intermediate 13 as racemic mixture (20.097 g, 0.060 mol) was dissolved to 90 mg/mL in MeOH and was then purified by SFC, by following Method 1. Combined fractions of each of 1^{st} eluting (Intermediate 14a) and 2^{nd} eluting (Intermediate 14b) peaks were then evaporated to near dryness using a rotary evaporator. The resultant solids were then transferred into final vessels with DCM which was removed under a stream of compressed air at 35°C before being stored in a vacuum oven at 35°C and 5 mbar until constant weight.

### 1^{st} eluted enantiomer (Intermediate 14a)

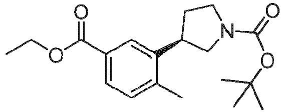

Amount: 9.6 g
Chiral analysis (SFC Method 2): t*_{R}* 1.238 min, ee 100%
LC-MS (ESI): Method 7 t*_{R}* = 1.723 min; m/z (M+1-tBu)= 278.2

### 2^{nd} eluted enantiomer (Intermediate 14b)

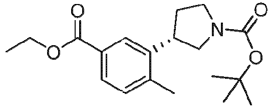

Amount: 9.2 g
Chiral analysis (SFC Method 2): t*_{R}* 1.562 min, ee 100%
LC-MS (ESI): Method 7 t*_{R}* = 1.722 min; m/z (M+1-tBu)= 278.2

Absolute configuration of Intermediate 14a and 14b was determined by IR and VCD Spectroscopy.

The following Intermediates 15a and 15b were synthesized from Intermediate 14a (1^{st} eluted) and 14b (2^{nd} eluted) respectively, under no racemizing conditions. Absolute configuration of Intermediate 14a and 14b was determined by IR and VCD Spectroscopy.

### Intermediate 15a: tert-butyl (R)-3-(2-methyl-5-((3-(trifluoromethyl)phenyl) carbamoyl)phenyl)pyrrolidine-1-carboxylate

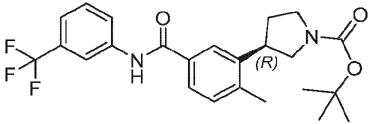

3-(trifluoromethyl)aniline (0.373 ml, 3.00 mmol) was dissolved in dry THF (25 ml)) under N₂. The mixture was stirred at -78°C for 15 min, then butyllithium 2.5M in hexanes (1.140 ml, 2.85 mmol) was added dropwise in 5 min and the reaction was stirred for 1 h at -78°C. A solution of Intermediate 14a in THF (10 ml) was added dropwise for 10 min, the temperature was increased up to RT and the reaction was stirred for 1 h. 10 mL of H₂O was added to quench the reaction and the solvent was evaporated by reduced pressure. The solid was dissolved in DCM (50 mL) and the organic layer was washed with H₂O (2x 20 mL). Purification was performed by FCC on silica gel eluting with a gradient of 0- 30% of EtOAc in n-Heptane. Appropriate fractions were combined and dried to afford the title compound (554 mg, 1.235 mmol, 82 % yield).
Chiral analysis (SFC Method 3): t*_{R}* 1.16 min, ee 100%
LC-MS (ESI): Method 7 t*_{R}* = 1.79 min; m/z (M+1-tBu)= 393.3
IR and VCD Spectroscopy: configuration R was assigned.

### Intermediate 15b: tert-butyl (S)-3-(2-methyl-5-((3-(trifluoromethyl)phenyl) carbamoyl)phenyl)pyrrolidine-1-carboxylate

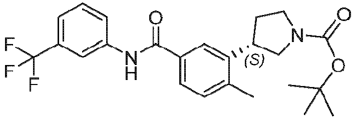

Analogously, Intermediate 15b was prepared by reacting Intermediate 13b with the suitable amine.
SM: Intermediate 14b: 4 g (1 eq.)
3-(trifluoromethyl)aniline: 3.87 g (2 eq.)
Amount/yield: 4.23 g/ 79%
Chiral analysis (SFC Method 2): t*_{R}* = 2.46 min, ee 99.8%
LC-MS (ESI): Method 7 t*_{R}* = 1.79 min; m/z (M+1- t-But)= 393.3
IR and VCD Spectroscopy: configuration S was assigned.

### Step 6 - ethyl (S)-4-methyl-3-(pyrrolidin-3-yl)benzoate (Intermediate 16)

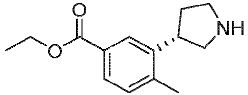

A 20 mL vial was loaded with Intermediate 14b (4.800 g, 14.40 mmol) and then TFA (8.32 ml, 108 mmol) was added dropwise. Solution was stirred for 10 min at rt. Solution was then diluted with DCM (10 mL) and organic phase was extracted with NaHCO₃ sat solution at 0 °C. Organic phase was evaporated under vacuum to give Intermediate 15 (3.40 g, 14.57 mmol, 101 % yield). LC-MS (ESI): (Method 1) t*_{R}* = 0.50 min; m/z (M+1) =234.0
¹H NMR (400 MHz, DMSO-d₆) δ ppm 7.86 (d, J=1.32 Hz, 1 H), 7.69 (dd, J=7.78, 1.64 Hz, 1 H), 7.30 (d, J=7.89 Hz, 1 H), 4.30 (q, J=7.09 Hz, 1 H), 4.24 - 4.34 (m, 1 H), 3.40 (quin, J=8.06 Hz, 1 H), 3.27 (dd, J=10.52, 7.67 Hz, 1 H), 2.93 -3.10 (m, 2 H), 2.69 (dd, J=10.52, 7.89 Hz, 1 H), 2.11 - 2.22 (m, 1 H), 1.69 (dq, J=12.30, 8.10 Hz, 1 H), 1.31 (t, J=7.13 Hz, 3 H)

### Step 7 - ethyl (S)-3-(1-(5-cyanopyridin-3-yl)pyrrolidin-3-yl)-4-methylbenzoate (Intermediate 17)

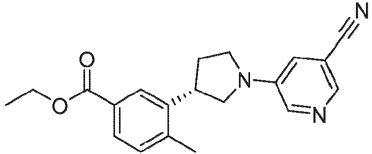

A 100 ml reactor was charged with a mixture of 5-bromonicotinonitrile (4.00 g, 21.86 mmol), Intermediate 16, RuPhos (1.360 g, 2.91 mmol), Pd(dppf)Cl₂ (1.066 g, 1.457 mmol) and Cs₂CO₃ (14.24 g, 43.7 mmol). The reactor was evacuated and backfilled with Ar, then DMA (35 ml) was added. The solution was heated at 120 °C and stirred for 2 h. Solution was diluted with EtOAc and washed twice with NaHCO₃ sat solution and organic layer was evaporated under vacuum. Purification by FCC on silica gel, eluting with 40% EtOAc in n-Heptane yielded the title compound (3.3 g, 9.84 mmol, 67.5 % yield).
LC-MS (ESI): (Method 1) t*_{R}* = 1.20 min; m/z (M+1) =336.0

The following Intermediates were prepared using the procedure described for the synthesis of Intermediate 17.

| **Intermediate N°** | **Structure** | **Product Amount (Yield)** |
|---|---|---|
| | **Analytical Data** | |
| **45** | 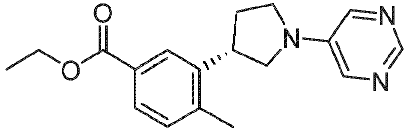 | 1.9 g (42%) |
| | LC-MS (ESI): Method 1, t*_{R}* = 1.03 min, m/z (M+1) = 312 | |
| **46** | 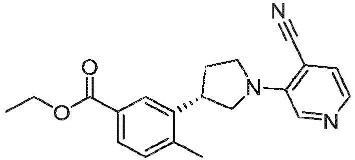 | 238 mg (82%) |
| | LC-MS (ESI, m/z): method 2, t*_{R}* = 1.7 min, m/z (M+1) = 336.2 | |
| **47** | 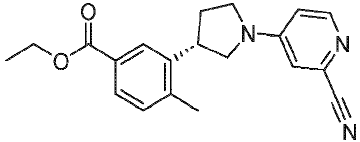 | 280 mg (65%) |
| | LC-MS (ESI, m/z): Method 16, t*_{R}* = 2.3 min, m/z (M+1) = 336.41 | |

### Step 8 - ethyl (S)-3-(1-(5-carbamoylpyridin-3-yl)pyrrolidin-3-yl)-4-methylbenzoate (Intermediate 18)

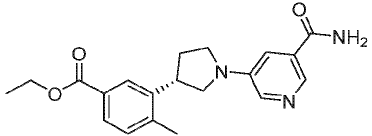

In a 100 mL flask, Intermediate 17 (2 g, 5.96 mmol) was dissolved in DMSO (20 ml), then K₂CO₃ (1.5 g, 10.85 mmol, 1.8 eq.) was added. The suspension was stirred 5 min at rt. H₂O₂ solution 30 % (w/w) in H₂O (1.827 ml, 17.89 mmol, 3 eq.) was added dropwise at 0 °C and the mixture was stirred for 2 h at rt. The reaction was quenched with 5 mL of H₂O and the desired product started precipitating as a white solid. Solid was filtered and washed with water and dried overnight to obtain Intermediate 18 in a quantitative yield.
LC-MS (ESI): (Method 1) t*_{R}* = 0.70 min; m/z (M+1) = 354.3
¹H NMR (400 MHz, DMSO-d₆) δ ppm 8.32 (d, *J*=1.53 Hz, 1 H), 8.10 (d, *J*=2.85 Hz, 1 H), 8.03 (br s, 1 H), 7.81 (d, *J*=1.53 Hz, 1H), 7.73 (dd, *J*=7.89, 1.75 Hz, 1 H), 7.45 (br s, 1H) 7.37 (s, 1 H), 7.33 - 7.35 (m, 1 H), 4.24 (q, *J*=7.09 Hz, 2 H), 3.73 - 3.82 (m, 2 H), 3.41 - 3.55(m, 2 H), 3.34 (br d, *J*=2.41 Hz, 1 H), 2.36 - 2.44 (m, 1 H), 2.46 (s, 3 H), 2.04 - 2.17 (m, 1 H), 1.23 (t, *J*=7.13 Hz, 3 H).

The following Examples and Intermediates were prepared using the procedure described for the synthesis of Intermediate 18 starting from related Intermediate.

| **Example/ Intermediate N°** | **Structure** | **Product Amount (Yield)** |
|---|---|---|
| | **Analytical Data** | |
| **51** | 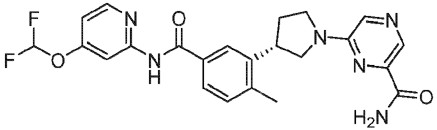 | 30 mg (45%) |
| | LC-MS (ESI): Method 8, t*_{R}* = 4.31 min, m/z (M+1) = 469.3 | |
| | ¹H NMR (400 MHz, DMSO-d₆) δ 11.04 (s, 1H), 8.36 (d, J=5.8 Hz, 1H), 8.31 (s, 1H), 8.20 (s, 1H), 8.10 (d, J=1.5 Hz, 1H), 8.04 (d, J=2.0 Hz, 1H), 7.97 - 7.95 (m, 1H), 7.82 (dd, J=1.8, 7.8 Hz, 1H), 7.61 (s, 1H), 7.47 (t, J=73.1 Hz, 1H), 7.35 (d, J=8.1 Hz, 1H), 6.97 (dd, J=2.0, 5.8 Hz, 1H), 4.12 - 4.06 (m, 1H), 3.92 - 3.81 (m, 1H), 3.81 - 3.72 (m, 1H), 3.65 - 3.49 (m, 2H), 2.47 (s, 3H), 2.38 - 2.27 (m, 2H). | |
| **52** | 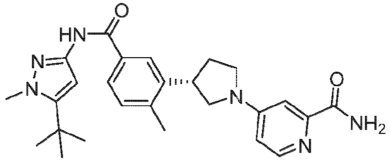 | 9 mg (6%) |
| | LC-MS (ESI): Method 11 *t_{R}* = 3.31 min, m/z (M+1) = 461.38 | |
| | ¹H NMR (400 MHz, DMSO-d₆) δ 10.69 (s, 1H), 8.15 (d, J = 5.8 Hz, 1H), 8.01 - 7.96 (m, 2H), 7.75 (dd, J = 7.9, 1.8 Hz, 1H), 7.48 (d, J = 3.5 Hz, 1H), 7.31 (d, J = 8.0 Hz, 1H), 7.20 (d, J = 2.5 Hz, 1H), 6.67 (dd, J = 5.8, 2.6 Hz, 1H), 6.45 (s, 1H), 3.82 (s, 3H), 3.78 (t, J = 5.5 Hz, 2H), 3.61 (t, J = 9.3 Hz, 1H), 3.48 (dd, J = 18.3, 9.9 Hz, 2H), 2.41 - 2.34 (m, 1H), 2.22 (t, J = 10.3 Hz, 1H), 1.33 (s, 9H). | |
| **53** | 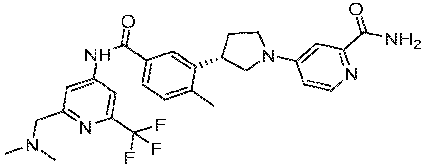 | 12 mg (16%) |
| | LC-MS (ESI): Method 10, t*_{R}* = 1.74 min, m/z (M+1) = 527.65 | |
| | ¹H NMR (400 MHz, DMSO-d₆) δ 10.74 (s, 1H), 8.25 (d, J = 1.9 Hz, 1H), 8.16 (d, J = 5.7 Hz, 1H), 8.12 (d, J = 1.9 Hz, 1H), 7.97 (dd, J = 6.2, 2.6 Hz, 2H), 7.82 (dd, J = 7.9, 1.8 Hz, 1H), 7.49 (d, J = 3.3 Hz, 1H), 7.41 (d, J = 8.0 Hz, 1H), 7.21 (d, J = 2.6 Hz, 1H), 6.68 (dd, J = 5.8, 2.6 Hz, 1H), 3.89 - 3.76 (m, 2H), 3.63 (t, J = 9.0 Hz, 1H), 3.56 (s, 2H), 3.51 (dd, J = 9.5, 7.0 Hz, 1H), 3.44 (d, J = 16.1 Hz, 1H), 2.40 (s, 1H), 2.25 (s, 1H), 2.22 (s, 6H). | |
| **54** | 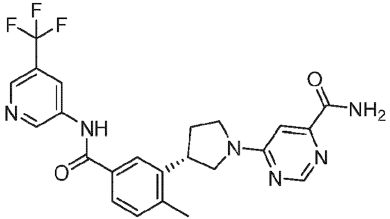 | 70 mg (40%) |
| | LC-MS (ESI): Method 9, t*_{R}* = 2.5 min, m/z (M+1) = 471.22 | |
| | ¹H NMR (400 MHz, DMSO-d₆) δ 10.65 (s, 1H), 9.13 (s, 1H), 8.64 (s, 1H), 8.62 - 8.52 (m, 2H), 8.07 (s, 1H), 7.96 (s, 1H), 7.82 (dd, J = 7.9, 1.8 Hz, 1H), 7.74 (d, J = 7.3 Hz, 1H), 7.39 (d, J = 7.9 Hz, 1H), 7.07 (d, J = 1.2 Hz, 1H), 4.12 (s, 1H), 4.00 - 3.69 (m, 2H), 3.55 (dd, J = 23.3, 11.7 Hz, 2H), 2.47 (s, 3H), 2.36 (s, 1H), 2.18 (d, J = 29.2 Hz, 1H). | |
| **Intermediate 48** | 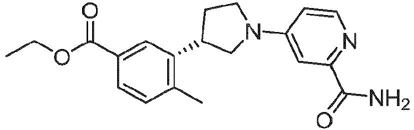 | 230 mg (84%) |
| | LC-MS (ESI): Method 16, t*_{R}* = 1.7 min, m/z (M+1) = 354.35 | |
| | ¹H NMR (300 MHz, DMSO-d₆) δ 8.16 (d, J = 5.7 Hz, 1H), 7.99 (d, J = 3.4 Hz, 1H), 7.78 (d, J = 1.8 Hz, 1H), 7.73 (dd, J = 7.9, 1.8 Hz, 1H), 7.49 (d, J = 3.4 Hz, 1H), 7.36 (d, J = 7.9 Hz, 1H), 7.19 (d, J = 2.5 Hz, 1H), 6.68 (dd, J = 5.8, 2.6 Hz, 1H), 4.23 (q, J = 7.1 Hz, 2H), 3.78 (dq, J = 13.4, 7.1 Hz, 2H), 3.49 (q, J = 7.7, 6.4 Hz, 2H), 2.45 (s, 3H), 2.42 - 2.35 (m, 1H), 2.10 (dq, J = 15.5, 7.8 Hz, 1H), 1.22 (t, J = 7.1 Hz, 3H). | |

### Step 9 - (S)-5-(3-(2-methyl-5-((5-(trifluoromethyl)pyridin-3-yl)carbamoyl) phenyl) pyrrolidin-1-yl)nicotinamide (Example 1)

5-(trifluoromethyl)pyridin-3-amine (68.8 mg, 0.424 mmol) was dissolved in dry THF (5 ml) under nitrogen the mixture was stirred at -78°C for 15 min, then butyllithium 2.5M in hexanes (0.191 ml, 0.478 mmol) was added dropwise in 5 min and the reaction was stirred for 1 h at -78°C. A solution of Intermediate 18 (50 mg, 0.141 mmol) in THF (5 ml) was added dropwise for 10 min, the temperature was increased at rt and the reaction was stirred for 1 h. 10 mL of H₂O was added to quench the reaction and the solvent was evaporated by reduced pressure. The solid was dissolved in EtOAc and washed with NH₄Cl sat solution. Organic phase was evaporated under vacuum. Purification by FCC, on amino silica gel eluting with DCM/EtOH 9:1, yielded the title compound (16.8 mg, 0.036 mmol, 25.3 % yield).
LC-MS (ESI): (Method 3) t*_{R}* = 1.23 min; m/z (M+1) = 470.2
¹H NMR (DMSO-d₆, 400 MHz) δ 10.60 (s, 1H), 9.1-9.2 (m, 1H), 8.66 (s, 1H), 8.56 (s, 1H), 8.28 (d, 1H, J=1.5 Hz), 8.08 (d, 1H, J=2.9 Hz), 7.99 (br s, 1H), 7.9-8.0 (m, 1H), 7.79 (dd, 1H, J=1.8, 7.9 Hz), 7.3-7.4 (m, 2H), 7.31 (t, 1H, J=2.2 Hz), 3.7-3.9 (m, 2H), 3.5-3.6 (m, 1H), 3.45 (br d, 1H, J=7.5 Hz), 3.3-3.4 (m, 1H), 2.45 (s, 3H), 2.3-2.4 (m, 1H), 2.22 (br d, 1H, J=8.6 Hz)

The following Examples and Intermediates were prepared using the reference procedure described for the synthesis of Example 1, step 9, by reacting the appropriate Intermediate with the suitable amine. In a few cases, n-Butyllithium 2.5M in hexanes was replaced by LiHMDS 1M THF solution.

| **Example N°** | **Structure** | **Product Amount (Yield)** |
|---|---|---|
| | **Analytical Data** | |
| **55** | 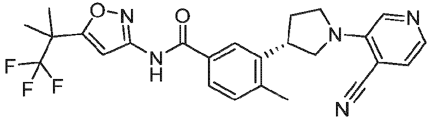 | 33 mg (55%) |
| | LC-MS (ESI): Method 8 t*_{R}* = 5.26 min; m/z (M+1) = 484.4 | |
| | ¹H NMR (400 MHz, DMSO-d₆) δ 11.48 (s, 1H), 8.32 (s, 1H), 8.06 (d, J=1.5 Hz, 1H), 7.91 (d, J=5.0 Hz, 1H), 7.82 (dd, J=1.8, 7.9 Hz, 1H), 7.48 (d, J=5.1 Hz, 1H), 7.38 (d, J=8.0 Hz, 1H), 7.12 (s, 1H), 4.08 - 4.02 (m, 1H), 3.88 - 3.72 (m, 3H), 3.65 (t, J=9.3 Hz, 1H), 2.46 (s, 3H), 2.39 - 2.22 (m, 2H), 1.58 (s, 6H). | |
| **56** | 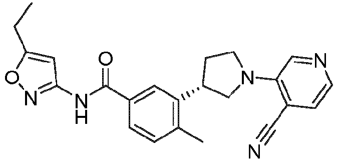 | 33 mg (55%) |
| | LC-MS (ESI): Method 5, *t_{R} =* 4.71 min; m/z (M+1) = 402.5 | |
| | ¹H NMR (400 MHz, DMSO-d₆) δ 11.28 (s, 1H), 8.32 (s, 1H), 8.05 (d, J=1.8 Hz, 1H), 7.90 (d, J=4.8 Hz, 1H), 7.81 (dd, J=1.8, 7.8 Hz, 1H), 7.47 (d, J=5.1 Hz, 1H), 7.36 (d, J=8.3 Hz, 1H), 6.76 (s, 1H), 4.07 - 4.01 (m, 1H), 3.87 - 3.60 (m, 4H), 2.76 (q, J=7.6 Hz, 2H), 2.46 (s, 3H), 2.37 - 2.25 (m, 2H), 1.23 (t, J=7.6 Hz, 3H). | |
| **57** | 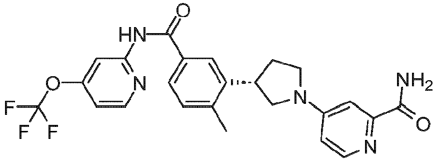 | 4 mg (6%) |
| | LC-MS (ESI): Method 10, t*_{R}* = 2.63 min; m/z (M+1) = 486.19 | |
| | ¹H NMR (400 MHz, DMSO-d₆) δ 11.23 (s, 1H), 8.48 (d, J = 5.7 Hz, 1H), 8.23 (s, 1H), 8.15 (d, J = 5.7 Hz, 1H), 8.08 (s, 1H), 7.98 (s, 1H), 7.84 - 7.78 (m, 1H), 7.48 (s, 1H), 7.36 (d, J = 7.9 Hz, 1H), 7.19 (dd, J = 10.7, 4.1 Hz, 2H), 6.67 (dd, J = 5.8, 2.6 Hz, 1H), 3.80 (d, J = 5.9 Hz, 2H), 3.63 (s, 1H), 3.48 (d, J = 9.8 Hz, 2H), 2.46 (s, 3H), 2.39 (t, 1H), 2.26 (t, 1H). | |
| **58** | 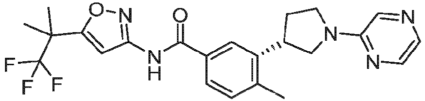 | 49 mg (66%) |
| | LC-MS (ESI): Method 8, t*_{R}* = 5.05 min; m/z (M+1) = 460.3 | |
| | ¹H NMR (400 MHz, DMSO) δ 11.47 (s, 1H), 8.06 (dd, J=1.5, 2.5 Hz, 1H), 8.03 (s, 2H), 7.81 - 7.77 (m, 2H), 7.36 (d, J=7.8 Hz, 1H), 7.10 (s, 1H), 3.94 (dd, J=7.7, 10.2 Hz, 1H), 3.79 - 3.72 (m, 2H), 3.58 - 3.45 (m, 2H), 2.46 (s, 3H), 2.38 - 2.32 (m, 1H), 2.25 - 2.14 (m, 1H), 1.57 (s, 6H). | |
| **59** | 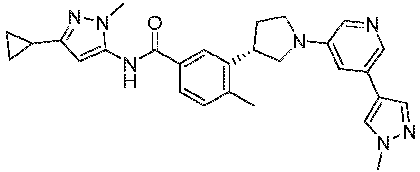 | 25 mg (24%) |
| | LC-MS (ESI): Method 5, t*_{R}* = 3.17 min; m/z (M+1) = 482.4 | |
| | ¹HNMR (400 MHz, DMSO-d₆) δ 10.13 (s, 1H), 8.21 (s, 1H), 8.10 (d, J=1.8 Hz, 1H), 7.92 (s, 2H), 7.83 (d, J=2.8 Hz, 1H), 7.74 (dd, J=1.5, 7.8 Hz, 1H), 7.36 (d, J=8.1 Hz, 1H), 7.09 (t, J=2.3 Hz, 1H), 5.89 (s, 1H), 3.86 (s, 3H), 3.82 - 3.75 (m, 2H), 3.62 - 3.55 (m, 1H), 3.51 (s, 3H), 3.50 - 3.33 (m, 2H), 2.47 (s, 3H), 2.40 - 2.31 (m, 1H), 2.25 - 2.17 (m, 1H), 1.83 - 1.75 (m, 1H), 0.81 (ddd, J=3.9, 6.3, 8.4 Hz, 2H), 0.62 - 0.57 (m, 2H). | |
| **60** | 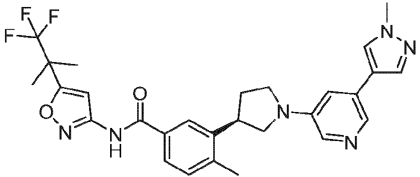 | 28 mg (28%) |
| | LC-MS (ESI): Method 5, t*_{R}* = 3.94 min; m/z (M+1) = 539.2 | |
| | ¹H NMR (400 MHz, DMSO-d₆) δ 11.46 (s, 1H), 8.21 (s, 1H), 8.11 (d, J=1.8 Hz, 1H), 8.06 (d, J=1.5 Hz, 1H), 7.92 (s, 1H), 7.84 - 7.79 (m, 2H), 7.36 (d, J=8.1 Hz, 1H), 7.09 (d, J=3.0 Hz, 2H), 3.86 (s, 3H), 3.80 - 3.74 (m, 2H), 3.64 - 3.57 (m, 1H), 3.51 - 3.35 (m, 2H), 2.47 (s 3H), 2.42 - 2.34 (m, 1H), 2.30 - 2.23 (m, 1H), 1.57 (s, 6H). | |
| **Interme diate 49** | 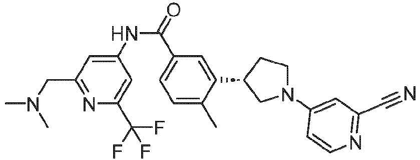 | 73 mg (33%) |
| | LC-MS (ESI): Method 16, t*_{R}* = 1.83 min; m/z (M+1) = 509.24 | |
| | ¹H NMR (300 MHz, DMSO-d₆) δ 10.74 (s, 1H), 8.25 (d, J = 1.9 Hz, 1H), 8.20 (d, J = 6.0 Hz, 1H), 8.12 (d, J = 1.8 Hz, 1H), 7.94 (s, 1H), 7.83 (dd, J = 7.9, 1.8 Hz, 1H), 7.41 (d, J = 8.0 Hz, 1H), 7.18 (d, J = 2.5 Hz, 1H), 6.77 (dd, J = 6.0, 2.6 Hz, 1H), 3.82 (p, J = 8.0 Hz, 2H), 3.63 (t, J = 8.5 Hz, 1H), 3.54 - 3.38 (m, 1H), 2.47 (s, 3H), 2.39 (s, 2H), 2.23 (s, 6H), 2.11 (d, J = 12.8 Hz, 1H). | |
| **Interme diate 50** | 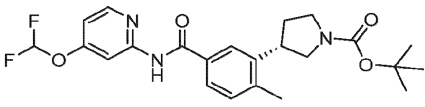 | 1240 mg (89%) |
| | LC-MS (ESI, m/z): Method 14, t*_{R}* = 1.8 min, m/z (M+1) = 448.3 | |
| | ¹H NMR (400 MHz, DMSO-d₆) δ 11.09 (d, J=9.0 Hz, 1H), 8.39 (d, J=5.6 Hz, 1H), 8.07 - 8.01 (m, 2H), 7.79 (d, J=7.8 Hz, 1H), 7.49 (t, J=74.7 Hz, 1H), 7.32 (d, J=7.5 Hz, 1H), 7.00 (dd, J=2.1, 5.6 Hz, 1H), 3.71 - 3.50 (m, 3H), 3.32 - 3.32 (m, 2H), 2.41 (s, 3H), 2.19 - 2.04 (m, 2H), 1.42 (s, 9H). | |
| **Interme diate 51** | 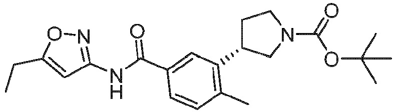 | 780 mg (76%) |
| | LC-MS (ESI): Method 2, t*_{R}* = 1.81 min; m/z (M-1) = 398.3 | |
| | ¹H NMR (400 MHz, DMSO-d₆) δ 11.30 (d, J=6.1 Hz, 1H), 8.00 (d, J=7.4 Hz, 1H), 7.78 - 7.75 (m, 1H), 7.32 (d. J=8.0 Hz, 1H), 6.77 (s, 1H), 3.68 - 3.32 (m, 5H), 2.77 (q, J=7.6 Hz, 2H), 2.41 (s, 3H), 2.23 - 2.02 (m, 2H), 1.42 (s, 9H), 1.24 (t, J=7.6 Hz, 3H). | |

### Example 2 - (R)-5-(3-(2-methyl-5-((5-(trifluoromethyl)pyridin-3-yl)carbamoyl)phenyl) pyrrolidin-1-yl)nicotinamide

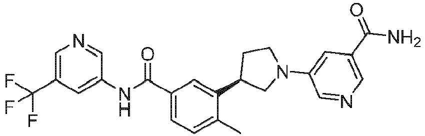

### Step 1 - Ethyl (S)-4-methyl-3-(pyrrolidin-3-yl)benzoate (Intermediate 16b)

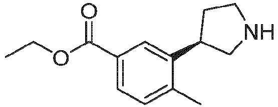

Intermediate 16b was prepared by following the same procedure reported for the synthesis of Intermediate 16.
SM: Intermediate 14a: 580 mg (1 eq.)
Amount/yield: 366 mg/ 90%
LC-MS (ESI): (Method 1) t*_{R}* = 0.50 min; m/z (M+1) = 234.0

### Step 2 - ethyl (S)-3-(1-(5-cyanopyridin-3-yl)pyrrolidin-3-yl)-4-methylbenzoate (Intermediate 17b)

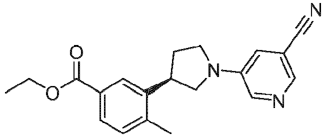

Intermediate 17b was prepared by following the same procedure reported for the synthesis of Intermediate 17.
SM: Intermediate 16b: 350 mg (1 eq.)
Amount/yield: 318 mg/ 63%
LC-MS (ESI): (Method 1) t*_{R}* =1.2 min; m/z (M+1) = 336.0

### Step 3 - ethyl (R)-3-(1-(5-carbamoylpyridin-3-yl)pyrrolidin-3-yl)-4-methylbenzoate (Intermediate 18b)

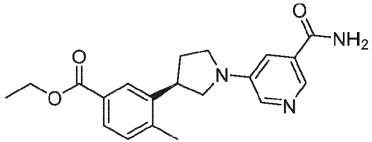

Intermediate 18b was prepared by following the same procedure reported for the synthesis of Intermediate 18.
SM: Intermediate 17b: 184 mg (1 eq.)
Amount/yield: 116 mg/ 60%
LC-MS (ESI): (Method 1) t*_{R}* = 0.68 min; m/z (M+1) = 354.3

### Step 4 - (R)-5-(3-(2-methyl-5-((5-(trifluoromethyl)pyridin-3-yl)carbamoyl)phenyl) pyrrolidin-1-yl)nicotinamide (Example 2)

Example 2 was prepared by following the same procedure described in step 9 for the synthesis of Example 1.
SM: Intermediate 18b: 50 mg (1 eq.)
5-(trifluoromethyl)pyridin-3-amine: 69 mg (3 eq.)
Amount/yield: 15 mg/ 22.58%
LC-MS (ESI): (Method 1) t*_{R}* = 0.71 min; m/z (M+1) = 470.0
¹H NMR (DMSO-d₆, 400 MHz) δ 10.60 (s, 1H), 9.15 (d, 1H, J=2.2 Hz), 8.66 (s, 1H), 8.56 (s, 1H), 8.29 (d, 1H, J=1.5 Hz), 8.08 (d, 1H, J=2.9 Hz), 8.01 (s, 1H), 7.9-8.0 (m, 1H), 7.79 (dd, 1H, J=1.5, 7.9 Hz), 7.44 (br s, 1H), 7.3-7.4 (m, 2H), 3.7-3.8 (m, 2H), 3.5-3.6 (m, 1H), 3.3-3.5 (m, 2H), 2.50 (s, 3 H), 2.3-2.4 (m, 1H), 2.1-2.3 (m, 1H)

The following Examples were prepared using the reference procedure described for the synthesis of Example 1, step 9, by reacting Intermediate 18 with the suitable amine. Butyllithium 2.5M in hexanes was replaced by LiHMDS 1M THF solution. Such procedure may involve minor variations, for example reaction temperature, reagent/solvent amount, reaction time, work-up conditions or chromatographic purification conditions, that will be appreciated by those skilled in the art.

| **Example N°** | **Structure** | **Product Amount (Yield)** |
|---|---|---|
| | **Analytical Data** | |
| **3** | 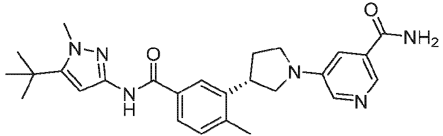 | 30 mg (46%) |
| | LC-MS (ESI): (Method1) t*_{R}* = 0.72 min; m/z (M+1) = 461.1 | |
| | ¹H NMR (DMSO-d*₆*, 400 MHz) Shift 10.65 (s, 1H), 8.28 (s, 1H), 8.07 (d, 1H, J=2.4 Hz), 8.00 (br s, 1H), 7.97 (s, 1H), 7.71 (d, 1H, J=7.9 Hz), 7.42 (br s, 1H), 7.31 (br s, 1H), 7.27 (d, 1H, J=7.9 Hz), 6.42 (s, 1H), 3.79 (s, 3H), 3.73 (br d, 2H, J=4.6 Hz), 3.5-3.6 (m, 1H), 3.3-3.5 (m, 2H), 2.41 (s, 3H), 2.1-2.3 (m, 2H), 1.30 (s, 9H) | |
| **4** | 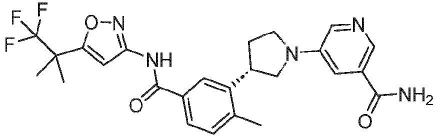 | 30 mg (42%) |
| | LC-MS (ESI): (Method 3) t*_{R}* = 1.48 min; m/z (M+1) = 502.2 | |
| | ¹H NMR (DMSO-d*₆*, 400 MHz) δ 11.43 (s, 1H), 8.28 (d, 1H, J=1.5 Hz), 8.07 (d, 1H, J=2.6 Hz), 8.00 (s, 2H), 7.76 (dd, 1H, J=1.5, 7.9 Hz), 7.42 (s, 1H), 7.3-7.3 (m, 2H), 7.06 (s, 1H), 3.7-3.8 (m, 2H), 3.5-3.6 (m, 1H), 3.3-3.5 (m, 2H), 2.43 (s, 3H), 2.3-2.4 (m, 1H), 2.2-2.3 (m, 1H), 1.53 (s, 6H) | |

### Example 5 - (S)-5-(3-(5-((3-(tert-butyl)-1-methyl-1H-pyrazol-5-yl)carbamoyl)-2-methylphenyl)pyrrolidin-1-yl)nicotinamide

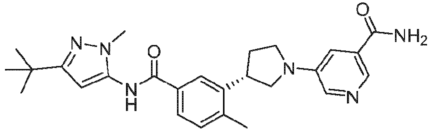

### Step 1 - (S)-3-(1-(5-carbamoylpyridin-3-yl)pyrrolidin-3-yl)-4-methylbenzoic acid (Intermediate 19)

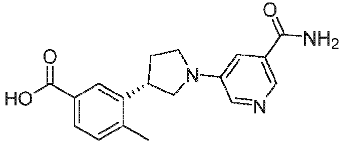

Intermediate 18 (500 mg, 1.415 mmol) was dissolved in THF (1 ml) and H₂O (1.5 ml). LiOH (67.8 mg, 2.83 mmol) was then added. The mixture was stirred overnight at rt. Solvent was evaporated under vacuum. Purification by FCC on reverse phase (gradient A:B from 100:0 to 75:25 with 10 CV, eluent A: H₂O:ACN:HCOOH 95:5:0.1 eluent B:H₂O:ACN:HCOOH 5:95:0.1) yielded the title compound (148 mg, 0.455 mmol, 32.2 % yield).
LC-MS (ESI): (Method 1) t*_{R}* = 0.51 min; m/z (M-1) = 324.2

The following Intermediate was prepared using the procedure described for the synthesis of Intermediate 19.

| **Interme diate N°** | **Structure** | **Product Amount (Yield)** |
|---|---|---|
| | **Analytical Data** | |
| **Interme diate 52** | 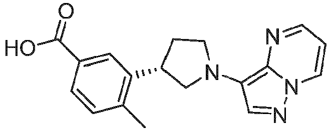 | 136 mg (100%) |
| | LC-MS (ESI): Method 2, t*_{R}* = 1.10 min, m/z (M+1) = 323.1 | |

### Step 2 - (S)-5-(3-(5-((3-(tert-butyl)-1-methyl-1H-pyrazol-5-yl)carbamoyl)-2-methyl phenyl)pyrrolidin-1-yl)nicotinamide (Example 5)

Intermediate 19 (60 mg, 0.184 mmol), HATU (140 mg, 0.369 mmol), DIPEA (0.097 ml, 0.553 mmol) and 3-(tert-butyl)-1-methyl-1H-pyrazol-5-amine (56.5 mg, 0.369 mmol) were dissolved in DMF (1.5 ml). The solution was stirred for 24 h at 50 °C. Then solution was diluted with EtOAc (5 mL) and washed with NaOH 2 M (2 mL). The organic layer was evaporated under vacuum. Purification was performed by FCC on amino silica gel, eluting system DCM/MeOH. The title compound was eluted with 3% of MeOH. Appropriate fractions were collected and evaporated under vacuum to give the title compound (30 mg, 0.065 mmol, 35.3 % yield).
LC-MS (ESI): (Method 3) t*_{R}* = 1.20 min; m/z (M+1) = 461.3
¹H NMR (Acetone-d₆, 400 MHz) δ 9.34 (br s, 1H), 8.39 (s, 1H), 8.11 (d, 1H, J=2.6 Hz), 8.01 (s, 1H), 7.79 (br d, 1H, J=7.9 Hz), 7.51 (br s, 1H), 7.3-7.4 (m, 2H), 6.67 (br s, 1H), 6.05 (s, 1H), 3.8-3.9 (m, 2H), 3.4-3.7 (m, 6H), 2.51 (s, 3H), 2.5-2.5 (m, 1H), 2.2-2.4 (m, 1H), 1.22 (s, 9H)

The following Example and Intermediate were prepared using the procedure described for the synthesis of Example 5 starting from suitable intermediates.

| **Example N°** | **Structure** | **Product Amount (Yield)** |
|---|---|---|
| | **Analytical Data** | |
| **62** | 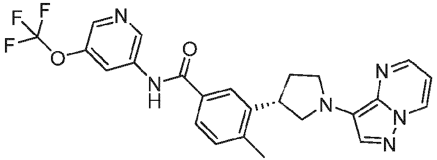 | 14 mg (16%) |
| | LC-MS (ESI): Method 5, t*_{R}* = 4.89 min, m/z (M+1) = 483.5 ¹H NMR (400 MHz, DMSO-d₆) d 10.68 (s, 1H), 8.99 (d, J=2.0 Hz, 1H), 8.86 (dd, J=1.6, 7.2 Hz, 1H), 8.45 (d, J=2.3 Hz, 1H), 8.41 (s, 1H), 8.25 (dd, J=1.5, 3.8 Hz, 1H), 8.03 (d, J=1.8 Hz, 1H), 7.88 (s, 1H), 7.84 (dd, J=1.6, 8.0 Hz, 1H), 7.44 (d, J=7.8 Hz, 1H), 6.83 (dd, J=3.9, 7.2 Hz, 1H), 3.92 - 3.73 (m, 3H), 3.62 - 3.47 (m, 2H), 2.53 (s, 1H), 2.50 - 2.43 (m, 1H), 2.20 - 2.12 (m, 1H). | |
| **Interme diate 53** | 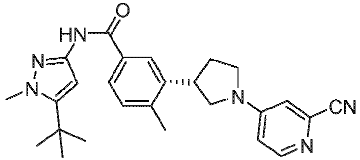 | 150 mg (97%) |
| | LC-MS (ESI): Method 16, t*_{R}* = 2.16 min, m/z (M+1) = 443.10 | |

### Example 6 - (S)-5-(3-(5-((3-(tert-butyl)isoxazol-5-yl)carbamoyl)-2-methylphenyl) pyrrolidin-1-yl)nicotinamide

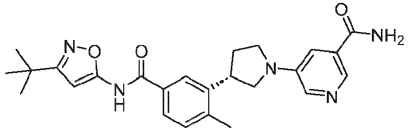

### Step 1 - (S)-N-(3-(tert-butyl)isoxazol-5-yl)-3-(1-(5-cyanopyridin-3-yl)pyrrolidin-3-yl)-4-methylbenzamide (Example 7)

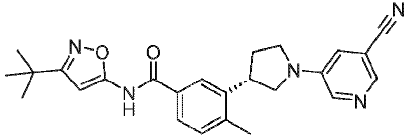

A 2-5 mL vial was loaded with 3-tert-butyl-1,2-oxazol-5-amine (125 mg, 0.894 mmol) and dissolved in 2 mL of THF. The vial was purged and backfilled with argon. Solution was then added of LDA 2M in THF (1.118 ml, 1.118 mmol) and stirred at rt for 6 h. Then Intermediate 17 was dissolved in 2 mL THF and added to the solution. Resulting solution was stirred overnight at rt. LC-MS analysis showed complete conversion. Solvent was evaporated. Resulting solid was dissolved in EtOAc (5mL) and washed with NaHCO₃ sat. solution (5 mL). Purification by FCC on silica gel eluting with a gradient of 40% acetone in n-heptane. Appropriate fractions were combined and evaporated under vacuum to give the title compound (95 mg, 0.221 mmol, 49.5 %). LC-MS (ESI): (Method 3) t*_{R}* = 2.28 min; m/z (M+1) = 430.2
¹H NMR (ACN-d₃, 400 MHz) δ 9.73 (br s, 1H), 8.21 (d, 1H, J=2.9 Hz), 8.13 (s, 1H), 7.83 (s, 1H), 7.74 (d, 1H, J=7.9 Hz), 7.38 (d, 1H, J=7.9 Hz), 7.21 (br s, 1H), 6.40 (s, 1H), 3.7-3.9 (m, 2H), 3.59 (dt, 1H, J=3.3, 8.9 Hz), 3.4-3.5 (m, 2H), 2.48 (s, 3H), 2.4-2.5 (m, 1H), 2.2-2.3 (m, 1H), 1.31 (s, 9H)

Analogously, the following Examples were prepared by reacting Intermediate 17 with the suitable amine, and by varying the strong base.

### Example 48: (S)-3-(1-(5-cyanopyridin-3-yl)pyrrolidin-3-yl)-4-methyl-N-(4-(trifluoromethoxy)pyridin-2-yl)benzamide

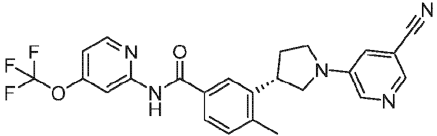

SM: Intermediate 17: 75 mg (1 eq.)
4-(trifluoromethoxy)pyridin-2-amine: 59.7 mg (1.5 eq.)
Base: LiHMDS
Amount/yield: 130 mg/ quantitative
LC-MS (ESI, m/z): method 1, t*_{R}* = 1.27 min, m/z (M+1) = 468.2

### Example 49: (S)-3-(1-(5-cyanopyridin-3-yl)pyrrolidin-3-yl)-4-methyl-N-(5-(trifluoromethoxy)pyridin-2-yl)benzamide

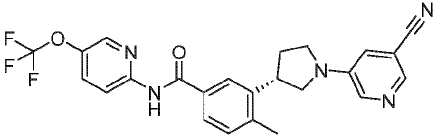

SM: Intermediate 17: 150 mg, (1 eq.)
5-(trifluoromethoxy)pyridin-2-amine: mg (2 eq.)
Base: LiHMDS
Amount/yield: 78 mg/ 37%
LC-MS (ESI, m/z): method 1, t*_{R}* = 1.27 min, m/z (M+1) = 468.08

### Example 50: (S)-N-(5-(tert-butyl)isoxazol-3-yl)-3-(1-(5-cyanopyridin-3-yl)pyrrolidin-3-yl)-4-methylbenzamide

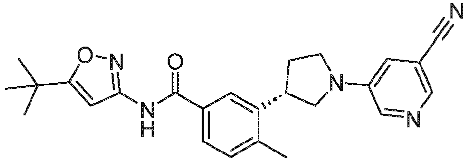

SM: Intermediate 17: 150 mg (1 eq.)
5-tert-butyl-1,2-oxazol-3-amine: 125 mg (2 eq.)
Base: LiHMDS
Amount/yield: 157 mg/ 82%
LC-MS (ESI, m/z): method 1, t*_{R}* = 1.24 min, m/z (M+1) = 430.3

### Example 120: 4-(difluoromethyl)-3- (1-(pyrimidin-5-yl)pyrrolidin-3-yl)-N-(5-(trifluoromethyl)pyridin-3-yl)benzamide

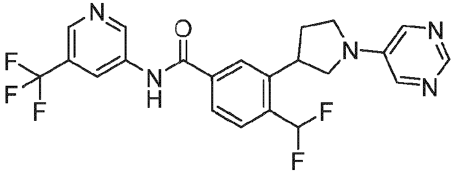

SM: Intermediate 102: 50 mg (1 eq.); 5-(trifluoromethyl)pyridin-3-amine: 58 mg (2.5 eq.)
Base: LiHMDS
Amount/yield: 18 mg (27%)
LC-MS (ESI, m/z): method 4, t*_{R}* = 5.91 min, m/z (M+1) = 464.2
¹H NMR (acetone-d₆, 400 MHz) δ 10.26 (br s, 1H), 9.13 (br d, 1H, J=2.2 Hz), 8.68 (br d, 2H, J=17.8 Hz), 8.45 (s, 1H), 8.31 (s, 1H), 8.14 (s, 2H), 8.07 (br d, 1H, J=8.1 Hz), 7.80 (br d, 1H, J=8.1 Hz), 7.35 (br t, 1H, J=54.7 Hz), 4.0-4.1 (m, 1H), 3.8-3.9 (m, 1H), 3.71 (dt, 1H, J=2.8, 8.9 Hz), 3.4-3.6 (m, 2H), 2.5-2.6 (m, 1H), 2.3-2.5 (m, 1H)

The following Examples were prepared by using the procedure described for Example 120 and the suitable Intermediate.

| **Example N°** | **Structure** | **Product Amount (Yield)** |
|---|---|---|
| | **Analytical Data** | |
| **124** | 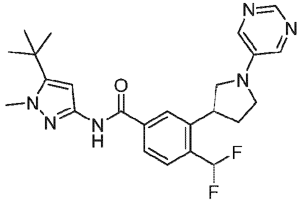 | 12 mg (18%) |
| | LC-MS (ESI, m/z): method 4, t*_{R}* = 5.91 min, m/z (M+1) = 455.3 ¹H NMR (400 MHz, acetone-d₆) δ ppm 9.75 (1 H, br s) 8.46 (1 H, s) 8.34 (1 H, s) 8.15 (2 H, s) 8.08 (1 H, d, J=8.11 Hz) 7.77 (1 H, d, J=8.11 Hz) 7.34 (1 H, t, J=54.81 Hz) 6.60 (1 H, s) 4.02 - 4.12 (1 H, m) 3.85 (3 H, s) 3.77 - 3.84 (1 H, m) 3.72 (1 H, td, J=8.82, 2.74 Hz) 3.52 - 3.61 (2 H, m) 2.48 - 2.57 (1 H, m) 2.36 - 2.48 (1 H, m) 1.38 (9 H, s) | |

### Example 8 - (S)-3-(1-(5-cyanopyridin-3-yl)pyrrolidin-3-yl)-4-methyl-N-(6-(trifluoromethyl)pyrimidin-4-yl)benzamide

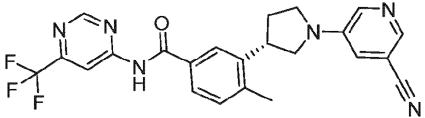

Analogously, Example 8 was prepared by reacting Intermediate 17 with the suitable amine.
SM: Intermediate 17: 150 mg (1 eq.)
6-(trifluoromethyl)pyrimidin-4-amine: 146 mg (2 eq.)
Base: LDA
Amount/yield: 107 mg/ 53%
LC-MS (ESI, m/z): method 3, t*_{R}* = 2.23 min, m/z (M+1) = 453.1
¹H NMR (ACN-d₃, 400 MHz) δ 9.57 (br s, 1H), 9.02 (s, 1H), 8.62 (s, 1H), 8.21 (d, 1H, J=2.8 Hz), 8.13 (s, 1H), 7.86 (s, 1H), 7.78 (dd, 1H, J=1.5, 7.9 Hz), 7.40 (d, 1H, J=8.1 Hz), 7.21 (br s, 1H), 3.7-3.9 (m, 2H), 3.4-3.6 (m, 3H), 2.50 (s, 3H), 2.4-2.5 (m, 1H), 2.2-2.3 (m, 1H)

### Step 2 - (S)-5-(3-(5-((3-(tert-butyl)isoxazol-5-yl)carbamoyl)-2-methylphenyl)pyrrolidin-1-yl)nicotinamide (Example 6)

Example 6 was prepared using the same procedure described for the synthesis of Intermediate 18.
SM: Example 7: 90 mg (1 eq.)
Amount/yield: 41.5 mg/ 44 %
LC-MS (ESI): (Method 3) t*_{R}* = 1.38 min; m/z (M+1) = 448.1
¹H NMR (ACN-d₃, 400 MHz) δ 9.90 (br s, 1H), 8.2-8.3 (m, 1H), 8.12 (d, 1H, J=2.8 Hz), 7.86 (s, 1H), 7.74 (d, 1H, J=7.6 Hz), 7.37 (d, 1H, J=8.1 Hz), 7.2-7.3 (m, 1H), 6.83 (br s, 1H), 6.40 (s, 1H), 6.04 (br s, 1H), 3.7-3.9 (m, 2H), 3.4-3.6 (m, 3H), 2.3-2.5 (m, 4H), 2.1-2.3 (m, 1H), 1.31 (s, 9H)

The following Examples were prepared by using the procedure described for Example 6 and the suitable Intermediate/Example.

| **Example N°** | **Structure** | **Product Amount (Yield)** |
|---|---|---|
| | **Analytical Data** | |
| **9** | 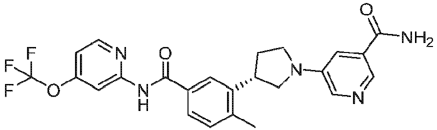 | 33 mg (24%) |
| | LC-MS (ESI, m/z): method 3, t*_{R}* = 1.48 min, m/z (M+1) = 486.2 ¹H NMR (DMSO-d*₆*, 400 MHz) δ 11.19 (s, 1H), 8.45 (d, 1H, J=5.7 Hz), 8.28 (s, 1H), 8.20 (s, 1H), 8.07 (d, 1H, J=2.4 Hz), 8.05 (s, 1H), 7.99 (br s, 1H), 7.79 (d, 1H, J=7.5 Hz), 7.42 (br s, 1H), 7.3-7.3 (m, 2H), 7.14 (br d, 1H, J=5.7 Hz), 3.7-3.8 (m, 2H), 3.5-3.6 (m, 1H), 3.4-3.5 (m, 2H), 2.43 (s, 3H), 2.2-2.4 (m, 2H) | |
| **10** | 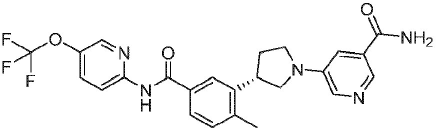 | 40 mg (49%) |
| | LC-MS (ESI, m/z): method 3, t*_{R}* = 1.47 min, m/z (M+1) = 486.1 ¹H NMR (DMSO-d*₆*, 400 MHz) δ 11.06 (s, 1H), 8.44 (d, 1H, J=2.6 Hz), 8.3-8.3 (m, 2H), 8.07 (d, 1H, J=2.6 Hz), 8.03 (s, 1H), 8.00 (br s, 1H), 7.9-7.9 (m, 1H), 7.78 (d, 1H, J=7.9 Hz), 7.42 (s, 1H), 7.3-7.4 (m, 2H), 3.7-3.8 (m, 2H), 3.5-3.6 (m, 1H), 3.4-3.5 (m, 2H), 2.43 (s, 3H), 2.2-2.4 (m, 2H) | |
| **11** | 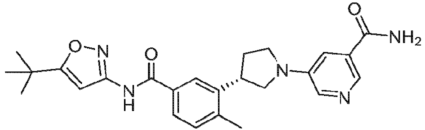 | 167 mg (60%) |
| | LC-MS (ESI, m/z): method 3, t*_{R}* = 1.43 min, m/z (M+1) = 448.1 ¹H NMR (DMSO-d₆, 400 MHz) δ 11.28 (s, 1H), 8.28 (s, 1H), 8.07 (d, 1H, J=2.6 Hz), 8.00 (s, 2H), 7.76 (d, 1H, J=7.9 Hz), 7.43 (s, 1H), 7.31 (s, 1H), 7.32 (d, 2H, J=8.1 Hz), 6.69 (s, 1H), 3.7-3.8 (m, 2H), 3.5-3.6 (m, 1H), 3.4-3.5 (m, 1H), 3.32-3.38 (m, 1H), 2.43 (s, 3H), 2.3-2.4 (m, 1H), 2.2-2.3 (m, 1H), 1.28 (s, 9H) | |
| **63** | 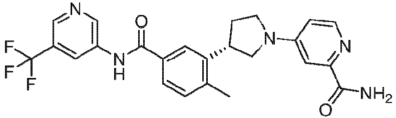 | 15 mg (34%) |
| | LC-MS (ESI, m/z): method 5, t*_{R}* = 3.34 min, m/z (M+1) = 470.4 ¹H NMR (400 MHz, DMSO-d₆) δ 10.63 (s, 1H), 9.18 (d, J=2.0 Hz, 1H), 8.69 (s, 1H), 8.59 (t, J=2.1 Hz, 1H), 8.16 (d, J=5.8 Hz, 1H), 7.99 - 7.94 (m, 2H), 7.82 (dd, J=1.8, 7.8 Hz, 1H), 7.48 (d, J=2.8 Hz, 1H), 7.41 (d, J=8.1 Hz, 1H), 7.21 (d, J=2.5 Hz, 1H), 6.68 (dd, J=2.7, 5.9 Hz, 1H), 3.88 - 3.79 (m, 2H), 3.66 - 3.59 (m, 1H), 3.54 - 3.39 (m, 2H), 2.48 (s, 3H), 2.45 - 2.39 (m, 1H), 2.28 - 2.19 (m, 1H). | |
| **64** | 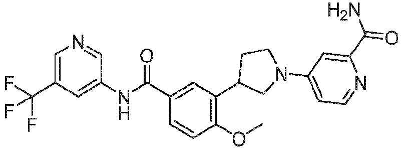 | 21 mg (81%) |
| | LC-MS (ESI, m/z): method 4, t*_{R}* = 3.96 min, m/z (M+1) = 486.2 ¹H NMR (MeOH-d₄, 400 MHz) δ 9.07 (d, 1H, J=2.0 Hz), 8.6-8.6 (m, 1H), 8.58 (s, 1H), 8.12 (d, 1H, J=5.9 Hz), 7.9-8.0 (m, 2H), 7.29 (d, 1H, J=2.4 Hz), 7.14 (d, 1H, J=8.6 Hz), 6.66 (dd, 1H, J=2.5, 6.0 Hz), 3.96 (s, 3H), 3.9-3.9 (m, 2H), 3.6-3.7 (m, 1H), 3.4-3.6 (m, 2H), 2.4-2.5 (m, 1H), 2.3-2.4 (m, 1H) | |
| **65** | 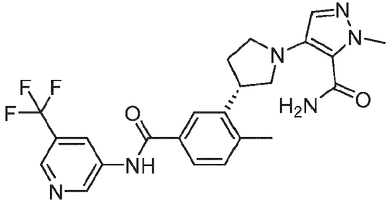 | 13 mg (10%) |
| | LC-MS (ESI, m/z): method 9, t*_{R}* = 3.06 min, m/z (M+1) = 473.87 ¹H NMR (400 MHz, DMSO-d6) δ 10.68 (s, 1H), 9.19 (d, J = 2.3 Hz, 1H), 8.70 (d, J = 2.0 Hz, 1H), 8.63 (t, J = 2.3 Hz, 1H), 7.97 (d, J = 1.9 Hz, 1H), 7.93 (s, 1H), 7.79 (dd, J = 7.9, 1.9 Hz, 1H), 7.65 (s, 1H), 7.48 (s, 1H), 7.37 (d, J = 8.0 Hz, 1H), 3.94 (s, 3H), 3.75 (p, J = 8.2 Hz, 1H), 3.48 - 3.38 (m, 1H), 3.26 (dd, J = 8.7, 5.4 Hz, 2H), 3.16 (dd, J = 9.2, 7.7 Hz, 1H), 2.43 (s, 3H), 2.38 (dd, J = 12.8, 7.5 Hz, 1H), 2.11 - 1.98 (m, 1H). | |
| **66** | 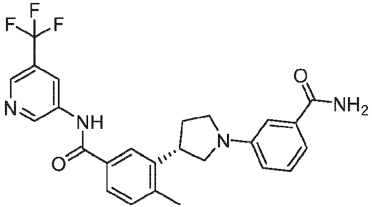 | 65 mg (33%) |
| | LC-MS (ESI, m/z): method 12, t*_{R}* = 2.52 min, m/z (M+1) = 469.24 ¹H NMR (400 MHz, DMSO-d₆) δ 10.64 (s, 1H), 9.19 (d, J = 2.4 Hz, 1H), 8.69 (d, J = 2.0 Hz, 1H), 8.59 (t, J = 2.3 Hz, 1H), 7.97 (d, J = 1.9 Hz, 1H), 7.87 - 7.79 (m, 2H), 7.41 (d, J = 8.0 Hz, 1H), 7.27 - 7.19 (m, 2H), 7.14 - 7.10 (m, 1H), 7.08 (t, J = 2.0 Hz, 1H), 6.73 (dd, J = 8.0, 2.5 Hz, 1H), 3.79 (dq, J = 22.9, 7.7 Hz, 2H), 3.55 (dt, J = 8.6, 5.3 Hz, 1H), 3.45 (td, J = 9.0, 7.0 Hz, 1H), 3.36 (d, J = 8.2 Hz, 1H), 2.49 (s, 3H), 2.44 - 2.36 (m, 1H), 2.28 - 2.17 (m, 1H). | |
| **Interme diate 54** | 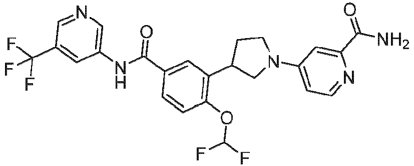 | 86 mg (50%) |
| | LC-MS (ESI, m/z): method 4, t*_{R}* = 4.83 min, m/z (M+1) = 522.2 | |

### Example 12 - (S)-4-methyl-3-(1-(pyrimidin-5-yl)pyrrolidin-3-yl)-N-(4-(trifluoro-methoxy)pyridin-2-yl)benzamide

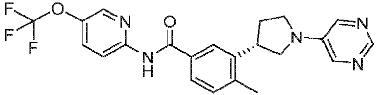

### Step 1 - ethyl (S)-4-methyl-3-(1-(pyrimidin-5-yl)pyrrolidin-3-yl)benzoate (Intermediate 20)

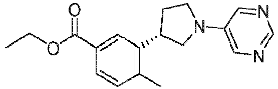

Intermediate 20 was prepared by following the procedure described for the synthesis of Intermediate 17 by reacting Intermediate 16 with the suitable bromo derivative.
SM: Intermediate 16: 3.4 g (1 eq.)
5-bromopyrimidine: 3.48 g (1.5 eq.)
Amount/yield: 3.4 g/ 42%
LC-MS (ESI, m/z): method 1, t*_{R}* = 1.03 min, m/z (M+1) = 312.2

### Step 2 - (S)-4-methyl-3-(1-(pyrimidin-5-yl)pyrrolidin-3-yl)-N-(4-(trifluoromethoxy) pyridin-2-yl)benzamide (Example 12)

Example 12 was prepared by following the procedure described for the synthesis of Example 7, step 1, by reacting Intermediate 20 with the suitable amine, replacing LDA with LiHDMS.
SM: Intermediate 20: 50 mg (1 eq.)
4-(trifluoromethoxy)pyridin-2-amine: (3.5 eq.)
Amount/yield: 52 mg/ 73%
LC-MS (ESI, m/z): method 3, t*_{R}* = 2.06 min, m/z (M+1) = 444.1
¹H NMR (acetone-d₆, 400 MHz) δ 9.92 (br s, 1H), 8.43 (s, 1H), 8.40 (d, 1H, J=5.7 Hz), 8.35 (s, 1H), 8.1-8.2 (m, 3H), 7.91 (dd, 1H, J=1.8, 7.9 Hz), 7.40 (d, 1H, J=7.9 Hz), 7.0-7.1 (m, 1H), 3.8-4.0 (m, 2H), 3.66 (dt, 1H, J=3.2, 8.8 Hz), 3.4-3.6 (m, 2H), 2.53 (s, 3H), 2.5-2.5 (m, 1H), 2.3-2.4 (m, 1H)

The following Examples were prepared by using the procedure described for Example 12 and the suitable amine in step 2.

| **Example N°** | **Structure** | **Product Amount (Yield)** |
|---|---|---|
| | **Analytical Data** | |
| **13** | 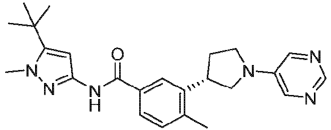 | 42 mg (62%) |
| | LC-MS (ESI, m/z): method 3, t*_{R}* = 1.79 min, m/z (M+1) = 419.3 ¹H NMR (DMSO-d*₆*, 400 MHz) δ 10.64 (s, 1H), 8.43 (s, 1H), 8.14 (s, 2H), 7.95 (s, 1H), 7.72 (d, 1H, J=7.9 Hz), 7.27 (d, 1H, J=7.9 Hz), 6.42 (s, 1H), 3.7-3.8 (m, 5H), 3.5-3.6 (m, 1H), 3.3-3.4 (m, 2H), 2.40 (s, 3H), 2.3-2.4 (m, 1H), 2.1-2.2 (m, 1H), 1.30 (s, 9H) | |
| **14** | 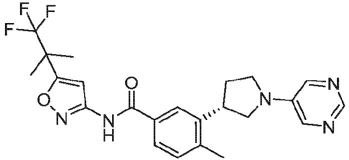 | 45 mg (61%) |
| | LC-MS (ESI, m/z): method 3, t*_{R}* = 2.06 min, m/z (M+1) = 460.2 ¹H NMR (DMSO-d*₆*, 400 MHz) δ 11.43 (s, 1H), 8.44 (s, 1H), 8.15 (s, 2H), 7.99 (s, 1H), 7.77 (d, 1H, J=7.5 Hz), 7.33 (d, 1H, J=7.9 Hz), 7.07 (s, 1H), 3.7-3.8 (m, 2H), 3.5-3.6 (m, 1H), 3.3-3.4 (m, 2H), 2.42 (s, 3H), 2.3-2.4 (m, 1H), 2.1-2.2 (m, 1H), 1.54 (s, 6H) | |
| **15** | 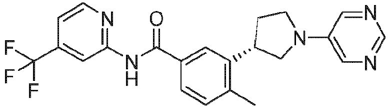 | 31 mg (45%) |
| | LC-MS (ESI, m/z): method 1, t*_{R}* = 1.1 min, m/z (M+1) = 428.2 ¹H NMR (DMSO-d₆, 400 MHz) δ 11.25 (s, 1H), 8.62 (d, 1H, J=5.3 Hz), 8.50 (s, 1H), 8.43 (s, 1H), 8.14 (s, 2H), 8.0-8.1 (m, 1H), 7.79 (dd, 1H, J=1.5, 7.9 Hz), 7.48 (d, 1H, J=5.0 Hz), 7.33 (d, 1H, J=7.9 Hz), 3.7-3.8 (m, 2H), 3.5-3.6 (m, 1H), 3.3-3.4 (m, 2H), 2.42 (s, 3H), 2.3-2.4 (m, 1H), 2.2-2.3 (m, 1H) | |
| **16** | 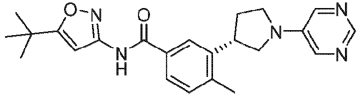 | 30 mg (46%) |
| | LC-MS (ESI, m/z): method 1, t*_{R}* = 1.10 min, m/z (M+1) = 406.3 ¹H NMR (DMSO-d*₆*, 400 MHz) δ 11.25 (s, 1H), 8.43 (s, 1H), 8.14 (s, 2H), 7.98 (s, 1H), 7.75 (d, 1H, J=7.5 Hz), 7.31 (d, 1H, J=7.9 Hz), 6.69 (s, 1H), 3.73 (br d, 2H, J=4.4 Hz), 3.5-3.6 (m, 1H), 3.4-3.5 (m, 1H), 2.41 (s, 3H), 2.1-2.4 (m, 2H), 1.27 (s, 9H) | |
| **17** | 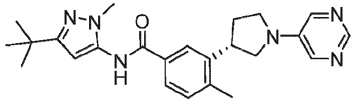 | 43 mg (64%) |
| | LC-MS (ESI, m/z): method 1, t*_{R}* = 0.96 min, m/z (M+1) = 419.3 ¹H NMR (DMSO-d*₆*, 400 MHz) δ 10.09 (s, 1H), 8.43 (s, 1H), 8.14 (s, 2H), 7.85 (s, 1H), 7.71 (d, 1H, J=7.9 Hz), 7.33 (d, 1H, J=7.9 Hz), 6.02 (s, 1H), 3.7-3.8 (m, 2H), 3.5-3.6 (m, 1H), 3.52 (s, 3H), 3.3-3.4 (m, 2H), 2.42 (s, 3H), 2.3-2.4 (m, 1H), 2.14 (br dd, 1H, J=8.6, 12.1 Hz). 1.18 (s, 9H) | |
| **18** | 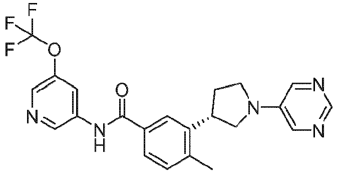 | 46 mg (65%) |
| | LC-MS (ESI, m/z): method 1, t*_{R}* = 1.04 min, m/z (M+1) = 444.2 ¹H NMR (DMSO-d*₆*, 400 MHz) δ 10.58 (s, 1H), 8.90 (d, 1H, J=2.0 Hz), 8.43 (s, 1H), 8.3-8.4 (m, 2H), 8.15 (s, 2H), 7.89 (s, 1H), 7.77 (dd, 1H, J=1.4, 8.0 Hz), 7.37 (d, 1H, J=7.9 Hz), 3.7-3.8 (m, 2H), 3.5-3.6 (m, 1H), 3.3-3.5 (m, 1H), 2.44 (s, 3H), 2.3-2.4 (m, 1H), 2.1-2.2 (m, 1H) | |
| **19** | 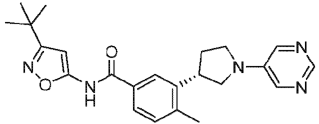 | 13 mg (20%) |
| | LC-MS (ESI, m/z): method 1, t*_{R}* = 1.09 min, m/z (M+1) = 406.2 ¹H NMR (DMSO-d*₆*, 400 MHz) δ 11.4-12.0 (m, 1H), 8.43 (s, 1H), 8.14 (s, 2H), 7.96 (s, 1H), 7.76 (dd, 1H, J=1.4, 7.8 Hz), 7.33 (d, 1H, J=7.9 Hz), 6.36 (s, 1H), 3.7-3.8 (m, 2H), 3.5-3.6 (m, 1H), 3.3-3.5 (m, 2H), 2.42 (s, 3H), 2.1-2.4 (m, 2H), 1.0-1.3 (s, 9H) | |
| **20** | 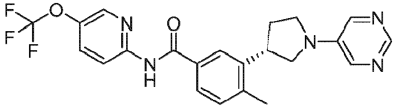 | 45 mg (63%) |
| | LC-MS (ESI, m/z): method 1, t*_{R}* = 1.13 min, m/z (M+1) = 444.2 ¹H NMR (DMSO-d*₆*, 400 MHz) δ 11.04 (s, 1H), 8.4-8.4 (m, 2H), 8.27 (d, 1H, J=9.2 Hz), 8.14 (s, 2H), 8.0-8.0 (m, 1H), 7.9-7.9 (m, 1H), 7.8-7.8 (m, 1H), 7.31 (d, 1H, J=7.9 Hz), 3.7-3.8 (m, 2H), 3.5-3.6 (m, 1H), 3.3-3.4 (m, 2H), 2.42 (s, 3H), 2.3-2.4 (m, 1H), 2.2-2.3 (m, 1H) | |
| **21** | 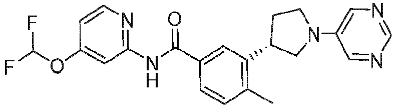 | 40 mg (59%) |
| | LC-MS (ESI, m/z): method 1, t*_{R}* = 1.00 min, m/z (M+1) = 426.1 ¹H NMR (DMSO-d*₆*, 400 MHz) δ 10.99 (s, 1H), 8.43 (s, 1H), 8.33 (d, 1H, J=5.7 Hz), 8.14 (s, 2H), 8.0-8.0 (m, 2H), 7.78 (dd, 1H, J=1.6, 8.0 Hz), 7.44 (t, 1H, J=75.0 Hz), 7.31 (d, 1H, J=7.9 Hz), 6.95 (dd, 1H, J=2.2, 5.7 Hz), 3.7-3.8 (m, 2H), 3.5-3.6 (m, 1H), 3.3-3.4 (m, 2H), 2.42 (s, 3H), 2.2-2.4 (m, 2H) | |
| **67** | 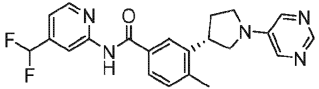 | 50 mg (76%) |
| | LC-MS (ESI, m/z): method 4, t*_{R}* = 6.3 min, m/z (M+1) = 410.3 ¹H NMR (acetone-d₆, 400 MHz) δ 11.09 (s, 1H), 8.53 (d, 1H, J=4.8 Hz), 8.46 (s, 1H), 8.40 (s, 1H), 8.18 (s, 2H), 8.07 (d, 1H, J=1.8 Hz), 7.82 (dd, 1H, J=1.6, 8.0 Hz), 7.3-7.4 (m, 2H), 7.14 (t, 1H, J=55 Hz), 3.7-3.8 (m, 2H), 3.6-3.6 (m, 1H), 3.4-3.5 (m, H), 2.46 (s, 3 H), 2.2-2.4 (m, 2H) | |
| **68** | 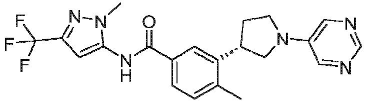 | 85 mg (62%) |
| | LC-MS (ESI, m/z): method 9, t*_{R}* = 2.9 min, m/z (M+1) = 431.2 ¹H NMR (400 MHz, DMSO-d₆) δ 10.48 (s, 1H), 8.47 (s, 1H), 8.18 (s, 2H), 7.91 (d, J = 1.8 Hz, 1H), 7.78 (dd, J = 7.9, 1.9 Hz, 1H), 7.39 (d, J = 8.0 Hz, 1H), 6.70 (s, 1H), 3.79 (p, J = 6.7, 5.9 Hz, 2H), 3.75 (s, 3H), 3.62 - 3.54 (m, 1H), 3.44 (td, J = 8.8, 6.9 Hz, 1H), 3.39 - 3.34 (m, 1H), 2.47 (s, 3H), 2.39 (t, J = 5.9 Hz, 1H), 2.24 - 2.14 (m, 1H). | |
| **69** | 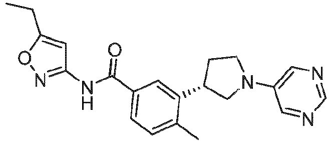 | 19 mg (30%) |
| | LC-MS (ESI, m/z): method 9, t*_{R}* = 2.9 min, m/z (M+1) = 378.2 ¹H NMR (400 MHz, DMSO-d₆) δ 11.27 (s, 1H), 8.47 (s, 1H), 8.18 (s, 2H), 8.02 (d, J = 1.9 Hz, 1H), 7.79 (dd, J = 7.9, 1.9 Hz, 1H), 7.34 (d, J = 8.0 Hz, 1H), 6.74 (d, J = 1.0 Hz, 1H), 3.82 - 3.71 (m, 2H), 3.63 - 3.56 (m, 1H), 3.44 (q, J = 8.9 Hz, 1H), 3.37 (d, J = 12.4 Hz, 1H), 2.80 - 2.69 (m, 2H), 2.45 (s, 3H), 2.37 (d, J = 12.1 Hz, 1H), 2.23 (dd, J = 12.1, 8.6 Hz, 1H), 1.22 (t, J = 7.6 Hz, 3H). | |
| **70** | 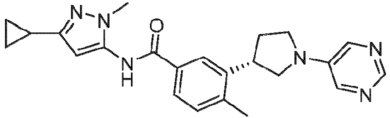 | 47 mg (36%) |
| | LC-MS (ESI, m/z): Method 10, t*_{R}* = 3.0 min, m/z (M+1) = 403.2 ¹H NMR (400 MHz DMSO-d₆) δ 10.13 (s, 1H), 8.47 (s, 1H), 8.18 (s, 2H), 7.94 - 7.85 (m, 1H), 7.74 (dd, J = 7.9, 1.8 Hz, 1H), 7.36 (d, J = 8.0 Hz, 1H), 5.89 (s, 1H), 3.78 (q, J = 7.0, 6.5 Hz, 2H), 3.57 (ddd, J = 11.6, 8.4, 3.3 Hz, 1H), 3.52 (s, 3H), 3.44 (q, J = 8.6 Hz, 1H), 3.40 - 3.33 (m, 1H), 2.46 (s, 3H), 2.39 (s, 1H), 2.24 - 2.14 (m, 1H), 1.80 (tt, J = 8.3, 5.0 Hz, 1H), 0.87 - 0.77 (m, 2H), 0.64 - 0.56 (m, 2H). | |

### Example 22 - (S)-3-(1-(5-(difluoromethyl)pyridin-3-yl)pyrrolidin-3-yl)-4-methyl-N-(5-(trifluoromethyl)pyridin-3-yl)benzamide

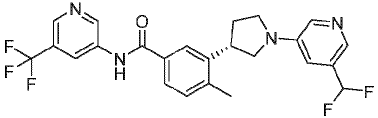

### Step 1 - tert-butyl (S)-3-(2-methyl-5-((5-(trifluoromethyl)pyridin-3-yl)carbamoyl)phenyl)pyrrolidine-1-carboxylate (Intermediate 21)

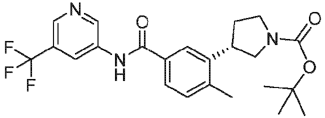

Intermediate 21 was prepared by following the same procedure described for the synthesis of Intermediate 15a and 15b.
SM: Intermediate 14b: 9.50 g (1 eq.)
5-(trifluoromethyl)pyridin-3-amine: 9.70 (2.1 eq.)
Amount/yield: 8.23 g/ 64%
LC-MS (ESI, m/z): method 3, t*_{R}* = 2.40 min, m/z (M+1) = 450.2
¹H NMR (ACN-d₃, 400 MHz) δ 9.08 (br s, 1H), 9.05 (d, 1H, *J*=2.0 Hz), 8.63 (s, 1H), 8.58 (s, 1H), 7.82 (d, 1H, *J*=1.3 Hz), 7.72 (dd, 1H, *J*=1.8, 7.9 Hz), 7.34 (d, 1H, *J*=7.9 Hz), 3.75 (dd, 1H, *J*=7.7, 10.3 Hz), 3.64 (br d, 1H, *J*=4.2 Hz), 3.54 (ddd, 1H, *J*=3.2, 8.0, 10.7 Hz), 3.3-3.4 (m, 2H), 2.43 (s, 3H), 2.22 (br d, 1H, *J*=2.4 Hz), 2.0-2.1 (m, 1H), 1.44 (br s, 9H)

### Step 2 - (S)-4-methyl-3-(pyrrolidin-3-yl)-N-(5-(trifluoromethyl)pyridin-3-yl)benzamide hydrochloride (Intermediate 22)

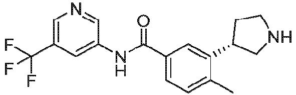

Intermediate 21 (8.2 g, 18.24 mmol) was dissolved in 22 mL of HCl (22.80 ml, 91 mmol) 4M in Dioxane and stirred at rt for 6 h. Since the reaction did not get to completion TFA (2 ml, 30.4 mmol) was added, and the solution stirred for 1 h at rt. Solvent was evaporated under vacuum to give the title compound in a quantitative yield which was used without further purifications in next steps.
LC-MS (ESI, m/z): method 1, t*_{R}* = 0.55 min, m/z (M+1) = 350.1

The following Intermediates were prepared using the procedure described for Intermediate 22.

| **Interme diate N°** | **Structure** | **Product Amount (Yield)** |
|---|---|---|
| | **Analytical Data** | |
| **Interme diate 55** | 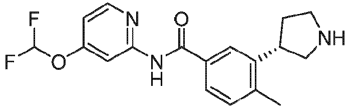 | 17 mg (14%) |
| | LC-MS (ESI): Method 2, t*_{R}* = 1.64 min, m/z (M+1) = 348.2 | |
| **Interme diate 56** | 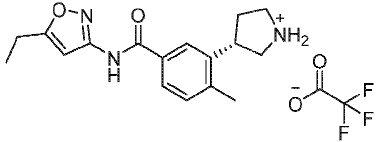 | 601 mg (74%) |
| | LC-MS (ESI): Method 2, t*_{R}* = 1.5 min, m/z (M+1) = 300.1 | |

### Step 3 - (S)-3-(1-(5-(difluoromethyl)pyridin-3-yl)pyrrolidin-3-yl)-4-methyl-N-(5-(trifluoromethyl)pyridin-3-yl)benzamide (Example 22)

Example 22 was prepared by following the procedure described for the synthesis of Intermediate 17 by reacting Intermediate 22 with the suitable bromo derivative.
SM: Intermediate 22: 80 mg (1 eq., 0.229 mmol)
3-bromo-5-(difluoromethyl)pyridine: 71.4 mg (1.5 eq)
Amount/ Yield (31 mg, 28%)
LC-MS (ESI, m/z): method 4, t*_{R}* = 7.79 min, m/z (M+1) = 477.2
¹H NMR (MeOH-d₄, 400 MHz) δ 9.06 (s, 1H), 8.58-8.60 (m, 2H), 8.0-8.1 (m, 1H), 7.94 (d, 2H, J=9.9 Hz), 7.77 (d, 1H, J=7.4 Hz), 7.37 (d, 1H, J=7.9 Hz), 7.14 (br s, 1H), 6.80 (t, 1H, J=55.7 Hz), 3.8-3.9 (m, 2H), 3.63 (dt, 1H, J=3.3, 8.8 Hz), 3.4-3.6 (m, 2H), 2.50 (s, 3H), 2.4-2.5 (m, 1H), 2.2-2.4 (m, 1H)

The following Examples were prepared using the procedure described for Example 22, step 3, using the suitable bromo derivative.

| **Example N°** | **Structure** | **Product Amount (Yield)** | **Catalyst-Ligand Base Solvent** |
|---|---|---|---|
| | **Analytical Data** | | |
| **23** | 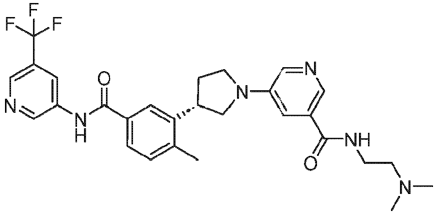 | 17 mg (14%) | RuPhos, Pd(dppf)Cl₂ Cs₂CO₃ DMA |
| | LC-MS (ESI, m/z): method 4, t*_{R}* = 4.90 min, m/z (M+1) = 541.3 | | |
| | ¹H NMR ( MeOH-d₄, 400 MHz) δ 9.0-9.1 (m, 1H), 8.57 and 8.58 (s, 2H, 1 H each), 8.23 (s, 1H), 8.02 (d, 1H, J=2.4 Hz), 7.91 (s, 1H), 7.75 (d, 1H, J=7.5 Hz), 7.41 (br s, 1H), 7.35 (d, 1H, J=8.1 Hz), 3.7-3.9 (m, 2H), 3.2-3.6 (m, 5H), 2.69 (t, 2H, J=6.5 Hz), 2.49 (s, 3H), 2.4-2.5 (m, 1H), 2.39 (s, 6H), 2.2-2.3 (m, 1H) | | |
| **24** | 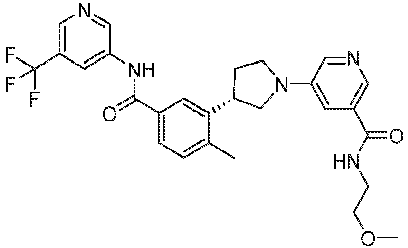 | 31 mg (27%) | RuPhos, Pd(dppf)Cl₂ Cs₂CO₃ DMA |
| | LC-MS (ESI, m/z): method 4, t*_{R}* = 6.47 min, m/z (M+1) = 528.2 | | |
| | ¹H NMR ( DMSO-d₆, 400 MHz) δ 10.61 (s, 1H), 9.1-9.2 (m, 1H), 8.66 (s, 1H), 8.5-8.6 (m, 2H), 8.26 (s, 1H), 8.08 (d, 1H, J=2.2 Hz), 7.93 (s, 1H), 7.79 (d, 1H, J=7.9 Hz), 7.50 (br s, 1H), 7.38 (d, 1H, J=8.1 Hz), 7.30 (br s, 1H), 3.7-3.8 (m, 2H), 3.5-3.6 (m, 1H), 3.3-3.5 (m, 6H), 3.23 (s, 3H), 2.1-2.4 (m, 2H) | | |
| **25** | 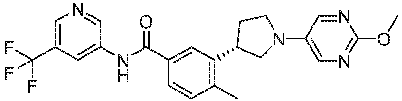 | 39 mg (20%) | Pd 170 Cs₂CO₃ Dioxane |
| | LC-MS (ESI, m/z): method 3, t*_{R}* = 2.06 min, m/z (M+1) = 458.1 | | |
| | ¹H NMR ( DMSO-d₆, 400 MHz) δ 10.61 (s, 1H), 9.1-9.2 (m, 1H), 8.66 (s, 1H), 8.5-8.6 (m, 2H), 8.26 (s, 1H), 8.08 (d, 1H, J=2.2 Hz), 7.93 (s, 1H), 7.79 (d, 1H, J=7.9 Hz), 7.50 (br s, 1H), 7.38 (d, 1H, J=8.1 Hz), 7.30 (br s, 1H), 3.7-3.8 (m, 2H), 3.5-3.6 (m, 1H), 3.3-3.5 (m, 6H), 3.23 (s, 3H), 2.1-2.4 (m, 2H) | | |
| **26** | 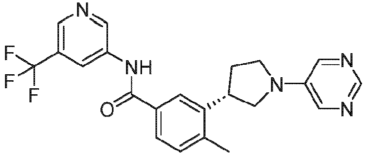 | 87 mg (35%) | RuPhos, Pd(dppf)Cl₂ Cs₂CO₃ DMA |
| | LC-MS (ESI, m/z): method 4, t*_{R}* = 7.11 min, m/z (M+1) = 428.2 | | |
| | ¹H NMR ( MeOH-d₄, 400 MHz) δ 9.06 (s, 1H), 8.60 (br d, 2H, J=8.6 Hz), 8.41 (s, 1H), 8.16 (s, 2H), 7.92 (s, 1H), 7.7-7.8 (m, 1H), 7.37 (d, 1H, J=8.1 Hz), 3.8-3.9 (m, 2H), 3.64 (dt, 1H, J=3.3, 8.8 Hz), 3.4-3.6 (m, 2H), 2.50 (s, 3H), 2.5-2.5 (m, 1H), 2.2-2.4 (m, 1H) | | |
| **27** | 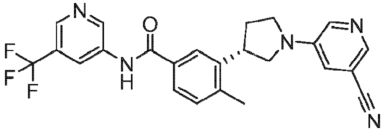 | 0.88 g (45%) | RuPhos, Pd(dppf)Cl₂ Cs₂CO₃ DMA |
| | LC-MS (ESI, m/z): method , t*_{R}* = 2.15 min, m/z (M+1) = 452.1 | | |
| | ¹H NMR ( ACN-d₃, 400 MHz) δ 9.0-9.1 (m, 2H), 8.64 (s, 1H), 8.57 (s, 1H), 8.22 (d, 1H, J=2.8 Hz), 8.13 (s, 1H), 7.86 (s, 1H), 7.75 (dd, 1H, J=1.8, 7.9 Hz), 7.39 (d, 1H, J=7.9 Hz), 7.2-7.2 (m, 1H), 3.8-3.9 (m, 2H), 3.60 (dt, 1H, J=3.3, 8.9 Hz), 3.4-3.5 (m, 2H), 2.49 (s, 3H), 2.4-2.5 (m, 1H), 2.2-2.3 (m, 1H) | | |
| **28** | 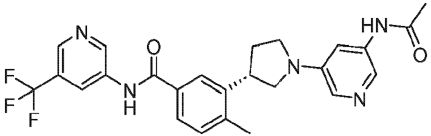 | 17 mg (17%) | RuPhos, Pd(dppf)Cl₂ Cs₂CO₃ DMA |
| | LC-MS (ESI, m/z): method 4, t*_{R}* = 5.49 min, m/z (M+1) = 484.3 | | |
| | ¹H NMR (MeOH-d₄, 400 MHz) δ 9.0-9.1 (m, 1H), 8.59 (br d, 2H, J=7.2 Hz), 7.9-8.0 (m, 2H), 7.76 (d, 1H, J=7.9 Hz), 7.66 (d, 1H, J=2.4 Hz), 7.45 (s, 1H), 7.36 (d, 1H, J=7.9 Hz), 3.8-3.9 (m, 1H), 3.75 (t, 1H, J=8.4 Hz), 3.4-3.6 (m, 3H), 2.50 (s, 3H), 2.4-2.5 (m, 1H), 2.2-2.3 (m, 1H), 2.12 (s, 3H) | | |
| **29** | 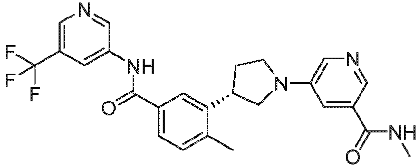 | 34 mg (31%) | RuPhos, Pd(dppf)Cl₂ Cs₂CO₃ DMA |
| | LC-MS (ESI, m/z): method 4, t*_{R}* = 5.71 min, m/z (M+1) = 484.3 | | |
| | ¹H NMR (MeOH-d₄, 400 MHz) δ 9.05 (d, 1H, J=1.8 Hz), 8.59 (br d, 2H, J=7.0 Hz), 8.19 (s, 1H), 8.04 (d, 1H, J=2.9 Hz), 7.92 (s, 1H), 7.77 (dd, 1H, J=1.5, 7.9 Hz), 7.3-7.4 (m, 2H), 3.8-3.9 (m, 2H), 3.64 (dt, 1H, J=3.6, 8.7 Hz), 3.4-3.6 (m, 2H), 2.90 (s, 3H), 2.51 (s, 3H), 2.5-2.5 (m, 1H), 2.2-2.4 (m, 1H) | | |
| **30** | 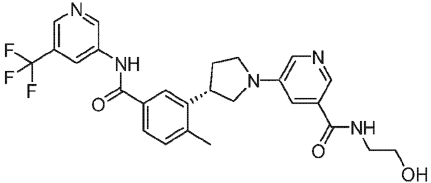 | 10 mg (16%) | RuPhos, Pd(dppf)Cl₂ Cs₂CO₃ DMA |
| | LC-MS (ESI, m/z): method 4, t*_{R}* = 4.98 min, m/z (M+1) = 514.2 | | |
| | ¹H NMR (MeOH-d₄, 400 MHz) δ 9.0-9.1 (m, 1H), 8.59 (br d, 2H, J=8.1 Hz), 8.23 (s, 1H), 8.0-8.1 (m, 1H), 7.92 (s, 1H), 7.76 (d, 1H, J=7.9 Hz), 7.42 (br s, 1H), 7.37 (d, 1H, J=7.9 Hz), 3.8-3.9 (m, 2H), 3.4-3.7 (m, 7H), 2.51 (s, 3H), 2.4-2.5 (m, 1H), 2.2-2.4 (m, 1H) | | |
| **31** | 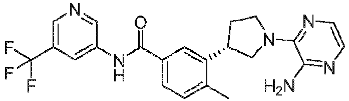 | 45 mg (21%) | RuPhos Pd G2 1 M lithium bis(trimethyls ilyl)amide THF |
| | LC-MS (ESI, m/z): method 5, t*_{R}* = 3.63 min, m/z (M+1) = 443.3 | | |
| | ¹H NMR (400 MHz, DMSO-d₆) δ 10.68 (s, 1H), 9.21 (d, J=2.3 Hz, 1H), 8.71 (s, 1H), 8.62 (t, J=2.3 Hz, 1H), 7.99 (d, J=1.6 Hz, 1H), 7.81 (dd, J=1.8, 7.9 Hz, 1H), 7.41 (d, J=5.8 Hz, 3H), 5.77 (s, 2H), 3.85 - 3.55 (m, 5H), 2.47 (s, 3H), 2.36 - 2.30 (m, 1H), 2.13 - 2.06 (m, 1H). | | |
| **32** | 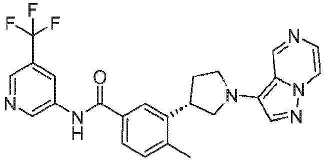 | 24 mg (12%) | tBuBrettPhos Pd G3 *t*BuONa Dioxane |
| | LC-MS (ESI, m/z): method 5, t*_{R}* = 3.95 min, m/z (M+1) = 467.2 | | |
| | ¹H NMR (400 MHz, DMSO-d₆) δ 10.64 (s, 1H), 9.16 (dd, J=1.8, 10.4 Hz, 2H), 8.68 (s, 1H), 8.59 (t, J=2.1 Hz, 1H), 8.38 (dd, J=1.4, 4.9 Hz, 1H), 7.99 (d, J=1.8 Hz, 1H), 7.81 (dd, J=1.6, 8.0 Hz, 1H), 7.66 (s, 1H), 7.53 (d, J=4.8 Hz, 1H), 7.40 (d, J=8.1 Hz, 1H), 3.87 - 3.81 (m, 2H), 3.72 - 3.59 (m, 2H), 3.53 - 3.49 (m, 1H), 2.48 (s, 3H), 2.46 - 2.38 (m, 1H), 2.24 - 2.17 (m, 1H). | | |
| **33** | 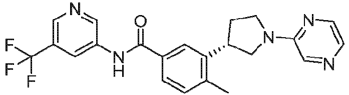 | 24 mg (39%) | RuPhos, Pd(dppf)Cl₂ Cs₂CO₃ DMA |
| | LC-MS (ESI, m/z): method 1, t*_{R}* = 1.06 min, m/z (M+1) = 428.32 | | |
| | ¹H NMR ( DMSO-d₆, 400 MHz) δ 10.66 (s, 1H), 9.19 (s, 1H), 8.69 (s, 1H), 8.59 (s, 1H), 8.0-8.1 (m, 2H), 7.95 (s, 1H), 7.8-7.8 (m, 2H), 7.41 (d, 1H, J=7.9 Hz), 3.97 (dd, 1H, J=7.7, 9.9 Hz), 3.7-3.8 (m, 2H), 3.55 (br d, 1H, J=7.2 Hz), 3.47 (t, 1H, J=9.4 Hz), 3.25 (d, 1H, J=7.2 Hz), 2.48 (s, 3H), 2.3-2.4 (m, 1H) | | |
| **34** | 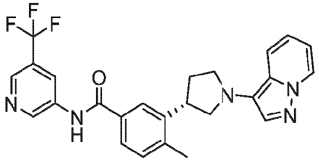 | 30 mg (15%) | tBuBrettPhos Pd G3 *t*BuONa Dioxane |
| | LC-MS (ESI, m/z): method 5, t*_{R}* = 4.92 min, m/z (M+1) = 466.2 | | |
| | ¹H NMR (400 MHz, DMSO-d₆) δ 10.68 (s, 1H), 9.19 (d, J=2.1 Hz, 1H), 8.69 (s, 1H), 8.61 (t, J=2.3 Hz, 1H), 8.41 (d, J=7.2 Hz, 1H), 8.03 (d, J=1.6 Hz, 1H), 7.80 (dd, J=1.8, 7.9 Hz, 1H), 7.74 (d, J=9.2 Hz, 1H), 7.65 (s, 1H), 7.39 (d, J=8.0 Hz, 1H), 6.88 (dd, J=6.6, 8.7 Hz, 1H), 6.67 (dd, J=6.3 Hz, 1H), 3.83 - 3.66 (m, 2H), 3.57 - 3.36 (m, 3H), 2.47 (s, 3H),2.45 - 2.39 (m, 1H),2.17 - 2.08 (m, 1H). | | |
| **35** | 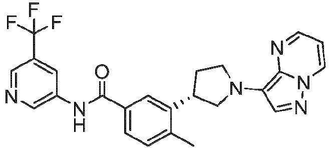 | 18 mg (9%) | tBuBrettPhos Pd G3 *t*BuONa Dioxane |
| | LC-MS (ESI, m/z): method 5, t*_{R}* = 4.79 min, m/z (M+1) = 467.2 | | |
| | ¹H NMR (400 MHz, DMSO-d₆) δ 10.65 (s, 1H), 9.18 (d, J=2.3 Hz, 1H), 8.81 (dd, J=1.8, 7.1 Hz, 1H), 8.68 (s, 1H), 8.59 (dd, J= 2.1 Hz, 1H), 8.19 (dd, J=1.6, 3.9 Hz, 1H), 7.99 (d, J=2.0 Hz, 1H), 7.82 (s, 1H), 7.80 (dd, J=2.1, 8.0 Hz, 1H), 7.39 (d, J=7.8 Hz, 1H), 6.78 (dd, J=3.9, 7.2 Hz, 1H), 3.87 - 3.76 (m, 2H), 3.73 - 3.68 (m, 1H), 3.56 - 3.46 (m, 2H), 2.47 (s, 3H), 2.13 - 2.07 (m, 1H). | | |
| **36** | 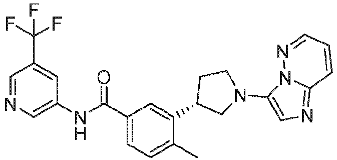 | 28 mg (13%) | tBuBrettPhos Pd G3 *t*BuONa Dioxane |
| | LC-MS (ESI, m/z): method 6, t*_{R}* = 3.84 min, m/z (M+1) = 467.2 | | |
| | ¹H NMR (400 MHz, DMSO-d₆) δ 10.66 (s, 1H), 9.19 (d, J=2.0 Hz, 1H), 8.70 - 8.68 (m, 1H), 8.60 (t, J=2.3, Hz, 1H), 8.39 (dd, J=1.5, 4.3 Hz, 1H), 8.04 (d, J=1.5 Hz, 1H), 7.94 (dd, J=1.6, 9.2 Hz, 1H), 7.80 (dd, J=1.9, 8.0 Hz, 1H), 7.39 (d, J=7.8 Hz, 1H), 7.29 (s, 1H), 6.98 (dd, J=4.4, 9.2 Hz, 1H), 3.86 - 3.79 (m, 2H), 3.72 - 3.52 (m, 3H), 2.47 (s, 3H), 2.17 - 2.11 (m, 1H). | | |
| **37** | 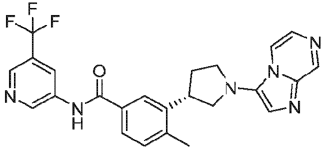 | 15 mg (7.5%) | tBuBrettPhos Pd G3 *t*BuONa Dioxane |
| | LC-MS (ESI, m/z): method 5, t*_{R}* = 3.74 min, m/z (M+1) = 467.4 | | |
| | ¹H NMR (400 MHz, DMSO-d₆) δ 10.67 (s, 1H), 9.20 (d, J=2.3 Hz, 1H), 8.88 (d, J=1.5 Hz, 1H), 8.71 - 8.69 (m, 1H), 8.61 (t, J=2.3 Hz, 1H), 8.33 (dd, J=1.4, 4.7 Hz, 1H), 8.04 (d, J=1.8 Hz, 1H), 7.81 (dd, J=1.8, 7.8 Hz, 1H), 7.75 (d, J=4.8 Hz, 1H), 7.50 (s, 1H), 7.39 (d, J=8.1 Hz, 1H), 3.86 - 3.72 (m, 2H), 3.57 (dd, J=5.8, 7.8 Hz, 2H), 3.48 - 3.41 (m, 1H), 2.47 (s, 3H), 2.22 - 2.12 (m, 1H). | | |
| **71** | 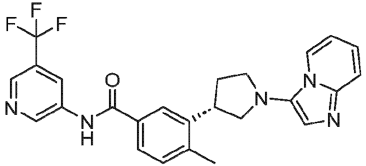 | 15 mg (7.5%) | tBuBrettPhos Pd G3 *t*BuONa Dioxane |
| | LC-MS (ESI, m/z): method 5, t*_{R}* = 6.22 min, m/z (M+1) = 466.4 | | |
| | ¹H NMR (400 MHz, DMSO-d₆) δ 10.68 (s, 1H), 9.21 (d, J=2.3 Hz, 1H), 8.71 - 8.70 (m, 1H), 8.62 (t, J=2.1 Hz, 1H), 8.26 (d, J=7.1 Hz, 1H), 8.08 (d, J=1.8 Hz, 1H), 7.80 (dd, J=1.8, 7.8 Hz, 1H), 7.46 (d, J=9.1 Hz, 1H), 7.39 (d, J=7.8 Hz, 1H), 7.31 (s, 1H), 7.13 (ddd, J=1.3, 6.6, 9.1 Hz, 1H), 6.88 - 6.83 (m, 1H), 3.87 - 3.80 (m, 1H), 3.65 - 3.59 (m, 1H), 3.51 - 3.41 (m, 2H), 2.47 (s, 3H), 2.16 - 2.07 (m, 1H). | | |
| **72** | 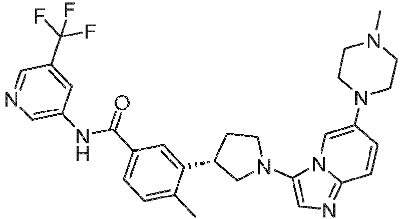 | 6 mg (2.5%) | tBuBrettPhos Pd G3 *t*BuONa Dioxane |
| | LC-MS (ESI, m/z): method 5, t*_{R}* = 2.86 min, m/z (M+1) = 564.4 | | |
| | ¹H NMR (400 MHz, DMSO-d₆) δ 10.68 (s, 1H), 9.19 (d, J=2.3 Hz, 1H), 8.70 - 8.69 (m, 1H), 8.63 (t, J=1.9 Hz, 1H), 8.16 (d, J=1.8 Hz, 1H), 7.82 (dd, J=1.8, 7.8 Hz, 1H), 7.40 - 7.33 (m, 3H), 7.25 (s, 1H), 7.13 (dd, J=2.5, 9.6 Hz, 1H), 3.88 - 3.80 (m, 1H), 3.52 - 3.34 (m, 4H), 3.00 (m, 4H), 2.46 (s, 3H), 2.38 (m, 4H), 2.13 (s, 3H), 2.10 - 2.04 (m, 1H). | | |
| **73** | 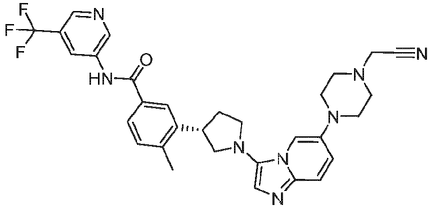 | 2 mg (2%) | tBuBrettPhos Pd G3 *t*BuONa Dioxane |
| | LC-MS (ESI, m/z): method 5, t*_{R}* = 3.82 min, m/z (M+1) = 589.5 | | |
| | ¹H NMR (400 MHz, DMSO-d₆) δ 10.68 - 10.68 (m, 1H), 9.19 (d, J=1.5 Hz, 1H), 8.69 (s, 1H), 8.62 (t, J=2.3 Hz, 1H), 8.14 (d, J=1.5 Hz, 1H), 7.82 (dd, J=1.6, 7.7 Hz, 1H), 7.40 - 7.35 (m, 3H), 7.25 (s, 1H), 7.15 (dd, J=2.0, 9.9 Hz, 1H), 3.86 - 3.81 (m, 2H), 3.72 (s, 2H), 3.54 - 3.36 (m, 2H), 3.10 - 3.02 (m, 4H), 2.62 - 2.53 (m, 6H), 2.46 (s, 3H), 2.13 - 2.06 (m, 1H). | | |
| **74** | 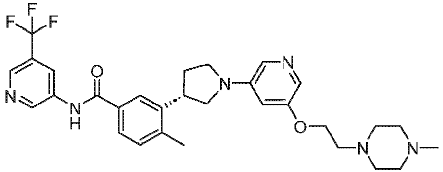 | 14 mg (9%) | Pd 170 Cs₂CO₃ Dioxane |
| | LC-MS (ESI, m/z): method 4, t*_{R}* = 4.33 min, m/z (M+1) = 569.4 | | |
| | ¹H NMR (MeOH-d₄, 600 MHz) δ 8.97 (d, 1H, J=2.3 Hz), 8.5-8.5 (m, 1H), 8.5-8.5 (m, 1H), 7.83 (d, 1H, J=1.8 Hz), 7.68 (dd, 2H, J=1.9, 7.9 Hz), 7.48 (d, 1H, J=2.2 Hz), 7.46 (d, 1H, J=2.3 Hz), 7.28 (d, 1H, J=7.9 Hz), 6.51 (t, 1H, J=2.3 Hz), 4.09 (t, 2H, J=5.4 Hz), 3.78 (dd, 1H, J=7.3, 8.7 Hz), 3.67 (dd, 1H, J=7.7, 9.3 Hz), 3.48 (br dd, 1H, J=3.6, 8.3 Hz), 3.3-3.4 (m, 2H), 2.73 (t, 3H, J=5.4 Hz), 2.3-2.6 (m, 12H), 2.26 (s, 3H), 2.16 (br dd, 1H, J=9.0, 12.3 Hz) | | |
| **75** | 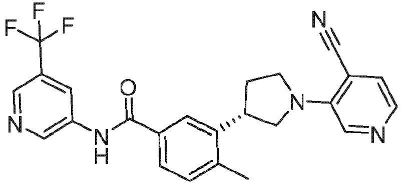 | mg (31%) | 62 RuPhos Pd(dppf)Cl₂ Cs₂CO₃ DMA |
| | LC-MS (ESI, m/z): method 8, t*_{R}* = 4.79 min, m/z (M+1) = 452.2 | | |
| | ¹H NMR (400 MHz, DMSO-d₆) δ 10.68 (d, J=0.9 Hz, 1H), 9.19 (d, J=1.9 Hz, 1H), 8.70 (s, 1H), 8.60 (t, J=2.3 Hz, 1H), 8.33 (s, 1H), 7.97 (s, 1H), 7.91 (d, J=5.0 Hz, 1H), 7.84 (dd, J=1.6, 7.9 Hz, 1H), 7.48 (d, J=5.0 Hz, 1H), 7.42 (d, J=7.9 Hz, 1H), 4.08 (dd, J=7.7, 9.3 Hz, 1H), 3.87 - 3.78 (m, 3H), 3.64 (t, J=9.4 Hz, 1H), 2.48 (s, 3H). 2.44 - 2.35 (m, 1H), 2.29 - 2.24 (m, 1H). | | |
| **76** | 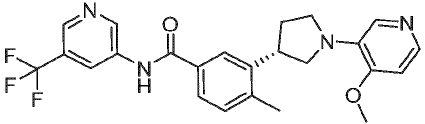 | 6 mg (3%) | RuPhos, Pd(dppf)Cl₂ Cs₂CO₃ DMA |
| | LC-MS (ESI, m/z): method 5, , t*_{R}* = 3.72 min, m/z (M+1) = 457.5 | | |
| | ¹H NMR (400 MHz, DMSO-d₆) δ 10.66 (s, 1H), 9.19 (d, J=2.3 Hz, 1H), 8.69 - 8.68 (m, 1H), 8.60 (t, J=1.9 Hz, 1H), 7.99 (d, J=1.8 Hz, 1H), 7.94 (d, J=5.4 Hz, 1H), 7.91 (s, 1H), 7.80 (dd, J=1.9, 8.0 Hz, 1H), 7.38 (d, J=7.8 Hz, 1H), 6.93 (d, J=5.3 Hz, 1H), 3.82 (s, 3H), 3.72 - 3.45 (m, 5H), 2.45 (s, 3H), 2.35 - 2.29 (m, 1H), 2.14 - 2.04 (m, 1H). | | |
| **77** | 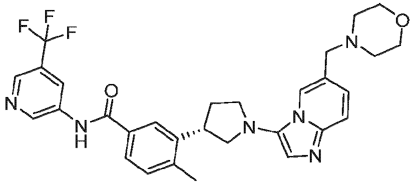 | 50 mg (21%) | tBuBrettPhos Pd G3 *t*BuONa Dioxane |
| | LC-MS (ESI, m/z): method 5, , t*_{R}* = 2.97 min, m/z (M+1) = 565.5 | | |
| | ¹H NMR (400 MHz, DMSO-d₆) δ 10.69 (s, 1H), 9.22 (d, J=2.3 Hz, 1H), 8.70 (s, 1H), 8.63 (t, J=2.4 Hz, 1H), 8.13 (d, J=1.5 Hz, 1H), 8.09 (s, 1H), 7.82 (dd, J=1.8, 7.8 Hz, 1H), 7.44 - 7.38 (m, 2H), 7.30 (s, 1H), 7.12 (dd, J=1.4, 9.2 Hz, 1H), 3.88 - 3.80 (m, 1H), 3.57 - 3.34 (m, 9H), 2.47 (s, 3H), 2.38 - 2.32 (m, 4H), 2.15 - 2.05 (m, 1H). | | |
| **78** | 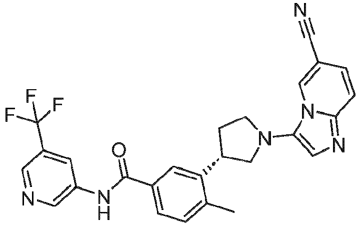 | 9 mg (5%) | tBuBrettPhos Pd G3 Cs₂CO₃ Dioxane |
| | LC-MS (ESI, m/z): method 5, t*_{R}* = 4.2 min, m/z (M+1) = 491.5 | | |
| | ¹H NMR (400 MHz, DMSO-d₆) δ 10.68 (s, 1H), 9.20 (d, J=2.0 Hz, 1H), 9.08 (s, 1H), 8.70 - 8.68 (m, 1H), 8.62 (t, J=2.1 Hz, 1H), 8.07 (d, J=1.5 Hz, 1H), 7.81 (dd, J=1.8, 7.8 Hz, 1H), 7.62 (d, J=9.3 Hz, 1H), 7.47 (s, 1H), 7.40 - 7.32 (m, 2H), 3.88 - 3.79 (m, 1H), 3.73 - 3.67 (m, 1H), 3.55 - 3.37 (m, 4H), 2.47 (s, 3H), 2.19 - 2.09 (m, 1H). | | |
| **79** | 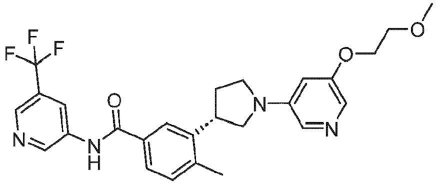 | 20 mg (17%) | RuPhos, Pd(dppf)Cl₂ Cs₂CO₃ DMA |
| | LC-MS (ESI, m/z): method 4, t*_{R}* = 5.76 min, m/z (M+1) = 501.3 | | |
| | ¹H NMR (MeOH-d₄, 400 MHz) δ 9.05 (d, 1H, J=2.2 Hz), 8.60 (d, 1H, J=1.8 Hz), 8.58 (s, 1H), 7.91 (d, 1H, J=1.8 Hz), 7.76 (dd, 1H, J=1.8, 7.9 Hz), 7.55 (br d, 2H, J=6.4 Hz), 7.36 (d, 1H, J=7.9 Hz), 6.60 (t, 1H, J=2.2 Hz), 4.1-4.2 (m, 2H), 3.8-3.9 (m, 1H), 3.7-3.8 (m, 3H), 3.58 (dt, 1H, J=3.6, 8.7 Hz), 3.4-3.5 (m, 2H), 3.39 (s, 3H), 2.50 (s, 3H), 2.4-2.5 (m, 1H), 2.2-2.3 (m, 1H) | | |
| **80** | 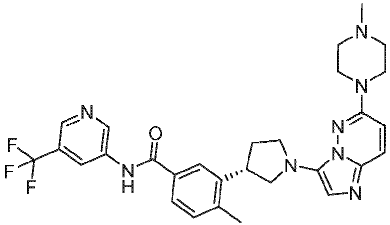 | 39 mg (16%) | tBuBrettPhos Pd G3 *t*BuONa Dioxane |
| | LC-MS (ESI, m/z): method 5, t*_{R}* = 2.9 min, m/z (M+1) = 565.5 | | |
| | ¹H NMR (400 MHz, DMSO-d₆) δ 10.71 (s, 1H), 9.13 (s, 1H), 8.64 (s, 2H), 8.13 (d, J=1.5 Hz, 1H), 7.80 (dd, J=1.8, 7.9 Hz, 1H), 7.68 (d, J=9.9 Hz, 1H), 7.36 (d, J=7.9 Hz, 1H), 7.02 (s, 1H), 6.95 (d, J=10.2 Hz, 1H), 3.82 - 3.69 (m, 3H), 3.63 - 3.38 (m, 7H), 2.46 (s, 3H), 2.38 - 2.32 (m, 4H), 2.11 (s, 3H), 2.08 - 1.99 (m, 1H). | | |
| **81** | 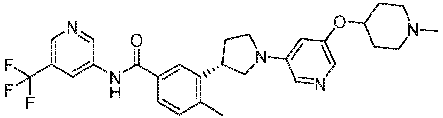 | 39 mg (16%) | RuPhos, Pd(dppf)Cl₂ Cs₂CO₃ DMA |
| | LC-MS (ESI, m/z): method 4, t*_{R}* = 4.4 min, m/z (M+1) = 540.32 | | |
| | ¹H NMR DMSO-d₆, 400 MHz) δ 10.60 (s, 1H), 9.15 (d, 1H, J=2.4 Hz), 8.6-8.7 (m, 1H), 8.5-8.6 (m, 1H), 7.90 (d, 1H, J=1.5 Hz), 7.78 (dd, 1H, J=1.8, 7.9 Hz), 7.58 (br d, 1H, J=2.2 Hz), 7.56 (br d, 1H, J=2.4 Hz), 7.37 (d, 1H, J=7.9 Hz), 6.48 (t, 1H, J=2.2 Hz), 4.4-4.4 (m, 1H), 3.6-3.8 (m, 2H), 3.4-3.6 (m, 1H), 3.3-3.4 (m, 1H), 3.2-3.3 (m, 4H), 2.5-2.7 (m, 4H), 2.3-2.4 (m, 1H), 2.0-2.2 (m, 4H), 1.8-1.9 (m, 2H), 1.5-1.7 (m, 2H) | | |
| **82** | 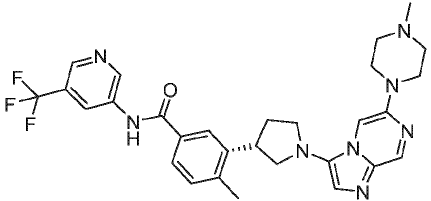 | 10 mg (4%) | tBuBrettPhos Pd G3 tBuONa Dioxane |
| | LC-MS (ESI, m/z): method 5, t*_{R}* = 3.2 min, m/z (M+1) = 565.2 | | |
| | ¹H NMR (400 MHz, DMSO-d₆) δ 10.67 (s, 1H), 9.19 (d, J=2.3 Hz, 1H), 8.71 - 8.68 (m, 2H), 8.62 (s, 1H), 8.11 (d, J=1.8 Hz, 1H), 7.81 (dd, J=1.8, 7.8 Hz, 1H), 7.48 (s, 1H), 7.39 (d, J=7.8 Hz, 1H), 7.24 (d, J=1.3 Hz, 1H), 3.83 (t, J=7.3 Hz, 1H), 3.62 - 3.38 (m, 4H), 3.29 - 3.25 (m, 4H), 2.46 (s, 3H), 2.42 - 2.39 (m, 4H), 2.18 (s, 3H), 2.14 - 2.07 (m, 1H). | | |
| **83** | 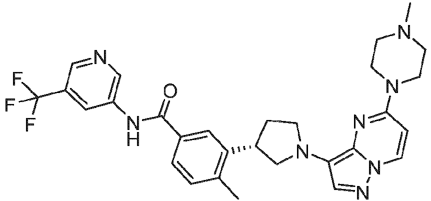 | 19 mg (7%) | tBuBrettPhos Pd G3 *t*BuONa Dioxane |
| | LC-MS (ESI, m/z): method 5, t*_{R}* = 3.0 min, m/z (M+1) = 565.2 | | |
| | ¹H NMR (400 MHz, DMSO-d₆) δ 10.66 (s, 1H), 9.18 (d, J=2.0 Hz, 1H), 8.70 - 8.68 (m, 1H), 8.61 (t, J=2.3 Hz, 1H), 8.46 (d, J=7.8 Hz, 1H), 8.03 (d, J=1.8 Hz, 1H), 7.77 (dd, J=1.8, 7.8 Hz, 1H), 7.60 (s, 1H), 7.37 (d, J=8.1 Hz, 1H), 6.60 (d, J=8.1 Hz, 1H), 3.78 - 3.68 (m, 1H), 3.62 - 3.52 (m, 6H), 3.46 - 3.34 (m, 2H), 2.45 (s, 3H), 2.43 - 2.38 (m, 1H), 2.34 (t, J=4.9 Hz, 4H), 2.15 (s, 3H), 2.01 - 1.92 (m, 1H). | | |
| **84** | 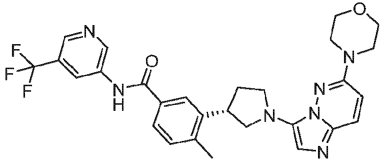 | 59 mg (25%) | tBuBrettPhos Pd G3 *t*BuONa Dioxane |
| | LC-MS (ESI, m/z): method 5, t*_{R}* = 3.8 min, m/z (M+1) = 552.2 | | |
| | ¹H NMR (400 MHz, DMSO-d₆) δ 10.66 (s, 1H), 9.18 (d, J=2.3 Hz, 1H), 8.70 - 8.69 (m, 1H), 8.61 (t, J=2.4 Hz, 1H), 8.09 (d, J=1.5 Hz, 1H), 7.79 (dd, J=1.6, 8.0 Hz, 1H), 7.71 (d, J=9.9 Hz, 1H), 7.39 (d, J=7.8 Hz, 1H), 7.03 (s, 1H), 6.94 (d, J=9.9 Hz, 1H), 3.83 - 3.74 (m, 1H), 3.69 - 3.66 (m, 5H), 3.64 - 3.50 (m, 3H), 3.43 - 3.35 (m, 4H), 2.46 (s, 3H), 2.08 - 2.01 (m, 1H). | | |
| **85** | 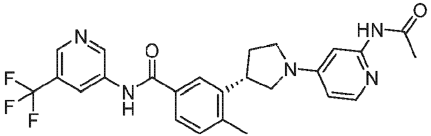 | 35 mg (25%) | RuPhos, Pd(dppf)Cl₂ Cs₂CO₃ DMA |
| | LC-MS (ESI, m/z): Method 10, t*_{R}* = 2.5 min, m/z (M+1) = 484.27 | | |
| | ¹H NMR (400 MHz, DMSO-d₆) δ 10.64 (s, 1H), 10.07 (s, 1H), 9.18 (d, J = 2.3 Hz, 1H), 8.69 (dd, J = 2.1, 1.0 Hz, 1H), 8.59 (t, J = 2.4 Hz, 1H), 7.95 (d, J = 1.9 Hz, 1H), 7.86 (d, J = 5.8 Hz, 1H), 7.81 (dd, J = 7.9, 1.9 Hz, 1H), 7.41 (d, J = 8.0 Hz, 1H), 7.36 (s, 1H), 6.30 (dd, J = 5.9, 2.3 Hz, 1H), 3.77 (dq, J = 16.1, 8.0 Hz, 2H), 3.53 (t, J = 8.9 Hz, 1H), 3.43 (q, J = 8.8 Hz, 1H), 3.35 (s, 1H), 2.47 (s, 3H), 2.43 - 2.34 (m, 1H), 2.26 - 2.15 (m, 1H), 2.04 (s, 3H). | | |
| **86** | 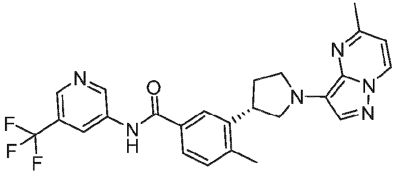 | 10 mg (7%) | BPC-305 Cs₂CO₃ Dioxane |
| | LC-MS (ESI, m/z): method 4, t*_{R}* = 7.3 min, m/z (M+1) = 481.4 | | |
| | ¹H NMR (acetone-d₆, 400 MHz) δ 9.99 (br s, 1H), 9.10 (d, 1H, J=2.2 Hz), 8.70 (s, 1H), 8.63 (s, 1H), 8.49 (d, 1H, J=7.2 Hz), 8.13 (d, 1H, J=1.5 Hz), 7.82 (dd, 1H, J=2.0, 7.9 Hz), 7.68 (s, 1H), 7.37 (d, 1H, J=7.9 Hz), 6.64 (d, 1H, J=7.2 Hz), 3.8-3.9 (m, 2H), 3.7-3.8 (m, 1H), 3.5-3.6 (m, 2H), 2.3-2.5 (m, 7H), 2.1-2.2 (m, 1H) | | |
| **87** | 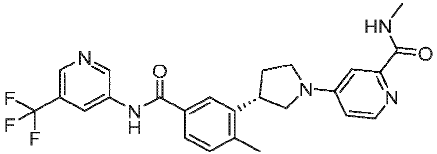 | 20 mg (14%) | RuPhos, Pd(dppf)Cl₂ Cs₂CO₃ DMA |
| | LC-MS (ESI, m/z): Method 10, t*_{R}* = 2.4 min, m/z (M+1) = 484.20 | | |
| | ¹H NMR (400 MHz, DMSO-d₆) δ 10.65 (s, 1H), 9.11 (d, J = 2.3 Hz, 1H), 8.71 - 8.50 (m, 3H), 8.15 (d, J = 5.7 Hz, 1H), 7.97 (d, J = 1.9 Hz, 1H), 7.82 (dd, J = 7.9, 1.8 Hz, 1H), 7.38 (d, J = 7.9 Hz, 1H), 7.18 (d, J = 2.5 Hz, 1H), 6.67 (dd, J = 5.8, 2.6 Hz, 1H), 3.89 - 3.75 (m, 2H), 3.62 (t, J = 8.9 Hz, 1H), 3.49 (q, J = 9.4, 8.9 Hz, 1H), 3.41 (d, J = 7.7 Hz, 1H), 2.79 (d, J = 4.9 Hz, 3H), 2.44 - 2.37 (m, 1H), 2.22 (dt, J = 12.4, 6.4 Hz, 1H). | | |
| **88** | 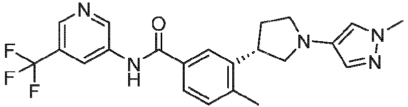 | 17 mg (28%) | tBuXPhos Pd G3 tBuOK Me-THF |
| | LC-MS (ESI, m/z): method 9, t*_{R}* = 2.72 min, m/z (M+1) = 430.19 | | |
| | ¹H NMR (400 MHz, DMSO-d₆) δ 10.65 (s, 1H), 9.18 (d, J = 2.3 Hz, 1H), 8.73 - 8.67 (m, 1H), 8.60 (t, J = 2.3 Hz, 1H), 7.93 (d, J = 1.9 Hz, 1H), 7.78 (dd, J = 7.9, 1.9 Hz, 1H), 7.37 (d, J = 7.9 Hz, 1H), 7.13 (d, J = 0.9 Hz, 1H), 7.02 (d, J = 0.9 Hz, 1H), 3.72 (s, 4H), 3.39 (t, J = 8.4 Hz, 1H), 3.27 (td, J = 8.6, 4.1 Hz, 1H), 3.15 (dd, J = 8.7, 7.3 Hz, 1H), 3.12 - 3.05 (m, 1H), 2.44 (s, 3H), 2.41 - 2.31 (m, 1H), 2.09 - 1.99 (m, 1H). | | |
| **89** | 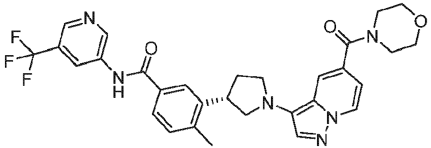 | 45 mg (28%) | tBuBrettPhos Pd G3 *t*BuONa Me-THF |
| | LC-MS (ESI, m/z): method 5, t*_{R}* = 4.51 min, m/z (M+1) = 579.2 | | |
| | ¹H NMR (400 MHz, DMSO-d₆) δ 10.69 (s, 1H), 9.22 (d, J=2.3 Hz, 1H), 8.73 (d, J=1.3 Hz, 1H), 8.64 (t, J=2.3 Hz, 1H), 8.47 (d, J=7.8 Hz, 1H), 8.05 (d, J=1.5 Hz, 1H), 7.86 - 7.80 (m, 2H), 7.74 (s, 1H), 7.43 (d, J=8.1 Hz, 1H), 6.70 (dd, J=1.6, 7.2 Hz, 1H), 3.88 - 3.75 (m, 2H), 3.67 - 3.62 (m, 5), 3.59 - 3.53 (m, 5), 3.49 - 3.45 (m, 1H), 2.51 (s, 3H), 2.50 - 2.45 (m, 1H), 2.23 - 2.13 (m, 1H). | | |
| **90** | 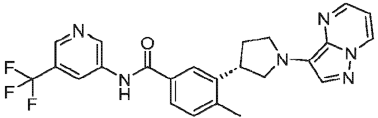 | 45 mg (28%) | tBuBrettPhos Pd G3 *t*BuONa Me-THF |
| | LC-MS (ESI, m/z): method 5, t*_{R}* = 4.77 min, m/z (M+1) = 467.1 | | |
| | ¹H NMR (400 MHz, DMSO-d₆) δ 10.66 (s, 1H), 9.18 (d, J=2.3 Hz, 1H), 8.80 (dd, J=1.6, 7.2 Hz, 1H), 8.68 (d, J=1.3 Hz, 1H), 8.59 (t, J=2.1 Hz, 1H), 8.19 (dd, J=1.5, 3.8 Hz, 1H), 7.99 (d, J=1.8 Hz, 1H), 7.82 (s, 1H), 7.80 (dd, J=1.9, 8.0 Hz, 1H), 7.39 (d, J=8.1 Hz, 1H), 6.78 (dd, J=3.9, 7.2 Hz, 1H), 3.87 - 3.67 (m, 3H), 3.57 - 3.38 (m, 2H), 2.47 (s, 3H), 2.45 - 2.40 (m, 1H), 2.14 - 2.07 (m, 1H). | | |
| **91** | 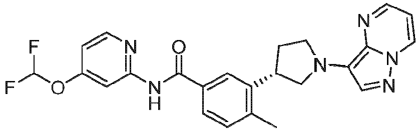 | 20 mg (10%) | tBuBrettPhos Pd G3 *t*BuONa Me-THF |
| | LC-MS (ESI, m/z): method 5, t*_{R}* = 4.79 min, m/z (M+1) = 465.2 | | |
| | ¹H NMR (400 MHz, DMSO-d₆) δ 11.08 (s, 1H), 8.84 (dd, J=1.6, 7.2 Hz, 1H), 8.40 (d, J=5.8 Hz, 1H), 8.23 (dd, J=1.6, 3.9 Hz, 1H), 8.12 - 8.06 (m, 2H), 7.86 (s, 1H), 7.83 (dd, J=1.8, 7.8 Hz, 1H), 7.51 (t, J=73.5 Hz, 1H), 7.36 (d, J=8.1 Hz, 1H), 7.01 (dd, J=2.3, 5.6 Hz, 1H), 6.81 (dd, J=3.9, 7.2 Hz, 1H), 3.90 - 3.70 (m, 3H), 3.62 - 3.48 (m, 2H), 2.49 (s, 3H), 2.45 - 2.36 (m, 1H), 2.24 - 2.11 (m, 1H). | | |
| **92** | 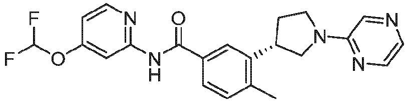 | 31 mg (17%) | RuPhos, Pd(dppf)Cl₂ Cs₂CO₃ DMA |
| | LC-MS (ESI, m/z): method 8, t*_{R}* = 4.66 min, m/z (M+1) = 426.5 | | |
| | ¹H NMR (400 MHz, DMSO-d₆) δ 11.06 (s, 1H), 8.37 (d, J=5.6 Hz, 1H), 8.07 - 8.03 (m, 4H), 7.83 - 7.77 (m, 2H), 7.48 (t, J=72.7 Hz, 1H), 7.35 (d, J=8.0 Hz, 1H), 6.97 (dd, J=0.7, 4.1 Hz, 1H), 3.97 - 3.91 (m, 1H), 3.82 - 3.72 (m, 2H), 3.59 - 3.48 (m, 2H), 2.46 (s, 3H), 2.39 - 2.19 (m, 2H) | | |
| **93** | 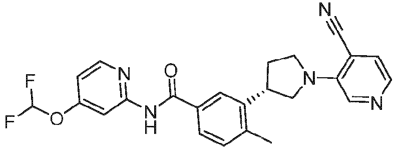 | 14 mg (7%) | RuPhos, Pd(dppf)Cl₂ Cs₂CO₃ DMA |
| | LC-MS (ESI, m/z): method 5, t*_{R}* = 4.95 min, m/z (M+1) = 450.5 | | |
| | ¹H NMR (400 MHz, DMSO-d₆) δ 11.03 (s, 1H), 8.38 (d, J=5.8 Hz, 1H), 8.32 (s, 1H), 8.10 (d, J=1.3 Hz, 1H), 8.05 (d, J=2.0 Hz, 1H), 7.90 (d, J=5.1 Hz, 1H), 7.83 (dd, J=1.6, 8.0 Hz, 1H), 7.48 (t, J=72.6 Hz, 1H) 7.47 - 7.45 (m, 1H), 7.36 (d, J=8.1 Hz, 1H), 6.99 (dd, J=2.3, 5.6 Hz, 1H), 4.07 - 4.01 (m, 1H), 3.88 - 3.65 (m, 4H), 2.45 (s, 3H), 2.37 - 2.28 (m, 2H). | | |
| **94** | 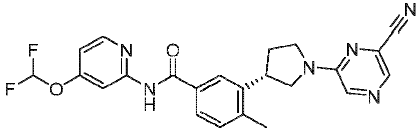 | 70 mg (54%) | tBuBrettPhos Pd G3 *t*BuONa Me-THF |
| | LC-MS (ESI, m/z): method 8, t*_{R}* = 5.05 min, m/z (M+1) = 451.4 | | |
| | ¹H NMR (400 MHz, DMSO-d₆) δ 11.07 (s, 1H), 8.38 (d, J=5.9 Hz, 2H), 8.28 (s, 1H), 8.05 (d, J=2.1 Hz, 2H), 7.81 (dd, J=1.6, 7.9 Hz, 1H), 7.48 (t, J=73.4 Hz, 1H) 7.36 (d, J=8.0 Hz, 1H), 7.00 (dd, J=2.3, 5.7 Hz, 1H), 4.02 - 3.96 (m, 1H), 3.87 - 3.76 (m, 2H), 3.62 - 3.54 (m, 2H), 2.46 (s, 3H), 2.42 - 2.30 (m, 1H), 2.30 - 2.19 (m, 1H). | | |
| **95** | 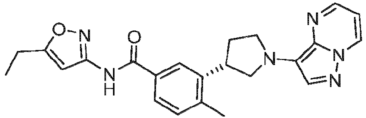 | 1.7 mg (1%) | tBuBrettPhos Pd G3 *t*BuONa Me-THF |
| | LC-MS (ESI, m/z): method 5, t*_{R}* = 4.53 min, m/z (M+1) = 417.4 | | |
| | ¹H NMR (400 MHz, DMSO-d₆) δ 11.33 (s, 1H), 8.85 (dd, J=1.6, 7.2 Hz, 1H), 8.24 (dd, J=1.8, 3.8 Hz, 1H), 8.08 (d, J=1.8 Hz, 1H), 7.86 (s, 1H), 7.82 (dd, J=1.9, 8.0 Hz, 1H), 7.37 (d, J=8.1 Hz, 1H), 6.84 - 6.78 (m, 2H), 3.91 - 3.70 (m, 3H), 3.63 - 3.55 (m, 1H), 3.52 - 3.46 (m, 1H), 2.80 (q, J=7.5 Hz, 2H), 2.49 (s, 3H), 2.46 - 2.36 (m, 1H), 2.22 - 2.12 (m, 1H), 1.27 (t, J=7.5, Hz, 3H). | | |
| **96** | 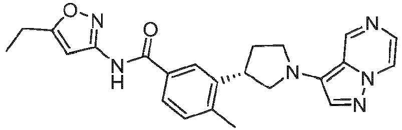 | 11 mg (6%) | tBuBrettPhos Pd G3 *t*BuONa Me-THF |
| | LC-MS (ESI, m/z): method 5, t*_{R}* = 3.72 min, m/z (M+1) = 417.3 | | |
| | ¹H NMR (400 MHz, DMSO-d₆) δ 11.33 (s, 1H), 9.20 (d, J=1.5 Hz, 1H), 8.44 (dd, J=1.4, 4.9 Hz, 1H), 8.11 (d, J=1.8 Hz, 1H), 7.84 (dd, J=1.8, 7.8 Hz, 1H), 7.71 (s, 1H), 7.58 (d, J=4.8 Hz, 1H), 7.39 (d, J=8.1 Hz, 1H), 6.80 (s, 1H), 3.90 - 3.83 (m, 2H), 3.76 - 3.62 (m, 2H), 3.55 (s, 1H), 2.81 (q, J=7.6, 2H), 2.52 (s, 3H), 2.50 - 2.38 (m, 1H), 2.33 - 2.22 (m, 1H), 1.28 (t, J=7.6, 3H). | | |
| **97** | 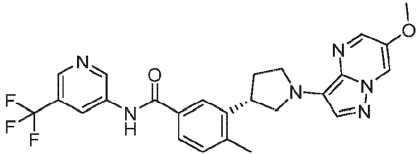 | 18 mg (16%) | BPC-305 Cs₂CO₃ DMA |
| | LC-MS (ESI, m/z): method 4, t*_{R}* = 6.6 min, m/z (M+1) = 497.2 | | |
| | ¹H NMR (400 MHz, acetone-d₆) δ ppm 10.01 (1 H, br s) 9.12 (1 H, s) 8.71 (1 H, s) 8.63 (1 H, s) 8.45 (1 H, d, J=7.67 Hz) 8.15 (1 H, s) 7.82 (1 H, dd, J=7.89, 1.75 Hz) 7.66 (1 H, s) 7.36 (1 H, d, J=7.89 Hz) 6.28 (1 H, d, J=7.45 Hz) 3.92 (3 H, s) 3.78 - 3.86 (2 H, m) 3.72 (1 H, td, J=8.66, 4.17 Hz) 3.47 - 3.56 (2 H, m) 2.51 (3 H, s) 2.43 - 2.50 (1 H, m) 2.08 - 2.18 (1 H, m) | | |
| **98** | 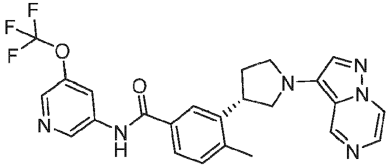 | 19 mg (15%) | tBuBrettPhos Pd G3 *t*BuONa Me-THF |
| | LC-MS (ESI, m/z): method 5, t*_{R}* = 4.01 min, m/z (M+1) = 483.2 | | |
| | ¹H NMR (400 MHz, DMSO-d₆) δ 10.63 (s, 1H), 9.16 (s, 1H), 8.94 (d, J=2.0 Hz, 1H), 8.41 - 8.36 (m, 3H), 7.98 (d, J=1.5 Hz, 1H), 7.80 (dd, J=1.8, 7.9 Hz, 1H), 7.67 (s, 1H), 7.53 (d, J=5.0 Hz, 1H), 7.40 (d, J=8.0 Hz, 1H), 3.87 - 3.81 (m, 2H), 3.70 - 3.48 (m, 3H), 2.48 (s, 3H), 2.46 - 2.43 (m, 1H), 2.23 - 2.18 (m, 1H) | | |

Analogously, the described procedure was followed to synthesize the following intermediates:

| | | | |
|---|---|---|---|
| **Interme diate 57** | 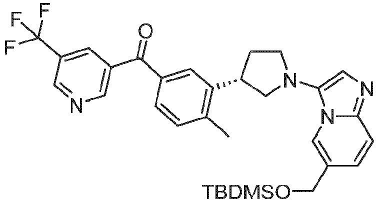 | 108 mg (41%) | tBuBrettPhos Pd G3 *t*BuONa Dioxane |
| | LC-MS (ESI, m/z): method 5, t*_{R}* = 2.07 min, m/z (M+1) = 610.3 | | |
| **Interme diate 58** | 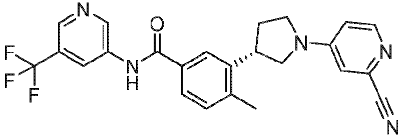 | 42 mg (22%) | RuPhos, Pd(dppf)Cl₂ Cs₂CO₃ DMA |
| | LC-MS (ESI, m/z): method 5, t*_{R}* = 4.6 min, m/z (M+1) = 452.4 | | |
| **Interme diate 59** | 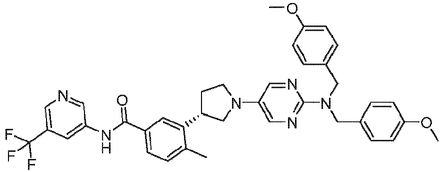 | 554 mg (28%) | BPC-305 Cs₂CO₃ Dioxane |
| | LC-MS (ESI, m/z): Method 13, t*_{R}* = 1.5 min, m/z (M+1) = 683.42 | | |
| **Interme diate 60** | 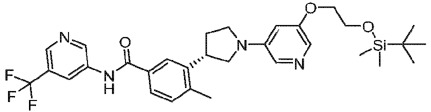 | 62 mg (48%) | RuPhos, Pd(dppf)Cl₂ Cs₂CO₃ DMA |
| | LC-MS (ESI, m/z): method 1, t*_{R}* = 1.2 min, m/z (M+1) = 601.3 | | |
| **Interme diate 61** | 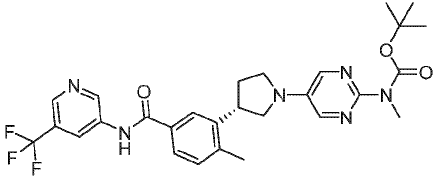 | 45 mg (28%) | RuPhos, Pd(dppf)Cl₂ Cs₂CO₃ DMA |
| | LC-MS (ESI, m/z): method 4 min, t*_{R}* = 1.16 min, m/z (M+1) = 557.24 | | |
| **Interme diate 62** | 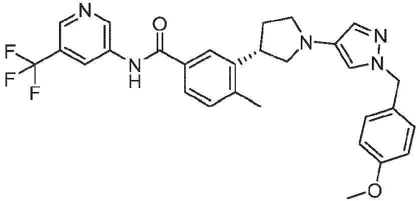 | 103 mg (21%) | tBuXPhos Pd G3 *t*BuOK Me-THF |
| | LC-MS (ESI, m/z): method 8, t*_{R}* = 1.8 min, m/z (M+1) = 536.3 | | |
| **Interme diate 63** | 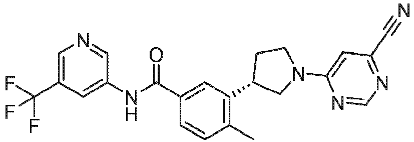 | 230 mg (89%) | RuPhos, Pd(dppf)Cl₂ Cs₂CO₃ DMA |
| | LC-MS (ESI, m/z): Method 16, t*_{R}* = 2.50 min, m/z (M+1) = 453.43 | | |
| **Interme diate 64** | 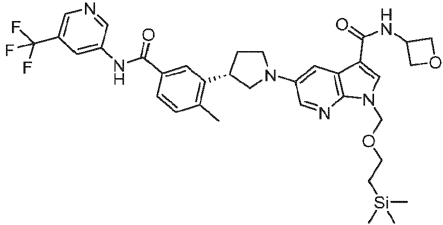 | 45 mg (28%) | tBuBrettPhos Pd G3 *t*BuONa Dioxane |
| | LC-MS (ESI, m/z): Method 16, t*_{R}* = 2.69 min, m/z (M+1) = 695.10 | | |
| **Interme diate 65** | 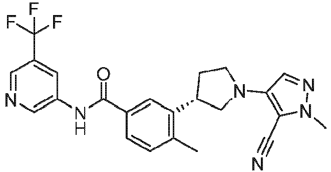 | | Pd-175 *t*BuONa Dioxane |
| | LC-MS (ESI, m/z): Method 16, t*_{R}* = 2.44 min, m/z (M+1) = 455.20 | | |
| **Interme diate 66** | 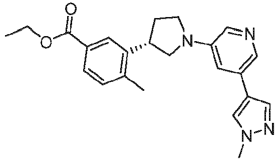 | (100%) | RuPhos, Pd(dppf)Cl₂ Cs₂CO₃ DMA |
| | LC-MS (ESI, m/z): method 2, t*_{R}* = 1.7 min, m/z (M+1) = 391.3 | | |
| **Interme diate 67** | 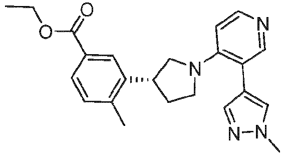 | 90 (56%) | RuPhos, Pd(dppf)Cl₂ Cs₂CO₃ DMA |
| | LC-MS (ESI, m/z): Method 15, t*_{R}* = 1.7 min, m/z (M+1) = 391.3 | | |
| **Interme diate 68** | 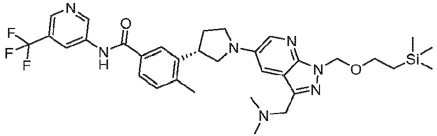 | 192 mg (75%) | tBuBrettPhos Pd G3 tBuONa Dioxane |
| | LC-MS (ESI, m/z): Method 16, t*_{R}* = 2.25 min, m/z (M+1) = 654.13 | | |
| **Interme diate 69** | 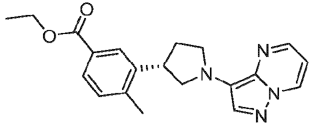 | 209 mg (35%) | tBuBrettPhos Pd G3 *t*BuONa Me-THF |
| | LC-MS (ESI, m/z): method 2, t*_{R}* = 1.8 min, m/z (M+1) = 351.1 | | |
| **Interme diate 70** | 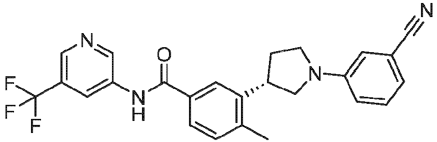 | 190 mg (98%) | RuPhos, Pd(dppf)Cl₂ Cs₂CO₃ DMA |
| | LC-MS (ESI, m/z): Method 16 min., t*_{R}* = 2.7 min, m/z (M+1) = 451.0 | | |

### Example 38 - (S)-5-(3-(2-methyl-5-((5-(trifluoromethyl)pyridin-3-yl)carbamoyl) phenyl)pyrrolidin-1-yl)-N-(oxetan-3-yl)nicotinamide

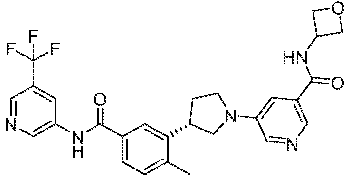

### Step 1 - Methyl (S)-5-(3-(2-methyl-5-((5-(trifluoromethyl)pyridin-3-yl)carbamoyl) phenyl)pyrrolidin-1-yl)nicotinate (Intermediate 23)

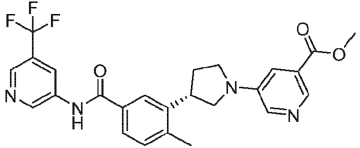

Intermediate 23 was prepared by following the same procedure described for the synthesis of Intermediate 17.
SM: Intermediate 22: 746 mg (1 eq.)
Methyl 5-bromonicotinate: 692 mg (1.5 eq.)
Amount/yield: 389 mg/ 38%
LC-MS (ESI, m/z): method 6, t*_{R}* = 1.74 min, m/z (M+1) = 485.5
¹H NMR(400 MHz, DMSO-d₆) δ 10.66 (s, 1H), 9.20 (d, J=2.1 Hz, 1H), 8.71 (s, 1H), 8.60 (t, J=2.3 Hz, 1H), 8.39 (d, J=1.8 Hz, 1H), 8.25 (d, J=2.9 Hz, 1H), 7.96 (d, J=1.6 Hz, 1H), 7.83 (dd, J=1.8, 7.9 Hz, 1H), 7.43 (d, J=8.0 Hz, 1H), 7.38 - 7.36 (m, 1H), 3.88 (s, 3H), 3.86 - 3.80 (m, 2H), 3.66 - 3.60 (m, 1H), 3.57 - 3.48 (m, 1H), 3.47 - 3.41 (m, 1H), 2.50 (s, 3H),2.42 - 2.41 (m, 1H), 2.31 - 2.21 (m, 1H).

### Step 2 - lithium (S)-5-(3-(2-methyl-5-((5-(trifluoromethyl)pyridin-3-yl) carbamoyl) phenyl)pyrrolidin-1-yl)nicotinate (Intermediate 24)

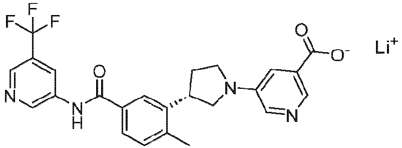

To a solution of Intermediate 23 (273 mg, 0.564 mmol, 1.00 eq) in THF (5.00 mL) a solution of LiOH•H₂O (24 mg, 0.564 mmol, 1.00 eq) in H₂O (2.00 mL) was added and the reaction mixture was stirred 65 °C for 4 h. The reaction mixture was concentrated *in vacuo* to give the title compound (228 mg, 0.479 mmol, 85%).
LC-MS (ESI, m/z): method 6, t*_{R}* = 1.45 min, m/z (M+1) = 471.5
¹H NMR (400 MHz, DMSO-d₆) δ 12.4 (s, 1H). 9.36 (d, J=1.4 Hz, 1H), 8.79 (s, 1H), 8.64 (s, 1H), 8.41 (s, 1H), 8.29 (s, 1H), 7.80 (dd, J=1.4, 7.9 Hz, 1H), 7.54 (d, J=2.1 Hz, 1H), 7.35 (d, J=8.0 Hz, 1H), 7.20 (s, 1H), 3.67 - 3.53 (m, 2H), 3.09 (t, J=9.1 Hz, 1H), 2.43 (s, 3H), 2.41 - 2.34 (m, 1H), 2.24 - 2.22 (m, 1H).

### Step 3 - (S)-5-(3-(2-methyl-5-((5-(trifluoromethyl)pyridin-3-yl)carbamoyl) phenyl)pyrrolidin-1-yl)-N-(oxetan-3-yl)nicotinamide (Example 38)

To a solution of Intermediate 24 (lithium salt, 88 mg, 0.185 mmol, 1.00 eq), HATU (105 mg, 0.277 mmol, 1.50 eq) and DIPEA (80 µL, 0.462 mmol, 2.50 eq) in DMF (2.50 mL) 3-aminooxetane (13 µL, 0.185 mmol, 1.00 eq) was added and the mixture stirred at rt overnight. The reaction mixture was diluted in EtOAc (25 mL) and washed in sequence with water (25 mL), H₂O:brine (1:1) (25 mL) and brine (25 mL), dried over MgSO₄, filtered and concentrated *in vacuo.* The material was submitted to purification by preparative HPLC to give the title compound (45 mg, 0.0844 mmol, 46%).
LC-MS (ESI, m/z): method 5, t*_{R}* = 3.54 min, m/z (M+1) = 526.2
¹H NMR (400 MHz, DMSO-d₆) δ 10.64 (s, 1H), 9.19 - 9.12 (m, 2H), 8.69 - 8.68 (m, 1H), 8.59 (t, J=2.1 Hz, 1H), 8.32 (d, J=1.8 Hz, 1H), 8.13 (d, J=3.0 Hz, 1H), 7.96 (d, J=1.5 Hz, 1H), 7.82 (dd, J=1.8, 7.8 Hz, 1H), 7.41 (d, J=8.1 Hz, 1H), 7.34 (t, J=2.3 Hz, 1H), 5.05 - 4.98 (m, 1H), 4.77 (t, J=6.9 Hz, 2H), 4.59 (t, J=6.4 Hz, 2H), 3.85 - 3.79 (m, 2H), 3.64 - 3.59 (m, 1H), 3.53 - 3.36 (m, 2H), 2.45 - 2.41 (m, 1H), 2.28 - 2.21 (m, 1H).

The following examples were prepared by using the procedure described for Example 38, using the suitable amine in step 3.

| **Example N°** | **Structure** | **Product Amount (Yield)** |
|---|---|---|
| | **Analytical Data** | |
| **39** | 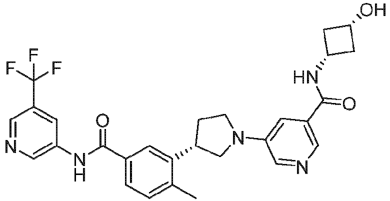 | 10 mg (12%) |
| | LC-MS (ESI, m/z): method 5, t*_{R}* = 3.43 min, m/z (M+1) = 540.2 ¹H NMR (400 MHz, DMSO-d₆) δ 10.64 (s, 1H), 9.16 (d, J=1.5 Hz, 1H), 8.68 - 8.65 (m, 1H), 8.59 (s, 1H), 8.58 (s, 1H), 8.28 (d, J=1.8 Hz, 1H), 8.10 (d, J=2.8 Hz, 1H), 7.97 (d, J=1.8 Hz, 1H), 7.82 (dd, J=1.6, 8.0 Hz, 1H), 7.40 (d, J=7.8 Hz, 1H), 7.31 (t, J=2.1, Hz, 1H), 5.09 (d, J=3.8 Hz, 1H), 3.94 - 3.78 (m, 4H), 3.64 - 3.58 (m, 1H), 3.52 - 3.36 (m, 2H), 2.58 - 2.52 (m, 2H), 2.49 (s, 3H), 2.44 - 2.39 (m, 1H), 2.28 - 2.20 (m, 1H), 1.95 - 1.86 (m, 2H). | |
| **40** | 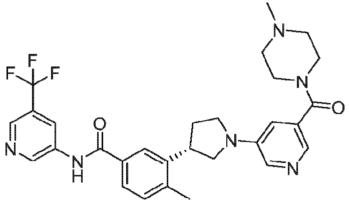 | 29 mg (49%) |
| | LC-MS (ESI, m/z): method 5, t*_{R}* = 3.03 min, m/z (M+1) = 553.2 ¹H NMR (400 MHz, DMSO-d₆) δ 10.63 (s, 1H), 9.18 (d, J=2.0 Hz, 1H), 8.69 (s, 1H), 8.59 (t, J=2.4 Hz, 1H), 8.05 (d, J=2.8 Hz, 1H), 7.93 (d, J=1.5 Hz, 1H), 7.83 - 7.79 (m, 2H), 7.41 (d, J=8.1 Hz, 1H), 6.90 (t, J=2.1 Hz, 1H), 3.83 - 3.76 (m, 2H), 3.62 - 3.42 (m, 5H), 2.48 (s, 3H), 2.43 - 2.21 (m, 6H), 2.18 (s, 3H). | |

### Example 41 - (S)-3-(3-(2-methyl-5-((5-(trifluoromethyl)pyridin-3-yl)carbamoyl) phenyl)pyrrolidin-1-yl)-N-(oxetan-3-yl)isonicotinamide

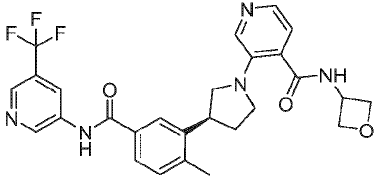

### Step 1 - methyl (S)-3-(3-(2-methyl-5-((5-(trifluoromethyl)pyridin-3-yl)carbamoyl) phenyl) pyrrolidin-1-yl)isonicotinate (Intermediate 25)

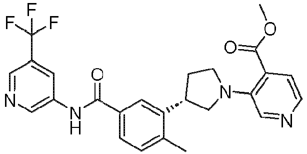

A suspension of Intermediate 22 (190 mg, 0.544 mmol, 1.00 eq), methyl 3-fluoroisonicotinate (253 mg, 1.63 mmol, 3.00 eq) and DIPEA (0.28 mL, 1.63 mmol, 3.00 eq) in DMSO (10.00 mL) was stirred at rt for 1 day. The reaction mixture was heated to 50 °C and stirred for 3 days. The reaction mixture was cooled to rt, diluted with EtOAc (50 mL) and washed with water (2×30 mL) and brine (sat. aq., 50 mL). The organic phase was dried over MgSO₄ and concentrated in vacuo. The residue was purified by FCC on silica gel, eluting with 0-100% 10% 7 NNH₃ in MeOH in EtOAc in cyclohexane to give the title compound (150 mg, 0.310 mmol, 57%).
LC-MS (ESI, m/z): method 6, t*_{R}* =1.68 min, m/z (M+1) = 485.5
¹H NMR (400 MHz, CDCl₃) δ 9.12 (s, 1H), 8.91 (d, J=2.3 Hz, 1H), 8.62 - 8.51 (m, 2H), 8.37 (s, 1H), 8.08 (d, J=5.0 Hz, 1H), 7.88 - 7.80 (m, 2H), 7.42 (d, J=4.9 Hz, 1H), 7.33 (d, J=8.2 Hz, 1H), 3.94 (s, 3H), 3.87 - 3.80 (m, 2H), 3.63 - 3.47 (m, 2H), 3.03 - 2.96 (m, 1H), 2.47 (s, 3H), 2.43 - 2.31 (m, 1H), 1.97 - 1.88 (m, 1H).

The following Examples and Intermediates were prepared by using the procedure described for Intermediate 25, starting from the suitable fluoro or chloro derivative and ACN as a solvent at a temperature varying from rt to 80 C°.

| **Example N°** | **Structure** | **Product Amount (Yield)** |
|---|---|---|
| | **Analytical Data** | |
| **61** | 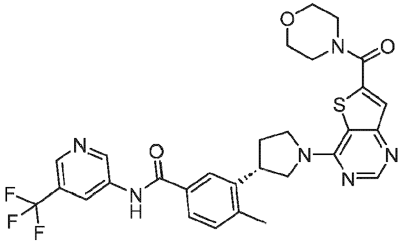 | 53 mg (19%) |
| | LC-MS (ESI): Method 4, t*_{R}* = 5.92 min, m/z (M+1) = 597.22 ¹H NMR ( ACN-d₃, 400 MHz) δ 9.22 (br s, 1H), 9.04 (d, 1H, J=2.2 Hz), 8.63 (s, 1H), 8.58 (s, 1H), 8.45 (s, 1H), 7.9-8.0 (m, 1H), 7.77 (dd, 1H, J=1.8, 7.9 Hz), 7.46 (s, 1H), 7.39 (d, 1H, J=8.1 Hz), 4.38 (br s, 1H), 4.18 (br s, 1H), 3.8-4.0 (m, 3H), 3.67 (s, 8H), 2.50 (s, 3H), 2.4-2.5 (m, 1H), 2.2-2.3 (m, 1H) | |
| **Interme diate 71** | 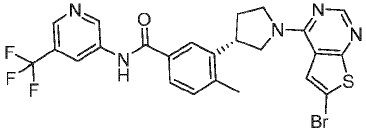 | 200 mg (62%) |
| | LC-MS (ESI, m/z): Method 14, t*_{R}* = 1.32 min, m/z (M+1) = 564.0 | |
| **125** | 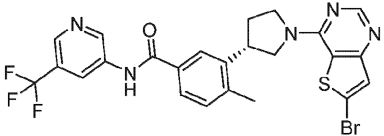 | 17 mg (14%) |
| | LC-MS (ESI, m/z): method , t*_{R}* = 7.43 min, m/z (M+1) = 563.2 ¹H NMR (400 MHz, acetone-d₆) δ ppm 10.01 (1 H, br s) 9.11 (1 H, s) 8.69 (1 H, s) 8.64 (1 H, s) 8.38 (1 H, s) 8.12 (1 H, s) 7.87 (1 H, dd, J=7.89, 1.75 Hz) 7.46 (1 H, s) 7.42 (1 H, d, J=7.89 Hz) 4.38 (1 H, br t, J=8.66 Hz) 4.20 (1 H, br t, J=8.33 Hz) 3.88 - 4.04 (2 H, m) 3.69 - 3.86 (1 H, m) 2.55 (3 H, s) 2.50 (1 H, br dd, J=7.34, 5.37 Hz) 2.29 - 2.40 (1 H, m). | |
| **Interme diate 72** | 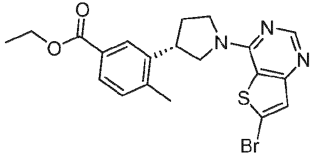 | 870 mg (quantitative yield) |
| | LC-MS (ESI, m/z): Method 13, t*_{R}* = 1.14 min, m/z (M+1) = 446.05/447.94 | |
| **129** | 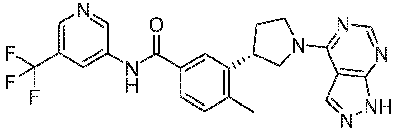 | 72 mg (54%) |
| | LC-MS (ESI, m/z): method 4, t*_{R}* = 5.10 min, m/z (M+1) = 468.24 ¹H NMR ( ACN-d₃, 400 MHz) δ 11.34 (br dd, 1H, J=4.2, 5.5 Hz), 9.09 (br s, 1H), 9.03 (br s, 1H), 8.63 (s, 1H), 8.58 (s, 1H), 8.26 (br d, 1H, J=10.3 Hz), 8.0-8.1 (m, 1H), 7.9-8.0 (m, 1H), 7.77 (br t, 1H, J=6.5 Hz), 7.41 (br d, 1H, J=7.7 Hz), 4.3-4.3 (m, 1H), 4.0-4.1 (m, 1H), 3.9-4.0 (m, 1H), 3.7-3.9 (m, 2H), 2.51 (br s, 3H), 2.2-2.4 (m, 2H) | |
| **126** | 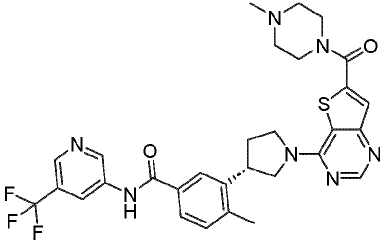 | 51 mg (18%) |
| | LC-MS (ESI, m/z): method 4, t*_{R}* = 4.63 min, m/z (M+1) = 610.23 ¹H NMR ( ACN-d₃, 400 MHz) δ 9.14 (br s, 1H), 9.03 (d, 1H, J=2.2 Hz), 8.6-8.7 (m, 1H), 8.6-8.6 (m, 1H), 8.45 (s, 1H), 7.92 (d, 1H, J=1.8 Hz), 7.77 (dd, 1H, J=1.8, 7.9 Hz), 7.43 (s, 1H), 7.40 (d, 1H, J=7.9 Hz), 4.39 (br s, 1H), 4.19 (br s, 1H), 3.8-4.1 (m, 3H), 3.6-3.7 (m, 4H), 2.50 (s, 3H), 2.4-2.5 (m, 1H), 2.38 (br s, 4H), 2.2-2.3 (m, 4H) | |
| **127** | 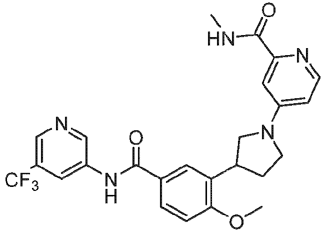 | 51 mg (18%) |
| | LC-MS (ESI, m/z): method 4, t*_{R}* = 4.24 min, m/z (M+1) = 500.19 ¹H NMR ( ACN-d₃, 400 MHz) δ 9.0-9.1 (m, 2H), 8.63 (s, 1H), 8.58 (s, 1H), 8.1-8.2 (m, 2H), 7.92 (dd, 1H, J=2.2, 8.6 Hz), 7.85 (d, 1H, J=2.2 Hz), 7.26 (d, 1H, J=2.4 Hz), 7.13 (d, 1H, J=8.6 Hz), 6.58 (dd, 1H, J=2.5, 5.8 Hz), 3.94 (s, 3H), 3.8-3.9 (m, 2H), 3.6-3.6 (m, 1H), 3.4-3.6 (m, 2H), 2.89 (d, 3H, J=5.1 Hz), 2.2-2.4 (m, 2H) | |

### Step 2 - lithium (S)-3-(3-(2-methyl-5-((5-(trifluoromethyl)pyridin-3-yl)carbamoyl) phenyl) pyrrolidin-1-yl)isonicotinate (Intermediate 26)

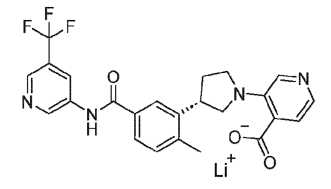

Intermediate 26 was prepared by following the same procedure described for Intermediate 24.
SM: Intermediate 25: 150 mg, 1.00 eq.
Amount/Yield: 148 mg/ 73.3%
LC-MS (ESI, m/z): method 6, t*_{R}* =1.33 min, m/z (M+1) = 471.5

### Step 3 - S)-3-(3-(2-methyl-5-((5-(trifluoromethyl)pyridin-3-yl)carbamoyl)phenyl) pyrrolidin-1-yl)-N-(oxetan-3-yl)isonicotinamide (Example 41)

Example 41 was prepared by following the same procedure described in step 3 for Example 38, varying the corresponding amine.
SM: Intermediate 26: 74 mg, 1.00 eq
oxetan-3-amine: 16.4 µL, 2 eq.
Amount/Yield: 5.1 mg/ 8.2%
LC-MS (ESI, m/z): method 6, t*_{R}* = 3.39 min, m/z (M+1) = 526.2
¹H NMR (400 MHz, DMSO-d₆) δ 10.63 (s, 1H), 9.22 (d, J=6.0 Hz) 9.17 (d, J=2.0), 8.69 (s, 1H), 8.59 (s, 1H), 8.17 (s, 1H), 7.93 - 7.89 (m, 2H), 7.81 (dd, J=1.8, 7.8 Hz, 1H), 7.40 (d, J=7.8 Hz, 1H), 7.14 (d, J=4.8 Hz, 1H), 4.92 - 4.86 (m, 1H), 4.74 - 4.68 (m, 2H), 4.54 - 4.44 (m, 2H), 3.74 - 3.66 (m, 2H) 3.51 - 3.54 (m, 2H), 2.44 (s, 3H), 2.36 - 2.29 (m, 1H), 2.18 - 2.12 (m, 1H).

### Example 128 - (S)-3-(1-(imidazo[1,5-a]pyrazin-8-yl)pyrrolidin-3-yl)-4-methyl-N-(5-(trifluoromethyl)pyridin-3-yl)benzamide

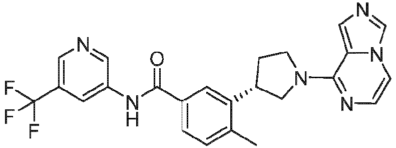

To a solution of Intermediate 22 (50 mg, 0.143 mmol) in DMF (1 mL) sodium hydride (17.17 mg, 0.429 mmol) was added at 0 °C. The mixture was stirred at 0 °C for 0.5 h, then 8-chloroimidazo[1,5-a]pyrazine (26.4 mg, 0.172 mmol) was added at 0 °C and the mixture was stirred overnight at rt. The mixture was quenched by water and extracted with DCM. Organic layers were combined and concentrated in vacuum. After purification by FCC, (NH silica, eluting with 50% of acetone in n-heptane) appropriate fractions were combined and evaporated under vacuum to give title compound (18 mg, 0.04 mmol, 27 % yield).
LC-MS (ESI, m/z): method 4, t*_{R}* = 4.43 min, m/z (M+1) = 467.3
¹H NMR (400 MHz, acetone-d₆) δ ppm 10.01 (1 H, br s) 9.11 (1 H, s) 8.70 (1 H, s) 8.63 (1 H, s) 8.22 (1 H, s) 8.11 (1 H, s) 7.86 (1 H, dd, J=7.89, 1.75 Hz) 7.77 (1 H, s) 7.55 (1 H, d, J=4.82 Hz) 7.42 (1 H, d, J=7.89 Hz) 7.04 (1 H, d, J=4.82 Hz) 4.33 (1 H, br dd, J=10.19, 7.78 Hz) 4.17 (1 H, br t, J=8.33 Hz) 3.88 - 4.02 (2 H, m) 3.75 - 3.86 (1 H, m) 2.55 (3 H, s) 2.48 (1 H, dtd, J=12.25, 6.43, 6.43, 2.52 Hz) 2.24 - 2.35 (1 H, m)

### Example 42 - (S)-4-methyl-3-(1-(2-nitropyridin-3-yl)pyrrolidin-3-yl)-N-(5-(trifluoromethyl)pyridin-3-yl)benzamide

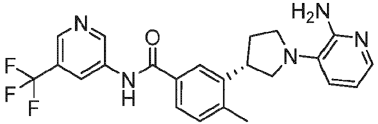

### Step 1 - (S)-4-methyl-3-(1-(2-nitropyridin-3-yl)pyrrolidin-3-yl)-N-(5-(trifluoromethyl) pyridin-3-yl)benzamide (Intermediate 27)

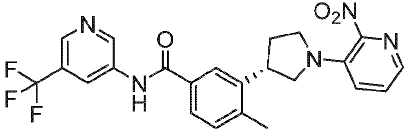

To a solution of 3-fluoro-2-nitropyridine (50.0 mg, 0.352 mmol, 1.00 eq) and Intermediate 22 (129 mg, 0.369 mmol, 1.05 eq) in ACN (3.50 mL) K₂CO₃ (anhydrous, 146 mg, 1.06 mmol, 3.00 eq) was added in one portion and the resulting mixture was stirred at 50 °C overnight. The cooled reaction mixture was diluted with H₂O (50 mL) and extracted with EtOAc (2×50 mL). The combined organic phase was washed with brine (100 mL), dried over MgSO₄, filtered and concentrated *in vacuo* to give the title compound (157 mg, 0.333 mmol, 95%).
LC-MS (ESI, m/z): method 6, t*_{R}* =1.55 min, m/z (M+1) = 472.3
¹H NMR (400 MHz, DMSO-d₆) δ 10.71 (s, 1H), 9.24 (d, J=1.8 Hz, 1H), 8.75 (s, 1H), 8.65 (s, 1H), 7.99 (d, J=1.0 Hz, 1H), 7.88 (d, J=5.6 Hz, 2H), 7.71 - 7.68 (m, 1H), 7.62 (dd, J=4.0, 8.6 Hz, 1H), 7.46 (d, J=7.8 Hz, 1H), 3.86 - 3.78 (m, 1H), 3.61 - 3.48 (m, 2H), 2.50 (s, 3H), 2.39 - 2.26 (m, 3H).

### Step 2 - (S)-3-(1-(2-aminopyridin-3-yl)pyrrolidin-3-yl)-4-methyl-N-(5-(trifluoromethyl)pyridin-3-yl)benzamide (Example 42)

A suspension of Intermediate 27 (150 mg, 0.318 mmol, 1.00 eq) and Pd/C (10% dry, 5.1 mg, 4.77 µmol, 0.0150 eq) in EtOH (3.0 mL) and EtOAc (0.5 mL) was stirred under an atmosphere of hydrogen at rt for 5 h. A further portion of Pd/C (10% dry, 29 mg, 0.0270 mmol, 0.0850 eq) was added and the solution stirred under an atmosphere of hydrogen at rt overnight. The reaction mixture was filtered through Celite^{®} and washed with EtOAc (50 mL), then concentrated *in vacuo.* The material was purified by preparative HPLC twice to give the title compound (16 mg, 0.0363 mmol, 11%).
LC-MS (ESI, m/z): method 5, t*_{R}* = 3.61 min, m/z (M+1) = 442.3
¹H NMR (400 MHz, DMSO-d₆) δ 10.68 (s, 1H), 9.21 (d, J=2.3 Hz, 1H), 8.71 (s, 1H), 8.64 (t, J=2.0 Hz, 1H), 8.04 (d, J=1.8 Hz, 1H), 7.80 (dd, J=1.9, 8.0 Hz, 1H), 7.62 (dd, J=1.4, 4.9 Hz, 1H), 7.39 (d, J=8.1 Hz, 1H), 7.17 (dd, J=1.5, 7.6 Hz, 1H), 6.55 (dd, J=4.8, 7.6 Hz, 1H), 5.48 (s, 2H), 3.79 - 3.72 (m, 1H), 3.50 - 3.44 (m, 1H), 3.28 - 3.22 (m, 1H), 3.15 (dd, J=7.8, 8.8 Hz, 1H), 2.46 (s, 3H), 2.44 - 2.41 (m, 1H), 2.10 - 2.09 (m, 1H).

### Example 43 - 4-methyl-3-[(3S)-1-(1H-pyrazolo[3,4-b]pyridin-5-yl)pyrrolidin-3-yl]-N-[5-(trifluoromethyl)-3-pyridyl]benzamide

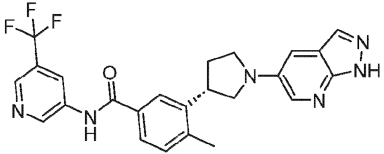

### Step 1 - 4-methyl-N-[5-(trifluoromethyl)-3-pyridyl]-3-[(3S)-1-[1-(2-trimethylsilylethoxymethyl) pyrazolo [3,4-b]pyridin-5-yl]pyrrolidin-3-yl]benzamide (Intermediate 28)

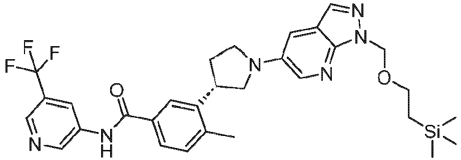

To a degassed solution of Intermediate 5 (94 mg, 0.286 mmol, 1.00 eq), Intermediate 22 (100 mg, 0.286 mmol, 1.00 eq) and NaOtBu (96 mg, 0.858 mmol, 3.00 eq) in dioxane (3 mL) tBuBrettPhos Pd G3 (49 mg, 0.057 mol, 0.20 eq) was added and the mixture was degassed for 5 min, heated to 80°C and stirred overnight. The solution was allowed to cool to rt, diluted in EtOAc and washed with water, brine, dried over MgSO₄, filtered and concentrated *in vacuo.*
LC-MS (ESI, m/z): method 6, t*_{R}* = 1.99 min, m/z (M+1) = 597.7

### Step 3 - 4-methyl-3-[(3S)-1-(1H-pyrazolo[3,4-b]pyridin-5-yl)pyrrolidin-3-yl]-N-[5-(trifluoromethyl)-3-pyridyl]benzamide (Example 43)

To a solution of Intermediate 28 in 1,4-dioxane (1 mL) was added HCl (4 M in 1,4-dioxane, 1.0 mL, 4.00 mmol, 62.8 eq) and the mixture was stirred at rt for 5 h, then concentrated *in vacuo.* The residue was purified by preparative HPLC to give the title compound (4.0 mg, 8.60 µmol, 13%). LC-MS (ESI, m/z): method 5, t*_{R}* = 4.67 min, m/z (M+1) = 467.3
¹H NMR (400 MHz, DMSO-d₆) δ 13.20 (s, 1H), 10.64 (s, 1H), 9.18 (d, J=2.3 Hz, 1H), 8.68 - 8.67 (m, 1H), 8.58 (t, J=2.1 Hz, 1H), 8.19 (d, J=2.5 Hz, 1H), 7.98 (d, J=1.5 Hz, 1H), 7.90 (s, 1H), 7.81 (dd, J=1.9, 8.0 Hz, 1H), 7.41 (d, J=8.1 Hz, 1H), 7.22 (d, J=2.3 Hz, 1H), 3.86 - 3.75 (m, 2H), 3.63 - 3.36 (m, 3H), 2.46 - 2.41 (m, 1H), 2.27 - 2.18 (m, 1H).

The following Example was prepared by using the procedure described for Example 43, using the suitable intermediate.

| **Example N°** | **Structure** | **Product Amount (Yield)** |
|---|---|---|
| | **Analytical Data** | |
| **99** | 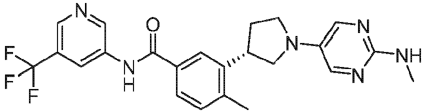 | 7 mg (18%) |
| | LC-MS (ESI, m/z): method 4, t*_{R}* = 6.16 min, m/z (M+1) = 457.31 ¹H NMR (400 MHz, DMSO-d₆) δ ppm 10.65 (1 H, s) 9.19 (1 H, d, J=2.19 Hz) 8.69 (1 H, s) 8.60 (1 H, t, J=1.86 Hz) 7.94 (1 H, s) 7.90 (2 H, s) 7.80 (1 H, dd, J=7.89, 1.75 Hz) 7.39 (1 H, d, J=8.11 Hz) 6.23 (1 H, q, J=4.82 Hz) 3.70 - 3.82 (1 H, m) 3.61 (1 H, t, J=8.33 Hz) 3.45 (1 H, td, J=8.66, 3.29 Hz) 3.33 - 3.36 (1 H, m) 3.24 (1 H, t, J=8.33 Hz) 2.74 (3 H, d, J=4.82 Hz) 2.45 - 2.47 (3 H, m) 2.34 - 2.41 (1 H, m) 2.11 - 2.17 (1 H, m) | |

### Example 130 - (S)-3-(1-(2-acetamidopyrimidin-5-yl)pyrrolidin-3-yl)-4-methyl-N-(5-(trifluoromethyl)pyridin-3-yl)benzamide

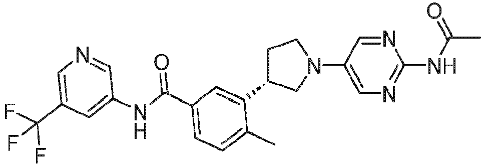

Intermediate 73 (100 mg, 0.226 mmol) was dissolved in DCM (3 ml) and DIPEA (0.118 ml, 0.68 mmol) was added then acetyl chloride (0.016 ml, 0.23 mmol) was added in one portion. Reaction mixture was stirred at rt for 1 hr. The solution was quenched with NaHCO3 sat solution and extracted with DCM. Purification was performed by FCC (silica NH eluting with 10% of MeOH in DCM). Appropriate fractions were combined and evaporated under vacuum to give title compound (23 mg, 0.047 mmol, 21.00 % yield).
LC-MS (ESI, m/z): method 5, t*_{R}* = 5.7 min, m/z (M+1) = 485.31
¹H NMR (400 MHz, DMSO-d₆) δ ppm 10.64 (1 H, s) 10.10 (1 H, s) 9.19 (1 H, d, J=2.41 Hz) 8.70 (1 H, s) 8.60 (1 H, s) 8.09 (2 H, s) 7.95 (1 H, d, J=1.75 Hz) 7.79 - 7.84 (1 H, m) 7.41 (1 H, d, J=8.11 Hz) 3.72 - 3.84 (2 H, m) 3.58 (1 H, td, J=8.71, 2.96 Hz) 3.40 - 3.48 (1 H, m) 3.35 (1 H, br s) 2.47 - 2.48 (3 H, m) 2.40 (1 H, br dd, J=6.14, 2.85 Hz) 2.15 - 2.27 (1 H, m) 2.06 (3 H, s)

### Example 100 - (S)-3-(1-(1H-pyrazol-4-yl)pyrrolidin-3-yl)-4-methyl-N-(5-(trifluoromethyl)pyridin-3-yl)benzamide

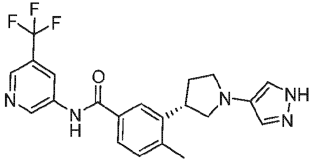

To a solution of Intermediate 62 ( (100 mg, 0.187 mmol, 1.00 eq) in DCM (1 mL) was added TFA (3.0 mL, 39.2 mmol, 210 eq) and the mixture was stirred at 80 °C for 3 h. The reaction mixture was concentrated in vacuo and the residue was purified by preparative HPLC (Xbridge Phenyl 19x150 mm, 10 µm 40-100% MeOH/H₂O (10 mM NH₄CO₃), 20 mL/min, RT) to give the title compound (34 mg, 0.0817 mmol, 44%).
LC-MS (ESI, m/z): method 5, t*_{R}* = 4.0 min, m/z (M+1) = 416.5
¹H NMR (400 MHz, DMSO-d₆) δ 12.22 (s, 1H), 10.64 (s, 1H), 9.18 (d, J=2.3 Hz, 1H), 8.69 - 8.67 (m, 1H), 8.60 (t, J=2.1 Hz, 1H), 7.95 (d, J=1.5 Hz, 1H), 7.78 (dd, J=1.8, 7.8 Hz, 1H), 7.37 (d, J=7.8 Hz, 1H), 7.14 (s, 2H), 3.76 - 3.70 (m, 1H), 3.41 (t, J=8.3 Hz, 1H), 3.20 - 3.07 (m, 2H), 2.44 (s, 3H), 2.41 - 2.31 (m, 1H), 2.10 - 2.00 (m, 1H).

The following Example was prepared by using the procedure described for Example 100, at a temperature varying from rt to 80 °C.

| **Example N°** | **Structure** | **Product Amount (Yield)** |
|---|---|---|
| | **Analytical Data** | |
| **101** | 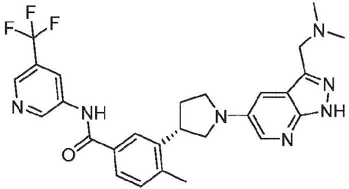 | 45 mg (30%) |
| | LC-MS (ESI, m/z): Method 10, t*_{R}* = 2.5 min, m/z (M+1) = 524.28 ¹H NMR (400 MHz, DMSO-d₆) δ 12.90 (s, 1H), 10.69 (s, 1H), 9.11 (d, J = 2.2 Hz, 1H), 8.60 (dd, J = 4.7, 2.4 Hz, 2H), 8.17 (d, J = 2.7 Hz, 1H), 8.01 (d, J = 1.9 Hz, 1H), 7.83 (dd, J = 7.9, 1.8 Hz, 1H), 7.37 (d, J = 8.0 Hz, 1H), 7.20 (d, J = 2.7 Hz, 1H), 3.83 (dd, J = 8.9, 7.0 Hz, 1H), 3.80 - 3.74 (m, 1H), 3.66 (s, 2H), 3.59 (td, J = 8.5, 3.1 Hz, 1H), 3.49 (q, J = 8.1 Hz, 1H), 3.38 (t, J = 8.1 Hz, 1H), 2.48 (s, 3H), 2.42 (p, J = 6.9, 6.0 Hz, 1H), 2.23 (dd, J = 12.1, 8.7 Hz, 1H), 2.16 (s, 6H). | |
| **102** | 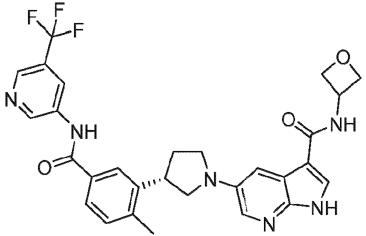 | 71 mg (36%) |
| | LC-MS (ESI, m/z): Method 10, t*_{R}* = 2.6 min, m/z (M+1) = 565.40 ¹H NMR (400 MHz, DMSO-d₆) δ 9.15 (d, J = 2.3 Hz, 1H), 8.66 (s, 1H), 8.59 (d, J = 2.3 Hz, 1H), 7.99 (s, 1H), 7.88 (d, J = 2.7 Hz, 1H), 7.82 (dd, J = 7.8, 1.8 Hz, 1H), 7.78 (s, 1H), 7.51 (t, J = 2.7 Hz, 1H), 7.40 (d, J = 8.0 Hz, 1H), 4.77 (s, 1H), 4.31 (dd, J = 9.2, 7.1 Hz, 1H), 4.23 (dt, J = 9.8, 5.0 Hz, 1H), 4.14 (t, J = 6.8 Hz, 1H), 3.84 (p, J = 7.7 Hz, 1H), 3.80 - 3.73 (m, 1H), 3.59 (dt, J = 16.7, 7.4 Hz, 2H), 3.49 (q, J = 8.0 Hz, 1H), 3.41 (t, J = 8.3 Hz, 2H), 2.45 (s, 1H), 2.22 (tt, J = 8.9, 4.7 Hz, 1H). | |
| **103** | 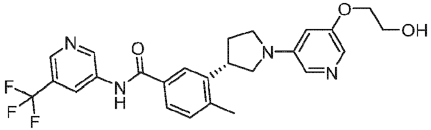 | 18 mg (37%) |
| | LC-MS (ESI, m/z): method 4, t*_{R}* = 5.06 min, m/z (M+1) = 487.4 ¹H NMR (400 MHz, DMSO-d₆) δ ppm 10.64 (1 H, s) 9.18 (1 H, d, J=2.19 Hz) 8.70 (1 H, s) 8.59 (1 H, t, J=1.86 Hz) 7.95 (1 H, d, J=1.75 Hz) 7.81 (1 H, dd, J=7.89, 1.75 Hz) 7.61 (2 H, dd, J=11.62, 2.41 Hz) 7.41 (1 H, d, J=7.89 Hz) 6.50 - 6.52 (1 H, m) 486 (1 H, t, J=5.48 Hz) 4.03 (2 H, t, J=4.93 Hz) 3.73 - 3.82 (2 H, m) 3.70 (2 H, q, J=5.33 Hz) 3.52 - 3.59 (1 H, m) 3.43 (1 H, br d, J=7.45 Hz) 3.33 - 3.37 (1 H, m) 2.47 - 2.48 (3 H, m) 2.38 (1 H, br dd, J=6.25, 2.96 Hz) 2.16 - 2.28 (1 H, m) | |
| **Interme diate 73** | 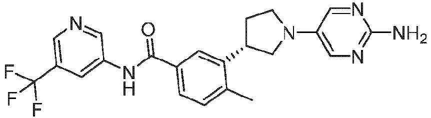 | 71 mg (36%) |
| | LC-MS (ESI, m/z): method 1, t*_{R}* = 0.86 min, m/z (M+1) = 443.27 | |

### Example 104 - 3-((3R,4S)-3-(4 -(hydroxymethyl)-1-(pyrimidin-5-yl)pyrrolidin-3-yl)-4-methyl-N-(5-(trifluoromethyl)pyridin-3-yl)benzamide and 3-((3S,4R)-3-(4-(hydroxymethyl) -1-(pyrimidin-5-yl)pyrrolidin-3-yl)-4-methyl-N-(5-(trifluoromethyl)pyridin-3-yl)benzamide3-(4-(hydroxymethyl)-1-(pyrimidin-5-yl)pyrrolidin-3-yl)-4-methyl-N-(5-(trifluoromethyl)pyridin-3-yl)benzamide

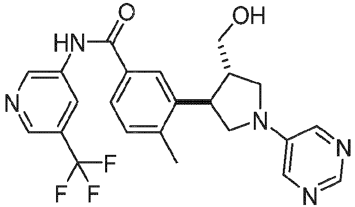

### Step 1 - (E)-3-(5-(ethoxycarbonyl)-2-methylphenyl)acrylic acid (Intermediate 74)

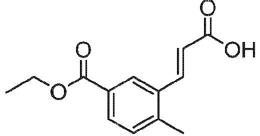

To a solution of ethyl 3-bromo-4-methylbenzoate (6.53 ml, 37.0 mmol) in DMF (45 mL), TEA (25.8 ml, 185 mmol), triphenylphosphine (1.942 g, 7.40 mmol), palladium acetate (0.416 g, 1.851 mmol) and prop-2-enoic acid (3.81 ml, 55.5 mmol) were added. The reaction was stirred at 110°C. The mixture was concentrated then it was basified with a saturated solution of NaHCO₃. The product was extracted with DCM after the acidification of the aqueous phase. The organic layer was dried with phase separator and the residual solvent was removed under reduced pressure. The crude was purified by FCC (100% heptane up to Ethyl acetate 100%) to give title compound (5.18 g, 22.11 mmol, 60%).
LC-MS (ESI): method 1, t*_{R}* = 0.94 min; m/z (M+1) = 235.23

### Step 2 - (3S,4R)-1-benzyl-4-(5-(ethoxycarbonyl)-2-methylphenyl)pyrrolidine-3-carboxylic acid and (3R,4S)-1-benzyl-4-(5-(ethoxycarbonyl)-2-methylphenyl)pyrrolidine-3-carboxylic acid (Intermediate 75)

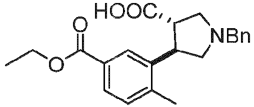

To Intermediate 74 (2.36 g, 10.07 mmol) in neat N-benzyl-1-methoxy-N-((trimethylsilyl)methyl)methanamine (10.31 ml, 40.3 mmol) was stirred at 80°C for 1 h. The crude mixture was concentrated and then diluted with a mixture of MTBE/Heptane 1:1 in order to precipitate the desired product which was filtered to give the title compound (3.63 g, 9.87 mmol, 98 %).
LC-MS (ESI): method 1, t*_{R}* = 0.66 min.

### Step 3 - ethyl 3-((3R,4S)-1-benzyl-4-(hydroxymethyl)pyrrolidin-3-yl)-4-methylbenzoate and ethyl 3-((3S,4R)-1-benzyl-4-(hydroxymethyl)pyrrolidin-3-yl)-4-methylbenzoate (Intermediate 76)

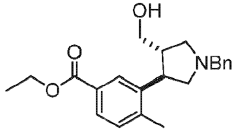

To a suspension of Intermediate 75 (3.17 g, 8.63 mmol) in THF (45 mL), oxalyl chloride (2.265 ml, 25.9 mmol) was added and the mixture was stirred until the formation of acyl chloride intermediate. Lithium borohydride (0.570 g, 26.2 mmol) was added and the mixture was stirred at rt overnight. This reaction crude was purified by FCC (100% heptane up to Ethyl acetate 100%) to give title compound (1.87 g, 5.29 mmol, 61%).

### Step 4 - ethyl 3-((3R,4S)-4-(hydroxymethyl)pyrrolidin-3-yl)-4-methylbenzoate and ethyl 3-((3S,4R)-4-(hydroxymethyl)pyrrolidin-3-yl)-4-methylbenzoate (Intermediate 77)

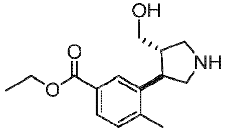

To a solution of Intermediate 76 (1.87 g, 5.29 mmol) in EtOH (25 mL) were added palladium on carbon (1.5 g, 14.10 mmol) and HCl 0.2M up to acid pH. The reaction flask was placed under vacuum and then under H₂ flow. The reaction crude was stirred overnight. The mixture was filtered and the residual solvent was removed under reduced pressure. The crude was used as such in the next step.
LC-MS (ESI): method 1, t*_{R}* = 0.45 min; m/z (M+1) = 264.22

### Step 5 - tert-butyl (3S,4R)-3-(((tert-butyldimethylsilyl)oxy)methyl)-4-(5-(ethoxycarbonyl)-2-methylphenyl)pyrrolidine-1-carboxylate and tert-butyl (3R,4S)-3-(((tert-butyldimethylsilyl)oxy)methyl)-4-(5-(ethoxycarbonyl)-2-methylphenyl) pyrrolidine-1-carboxylate (Intermediate 78)

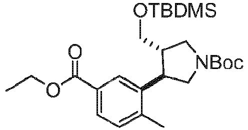

To a solution of Intermediate 77 (1.39 g, 5.3 mmol) in DCM (30 mL), TEA (2.72 ml, 19.6 mmol), 4-dimethylamino pyridine (0.065 g, 0.53 mmol) and di-tert-butyl dicarbonate (1.732 g, 7.9 mmol) were added. After the Boc protection was completed, tert-butyldimethylchlorosilane (2.291 ml, 13.2 mmol) and TEA were added. The reaction was stirred at rt for 30 min. The product was extracted with DCM after washing the crude with HCl 2N. The organic layer was dried with phase separator cartridge and the residual solvent removed under pressure. The crude was first purified by FCC (Gradient: from 100 % heptane to 100 % AcOEt) to give title compound (230 g, 0.48 mmol, 9.1 %).
LC-MS (ESI): Method 14, t*_{R}* = 3.60 min; m/z (M+1) = not ionized

### Step 6 - tert-butyl (3S,4R)-3-(((tert-butyldimethylsilyl)oxy)methyl)-4-(2-methyl-5-((5-(trifluoromethyl)pyridin-3-yl)carbamoyl)phenyl)pyrrolidine-1-carboxylate and tert-butyl (3R,4S)-3-(((tert-butyldimethylsilyl)oxy)methyl)-4-(2-methyl-5-((5-(trifluoromethyl)pyridin-3-yl)carbamoyl)phenyl)pyrrolidine-1-carboxylate (Intermediate 79)

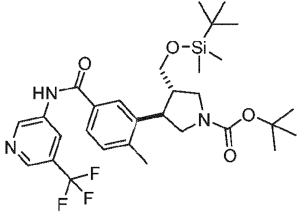

The intermediate was prepared following the procedure of Example 1, step 9.
SM: Intermediate 78: 230 mg (1 eq.); 5-(trifluoromethyl)pyridin-3-amine: 195 mg (2.5 eq.)
Amount/yield: 130 mg/ 46%
LC-MS (ESI): Method 14, t*_{R}* = 1.06 min; m/z (M+1)= not ionized

### Step 7 - 3-((3R,4S)-4-(hydroxymethyl)pyrrolidin-3-yl)-4-methyl-N-(5-(trifluoro methyl)pyridin-3-yl)benzamide and 3-((3S,4R)-4-(hydroxymethyl)pyrrolidin-3-yl)-4-methyl-N-(5-(trifluoromethyl)pyridin-3-yl)benzamide (Intermediate 80)

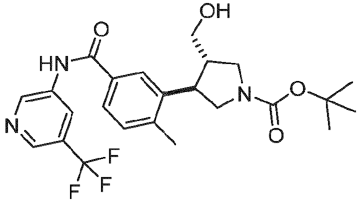

Intermediate 80 was prepared by following the procedure described for Intermediate 16.
SM: Intermediate 79: 133 mg
Amount/yield: 85 mg/ 100%
LC-MS (ESI): Method 13, t*_{R}* = 0.53 min; m/z (M+1)= 380.17

### Step 8 - 3-((3R,4S)-4-(((tert-butyldimethylsilyl)oxy)methyl)pyrrolidin-3-yl)-4-methyl-N-(5-(trifluoromethyl)pyridin-3-yl)benzamide and 3-((3S,4R)-4-(((tert-butyldimethylsilyl) oxy)methyl)pyrrolidin-3-yl)-4-methyl-N-(5-(trifluoromethyl) pyridin-3-yl)benzamide (Intermediate 81)

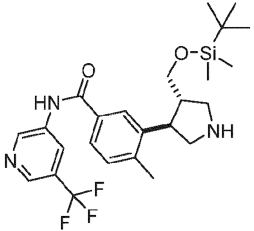

Intermediate 80 (85 mg, 0.224 mmol) was dissolved in DCM (2 mL) and added with TEA (0.120 ml, 0.861 mmol). Then tert-butyldimethylchlorosilane (0.092 ml, 0.53 mmol) was added. Solution was quenched with NaHCO₃ solution and extracted with DCM. Organic phase was evaporated under vacuum to give title compound (110 mg, 0.223 mmol, 99 %).
LC-MS (ESI): method 1, t*_{R}* = 0.84 min; m/z (M+1)= 494.26

### Step 9 - 3-((3R,4S)-4-(((tert-butyldimethylsilyl)oxy)methyl)-1-(pyrimidin-5-yl)pyrrolidine-3-yl)-4-methyl-N-(5-(trifluoromethyl)pyridin-3-yl)benzamide and 3-((3S,4R) -4-(((tert-butyldimethylsilyl)oxy)methyl)-1-(pyrimidin-5-yl)pyrrolidin-3-yl)-4-methyl-N-(5-(trifluoromethyl)pyridin-3-yl)benzamide (Intermediate 82)

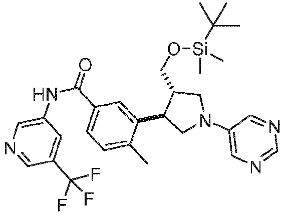

Intermediate 82 was prepared following the procedure of Intermediate 17, using RuPhos/Pd(dppf)Cl₂ as catalyst.
SM: Intermediate 81: 110 mg (1 eq.); 5-bromopyrimidine: 53 mg (1.5 eq.)
Amount/yield: 29 mg/ 25%
LC-MS (ESI): method 1, t*_{R}* = 1.47 min; m/z (M+1- t-But)= 572.35

### Step 10 - 3-((3R,4S)-3-(4-(hydroxymethyl)-1-(pyrimidin-5-yl)pyrrolidin-3-yl)-4-methyl-N-(5-(trifluoromethyl)pyridin-3-yl)benzamide and 3-((3S,4R)-3-(4-(hydroxymethyl) -1-(pyrimidin-5-yl)pyrrolidin-3-yl)-4-methyl-N-(5-(trifluoromethyl)pyridin-3-yl)benzamide (Example 104)

Intermediate 82 (29 mg, 0.05 mmol) was dissolved in water (0.5 mL) and ethanol (1.5 mL) and added of HCl 37% (4.17 µl, 0.051 mmol). Solution was stirred at rt overnight. Solvents were concentrated under vacuum, added of 1 mL of NaHCO₃ sat solution and diluted with DCM. The organic phase was washed with H₂O, then it was evaporated and purified by SCX eluting with NH₃ 7 MeOH to give title compound (10 mg, 0.022 mmol, 43%).
LC-MS (ESI, m/z): method 4, t*_{R}* = 5.32 min, m/z (M+1) = 458.3
¹H NMR (400 MHz, acetone-d₆) δ ppm 10.03 (1 H, br s) 9.11 (1 H, d, J=1.75 Hz) 8.69 (1 H, s) 8.63 (1 H, s) 8.45 (1 H, s) 8.13 (2 H, s) 8.11 (1 H, s) 7.85 (1 H, dd, J=7.89, 1.75 Hz) 7.40 (1 H, d, J=7.89 Hz) 3.88 - 3.94 (1 H, m) 3.83 (1 H, d, J=8.77 Hz) 3.78 (1 H, dd, J=9.43, 8.11 Hz) 3.71 (1 H, br d, J=4.38 Hz) 3.54 - 3.64 (2 H, m) 3.40 - 3.49 (2 H, m) 2.53 (3 H, s)

### Example 105 - 3-((3S,4R)-3-(4-hydroxy-1-(pyrimidin-5-yl)pyrrolidin-3-yl)-4-methyl-N-(5-(trifluoromethyl)pyridin-3-yl)benzamide and 3-((3R,4S)-4-hydroxy-1-(pyrimidin-5-yl)pyrrolidin-3-yl)-4-methyl-N-(5-(trifluoromethyl)pyridin-3-yl)benzamide

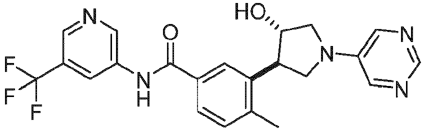

### Step 1 - ethyl (E)-4-methyl-3-(3-oxobut-1-en-1-yl)benzoate (Intermediate 83)

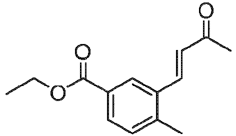

The intermediate 83 was prepared by following the procedure of Example 104, step 1.
SM: ethyl 3-bromo-4-methylbenzoate: 6 g (1 eq.); but-3-en-2-one 5.19 g (3 eq.)
Amount/yield: 5 g/87%
LC-MS (ESI): Method 13, t*_{R}* = 1.09 min; m/z (M+1)= 233.22

### Step 2 - ethyl 3-((3R,4S)-4-acetyl-1-benzylpyrrolidin-3-yl)-4-methylbenzoate and ethyl 3-(4-acetyl-1-benzylpyrrolidin-3-yl)-4-methylbenzoate and ethyl 3-((3S,4R)-4-acetyl-1-benzylpyrrolidin-3-yl)-4-methylbenzoate (Intermediate 84)

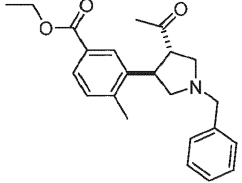

The intermediate 84 was prepared by following the procedure of Example 104, step 2.
SM: Intermediate 83: 5 g (1 eq.); N-benzyl-1-methoxy-N-((trimethylsilyl)methyl)methanamine 7.67 g (1.5 eq.)
Amount/yield: 7.7 g/98%
LC-MS (ESI): Method 13, t*_{R}* = 0.67 min; m/z (M+1)= 366.29

### Step 3 - ethyl 3-((3R,4S)-4-acetylpyrrolidin-3-yl)-4-methylbenzoate and ethyl 3-((3S,4R)-4-acetylpyrrolidin-3-yl)-4-methylbenzoate (Intermediate 85)

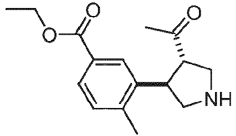

The intermediate 85 was prepared by following the procedure of Example 104, step 4 in quantitative yield.
SM: Intermediate 84: 7.7 g (1 eq.);
LC-MS (ESI): Method 13, t*_{R}* = 0.51 min; m/z (M+1)= 276.26

### Step 4 - tert-butyl (3S,4R)-3-acetyl-4-(5-(ethoxycarbonyl)-2-methylphenyl)pyrrolidine-1-carboxylate and tert-butyl (3R,4S)-3-acetyl-4-(5-(ethoxycarbonyl)-2-methylphenyl) pyrrolidine-1-carboxylate (Intermediate 86)

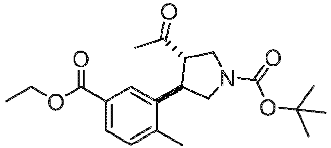

Intermediate 86 was prepared by following the procedure of Example 104, step 3 in quantitative yield.
SM: Intermediate 85: 2.8 g (1 eq.);
Amount/yield: 1.5 g/40%
LC-MS (ESI): Method 13, t*_{R}* = 1.27 min; m/z (M+1-BOC)= 276.26

### Step 5 - tert-butyl (3R,4S)-3-acetoxy-4-(5-(ethoxycarbonyl)-2-methylphenyl) pyrrolidine-1-carboxylate and tert-butyl (3S,4R)-3-acetoxy-4-(5-(ethoxycarbonyl)-2-methylphenyl)pyrrolidine-1-carboxylate (Intermediate 87)

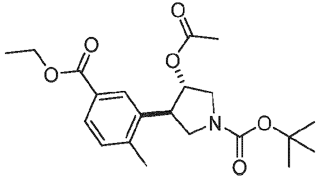

To a solution of Intermediate 86 (1.56 g, 4.15 mmol) in DCM (15 mL), trifluoroacetic anhydride (3465 ml, 24.93 mmol), urea compound with hydrogen peroxide (1:1) (2.345g, 24.9 mmol) and sodium phosphate, dibasic (1.769 g, 12.46 mmol) were added. The crude was diluted with DCM, washed with a solution of NaHCO₃ and then the organic layer was dried with phase separator cartridge. The residual solvent was evaporated under vacuum and the crude was used as it was for the following step. (810 mg, 2.069 mmol, 50%).
LC-MS (ESI): Method 13, t_{R} = 1.32 min; m/z (M+1- t-Butyl)= 292.13

### Step 6 - tert-butyl (3R,4S)-3-(5-(ethoxycarbonyl)-2-methylphenyl)-4-hydroxypyrrolidine-1-carboxylate and tert-butyl (3S,4R)-3-(5-(ethoxycarbonyl)-2-methylphenyl)-4-hydroxypyrrolidine-1-carboxylate (Intermediate 88)

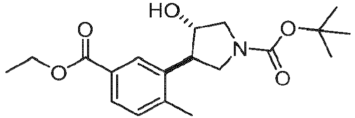

To a solution of Intermediate 87 (0.70 g, 1.79 mmol) in EtOH (20 mL), potassium carbonate (0.741 g, 5.36 mmol) was added and the reaction was stirred at 40°C overnight. The mixture was concentrated then it was diluted with DCM and washed with a solution of NaHCO₃. The organic layer was dried with phase separator cartridge and purified by FCC (1/1 Heptane/AcOEt) to give title compound (480 mg, 1.39 mmol, 78 %).
LC-MS (ESI): Method 14, t*_{R}* = 1.12 min; m/z (M+1- t-Butyl)= 250.22

### Step 7 - ethyl 3-((3R,4S)-4-hydroxypyrrolidin-3-yl)-4-methylbenzoate and ethyl 3-((3S,4R)-4-hydroxypyrrolidin-3-yl)-4-methylbenzoatem (Intermediate 89)

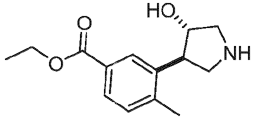

Intermediate 89 was prepared following the procedure of Example 43, step 3.
SM: Intermediate 88: 213 mg (1 eq.);
Amount/yield: 152 mg/100%
LC-MS (ESI): Method 13, t*_{R}* = 0.44 min; m/z (M+1)= 250.27

### Step 8 - ethyl 3-((3R,4S)-4-hydroxy-1-(pyrimidin-5-yl)pyrrolidin-3-yl)-4-methylbenzoate and ethyl 3-((3S,4R)-4-hydroxy-1-(pyrimidin-5-yl)pyrrolidin-3-yl)-4-methylbenzoate (Intermediate 90)

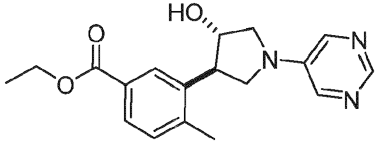

The intermediate was prepared following the procedure of Intermediate 17, using RuPhos/Pd(dppf)Cl₂ as catalyst.
SM: Intermediate 89: 152 mg (1 eq.); 5-bromopyrimidine: 116 mg (1.2 eq.)
Amount/yield: 76 mg/ 38%
LC-MS (ESI, m/z): Method 13, t*_{R}* = 0.80 min, m/z (M+1) = 328

### Step 9 - 3-((3S,4R)-3-(4-hydroxy-1-(pyrimidin-5-yl)pyrrolidin-3-yl)-4-methyl-N-(5-(trifluoromethyl)pyridin-3-yl)benzamide and 3-((3R,4S)-4-hydroxy-1-(pyrimidin-5-yl)pyrrolidin-3-yl)-4-methyl-N-(5-(trifluoromethyl)pyridin-3-yl)benzamide (Example 105)

The Example 105 was prepared following the procedure of Example104, step 6.
SM: ntermediate 90: 75 mg (1 eq.); 5-(trifluoromethyl)pyridin-3-amine: 130 mg (3.5 eq.)
Amount/yield: 30 mg/ 30%
LC-MS (ESI): Method 4, t*_{R}* = 4.44 min; m/z (M+1) = 444.2
¹H NMR (DMSO-d₆, 400 MHz) δ 10.59 (br s, 1H), 9.12 (d, 1H, J=2.4 Hz), 8.65 (s, 1H), 8.53 (t, 1H, J=1.9 Hz), 8.16 (s, 2H), 7.8-7.8 (m, 2H), 7.38 (d, 1H, J=8.6 Hz), 6.8-7.0 (m, 1H), 5.45 (d, 1H, J=5.3 Hz), 4.44 (quin, 1H, J=5.5 Hz), 3.8-3.9 (m, 1H), 3.6-3.7 (m, 2H), 3.45 (dd, 1H, J=6.7, 9.8 Hz), 3.2-3.3 (m, 1H), 2.47 (s, 3H)

### Example 106 - (S)-4-methyl-3-(1-(thieno[2,3-d]pyrimidin-4-yl)pyrrolidin-3-yl)-N-(5-(trifluoromethyl)pyridin-3-yl)benzamide

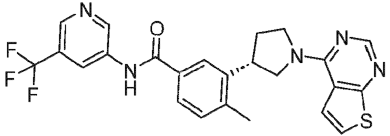

Intermediate 71 (200 mg, 0.36 mmol), XPhos Pd G2 (28.0 mg, 0.04 mmol), molybdenum hexacarbonyl (94 mg, 0.36 mmol) and DIPEA (0.095 mL, 0.53 mmol) were dissolved in methanamine 2.0 N in THF (2.0 mL, 4.0 mmol). The mixture was heated for 10 min at 150 °C. Solvent was evaporated under vacuum and crude was submitted to reverse phase FCC. Appropriate fractions were combined to give title compound (20 mg, 0.04 mmol, 11.6 % yield).
LC-MS (ESI, m/z): method 4, t*_{R}* = 7.08 min, m/z (M+1) = 484.21
¹H NMR (DMSO-d₆, 400 MHz) δ 10.62 (s, 1H), 9.15 (d, 1H, J=2.2 Hz), 8.66 (s, 1H), 8.55 (s, 1H), 8.33 (s, 1H), 7.97 (s, 1H), 7.79 (dd, 1H, J=1.8, 7.9 Hz), 7.66 (d, 1H, J=6.1 Hz), 7.50 (d, 1H, J=6.1 Hz), 7.39 (d, 1H, J=7.9 Hz), 4.3-4.3 (m, 1H), 4.1-4.1 (m, 1H), 3.7-4.0 (m, 3H), 2.3-2.4 (m, 1H), 2.2-2.3 (m, 1H).

The following Examples were prepared by using the procedure described for Example 106, starting from suitable intermediate.

| **Example N°** | **Structure** | **Product Amount (Yield)** |
|---|---|---|
| | **Analytical Data** | |
| **107** | 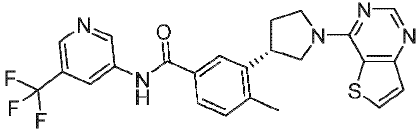 | 38 mg (30 %) |
| | LC-MS (ESI, m/z): method 4, t*_{R}* = 4.93 min, m/z (M+1) = 484.2 | |
| | ¹H NMR (400 MHz, DMSO-d₆) δ ppm 10.66 (1 H, s) 9.18 (1 H, s) 8.69 (1 H, s) 8.59 (1 H, s) 8.42 (1 H, s) 8.19 (1 H, d, J=5.48 Hz) 7.97 - 8.02 (1 H, m) 7.83 (1 H, dd, J=7.78, 1.64 Hz) 7.43 (1 H, s) 7.40 (1 H, d, J=5.70 Hz) 4.38 (1 H, br s) 4.17 (1 H, br s) 3.96 (1 H, br dd, J=6.03, 3.18 Hz) 3.77 - 3.91 (2 H, m) 2.41 - 2.46 (1 H, m) 2.20 - 2.32 (1 H, m) | |
| **108** | 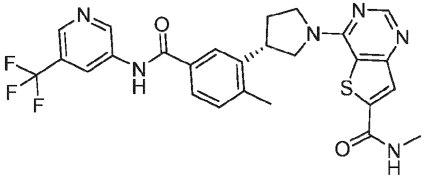 | 38 mg (30%) |
| | LC-MS (ESI, m/z): method 4, t*_{R}* = 5.14 min, m/z (M+1) = 541.1 | |
| | ¹H NMR (400 MHz, DMSO-d₆) δ ppm 10.66 (1 H, s) 9.18 (1 H, d, J=2.19 Hz) 8.88 (1 H, q, J=4.24 Hz) 8.69 (1 H, s) 8.57 - 8.61 (1 H, m) 8.44 (1 H, s) 8.00 (1 H, s) 7.96 (1 H, s) 7.83 (1 H, dd, J=7.89, 1.53 Hz) 7.42 (1 H, d, J=8.11 Hz) 4.29 - 4.51 (1 H, m) 4.09 - 4.28 (1 H, m) 3.90 - 4.02 (1 H, m) 3.84 (2 H, br s) 2.82 (3 H, d, J=4.38 Hz) 2.48 - 2.49 (3 H, m) 2.40 - 2.46 (1 H, m) 2.21 - 2.31 (1 H, m) | |
| **131** | 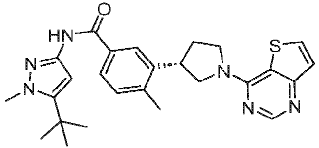 | 45 mg (30%) |
| | LC-MS (ESI, m/z): method 4, t*_{R}* = 5.7 min, m/z (M+1) = 475.29 | |
| | ¹H NMR (acetone-d₆, 400 MHz) δ 9.59 (br s, 1H), 8.39 (s, 1H), 8.1-8.2 (m, 1H), 8.01 (d, 1H, J=5.5 Hz), 7.87 (dd, 1H, J=1.8, 7.9 Hz), 7.36 (d, 1H, J=7.9 Hz), 7.33 (d, 1H, J=5.5 Hz), 6.57 (s, 1H), 4.42 (br s, 1H), 4.22 (br s, 1H), 3.9-4.0 (m, 3H), 3.81 (s, 3H), 2.52 (s, 3H), 2.48 (br dd, 1H, J=2.5, 5.8 Hz), 2.35 (br d, 1H, J=9.6 Hz), 1.36 (s, 9H) | |

### Example 109 and Example 110 - 4-methoxy-3-(1-(pyrimidin-5-yl)pyrrolidin-3-yl)-N-(5-(trifluoromethyl)pyridin-3-yl)benzamide

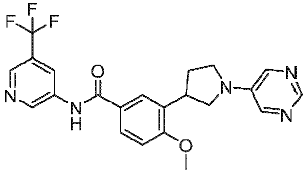

### Step 1 - tert-butyl 3-(5-(ethoxycarbonyl)-2-methoxyphenyl)-2,5-dihydro-1H-pyrrole-1-carboxylate (Intermediate 91)

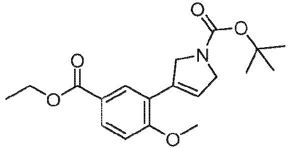

Ethyl 3-bromo-4-methoxybenzoate (5.0 g, 19.30 mmol), (tert-butyl 3-(4,4,5,5-tetramethyl-1,3,2-dioxaborolan-2-yl)-2,5-dihydropyrrole-1-carboxylate (6.27 g, 21.23 mmol), Pd(dppf)Cl₂ (0.706 g, 0.965 mmol) were dissolved in 1,4-Dioxane (50 ml), then the mixture was degassed with Ar for 5 min. Cesium carbonate (18.86 g, 57.9 mmol) was dissolved in 20 mL of water and this solution was added to the reaction mixture which was stirred at 90°C for 3 hrs.

The reaction mixture was filtered through celite pad, then the solvent was evaporated; the residue was purified via FCC (Eluent A:n-Heptane Eluent B: Ethyl Acetate, gradient A:B from 100:0 to 60:40). The relevant fractions were collected and desiccated until dryness to afford desired product (8.06 g, 23.20 mmol, 100 % yield).

¹H NMR (acetone-d₆, 400 MHz) δ 7.96 (d, 1H, *J*=8.6 Hz), 7.86 (dd, 1H, *J*=2.0, 16.0 Hz), 7.18 (d, 1H, *J*=8.8 Hz), 6.5-6.6 (m, 1H), 4.5-4.6 (m, 2H), 4.33 (q, 2H, *J*=7.0 Hz), 4.2-4.3 (m, 2H), 4.01 (s, 3H), 1.49 (d, 9H, *J*=5.7 Hz), 1.35 (t, 3H, *J*=7.1 Hz)
The following intermediates were prepared by using the procedure described for Intermediate **91,** using the suitable bromo intermediate.

| **Interme diate N°** | **Structure** | **Product Amount (Yield)** |
|---|---|---|
| | **Analytical Data** | |
| **Interme diate 92** | 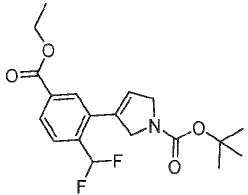 | 790 mg (100%) |
| | LC-MS (ESI, m/z): Method 14, t*_{R}* = 1.37 min, m/z (M+1-tButyl) = 312.2 | |
| **Interme diate 93** | 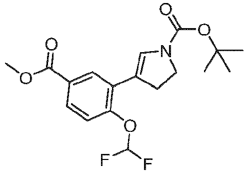 | 312 mg (95%) |
| | LC-MS (ESI, m/z): Method 14, t*_{R}* = 2.4 min, m/z (M+1) = 314.0 | |

### Step 2 -tert-butyl 3-(5-(ethoxycarbonyl)-2-methoxyphenyl)pyrrolidine-1-carboxylate (Intermediate 94)

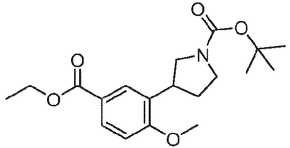

Intermediate 91 (8 g, 23.03 mmol) was dissolved in EtOH (25 ml) and Ethyl acetate (25 ml), then Pd/C 10R424 (Johnson Matthey, 50% wet) (2.4 g, 1.15 mmol) was added in one portion. The vessel was closed and 3 cycles vacuum-H₂ were performed to set the reaction which was run with 5 bar of hydrogen at 40°C, and stirred until completion. The mixture was filtered through celite pad then the solvent was removed by reduced pressure to afford desired product (8 g, 22.89 mmol, 99 %).

¹H NMR (acetone-d₆, 400 MHz) δ 7.93 (dd, 1H, *J*=2.0, 8.6 Hz), 7.88 (s, 1H), 7.1-7.1 (m, 1H), 4.3-4.4 (m, 2H), 3.9-4.0 (m, 3H), 3.6-3.8 (m, 2H), 3.5-3.5 (m, 1H), 3.3-3.4 (m, 1H), 3.2-3.3 (m, 1H), 2.1-2.3 (m, 1H), 2.06-2.04 (m, 1H), 1.46 (s, 9H), 1.3-1.4 (m, 3H)
The following intermediates were prepared by using the procedure described for Intermediate 94, using the corresponding intermediate.

| **Interme diate N°** | **Structure** | **Product Amount (Yield)** |
|---|---|---|
| | **Analytical Data** | |
| **Interme diate 95** | 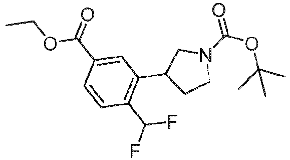 | 744 mg (82%) |
| | LC-MS (ESI, m/z): Method 13, *t_{R} =* 1.34 min, m/z (M+1-tButyl) = 316.1 | |
| **Interme diate 96** | 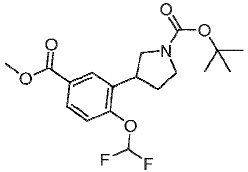 | 300 mg (98%) |
| | LC-MS (ESI, m/z): Method 14, t*_{R}* = 2.35 min, m/z (M+1-tButyl) = 316.2 | |

### Step 3 - tert-butyl 3-(2-methoxy-5-((5-(trifluoromethyl)pyridin-3-yl)carbamoyl) phenyl)pyrrolidine-1-carboxylate (Intermediate 97)

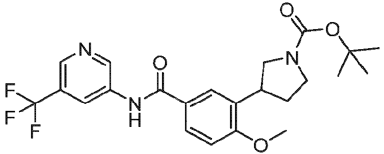

The Intermediate 97 was prepared by following the procedure for Example 1, step 9, replacing LDA with LiHDMS.
SM: Intermediate 94: 2.0 g, 5.7 mmol (1 eq.)4-(trifluoromethoxy)pyridin-2-amine: 1.9 (2 eq.)
Amount/yield: 2.47 g /93
LC-MS (ESI, m/z): Method 14, t*_{R}* = 2.41 min, m/z (M+1) =
¹H NMR (acetone-d₆, 400 MHz) δ 9.94 (br s, 1H), 9.13 (d, 1H, *J*=1.5 Hz), 8.71 (s, 1H), 8.63 (s, 1H), 7.9-8.0 (m, 2H), 7.16 (d, 1H, *J*=8.6 Hz), 3.97 (s, 3H), 3.7-3.8 (m, 1H), 3.70 (br dd, 1H, *J*=7.5, 14.5 Hz), 3.5-3.6 (m, 1H), 3.37 (br dd, 1H, *J*=7.3, 17.0 Hz), 3.1-3.3 (m, 1H), 2.2-2.3 (m, 1H), 2.1-2.2 (m, 1H), 1.46 (s, 9H)

The following Examples/intermediates were prepared by using the procedure described for Intermediate 97, using the corresponding intermediate.

| **Interme diate N°** | **Structure** | **Product Amount (Yield)** |
|---|---|---|
| | **Analytical Data** | |
| **Interme diate 98** | 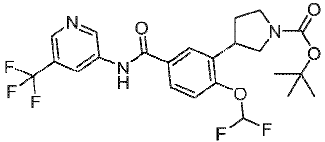 | 383 mg (98%) |
| | LC-MS (ESI, m/z): Method 14, t*_{R}* = 2.45 min, m/z (M+1-tButyl) = 446.2 | |

### Step 4 - 4-methoxy-3-(pyrrolidin-3-yl)-N-(5-(trifluoromethyl)pyridin-3-yl)benzamide (Intermediate 99)

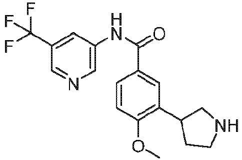

Intermediate 99 was prepared following the same procedure described for the synthesis of Intermediate 22.
SM Intermediate 97: 2.5 g (1.00 eq);
Amount/Yield: 1.9 g/ 100%
LC-MS (ESI, m/z): Method 13, t*_{R}* =0.57 min, m/z (M+1) = 366.2

The following intermediates were prepared by using the procedure described for Intermediate 99, using the corresponding intermediate.

| **Interme diate N°** | **Structure** | **Product Amount (Yield)** |
|---|---|---|
| | **Analytical Data** | |
| **Interme diate 100** | 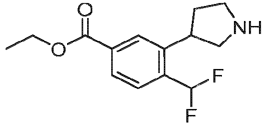 | <625 mg (95%) |
| | LC-MS (ESI, m/z): method 16, t*_{R}* = 0.78 min, m/z (M+1) = 270.2 | |
| **Interme diate 101** | 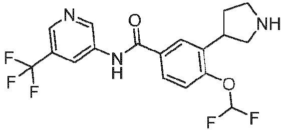 | 280 mg (91%) |
| | LC-MS (ESI, m/z): Method 13, t*_{R}* = 0.60 min, m/z (M+1) = 402.3 | |

### Step 5 - 4-methoxy-3-(1-(pyrimidin-5-yl)pyrrolidin-3-yl)-N-(5-(trifluoromethyl) pyridin-3-yl)benzamide (Example 111)

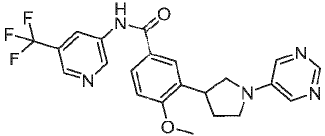

Example 111 was prepared following the same procedure described for the synthesis of Intermediate 31.
SM Intermediate 99: 0.18 g (1.00 eq);
5-bromopyrimidine : 0.12 g (1.5 eq); Pd-170: 33 mg (0.1 eq)
Amount/Yield: 53 mg/ 24%
LC-MS (ESI, m/z): method 4, t*_{R}* =6.58 min, m/z (M+1) = 444.31
¹H NMR (ACN-d₃, 400 MHz) δ 9.0-9.1 (m, 2H), 8.63 (s, 1H), 8.57 (s, 1H), 8.45 (s, 1H), 8.12 (s, 2H), 7.92 (dd, 1H, J=2.2, 8.6 Hz), 7.86 (d, 1H, J=2.2 Hz), 7.13 (d, 1H, J=8.6 Hz), 3.95 (s, 3H), 3.8-3.9 (m, 2H), 3.5-3.6 (m, 1H), 3.3-3.5 (m, 2H), 2.4-2.5 (m, 1H), 2.2-2.3 (m, 1H)

The following Examples were prepared by using the procedure described for Example 111, using the corresponding intermediate.

| **Example N°** | **Structure** | **Product Amount (Yield)** | Catalyst-**Ligand Base Solvent** |
|---|---|---|---|
| | **Analytical Data** | | |
| **112** | 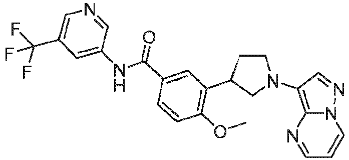 | 15 mg (8%) | BPC-305 Cs₂CO₃ DMA |
| | LC-MS (ESI, m/z): method 4, t*_{R}* =6.91 min, m/z (M+1) = 483.3 | | |
| | ¹H NMR (400 MHz, acetone-d₆) δ ppm 9.94 (1 H, br s) 9.11 (1 H, s) 8.71 (1 H, s) 8.59 - 8.64 (2 H, m) 8.16 (1 H, dd, J=3.73, 1.75 Hz) 8.10 (1 H, d, J=2.19 Hz) 7.99 (1 H, dd, J=8.66, 2.30 Hz) 7.71 (1 H, s) 7.17 (1 H, d, J=8.55 Hz) 6.74 (1 H, dd, J=7.23, 3.73 Hz) 3.98 (3 H, s) 3.90 - 3.94 (2 H, m) 3.72 (1 H, td, J=8.71, 4.28 Hz) 3.59 (1 H, dt, J=9.15, 7.48 Hz) 3.45 - 3.51 (1 H, m) 2.36 - 2.45 (1 H, m) 2.12 - 2.20 (1 H, m) | | |
| **113** | 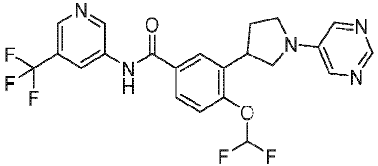 | 48 mg (40%) | RuPhos, Pd(dppf)Cl₂ CS₂CO₃ DMA |
| | LC-MS (ESI, m/z): method 4, t*_{R}* = 6.10 min, m/z (M+1) = 480.2 | | |
| | ¹H NMR (acetone-d₆, 400 MHz) δ 10.07 (br s, 1H), 9.09 (d, 1H, J=2.2 Hz), 8.66 (d, 2H, J=11.6 Hz), 8.44 (s, 1H), 8.1-8.2 (m, 3H), 8.03 (dd, 1H, J=2.2, 8.6 Hz), 7.4-7.4 (m, 1H), 7.20 (t, 1H, J = 73.4 Hz), 4.0-4.0 (m, 1H), 3.8-3.9 (m, 1H), 3.66 (dt, 1H, J=3.1, 8.9 Hz), 3.4-3.6 (m, 2H), 2.50 (tdd, 1H, J=3.1, 6.2, 9.5 Hz), 2.3-2.4 (m, 1H) | | |
| **Intermed iate 102** | 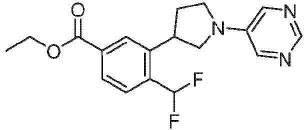 | 407 mg (57%) | RuPhos, Pd(dppf)Cl₂ CS₂CO₃ DMA |
| | LC-MS (ESI, m/z): Method 13, t*_{R}* = 1.05 min, m/z (M+1) = 348.21 | | |

### Step 6 - 4-methoxy-3-(1-(pyrimidin-5-yl)pyrrolidin-3-yl)-N-(5-(trifluoromethyl) pyridin-3-yl)benzamide and 4-methoxy-3-(1-(pyrimidin-5-yl)pyrrolidin-3-yl)-N-(5-(trifluoromethyl)pyridin-3-yl)benzamide (Example 109 and Example 110 )

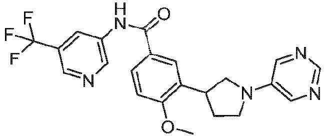

A racemic mixture of Example 111 (43 mg, 0.098 mmol) was separated by chiral SFC (LUX Cellulose-1 10x250 mm, 5 µm 35/65 MeOH (0.1% NH₄OH)/CO₂, 15 mL/minute, 120 bar, 40 °C, DAD 260 nm) to give the desired products.
1^{st} eluted enantiomer (12 mg, 0.0271 mmol, 28%).
Chiral analysis (SFC Method 4): t*_{R}* 2.41 min, ee 99.93%
LC-MS (ESI): Method 5, t*_{R}* = 4.45 min, m/z (M+1) = 444.6
2^{st} eluted enantiomer (7 mg, 16%):
   Chiral analysis (SFC Method 4): t*_{R}* 3.65 min, ee 99.88%
   LC-MS (ESI): Method 5, t*_{R}* = 4.44 min, m/z (M+1) = 444.5

The ¹H NMR spectra of first and second eluted isomers were superimposable with the ¹H NMR spectrum of the racemic mixture.

The following Examples, as single enantiomers, were prepared by using the procedure described for Example 109 and Example 110 , and by appropriate chiral SFC method, identified by conditions separation screening.

| **Example N°** | **Structure** | **Product Amount (Yield)/** |
|---|---|---|
| | **Analytical Data** | |
| **114** | 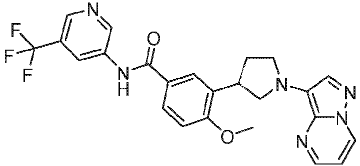 1^{st} eluted enantiomer | 11 mg (18%) |
| | Chiral analysis (SFC Method 6): t*_{R}* 2.34 min, ee 100% | |
| **115** | 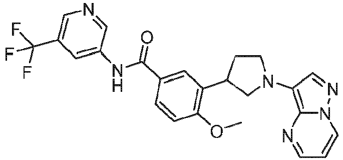 2^{nd} eluted enantiomer | 10 mg (17%) |
| | Chiral analysis (SFC Method 6): t*_{R}* 3.38 min, ee 98.6% | |
| | LC-MS (ESI, m/z): method 5, t*_{R}* =4.44 min, m/z (M+1) = 483.0 | |
| **116** | 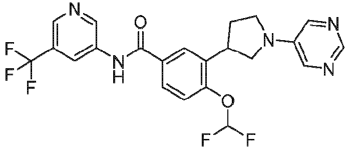 1^{st} eluted enantiomer | 13.6 mg (34%) |
| | Chiral analysis (SFC Method 5): t*_{R}* 1.77 min, ee 99% | |
| | LC-MS (ESI): Method 5, t*_{R}* = 4.50 min, m/z (M+1) = 480.0 | |
| **117** | 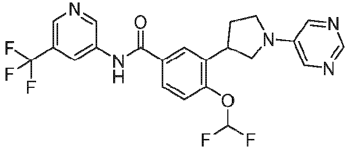 2^{nd} eluted enantiomer | 13.4 mg (33%) |
| | Chiral analysis (SFC Method 5): t*_{R}* 3.93 min, ee 99% | |
| | LC-MS (ESI): Method 5, t*_{R}* = 4.50 min, m/z (M+1) = 480.0 | |
| **118** | 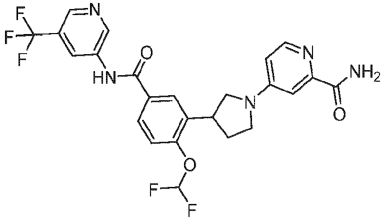 1^{st} eluted enantiomer | 34 mg (39%) |
| | Chiral analysis (SFC Method 7): t*_{R}* 7.23 min, ee 97.6% | |
| | LC-MS (ESI): Method 8, t*_{R}* = 4.63 min, m/z (M+1) = 522.3 | |
| **119** | 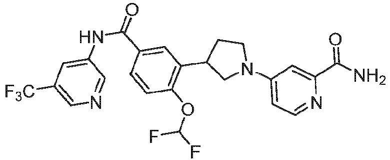 2^{nd}eluted enantiomer | 36 mg (41%) |
| | Chiral analysis (SFC Method 7): t*_{R}* 8.99 min, ee 94.6% | |
| | LC-MS (ESI): Method 8, t*_{R}* = 4.62 min, m/z (M+1) = 522.3 | |
| **121** | 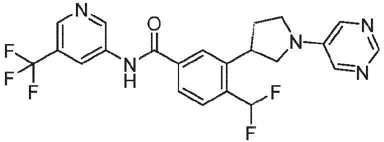 2^{nd} eluted enantiomer | 4.6 mg (26%) |
| | Chiral analysis (SFC Method 8): t*_{R}* 2.47 min, ee 99.8% | |
| | LC-MS (ESI): Method 5, t*_{R}* = 4.47 min, m/z (M+1) = 464.4 | |
| **122** | 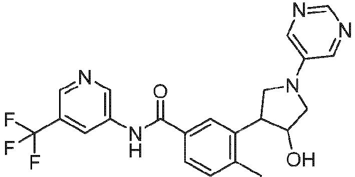 2^{nd} eluted enantiomer | 4.0 mg, (17%) |
| | Chiral analysis (SFC Method 4): t*_{R}* 4.20 min, ee 99% | |
| | LC-MS (ESI): Method 8, t*_{R}* = 3.83 min, m/z (M+1) = 444.3 | |

### Example 123 - (S)-(3-(1-(6-(hydroxymethyl)imidazo[1,2-a]pyridin-3-yl)pyrrolidin-3-yl)-4-methylphenyl)(5-(trifluoromethyl)pyridin-3-yl)methanone

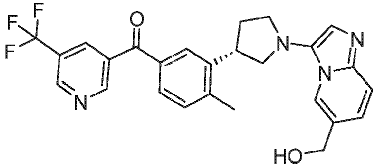

To a solution of Intermediate 57 (105 mg, 0.172 mmol) in THF (1.0 mL) was added TBAF 1M in THF (0.52 mL, 0.52 mmol) and the mixture was stirred at rt for 2 h. The reaction mixture was diluted with EtOAc and washed with NaHCO₃, water and brine. The organic phase was dried over MgSO₄, filtered and concentrated *in vacuo.* The residue was purified by achiral SFC (GREENSEP 2-EP 20x250 mm, 5 µm 10-20% MeOH (0.1% NH₄OH)/CO₂, 100 mL/minute, 120 bar, 40 °C, DAD 240 nm) to give the title compound (32 mg, 0.0646 mmol, 38%).
LC-MS (ESI): Method 5, t*_{R}* = 3.53 min, m/z (M+1) = 496.5
¹H NMR (400 MHz, DMSO-d₆) δ 10.68 (s, 1H), 9.21 (d, J=2.3 Hz, 1H), 8.71 - 8.70 (m, 1H), 8.63 (t, J=2.4 Hz, 1H), 8.13 (s, 1H), 8.09 (d, J=1.5 Hz, 1H), 7.81 (dd, J=1.8, 8.1 Hz, 1H), 7.45 - 7.38 (m, 2H), 7.29 (s, 1H), 7.11 (dd, J=1.5, 9.3 Hz, 1H), 5.25 (t, J=5.8 Hz, 1H), 4.51 (d, J=5.8 Hz, 2H), 3.87 - 3.80 (m, 1H), 3.64 - 3.58 (m, 1H), 3.48 - 3.41 (m, 2H), 3.39 - 3.33 (m, 2H), 2.47 (s, 3H), 2.19 - 2.09 (m, 1H).

### Example 44 - 3-(3-((6-aminopyridin-3-yl)oxy)azetidin-1-yl)-4-methyl-N-(5-(trifluoromethyl)pyridin-3-yl)benzamide

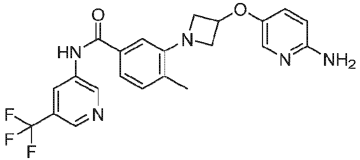

### Step 1 - tert-butyl 3-((6-nitropyridin-3-yl)oxy)azetidine-1-carboxylate (Intermediate 29)

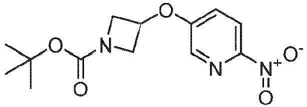

In a 20 mL vessel, 5-fluoro-2-nitropyridine (0.902 g, 6.35 mmol) and tert-butyl 3-hydroxyazetidine-1-carboxylate (1.00 g, 5.77 mmol) were dissolved in DMSO (10 ml), then Cs₂CO₃ (3.76 g, 11.55 mmol) was added in one portion. The reaction was stirred overnight at rt. The crude was quenched with H₂O and the product was extracted with DCM (2x50 mL). The organic layer was washed with brine (1 x50 mL) and desiccated under reduced pressure to give Intermediate 29 (2.01 g, 6.81 mmol, quantitative yield). The compound was used in the next step without further purification.
LC-MS (ESI, m/z): method 1, t*_{R}* = 0.95 min, m/z (M+1) = 239.9

### Step 2 - 5-(azetidin-3-yloxy)-2-nitropyridine hydrochloride (Intermediate 30)

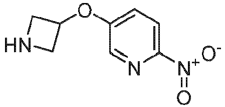

Intermediate 29 (1.20 g, 4.06 mmol) was dissolved in dioxane (10 ml) then HCl 3.7M in dioxane (10.000 ml, 37.0 mmol) was added in one portion. The reaction was stirred overnight at rt and monitored by UPLC-MS. The solvent was evaporated under reduced pressure to afford the title compound in quantitative yield (0.941 g, 4.06 mmol) which was used in the next step without further purifications.
LC-MS (ESI, m/z): method 1, t*_{R}* = 0.14 min, m/z (M+1) = 237.0

### Step 3 - 4-methyl-3-(3-((6-nitropyridin-3-yl)oxy)azetidin-1-yl)-N-(5-(trifluoromethyl)pyridin-3-yl)benzamide (Intermediate 31)

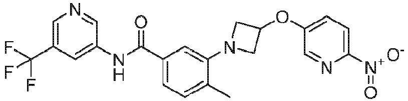

In a 20 mL microwave vial, Intermediate 8 (300 mg, 0.835 mmol), Intermediate 30 (290 mg, 1.253 mmol), cesium carbonate (816 mg, 2.506 mmol) and Pd-170 (56.3 mg, 0.084 mmol) were dissolved in DMA (4 ml), the vial was closed with sealed cap and backfilled with Ar. The reaction was stirred at 120 °C until complete conversion. The reaction was quenched with H₂O (5 mL) and the product was extracted with DCM (2 x 20 mL), the organic layers were combined and washed with brine (1 x 20 mL) and the solvent was evaporated until dryness. The crude product was purified by FCC on silica NH gel, eluting with a gradient of 40 % acetone in n-heptane. The relevant fractions were combined and exsiccated until dryness to afford the title compound (330 mg, 0.697 mmol, 83% yield).
LC-MS (ESI, m/z): method 3, t*_{R}* = 2.10 min, m/z (M+1) = 474.2
¹H NMR (acetone-d₆, 400 MHz) δ 9.88 (br s, 1H), 9.1-9.2 (m, 1H), 8.71 (s, 1H), 8.63 (s, 1H), 8.3-8.3 (m, 2H), 7.69 (dd, 1H, J=2.9, 9.0 Hz), 7.43 (dd, 1H, J=1.4, 7.8 Hz), 7.2-7.2 (m, 2H), 5.4-5.5 (m, 1H), 4.6-4.6 (m, 2H), 4.08 (dd, 2H, J=4.3, 8.9 Hz), 2.30 (s, 3H)

### Step 4 - 3-(3-((6-aminopyridin-3-yl)oxy)azetidin-1-yl)-4-methyl-N-(5-(trifluoromethyl)pyridin-3-yl)benzamide (Example 44)

Example 44 was prepared by following the procedure described for the synthesis of Example 42, step 2.
SM: Intermediate 31: 230 mg, 1.00 eq
Amount/Yield: 135 mg/ 63%
LC-MS (ESI, m/z): method 3, t*_{R}* = 1.18 min, m/z (M+1) = 444.2
¹H NMR (acetone-d₆, 400 MHz) δ 9.88 (br s, 1H), 9.13 (d, 1H, J=2.0 Hz), 8.71 (s, 1H), 8.62 (s, 1H), 7.65 (d, 1H, J=3.1 Hz), 7.40 (dd, 1H, J=1.4, 7.8 Hz), 7.16-7.18 (m, 2H), 7.10 (dd, 1H, J=3.1, 8.8 Hz), 6.53 (d, 1H, J=9.0 Hz), 5.0-5.1 (m, 3H), 4.4-4.5 (m, 2H), 3.91 (dd, 2H, J=4.7, 8.4 Hz), 2.28 (s, 3H)

### Example 45 - 3-(3-((6-acetamidopyridin-3-yl)oxy)azetidin-1-yl)-4-methyl-N-(5-(trifluoromethyl)pyridin-3-yl)benzamide

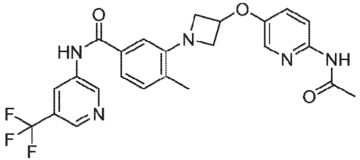

### Step 1 - 3-(3-((6-acetamidopyridin-3-yl)oxy)azetidin-1-yl)-4-methyl-N-(5-(trifluoromethyl)pyridin-3-yl)benzamide (Example 45)

Example 44 (60 mg, 0.135 mmol) and TEA (0.057 ml, 0.406 mmol) were dissolved in DCM (1 mL) then acetic anhydride (0.038 ml, 0.406 mmol) was added in one portion and the reaction was stirred at rt until completion. The solvent was evaporated and the crude was purified by FCC (gradient A:B from 100:0 to 0:10 in 12 CV eluent A: H₂O/ACN/HCOOH 95:5:0.1 Eluent B: H₂O/ACN/HCOOH 5:95:0.1). Relevant fractions were collected, the organic solvent was evaporated by reduced pressure. Then the aqueous phase was basified until pH 7-8 with NaHCO₃ sat. sol. and was extracted with Me-THF. Organic layer was dried to afford the title compound (52 mg, 0.107, 79% yield).
LC-MS (ESI, m/z): method 3, t*_{R}* = 1.81 min, m/z (M+1) = 486.2
¹H NMR (acetone-d₆, 400 MHz) δ 9.90 (br s, 1H), 9.34 (br s, 1H), 9.15 (s, 1H), 8.73 (s, 1H), 8.65 (s, 1H), 8.17 (d, 1H, J=9.0 Hz), 7.95 (d, 1H, J=3.1 Hz), 7.43 (dd, 1H, J=1.5, 7.7 Hz), 7.37 (dd, 1H, J=3.1, 9.0 Hz), 7.20 (dd, 2H, J=3.1, 4.6 Hz), 5.2-5.2 (m, 1H), 4.5-4.6 (m, 2H), 3.99 (dd, 2H, J=4.6, 8.8 Hz), 2.31 (s, 3H), 2.16 (s, 3H)

### Example 46 - 3-(3-((6-(2-methoxyacetamido)pyridin-3-yl)oxy)azetidin-1-yl)-4-methyl-N-(5-(trifluoromethyl)pyridin-3-yl)benzamide

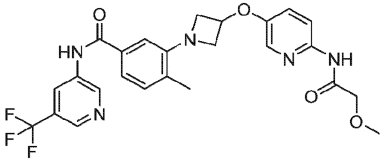

### Step 1 - 3-(3-((6-(2-methoxyacetamido)pyridin-3-yl)oxy)azetidin-1-yl)-4-methyl-N-(5-(trifluoromethyl)pyridin-3-yl)benzamide

Example 44 (60 mg, 0.135 mmol) was dissolved in pyridine (1.0 mL, 12.36 mmol) then methoxy acetyl chloride (22.03 mg, 0.203 mmol) was added in one portion and the solution was stirred at rt until completion. The crude was purified by FCC (gradient A:B from 100:0 to 0:10 in 12 CV eluent A: H₂O/ACN/HCOOH 95:5:0.1 Eluent B: H₂O/ACN/HCOOH 5:95:0.1). Relevant fractions were collected, and the organic solvent was evaporated by reduced pressure. The remaining aqueous phase was basified until pH 7-8 with NaHCO₃ and extracted with DCM. The organic layer was dried to afford the title compound (57.3 mg, 0.111 mmol, 82 % yield).
LC-MS (ESI, m/z): method 4, t*_{R}* = 6.79 min, m/z (M+1) = 516.2
¹H NMR (acetone-d₆, 400 MHz) δ 9.88 (br s, 1H), 9.13 (s, 1H), 8.81 (br s, 1H), 8.71 (s, 1H), 8.63 (s, 1H), 8.16 (d, 1H, J=9.0 Hz), 7.98 (d, 1H, J=2.9 Hz), 7.4-7.4 (m, 2H), 7.18 (dd, 2H, J=3.1, 4.6 Hz), 5.2-5.2 (m, 1H), 4.52 (dd, 2H, J=6.9, 8.0 Hz), 4.02 (s, 2H), 3.98 (dd, 2H, J=4.5, 8.7 Hz), 3.49 (s, 3H), 2.29 (s, 3H)

### Example 47 - 3-(3-((5-aminopyridin-3-yl)oxy)azetidin-1-yl)-4-methyl-N-(5-(trifluoromethyl)pyridin-3-yl)benzamide

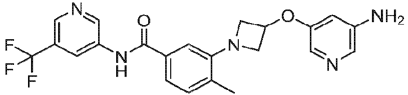

### Step 1 - tert-butyl 3-((5-nitropyridin-3-yl)oxy)azetidine-1-carboxylate (Intermediate 32)

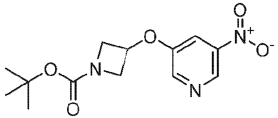

Intermediate 32 was prepared by following the same procedure described for the synthesis of Intermediate 29 and by varying the corresponding fluorine derivative.
SM: tert-butyl 3-hydroxyazetidine-1-carboxylate, 1.00 g (1.00 eq)
3-fluoro-5-nitropyridine: 0.902 g (1.1 eq)
Amount/Yield: 1.2 g/ 70%
LC-MS (ESI, m/z): method 1, t*_{R}* = 0.99 min, m/z (M+1) = 240.0

### Step 2: 3-(azetidin-3-yloxy)-5-nitropyridine hydrochloride (Intermediate 33)

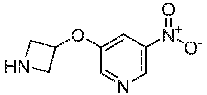

Intermediate 33 was prepared by following the same procedure described for the synthesis of Intermediate 30.
SM: Intermediate 32 g, 1.20 (1.00 eq)
Amount/Yield: 0.94 g/ 100%
LC-MS (ESI, m/z): method 1, t*_{R}* = 0.14 min, m/z (M+1) = 237.0

### Step 3: 4-methyl-3-(3-((5-nitropyridin-3-yl)oxy)azetidin-1-yl)-N-(5-(trifluoromethyl) pyridin-3-yl)benzamide (Intermediate 34)

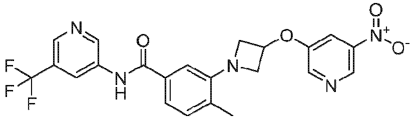

Intermediate 34 was prepared following the same procedure described for the synthesis of Intermediate 31.
SM: Intermediate 8, 300 mg (1.00 eq)
Intermediate 33, 290 mg (1.5 eq)
Amount/Yield: 230 mg/ 58%
LC-MS (ESI, m/z): method 3, t*_{R}* = 2.16 min, m/z (M+1) = 474.2

### Step 4 - 3-(3-((5-aminopyridin-3-yl)oxy)azetidin-1-yl)-4-methyl-N-(5-(trifluoromethyl) pyridin-3-yl)benzamide (Example 47)

Example 47 was prepared following the same procedure described for the synthesis of Example 42, step 2.
SM: Intermediate 34, 230 mg (1.00 eq)
Amount/Yield: 82 mg/ 38 %
LC-MS (ESI, m/z): method 3, t*_{R}* =1.13 min, m/z (M+1) = 444.1
¹H NMR (acetone-d₆, 400 MHz) δ 9.89 (br s, 1H), 9.13 (d, 1H, J=2.0 Hz), 8.7-8.7 (m, 1H), 8.63 (s, 1H), 7.72 (d, 1H, J=2.2 Hz), 7.52 (d, 1H, J=2.4 Hz), 7.41 (dd, 1H, J=1.6, 7.8 Hz), 7.2-7.2 (m, 2H), 6.57 (t, 1H, J=2.3 Hz), 5.1-5.1 (m, 1H), 4.90 (br s, 2H), 4.4-4.5 (m, 2H), 3.93 (dd, 2H, J=4.6, 8.6 Hz), 2.29 (s, 3H)

The following Examples and Intermediates were prepared using the procedure described for the synthesis of Intermediate 18 starting from the appropriate Intermediate.

| **Example N°** | **Structure** | **Product Amount (Yield)** |
|---|---|---|
| | **Analytical Data** | |
| **132** | 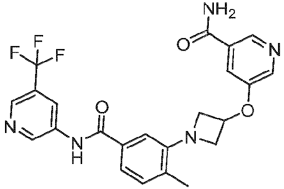 | 110 mg (35%) |
| | LC-MS (ESI): Method 9, t*_{R}* = 2.61 min, m/z (M+1) = 472.18 | |
| | ¹H NMR (300 MHz, DMSO-d₆) δ 10.60 (s, 1H), 9.19 (d, J = 2.3 Hz, 1H), 8.69 (d, J = 1.7 Hz, 2H), 8.61 (d, J = 2.3 Hz, 1H), 8.43 (d, J = 2.8 Hz, 1H), 8.18 (s, 1H), 7.69 (dd, J = 2.9, 1.7 Hz, 1H), 7.66 (s, 1H), 7.39 (dd, J = 7.7, 1.7 Hz, 1H), 7.21 (d, J = 7.8 Hz, 1H), 7.10 (d, J = 1.7 Hz, 1H), 5.37 - 5.19 (m, 1H), 4.57 - 4.44 (m, 2H), 3.97 (dd, J = 8.7, 4.2 Hz, 2H), 2.26 (s, 3H). | |

The following Examples and Intermediates were prepared using the procedure described for the synthesis of Example 130 (acetyl chloride) starting from the appropriate Intermediate.

| **Example N°** | **Structure** | **Product Amount (Yield)** |
|---|---|---|
| | **Analytical Data** | |
| **133** | 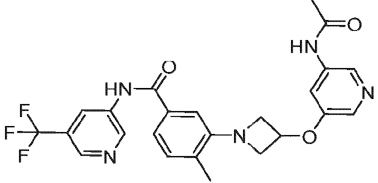 | 35 mg (93%) |
| | LC-MS (ESI): Method 4, t*_{R}* = 5.98 min, m/z (M+1) = 486.30 | |
| | ¹H NMR (ACN-d₃, 400 MHz) δ 9.05 (d, 1H, J=2.0 Hz), 9.00 (br s, 1H), 8.64 (s, 1H), 8.59 (s, 1H), 8.48 (br s, 1H), 8.26 (d, 1H, J=2.0 Hz), 7.96 (d, 1H, J=2.6 Hz), 7.74 (t, 1H, J=2.2 Hz), 7.36 (dd, 1H, J=1.6, 7.8 Hz), 7.20 (d, 1H, J=7.9 Hz), 7.07 (d, 1H, J=1.5 Hz), 5.1-5.2 (m, 1H), 4.48 (dd, 2H, J=7.0, 7.9 Hz), 4.01 (dd, 2H, J=4.4, 8.6 Hz), 2.29 (s, 3H), 2.10 (s, 3H) | |

The following compounds of formula (I) were prepared by applying the experimental conditions described above:
(*S*)-3-(1-(4-((dimethylamino)methyl)pyridin-3-yl)pyrrolidin-3-yl)-4-methyl-*N*-(5-(trifluoromethyl)pyridin-3-yl)benzamide;
(S)-4-methyl-3-(1-(pyrimidin-5-yl)pyrrolidin-3-yl)-N-(3-(trifluoromethyl)-1,2,4-oxadiazol-5-yl)benzamide;
(S)-4-(3-(2-methyl-5-((5-(trifluoromethyl)pyridin-3-yl)carbamoyl)phenyl)pyrrolidin-1-yl)pyrimidine-2-carboxamide;
(S)-N-methyl-3-(3-(2-methyl-5-((5-(trifluoromethyl)pyridin-3-yl)carbamoyl)phenyl) pyrrolidin-1-yl)isonicotinamide;
(S)-4-methyl-3-(1-(3-(methylamino)pyrazin-2-yl)pyrrolidin-3-yl)-N-(5-(trifluoromethyl) pyridin-3-yl)benzamide;
(S)-3-(1-(8-aminoimidazo[1,2-a]pyridin-3-yl)pyrrolidin-3-yl)-4-methyl-N-(5-(trifluoromethyl)pyridin-3-yl)benzamide;
(S)-3-(1-(6-cyanopyrimidin-4-yl)pyrrolidin-3-yl)-4-methyl-N-(5-(trifluoromethyl)pyridin-3-yl)benzamide;
(S)-4-methyl-3-(1-(1-methyl-1H-imidazol-5-yl)pyrrolidin-3-yl)-N-(5-(trifluoromethyl)pyridin-3-yl)benzamide;
(S)-3-(1-(5,6-dihydro-8H-imidazo[2,1-c][1,4]oxazin-3-yl) pyrrolidin-3-yl)-4-methyl-N-(5-(trifluoromethyl)pyridin-3-yl)benzamide;
(S)-3-(1-(4-cyanopyrimidin-5-yl)pyrrolidin-3-yl)-N-(4-(difluoromethoxy)pyridin-2-yl)-4-methylbenzamide;
(S)-5-(3-(2-methyl-5-((5-(trifluoromethyl)pyridin-3-yl) carbamoyl)phenyl)pyrrolidin-1-yl)-N-(2-(4-methylpiperazin-1-yl)ethyl)nicotinamide;
(S)-3-(1-(6-bromo-5H-pyrrolo[3,2-d]pyrimidin-4-yl)pyrrolidin-3-yl)-4-methyl-N-(5-(trifluoromethyl)pyridin-3-yl)benzamide;
3-((1-(imidazo[1,5-a]pyrazin-8-yl)azetidin-3-yl)oxy)-4-methyl-N-(5-(trifluoromethyl) pyridin-3-yl)benzamide;
(S)-4-methyl-3-(1-(pyrimidin-4-yl)pyrrolidin-3-yl)-N-(5-(trifluoromethyl)pyridin-3-yl)benzamide;
(S)-N-methyl-4-(3-(2-methyl-5-((5-(trifluoromethyl)pyridin-3-yl)carbamoyl)phenyl) pyrrolidin-1-yl)thieno[3,2-d]pyrimidine-7-carboxamide;
(S)-N-methyl-4-(3-(2-methyl-5-((5-(trifluoromethyl)pyridin-3-yl)carbamoyl)phenyl) pyrrolidin-1-yl)-5H-pyrrolo[3,2-d]pyrimidine-6-carboxamide;
(S)-4-methyl-3-(1-(2-(methylamino)thieno[3,2-d]pyrimidin-4-yl)pyrrolidin-3-yl)-N-(5-(trifluoromethyl)pyridin-3-yl)benzamide;
(S)-N-methyl-4-(3-(2-methyl-5-((5-(trifluoromethyl)pyridin-3-yl)carbamoyl)phenyl) pyrrolidin-1-yl)-1H-pyrazolo[3,4-d]pyrimidine-3-carboxamide;
(S)-N-methyl-4-(3-(2-methyl-5-((5-(trifluoromethyl)pyridin-3-yl)carbamoyl)phenyl) pyrrolidin-1-yl)thieno[2,3-d]pyrimidine-6-carboxamide;
4-hydroxy-3-(1-(pyrimidin-5-yl)pyrrolidin-3-yl)-N-(5-(trifluoromethyl)pyridin-3-yl)benzamide;
3-(1-(2-cyanopyridin-4-yl)pyrrolidin-3-yl)-4-methoxy-N-(5-(trifluoromethyl)pyridin-3-yl)benzamide;
4-(hydroxymethyl)-3-(1-(pyrimidin-5-yl)pyrrolidin-3-yl)-N-(5-(trifluoromethyl)pyridin-3-yl)benzamide;
4-cyclopropyl-3-(1-(pyrimidin-5-yl)pyrrolidin-3-yl)-N-(5-(trifluoromethyl)pyridin-3-yl)benzamide;
4-cyclopropyl-3-(1-(pyrazolo[1,5-a]pyrimidin-3-yl)pyrrolidin-3-yl)-N-(5-(trifluoromethyl) pyridin-3-yl)benzamide;
4-hydroxy-3-(1-(pyrimidin-5-yl)pyrrolidin-3-yl)-N-(5-(trifluoromethyl)pyridin-3-yl)benzamide, 1^{st} eluted;
4-hydroxy-3-(1-(pyrimidin-5-yl)pyrrolidin-3-yl)-N-(5-(trifluoromethyl)pyridin-3-yl)benzamide, 2^{nd} eluted;
3-(4-hydroxy-1-(pyrimidin-5-yl)pyrrolidin-3-yl)-4-methyl-N-(5-(trifluoromethyl)pyridin-3-yl)benzamide, 1^{st} eluted;
4-cyclopropyl-3-(1-(pyrimidin-5-yl)pyrrolidin-3-yl)-N-(5-(trifluoromethyl)pyridin-3-yl)benzamide, 1^{st} eluted;
4-cyclopropyl-3-(1-(pyrimidin-5-yl)pyrrolidin-3-yl)-N-(5-(trifluoromethyl)pyridin-3-yl)benzamide, 2^{nd} eluted;
4-cyclopropyl-3-(1-(pyrazolo[1,5-a]pyrimidin-3-yl)pyrrolidin-3-yl)-N-(5-(trifluoromethyl) pyridin-3-yl)benzamide, 1^{st} eluted; and
4-cyclopropyl-3-(1-(pyrazolo[1,5-a]pyrimidin-3-yl)pyrrolidin-3-yl)-N-(5-(trifluoromethyl) pyridin-3-yl)benzamide, 2^{nd} eluted.

Comparative newly synthesized compounds, characterized by having a -CH₂- linker between L and heteroaryl ring B (C1) or characterized by the replacement of heteroaryl ring B with an alkyl group (C2), were prepared as following:

### Example C1: (S)-3-(1-(imidazo[1,2-a]pyridin-3-ylmethyl)pyrrolidin-3-yl)-4-methyl-N-(5 (trifluoromethyl)pyridin-3-yl)benzamide

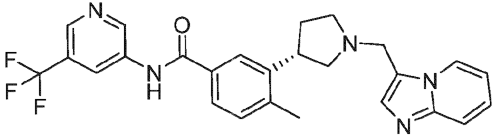

### Step 1 - (S)-3-(1-(imidazo[1,2-a]pyridin-3-ylmethyl)pyrrolidin-3-yl)-4-methyl-N-(5 (trifluoromethyl)pyridin-3-yl)benzamide (Example C1)

Intermediate 22 (100 mg, 0.286 mmol, 1.00 eq) was added to a solution of imidazo[1,2-*a*]pyridine-3-carbaldehyde (42 mg, 0.286 mmol, 1.00 eq) and titanium(IV) isopropoxide (0.25 mL, 0.859 mmol, 3.00 eq) in MeOH (3.00 mL) and the reaction mixture was stirred at 70 °C for 2 h. The reaction mixture was then allowed to cool to rt, NaBH₃CN (45 mg, 0.716 mmol, 2.50 eq) was added and the resulting mixture was stirred at rt overnight. It was then diluted with EtOAc (20 mL) and quenched with NH₄Cl (aq. sat., 20 mL), the precipitate was filtered off and the layers separated. The organic phase was washed with water (20 mL), HzO:brine (1:1) (20 mL) and brine (sat aq., 20 mL) then dried over MgSO₄, filtered and concentrated *in* vacuo. The residue was purified by preparative HPLC to give the title compound (67 mg, 0.129 mmol, 45%).
LC-MS (ESI, m/z): method 5, t*_{R}* = min, m/z (M+1) = 480.2

¹H NMR (400 MHz, DMSO-d₆) δ 10.63 (s, 1H), 9.20 (d, J=2.3 Hz, 1H), 8.72 - 8.71 (m, 1H), 8.63 (t, J=2.0 Hz, 1H), 8.53 (d, J=6.8 Hz, 1H), 7.96 (d, J=1.8 Hz, 1H), 7.72 (dd, J=1.8, 7.8 Hz, 1H), 7.52 (d, J=7.3 Hz, 2H), 7.30 (d, J=8.1 Hz, 1H), 7.17 - 7.12 (m, 1H), 6.84 - 6.80 (m, 1H), 4.08 - 3.96 (m, 2H), 3.62 - 3.54 (m, 1H), 2.89 - 2.76 (m, 2H), 2.71 - 2.57 (m, 2H), 2.35 (s, 3H), 2.33 - 2.29 (m, 1H), 1.78 - 1.69 (m, 1H).

### Example C2: (S)-3-(1-(2-amino-2-oxoethyl)pyrrolidin-3-yl)-4-methyl-N-(5-(trifluoromethyl)pyridin-3-yl)benzamide (Example C2)

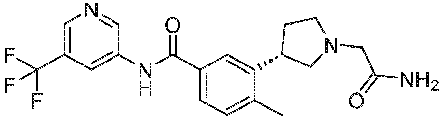

To a suspension of Intermediate 22 (50 mg, 0.143 mmol, 1.00 eq) and K₂CO₃ (30 mg, 0.215 mmol, 1.50 eq) in ACN (2.00 mL) 2-bromoacetamide (20 mg, 0.143 mmol, 1.00 eq) was added and the reaction mixture was stirred at rt for 3 days. The reaction mixture was diluted with EtOAc (15 mL) and washed with water (2×10 mL) and brine (sat. aq., 15 mL). The organic phase was dried over MgSO₄ and concentrated *in vacuo.* The residue was purified by preparative HPLC to give the title compound (31 mg, 0.0763 mmol, 53%).
LC-MS (ESI, m/z): method 8, t*_{R}* = 3.98 min, m/z (M+1) = 407.4
¹H NMR (400 MHz, DMSO-d₆) δ 10.67 (s, 1H), 9.22 (d, J=2.3 Hz, 1H), 8.71 (s, 1H), 8.66 (t, J=2.3 Hz, 1H), 8.03 (d, J=1.8 Hz, 1H), 7.77 (dd, J=1.8, 7.8 Hz, 1H), 7.35 (d, J=8.0 Hz, 1H), 7.28 - 7.23 (m, 1H), 7.18 - 7.14 (m, 1H), 3.65 - 3.57 (m, 1H), 3.17 - 3.05 (m, 2H), 2.98 (t, J=8.4 Hz, 1H), 2.90 - 2.69 (m, 3H), 2.41 (s, 3H), 2.37 - 2.28 (m, 1H), 1.87 - 1.77 (m, 1H).

### PHARMACOLOGICAL ACTIVITY OF THE COMPOUNDS OF THE INVENTION

### In vitro Assays

### Binding Assays

DDR1 and DDR2 binding assays were performed using Life Technologies LanthaScreen^{™} Europium Kinase Binding assay. The compounds were incubated with 5 nM DDR1 (Carna Biosciences) or 5 nM DDR2 (Life Technologies) for 1 h at rt in white 384-well OptiPlate (PerkinElmer), containing 20 nM or 10 nM Kinase Tracer 178 respectively and 2 nM Europium labelled anti-GST antibody (Life Technologies) in assay buffer (50 mM HEPES pH 7.5, 10 mM MgCI2, 1 mM EGTA and 0.01% BRIJ35). The ratio of fluorescence emission 665 nm/ 615 nm after excitation at 340 nm was obtained using the Tecan Spark 20M plate reader. IC₅₀ values were determined in GraphPad Prism 7.0 software, using 4 parameter model: log(inhibitor) vs. response. IC50 values were converted in Ki using the Cheng-Prusoff equation (Ki=IC50/(1+[Tracer]/Kd).

The results for individual compounds are provided herebelow in Table 3, wherein the compounds are classified in term of potency (nM) in binding with respect to their inhibitory activity on DDR1 and DDR2:

**Table 3**

| **Example N°** | **Ki DDR1 (nM)** | **Ki DDR2 (nM)** |
|---|---|---|
| **1** | +++ | +++ |
| **2** | +++ | ++ |
| **3** | +++ | +++ |
| **4** | +++ | +++ |
| **5** | +++ | ++ |
| **6** | +++ | +++ |
| **7** | +++ | ++ |
| **8** | +++ | ++ |
| **9** | +++ | +++ |
| **10** | +++ | ++ |
| **11** | +++ | +++ |
| **12** | +++ | +++ |
| **13** | +++ | +++ |
| **14** | +++ | +++ |
| **15** | +++ | +++ |
| **16** | +++ | +++ |
| **17** | +++ | ++ |
| **18** | +++ | +++ |
| **19** | +++ | +++ |
| **20** | +++ | ++ |
| **21** | +++ | +++ |
| **22** | +++ | ++ |
| **23** | +++ | ++ |
| **24** | +++ | +++ |
| **25** | +++ | ++ |
| **26** | +++ | +++ |
| **27** | +++ | +++ |
| **28** | +++ | +++ |
| **29** | +++ | +++ |
| **30** | +++ | ++ |
| **31** | +++ | +++ |
| **32** | +++ | +++ |
| **33** | +++ | +++ |
| **34** | +++ | ++ |
| **35** | +++ | +++ |
| **36** | +++ | ++ |
| **37** | +++ | +++ |
| **38** | +++ | +++ |
| **39** | +++ | +++ |
| **40** | +++ | ++ |
| **41** | +++ | + |
| **42** | +++ | +++ |
| **43** | +++ | +++ |
| **44** | -+ | + |
| **45** | +++ | ++ |
| **46** | -+ | + |
| **47** | -+ | ++ |
| **51** | +++ | +++ |
| **52** | +++ | ++ |
| **53** | +++ | ++ |
| **54** | +++ | +++ |
| **55** | +++ | +++ |
| **56** | +++ | +++ |
| **57** | +++ | ++ |
| **58** | +++ | +++ |
| **59** | +++ | ++ |
| **60** | +++ | +++ |
| **61** | +++ | ++ |
| **62** | +++ | ++ |
| **63** | +++ | +++ |
| **64** | +++ | ++ |
| **65** | +++ | ++ |
| **66** | -+ | + |
| **67** | +++ | +++ |
| **68** | -+ | ++ |
| **69** | +++ | ++ |
| **70** | -+ | + |
| **71** | +++ | +++ |
| **72** | +++ | ++ |
| **73** | +++ | ++ |
| **74** | +++ | ++ |
| **75** | +++ | +++ |
| **76** | +++ | +++ |
| **77** | +++ | ++ |
| **78** | +++ | +++ |
| **79** | +++ | ++ |
| **80** | -+ | ++ |
| **81** | +++ | ++ |
| **82** | +++ | ++ |
| **83** | +++ | ++ |
| **84** | +++ | ++ |
| **85** | +++ | ++ |
| **86** | +++ | ++ |
| **87** | +++ | ++ |
| **88** | -+ | ++ |
| **89** | +++ | ++ |
| **90** | +++ | ++ |
| **91** | +++ | +++ |
| **92** | +++ | ++ |
| **93** | +++ | +++ |
| **94** | +++ | ++ |
| **95** | +++ | +++ |
| **96** | +++ | +++ |
| **97** | +++ | ++ |
| **98** | +++ | +++ |
| **99** | +++ | ++ |
| **100** | +++ | ++ |
| **101** | +++ | ++ |
| **102** | +++ | ++ |
| **103** | +++ | +++ |
| **104** | +++ | ++ |
| **105** | +++ | ++ |
| **106** | -+ | ++ |
| **107** | +++ | +++ |
| **108** | +++ | +++ |
| **109** | +++ | +++ |
| **110** | +++ | ++ |
| **111** | +++ | ++ |
| **112** | +++ | ++ |
| **113** | +++ | +++ |
| **114** | +++ | ++ |
| **115** | +++ | ++ |
| **116** | +++ | +++ |
| **117** | +++ | +++ |
| **118** | +++ | ++ |
| **119** | +++ | ++ |
| **120** | -+ | + |
| **121** | +++ | +++ |
| **122** | +++ | ++ |
| **123** | +++ | +++ |
| **124** | +++ | ++ |
| **125** | -+ | ++ |
| **126** | +++ | ++ |
| **127** | +++ | ++ |
| **128** | +++ | ++ |
| **129** | +++ | +++ |
| **130** | +++ | ++ |
| **131** | +++ | +++ |
| **132** | +++ | ++ |
| **133** | +++ | +++ |

| | | |
|---|---|---|
| +: Ki between 25 and 100 nM ++: Ki between 5 nM and 25 nM +++: Ki lower than 5 nM | | |

As it can be appreciated, the compounds of Table 3, i.e. compounds according to the invention, show a good activity as antagonists of DDR1 and DDR2. Accordingly, the compounds of the invention can be effectively used for treating diseases, disorders or conditions associated with DDR receptors, such as fibrosis, e.g. pulmonary fibrosis, idiopathic pulmonary fibrosis (IPF), hepatic fibrosis, renal fibrosis, ocular fibrosis, cardiac fibrosis, arterial fibrosis and systemic sclerosis.

### Comparative Examples

Compounds of the Examples C1 and C2 were tested in the same binding assay described above.

**Table 4**

| **Example N°** | **DDR1 Ki (nM)** | **DDR2 Ki (nM)** |
|---|---|---|
| C1 | 148 | 588 |
| C2 | 487 | 588 |

The compounds of the present invention, as shown in Table 3, have a binding affinity for DDR1 and DDR2 receptors expressed as Ki lower than 100 nM, and, for most of the compounds, lower than 25 nM or even lower than 5 nM. To the contrary, comparative Example C1 has a binding affinity higher than 140 nM on DDR1 receptor, and even of 588 nM on DDR2; and Example C2 has a binding affinity higher than 480 nM on DDR1 receptor, and even of 588 nM on DDR2.

Thus, both the presence of a direct link between L and heteroaryl ring B and the presence of said heteroaryl ring B in the compounds of the present invention unexpectedly and noteworthy determines a remarkable increase in the inhibitory activity on the DDR1 and DDR2 receptors.

## Claims

1. A compound of formula (I) 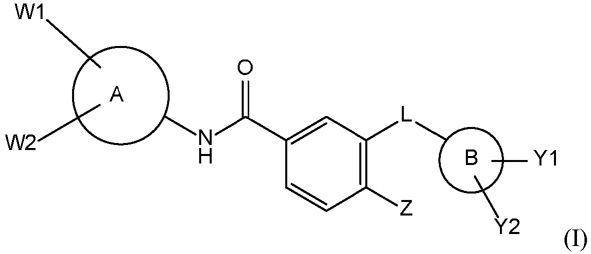 wherein
A is a ring selected from the group consisting of : 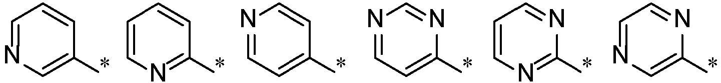 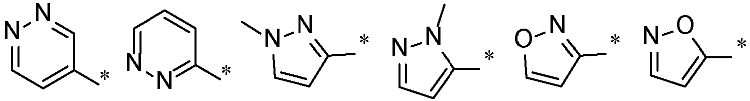 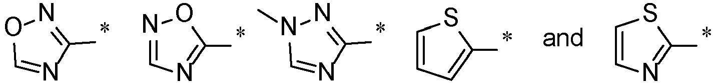 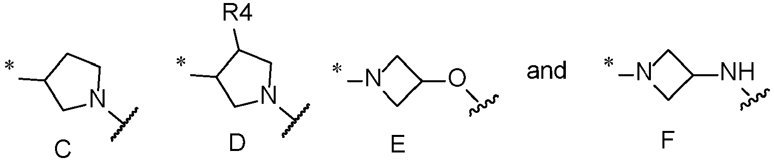
wherein 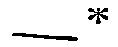 indicates a direct bond to the phenyl ring and 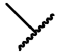 indicates a direct bond to B;
B is mono- or bi-cyclic heteroaryl ring;
Y1 and Y2 are substituents of ring B independently selected from the group consisting of hydrogen, (C₁-C₄)alkyl, (C₁-C₄)haloalkyl, (C₁-C₄)alkoxy, (C₁-C₄)alkoxy-(C₁-C₄)alkoxy, hydroxy-(C₁-C₄)alkoxy, hydroxy-(C₁-C₄)alkyl, (C₁-C₄)alkyl-heterocycloalkyl-(C₀-C₄)alkoxy, (C₁-C₄)haloalkoxy, halogen, cyano, cyano-(C₁-C₄)alkyl, cyano-(C₁-C₄)alkyl-heterocycloalkyl, CONR1R2, NHCOR1, NR1R2, R1R2N-(C₁-C₄)alkyl, heterocycloalkyl, (C₁-C₄)alkyl-heterocycloalkyl, heterocycloalkyl-(C₁-C₄)alkyl, (C₁-C₄)alkyl-heterocycloalkyl-carbonyl, (C₀-C₄)alkyl-heterocycloalkyl-carbonyl (C₁-C₄)alkyl-phenyl and monocyclic (C₁-C₄)alkyl-heteroaryl;
R1 and R2 are independently selected from the group consisting of hydrogen, (C₁-C₄)alkyl, (C₁-C₄)hydroxyalkyl, (C₁-C₄)alkoxy(C₁-C₄)alkyl, (C₁-C₄)alkylamino-(C₁-C₄)alkyl, di-(C₁-C₄)alkylamino-(C₁-C₄)alkyl, optionally substituted (C₃-C₆)cycloalkyl, optionally substituted heterocycloalkyl and optionally substituted heterocycloalkyl-(C₁-C₄)alkoxy, wherein optional substituents are one or more and are selected from (C₁-C₄)alkyl, (C₁-C₄)alkoxy, (C₁-C₄)haloalkyl, hydroxy and (C₁-C₄)haloalkoxy;
R4 is selected from hydroxy and hydroxymethyl;
or a stereoisomer, tautomer, solvate and pharmaceutically acceptable salt thereof.

2. The compound of formula (I) according to claim 1, wherein
L is selected from the group consisting of 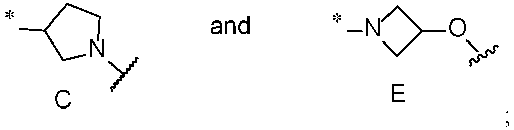
or a stereoisomer, tautomer, solvate and pharmaceutically acceptable salt thereof.

3. The compound of formula (I) according to claim 1 or 2, wherein
A is a ring selected from the group consisting of: 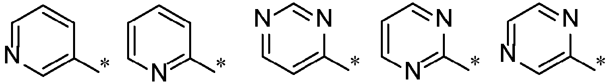 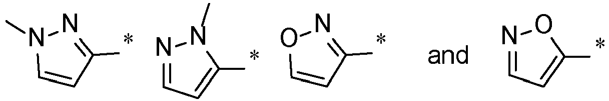
W1 and W2 are independently selected from the group consisting of H, F, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ and cyclopropyl;
Z is selected from the group consisting of CH₃, CF₂H, OCH₃, CH₂OH and cyclopropyl;
B is selected from the group consisting of pyridinyl, pyrimidinyl, pyrazinyl, pyrazolo[1,5-a]pyrazinyl, 1H-pyrazolo[3,4-b]pyridinyl, pyrazolo[1,5-a]pyridinyl, pyrazolo[1,5-a]pyrimidinyl, imidazo[1,2-b]pyridazinyl, imidazo[1,2-a]pyrazinyl, imidazo[1,2-a]pyridinyl, thieno[3,2-d]pyrimidinyl, 1H-pyrrolo[2,3-b]pyridinyl and pyrazolyl;
Y1 is selected from the group consisting of hydrogen, (C₁-C₄)haloalkyl, (C₁-C₄)alkoxy, cyano, CONR1R2, NHCOR1, NR1R2, (C₁-C₄)alkyl-heterocycloalkyl-carbonyl and monocyclic (C₁-C₄)alkyl-heteroaryl; and Y2 is hydrogen;
R1 and R2 are independently selected from the group consisting of CH₂CH₂N(CH₃)₂, CH₂CH₂OCH₃, CH₃, CH₂CH₂OH, oxetan-3-yl and 3-hydroxycyclobutyl;
or a stereoisomer, tautomer, solvate and pharmaceutically acceptable salt thereof.

4. The compound of formula (I) according to claim 3, wherein
Y1 is selected from the group consisting of CONH₂, CF₂H, OCH₃, cyano, NHCOCH₃, NH₂, 4-methylpiperazine-1-carbonyl, 4-methylpiperazin-1-yl and 1-methyl-1H-pyrazol-4-yl; and Y2 is hydrogen;
or a stereoisomer, tautomer, solvate and pharmaceutically acceptable salt thereof.

5. The compound according to any one of the preceding claims, wherein L is C 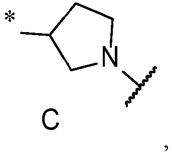 represented by formula (Ia) 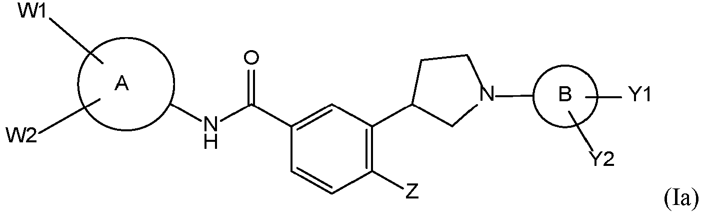 or a stereoisomer, tautomer, solvate and pharmaceutically acceptable salt thereof.

6. The compound according to any of the preceding claims, wherein L or C is C* 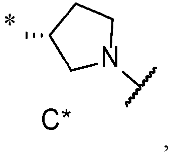 or a stereoisomer, tautomer, solvate and pharmaceutically acceptable salt thereof.

7. The compound according to any one of claims 1 to 4, wherein L is 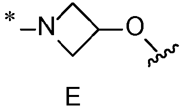 and which is referred to as a compound of formula (Ib1).

8. The compound of formula (Ia) according to claim 5 selected from the group consisting of:
(S)-5-(3-(2-methyl-5-((5-(trifluoromethyl)pyridin-3-yl)carbamoyl)phenyl)pyrrolidin-1-yl)nicotinamide (Example 1);
(R)-5-(3-(2-methyl-5-((5-(trifluoromethyl)pyridin-3-yl)carbamoyl)phenyl)pyrrolidin-1-yl)nicotinamide (Example 2);
(S)-5-(3-(5-((5-(tert-butyl)-1-methyl-1H-pyrazol-3-yl)carbamoyl)-2-methylphenyl)pyrrolidin-1-yl)nicotinamide (Example 3);
(S)-5-(3-(2-methyl-5-((5-(1,1,1-trifluoro-2-methylpropan-2-yl)isoxazol-3-yl)carbamoyl)phenyl)pyrrolidin-1-yl)nicotinamide (Example 4);
(S)-5-(3-(5-((3-(tert-butyl)-1-methyl-1H-pyrazol-5-yl)carbamoyl)-2-methylphenyl)pyrrolidin-1-yl)nicotinamide (Example 5);
(S)-5-(3-(5-((3-(tert-butyl)isoxazol-5-yl)carbamoyl)-2-methylphenyl)pyrrolidin-1-yl)nicotinamide (Example 6);
(S)-N-(3-(tert-butyl)isoxazol-5-yl)-3-(1-(5-cyanopyridin-3-yl)pyrrolidin-3-yl)-4-methylbenzamide (Example 7);
(S)-3-(1-(5-cyanopyridin-3-yl)pyrrolidin-3-yl)-4-methyl-N-(6-(trifluoromethyl)pyrimidin-4-yl)benzamide (Example 8);
(S)-5-(3-(2-methyl-5-((4-(trifluoromethoxy)pyridin-2-yl)carbamoyl)phenyl)pyrrolidin-1-yl)nicotinamide (Example 9);
(S)-5-(3-(2-methyl-5-((5-(trifluoromethoxy)pyridin-2-yl)carbamoyl)phenyl)pyrrolidin-1-yl)nicotinamide (Example 10);
(S)-5-(3-(5-((5-(tert-butyl)isoxazol-3-yl)carbamoyl)-2-methylphenyl)pyrrolidin-1-yl)nicotinamide (Example 11);
(S)-4-methyl-3-(1-(pyrimidin-5-yl)pyrrolidin-3-yl)-N-(4-(trifluoromethoxy)pyridin-2-yl)benzamide (Example 12);
(S)-N-(5-(tert-butyl)-1-methyl-1H-pyrazol-3-yl)-4-methyl-3-(1-(pyrimidin-5-yl)pyrrolidin-3-yl)benzamide (Example 13);
(S)-4-methyl-3-(1-(pyrimidin-5-yl)pyrrolidin-3-yl)-N-(5-(1,1,1-trifluoro-2-methylpropan-2-yl)isoxazol-3-yl)benzamide (Example 14);
(S)-4-methyl-3-(1-(pyrimidin-5-yl)pyrrolidin-3-yl)-N-(4-(trifluoromethyl)pyridin-2-yl)benzamide (Example 15);
(S)-N-(5-(tert-butyl)isoxazol-3-yl)-4-methyl-3-(1-(pyrimidin-5-yl)pyrrolidin-3-yl)benzamide (Example 16);
(S)-N-(3-(tert-butyl)-1-methyl-1H-pyrazol-5-yl)-4-methyl-3-(1-(pyrimidin-5-yl)pyrrolidin-3-yl)benzamide (Example 17);
(S)-4-methyl-3-(1-(pyrimidin-5-yl)pyrrolidin-3-yl)-N-(5-(trifluoromethoxy)pyridin-3-yl)benzamide (Example 18);
(S)-N-(3-(tert-butyl)isoxazol-5-yl)-4-methyl-3-(1-(pyrimidin-5-yl)pyrrolidin-3-yl)benzamide (Example 19);
(S)-4-methyl-3-(1-(pyrimidin-5-yl)pyrrolidin-3-yl)-N-(5-(trifluoromethoxy)pyridin-2-yl)benzamide (Example 20);
(S)-N-(4-(difluoromethoxy)pyridin-2-yl)-4-methyl-3-(1-(pyrimidin-5-yl)pyrrolidin-3-yl)benzamide (Example 21);
(S)-3-(1-(5-(difluoromethyl)pyridin-3-yl)pyrrolidin-3-yl)-4-methyl-N-(5-(trifluoromethyl)pyridin-3-yl)benzamide (Example 22);
(S)-N-(2-(dimethylamino)ethyl)-5-(3-(2-methyl-5-((5-(trifluoromethyl)pyridin-3-yl)carbamoyl)phenyl)pyrrolidin-1-yl)nicotinamide (Example 23);
(S)-N-(2-methoxyethyl)-5-(3-(2-methyl-5-((5-(trifluoromethyl)pyridin-3-yl)carbamoyl)phenyl)pyrrolidin-1-yl)nicotinamide (Example 24);
(S)-3-(1-(2-methoxypyrimidin-5-yl)pyrrolidin-3-yl)-4-methyl-N-(5-(trifluoromethyl) pyridin-3-yl)benzamide (Example 25);
(S)-4-methyl-3-(1-(pyrimidin-5-yl)pyrrolidin-3-yl)-N-(5-(trifluoromethyl)pyridin-3-yl)benzamide (Example 26);
(S)-3-(1-(5-cyanopyridin-3-yl)pyrrolidin-3-yl)-4-methyl-N-(5-(trifluoromethyl)pyridin-3-yl)benzamide (Example 27);
(S)-3-(1-(5-acetamidopyridin-3-yl)pyrrolidin-3-yl)-4-methyl-N-(5-(trifluoromethyl)pyridin-3-yl)benzamide (Example 28);
(S)-N-methyl-5-(3-(2-methyl-5-((5-(trifluoromethyl)pyridin-3-yl)carbamoyl)phenyl)pyrrolidin-1-yl)nicotinamide (Example 29);
(S)-N-(2-hydroxyethyl)-5-(3-(2-methyl-5-((5-(trifluoromethyl)pyridin-3-yl) carbamoyl)phenyl)pyrrolidin-1-yl)nicotinamide (Example 30);
(S)-3-(1-(3-aminopyrazin-2-yl)pyrrolidin-3-yl)-4-methyl-N-(5-(trifluoromethyl) pyridin-3-yl)benzamide (Example 31);
(S)-4-methyl-3-(1-(pyrazolo[1,5-a]pyrazin-3-yl)pyrrolidin-3-yl)-N-(5-(trifluoromethyl)pyridin-3-yl)benzamide (Example 32);
(S)-4-methyl-3-(1-(pyrazin-2-yl)pyrrolidin-3-yl)-N-(5-(trifluoromethyl)pyridin-3-yl)benzamide (Example 33);
(S)-4-methyl-3-(1-(pyrazolo[1,5-a]pyridin-3-yl)pyrrolidin-3-yl)-N-(5-(trifluoromethyl) pyridin-3-yl)benzamide (Example 34);
(S)-4-methyl-3-(1-(pyrazolo[1,5-a]pyrimidin-3-yl)pyrrolidin-3-yl)-N-(5-(trifluoromethyl)pyridin-3-yl)benzamide (Example 35);
(S)-3-(1-(imidazo[1,2-b]pyridazin-3-yl)pyrrolidin-3-yl)-4-methyl-N-(5-(trifluoromethyl)pyridin-3-yl)benzamide (Example 36);
(S)-3-(1-(imidazo[1,2-a]pyrazin-3-yl)pyrrolidin-3-yl)-4-methyl-N-(5-(trifluoromethyl) pyridin-3-yl)benzamide (Example 37);
(S)-5-(3-(2-methyl-5-((5-(trifluoromethyl)pyridin-3-yl)carbamoyl)phenyl)pyrrolidin-1-yl)-N-(oxetan-3-yl)nicotinamide (Example 38);
N-((1s,3R)-3-hydroxycyclobutyl)-5-((S)-3-(2-methyl-5-((5-(trifluoromethyl)pyridin-3-yl)carbamoyl)phenyl)pyrrolidin-1-yl)nicotinamide (Example 39);
(S)-4-methyl-3-(1-(5-(4-methylpiperazine-1-carbonyl)pyridin-3-yl)pyrrolidin-3-yl)-N-(5-(trifluoromethyl)pyridin-3-yl)benzamide (Example 40);
(S)-3-(3-(2-methyl-5-((5-(trifluoromethyl)pyridin-3-yl)carbamoyl)phenyl)pyrrolidin-1-yl)-N-(oxetan-3-yl)isonicotinamide (Example 41);
(S)-3-(1-(2-aminopyridin-3-yl)pyrrolidin-3-yl)-4-methyl-N-(5-(trifluoromethyl) pyridin-3-yl)benzamide (Example 42);
(S)-3-(1-(1H-pyrazolo[3,4-b]pyridin-5-yl)pyrrolidin-3-yl)-4-methyl-N-(5-(trifluoromethyl)pyridin-3-yl)benzamide (Example 43);
(*S*)-6-(3-(5-((4-(difluoromethoxy)pyridin-2-yl)carbamoyl)-2-methylphenyl)pyrrolidin-1-yl) pyrazine-2-carboxamide (Example 51);
(S)-4-(3-(5-((5-(tert-butyl)-1-methyl-1H-pyrazol-3-yl)carbamoyl)-2-methylphenyl) pyrrolidin-1-yl)picolinamide (Example 52);
(S)-4-(3-(5-((2-((dimethylamino)methyl)-6-(trifluoromethyl)pyridin-4-yl)carbamoyl)-2-methylphenyl)pyrrolidin-1-yl)picolinamide (Example 53);
(S)-6-(3-(2-methyl-5-((5-(trifluoromethyl)pyridin-3-yl)carbamoyl)phenyl)pyrrolidin-1-yl)pyrimidine-4-carboxamide (Example 54);
(S)-3-(1-(4-cyanopyridin-3-yl)pyrrolidin-3-yl)-4-methyl-N-(5-(1,1,1-trifluoro-2-methylpropan-2-yl)isoxazol-3-yl)benzamide (Example 55);
(S)-3-(1-(4-cyanopyridin-3-yl)pyrrolidin-3-yl)-N-(5-ethylisoxazol-3-yl)-4-methylbenzamide (Example 56);
(S)-4-(3-(2-methyl-5-((4-(trifluoromethoxy)pyridin-2-yl)carbamoyl)phenyl)pyrrolidin-1-yl)picolinamide (Example 57);
(*S*)-4-methyl-3-(1-(pyrazin-2-yl)pyrrolidin-3-yl)-*N*-(5-(1,1,1-trifluoro-2-methylpropan-2-yl)isoxazol-3-yl)benzamide (Example 58);
(*S*)-*N*-(3-cyclopropyl-1-methyl-1*H*-pyrazol-5-yl)-4-methyl-3-(1-(5-(1-methyl-1*H-*pyrazol-4-yl)pyridin-3-yl)pyrrolidin-3-yl)benzamide (Example 59);
(R)-4-methyl-3-(1-(5-(1-methyl-1H-pyrazol-4-yl)pyridin-3-yl)pyrrolidin-3-yl)-N-(5-(1,1,1-trifluoro-2-methylpropan-2-yl)isoxazol-3-yl)benzamide (Example 60);
(S)-4-methyl-3-(1-(6-(morpholine-4-carbonyl)thieno[3,2-d]pyrimidin-4-yl)pyrrolidin-3-yl)-N-(5-(trifluoromethyl)pyridin-3-yl)benzamide (Example 61);
(S)-4-methyl-3-(1-(pyrazolo[1,5-a]pyrimidin-3-yl)pyrrolidin-3-yl)-N-(5-(trifluoromethoxy)pyridin-3-yl)benzamide (Example 62);
(S)-4-(3-(2-methyl-5-((5-(trifluoromethyl)pyridin-3-yl)carbamoyl)phenyl)pyrrolidin-1-yl)picolinamide (Example 63);
4-(3-(2-methoxy-5-((5-(trifluoromethyl)pyridin-3-yl)carbamoyl)phenyl)pyrrolidin-1-yl)picolinamide (Example 64);
(S)-1-methyl-4-(3-(2-methyl-5-((5-(trifluoromethyl)pyridin-3-yl)carbamoyl)phenyl)pyrrolidin-1-yl)-1H-pyrazole-5-carboxamide (Example 65);
(S)-3-(1-(3-carbamoylphenyl)pyrrolidin-3-yl)-4-methyl-N-(5-(trifluoromethyl) pyridin-3-yl)benzamide (Example 66);
(S)-N-(4-(difluoromethyl)pyridin-2-yl)-4-methyl-3-(1-(pyrimidin-5-yl)pyrrolidin-3-yl)benzamide (Example 67);
(S)-4-methyl-N-(1-methyl-3-(trifluoromethyl)-1H-pyrazol-5-yl)-3-(1-(pyrimidin-5-yl)pyrrolidin-3-yl)benzamide (Example 68);
(S)-N-(5-ethylisoxazol-3-yl)-4-methyl-3-(1-(pyrimidin-5-yl)pyrrolidin-3-yl)benzamide (Example 69);
(S)-N-(3-cyclopropyl-1-methyl-1H-pyrazol-5-yl)-4-methyl-3-(1-(pyrimidin-5-yl)pyrrolidin-3-yl)benzamide (Example 70);
(S)-3-(1-(imidazo[1,2-a]pyridin-3-yl)pyrrolidin-3-yl)-4-methyl-N-(5-(trifluoromethyl)pyridin-3-yl)benzamide (Example 71);
(S)-4-methyl-3-(1-(6-(4-methylpiperazin-1-yl)imidazo[1,2-a]pyridin-3-yl)pyrrolidin-3-yl)-N-(5-(trifluoromethyl)pyridin-3-yl)benzamide (Example 72);
(S)-3-(1-(6-(4-(cyanomethyl)piperazin-1-yl)imidazo[1,2-a]pyridin-3-yl)pyrrolidin-3-yl)-4-methyl-N-(5-(trifluoromethyl)pyridin-3-yl)benzamide (Example 73);
(S)-4-methyl-3-(1-(5-(2-(4-methylpiperazin-1-yl)ethoxy)pyridin-3-yl)pyrrolidin-3-yl)-N-(5-(trifluoromethyl)pyridin-3-yl)benzamide (Example 74);
(S)-3-(1-(4-cyanopyridin-3-yl)pyrrolidin-3-yl)-4-methyl-N-(5-(trifluoromethyl) pyridin-3-yl)benzamide (Example 75);
(S)-3-(1-(4-methoxypyridin-3-yl)pyrrolidin-3-yl)-4-methyl-N-(5-(trifluoromethyl) pyridin-3-yl)benzamide (Example 76);
(S)-4-methyl-3-(1-(6-(morpholinomethyl)imidazo[1,2-a]pyridin-3-yl)pyrrolidin-3-yl)-N-(5-(trifluoromethyl)pyridin-3-yl)benzamide (Example 77);
(S)-3-(1-(6-cyanoimidazo[1,2-a]pyridin-3-yl)pyrrolidin-3-yl)-4-methyl-N-(5-(trifluoromethyl)pyridin-3-yl)benzamide (Example 78);
(S)-3-(1-(5-(2-methoxyethoxy)pyridin-3-yl)pyrrolidin-3-yl)-4-methyl-N-(5-(trifluoromethyl)pyridin-3-yl)benzamide (Example 79);
(S)-4-methyl-3-(1-(6-(4-methylpiperazin-1-yl)imidazo[1,2-b]pyridazin-3-yl)pyrrolidin-3-yl)-N-(5-(trifluoromethyl)pyridin-3-yl)benzamide (Example 80);
(S)-4-methyl-3-(1-(5-((1-methylpiperidin-4-yl)oxy)pyridin-3-yl)pyrrolidin-3-yl)-N-(5-(trifluoromethyl)pyridin-3-yl)benzamide (Example 81);
(S)-4-methyl-3-(1-(6-(4-methylpiperazin-1-yl)imidazo[1,2-a]pyrazin-3-yl)pyrrolidin-3-yl)-N-(5-(trifluoromethyl)pyridin-3-yl)benzamide (Example 82);
(S)-4-methyl-3-(1-(5-(4-methylpiperazin-1-yl)pyrazolo[1,5-a]pyrimidin-3-yl)pyrrolidin-3-yl)-N-(5-(trifluoromethyl)pyridin-3-yl)benzamide (Example 83);
(S)-4-methyl-3-(1-(6-morpholinoimidazo[1,2-b]pyridazin-3-yl)pyrrolidin-3-yl)-N-(5-(trifluoromethyl)pyridin-3-yl)benzamide (Example 84);
(S)-3-(1-(2-acetamidopyridin-4-yl)pyrrolidin-3-yl)-4-methyl-N-(5-(trifluoromethyl) pyridin-3-yl)benzamide (Example 85);
(S)-4-methyl-3-(1-(5-methylpyrazolo[1,5-a]pyrimidin-3-yl)pyrrolidin-3-yl)-N-(5-(trifluoromethyl)pyridin-3-yl)benzamide (Example 86);
(S)-N-methyl-4-(3-(2-methyl-5-((5-(trifluoromethyl)pyridin-3-yl)carbamoyl) phenyl)pyrrolidin-1-yl)picolinamide (Example 87);
(S)-4-methyl-3-(1-(1-methyl-1H-pyrazol-4-yl)pyrrolidin-3-yl)-N-(5-(trifluoromethyl) pyridin-3-yl)benzamide (Example 88);
(S)-4-methyl-3-(1-(5-(morpholine-4-carbonyl)pyrazolo[1,5-a]pyridin-3-yl)pyrrolidin-3-yl)-N-(5-(trifluoromethyl)pyridin-3-yl)benzamide (Example 89);
4-methyl-3-[(3R)-1-pyrazolo[1,5-a]pyrimidin-3-ylpyrrolidin-3-yl]-N-[5-(trifluoromethyl)-3-pyridyl]benzamide (Example 90);
N-[4-(difluoromethoxy)-2-pyridyl]-4-methyl-3-[(3S)-1-pyrazolo[1,5-a]pyrimidin-3-ylpyrrolidin-3-yl]benzamide (Example 91);
N-[4-(difluoromethoxy)-2-pyridyl]-4-methyl-3-[(3S)-1-pyrazin-2-ylpyrrolidin-3-yl]benzamide (Example 92);
3-[(3S)-1-(4-cyano-3-pyridyl)pyrrolidin-3-yl]-N-[4-(difluoromethoxy)-2-pyridyl]-4-methyl-benzamide (Example 93);
(S)-3-(1-(6-cyanopyrazin-2-yl)pyrrolidin-3-yl)-N-(4-(difluoromethoxy) pyridin-2-yl)-4-methyl-benzamide (Example 94);
(S)-N-(5-ethylisoxazol-3-yl)-4-methyl-3-(1-(pyrazolo[1,5-a]pyrimidin-3-yl)pyrrolidin-3-yl)benzamide (Example 95);
(S)-N-(5-ethylisoxazol-3-yl)-4-methyl-3-(1-(pyrazolo[1,5-a]pyrazin-3-yl)pyrrolidin-3-yl)benzamide (Example 96);
(S)-3-(1-(6-methoxypyrazolo[1,5-a]pyrimidin-3-yl)pyrrolidin-3-yl)-4-methyl-N-(5-(trifluoromethyl)pyridin-3-yl)benzamide (Example 97);
(S)-4-methyl-3-(1-(pyrazolo[1,5-a]pyrazin-3-yl)pyrrolidin-3-yl)-N-(5-(trifluoromethoxy)pyridin-3-yl)benzamide (Example 98);
(S)-4-methyl-3-(1-(2-(methylamino)pyrimidin-5-yl)pyrrolidin-3-yl)-N-(5-(trifluoromethyl)pyridin-3-yl)benzamide (Example 99);
(S)-3-(1-(1H-pyrazol-4-yl)pyrrolidin-3-yl)-4-methyl-N-(5-(trifluoromethyl)pyridin-3-yl)benzamide (Example 100);
(S)-3-(1-(3-((dimethylamino)methyl)-1H-pyrazolo[3,4-b]pyridin-5-yl)pyrrolidin-3-yl)-4-methyl-N-(5-(trifluoromethyl)pyridin-3-yl)benzamide (Example 101);
(S)-5-(3-(2-methyl-5-((5-(trifluoromethyl)pyridin-3-yl)carbamoyl)phenyl)pyrrolidin-1-yl)-N-(oxetan-3-yl)-1H-pyrrolo[2,3-b]pyridine-3-carboxamide (Example 102);
(S)-3-(1-(5-(2-hydroxyethoxy)pyridin-3-yl)pyrrolidin-3-yl)-4-methyl-N-(5-(trifluoromethyl)pyridin-3-yl)benzamide (Example 103);
3-(4-(hydroxymethyl)-1-(pyrimidin-5-yl)pyrrolidin-3-yl)-4-methyl-N-(5-(trifluoromethyl)pyridin-3-yl)benzamide (Example 104);
3-(4-hydroxy-1-(pyrimidin-5-yl)pyrrolidin-3-yl)-4-methyl-N-(5-(trifluoromethyl) pyridin-3-yl)benzamide (Example 105);
(S)-4-methyl-3-(1-(thieno[2,3-d]pyrimidin-4-yl)pyrrolidin-3-yl)-N-(5-(trifluoromethyl)pyridin-3-yl)benzamide (Example 106);
(S)-4-methyl-3-(1-(thieno[3,2-d]pyrimidin-4-yl)pyrrolidin-3-yl)-N-(5-(trifluoromethyl)pyridin-3-yl)benzamide (Example 107);
(S)-N-methyl-4-(3-(2-methyl-5-((5-(trifluoromethyl)pyridin-3-yl)carbamoyl) phenyl)pyrrolidin-1-yl)thieno[3,2-d]pyrimidine-6-carboxamide (Example 108);
4-methoxy-3-(1-(pyrimidin-5-yl)pyrrolidin-3-yl)-N-(5-(trifluoromethyl)pyridin-3-yl)benzamide, 1^{st} eluted enantiomer (Example 109);
4-methoxy-3-(1-(pyrimidin-5-yl)pyrrolidin-3-yl)-N-(5-(trifluoromethyl)pyridin-3-yl)benzamide, 2^{nd} eluted enantiomer (Example 110);
4-methoxy-3-(1-(pyrimidin-5-yl)pyrrolidin-3-yl)-N-(5-(trifluoromethyl) pyridin-3-yl) benzamide, racemic mixture (Example 111);
4-methoxy-3-(1-(pyrazolo[1,5-a]pyrimidin-3-yl)pyrrolidin-3-yl)-N-(5-(trifluoromethyl)pyridin-3-yl)benzamide (Example 112);
4-(difluoromethoxy)-3-(1-(pyrimidin-5-yl)pyrrolidin-3-yl)-N-(5-(trifluoromethyl)pyridin-3-yl)benzamide (Example 113);
4-methoxy-3-(1-(pyrazolo[1,5-a]pyrimidin-3-yl)pyrrolidin-3-yl)-N-(5-(trifluoromethyl)pyridin-3-yl)benzamide, 1^{st} eluted enantiomer (Example 114);
4-methoxy-3-(1-(pyrazolo[1,5-a]pyrimidin-3-yl)pyrrolidin-3-yl)-N-(5-(trifluoromethyl) pyridin-3-yl)benzamide, 2^{nd} eluted enantiomer (Example 115);
4-(difluoromethoxy)-3-(1-(pyrimidin-5-yl)pyrrolidin-3-yl)-N-(5-(trifluoromethyl) pyridin-3-yl)benzamide, 1st eluted enantiomer (Example 116);
4-(difluoromethoxy)-3-(1-(pyrimidin-5-yl)pyrrolidin-3-yl)-N-(5-(trifluoromethyl) pyridin-3-yl)benzamide, 2^{nd} eluted enantiomer(Example 117);
4-(3-(2-(difluoromethoxy)-5-((5-(trifluoromethyl)pyridin-3-yl)carbamoyl)phenyl) pyrrolidin-1-yl)picolinamide, 1^{st} eluted enantiomer (Example 118);
4-(3-(2-(difluoromethoxy)-5-((5-(trifluoromethyl)pyridin-3-yl)carbamoyl)phenyl) pyrrolidin-1-yl)picolinamide, 2^{nd} eluted enantiomer (Example 119);
4-(difluoromethyl)-3-(1-(pyrimidin-5-yl)pyrrolidin-3-yl)-N-(5-(trifluoromethyl) pyridin-3-yl)benzamide, (Example 120);
(1-(pyrimidin-5-yl)pyrrolidin-3-yl)-N-(5-(trifluoromethyl)pyridin-3-yl)benzamide, 2^{nd} eluted enantiomer (Example 121);
3-(4-hydroxy-1-(pyrimidin-5-yl)pyrrolidin-3-yl)-4-methyl-N-(5-(trifluoromethyl) pyridin-3-yl)benzamide, 2^{nd} eluted enantiomer (Example 122);
(S)-(3-(1-(6-(hydroxymethyl)imidazo[1,2-a]pyridin-3-yl)pyrrolidin-3-yl)-4-methylphenyl)(5-(trifluoromethyl)pyridin-3-yl)methanone (Example 123);
N-(5-(tert-butyl)-1-methyl-1H-pyrazol-3-yl)-4-(difluoromethyl)-3-(1-(pyrimidin-5-yl)pyrrolidin-3-yl)benzamide (Example 124);
(S)-3-(1-(6-bromothieno[3,2-d]pyrimidin-4-yl)pyrrolidin-3-yl)-4-methyl-N-(5-(trifluoromethyl)pyridin-3-yl)benzamide (Example 125);
(S)-4-methyl-3-(1-(6-(4-methylpiperazine-1-carbonyl)thieno[3,2-d]pyrimidin-4-yl) pyrrolidin-3-yl)-N-(5-(trifluoromethyl)pyridin-3-yl)benzamide, (Example 126);
4-(3-(2-methoxy-5-((5-(trifluoromethyl)pyridin-3-yl)carbamoyl)phenyl)pyrrolidin-1-yl)-N-methylpicolinamide, (Example 127);
(S)-3-(1-(imidazo[1,5-a]pyrazin-8-yl)pyrrolidin-3-yl)-4-methyl-N-(5-(trifluoromethyl) pyridin-3-yl)benzamide (Example 128);
(S)-3-(1-(1H-pyrazolo [3,4-d]pyrimidin-4-yl)pyrrolidin-3-yl)-4-methyl-N-(5-(trifluoromethyl)pyridin-3-yl)benzamide (Example 129);
(S)-3-(1-(2-acetamidopyrimidin-5-yl)pyrrolidin-3-yl)-4-methyl-N-(5-(trifluoromethyl) pyridin-3-yl)benzamide (Example 130); and
(S)-N-(5-(tert-butyl)-1-methyl-1H-pyrazol-3-yl)-4-methyl-3-(1-(thieno[3,2-d]pyrimidin-4-yl)pyrrolidin-3-yl)benzamide (Example 131).

9. The compound of formula (Ib1) according to claim 7, selected from the group consisting of:
3-(3-((6-aminopyridin-3-yl)oxy)azetidin-1-yl)-4-methyl-N-(5-(trifluoromethyl) pyridin-3-yl)benzamide (Example 44);
3-(3-((6-acetamidopyridin-3-yl)oxy)azetidin-1-yl)-4-methyl-N-(5-(trifluoromethyl) pyridin-3-yl)benzamide (Example 45);
3-(3-((6-(2-methoxyacetamido)pyridin-3-yl)oxy)azetidin-1-yl)-4-methyl-N-(5-(trifluoromethyl)pyridin-3-yl)benzamide (Example 46);
3-(3-((5-aminopyridin-3-yl)oxy)azetidin-1-yl)-4-methyl-N-(5-(trifluoromethyl) pyridin-3-yl)benzamide (Example 47);
5-((1-(2-methyl-5-((5-(trifluoromethyl)pyridin-3-yl)carbamoyl) phenyl)azetidin-3-yl)oxy)nicotinamide (Example 132); and
3-(3-((5-acetamidopyridin-3-yl)oxy)azetidin-1-yl)-4-methyl-N-(5-(trifluoromethyl) pyridin-3-yl)benzamide (Example 133).

10. A pharmaceutical composition comprising a compound of formula (I) according to any one of claims 1 to 9, in admixture with at least one or more pharmaceutically acceptable carrier and/or excipient.

11. The pharmaceutical composition according to claim 10 for administration by inhalation.

12. The pharmaceutical composition according to claim 10 or 11 for use as a medicament.

13. The pharmaceutical composition for use according to claim 12 in the prevention and/or treatment of a disease, disorder or condition associated with dysregulation of DDR.

14. The pharmaceutical composition for use according to claim 12 or 13 in the prevention and/or treatment of fibrosis and/or diseases, disorders or conditions that involve fibrosis.

15. The pharmaceutical composition for use according to claim 14 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

16. The pharmaceutical composition for use according to claim 15 in the prevention and/or treatment of idiopathic pulmonary fibrosis (IPF).

17. An intermediate compound selected from the compound of formula (IV) 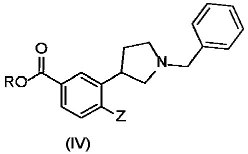 and the compound of formula (VI) 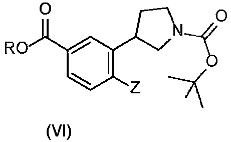 wherein R is (C₁-C₄)alkyl and Z is selected from the group consisting of (C₁-C₄)alkyl, (C₁-C₄)haloalkyl, (C₁-C₄)alkoxy, (C₁-C₄)haloalkoxy, (C₁-C₄)hydroxyalkyl and (C₃-C₆)cycloalkyl, and wherein the compound of formula (VI) is in its racemic or enantiomeric form, (R) or (S).

18. Use of the intermediate compound of formula (IV) and/or the intermediate compound of formula (VI), as defined in claim 17, in the preparation of a compound of formula (Ia) as defined in claim 5.

19. A process for the preparation of a compound of formula (Ia), as defined in claim 5, or a pharmaceutically acceptable salt thereof, comprising the step of:
a) reacting a compound of formula (III) 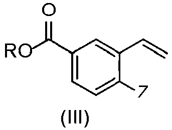 with N-benzyl-1-methoxy-N-((trimethylsilyl)methyl)methanamine 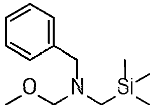 under acid catalysis in the presence of a solvent such as to obtain an intermediate compound of formula (IV) 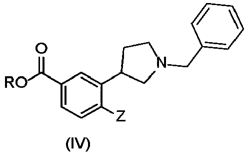 wherein R and Z are as defined in claim 17.

20. The process according to claim 19, further comprising the steps of:
b) cleaving the benzyl group of the intermediate compound of formula (IV) such as to obtain an intermediate compound of formula (V): 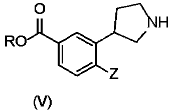 by reduction under hydrogen atmosphere in the presence of a Pd catalyst; and
c) Boc-protecting the free nitrogen of the intermediate compound of formula (V) such as to obtain an intermediate compound of formula (VI) 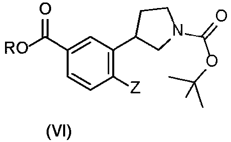 by reaction with di-*tert*-butyl-dicarbonate.

## Patentansprüche

1. Verbindung der Formel (I) 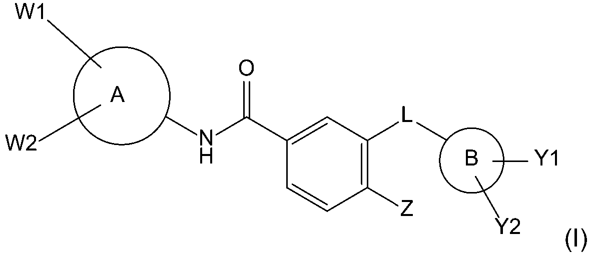 wobei
A ein Ring ist, der ausgewählt ist aus der Gruppe, bestehend aus: 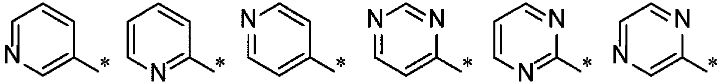 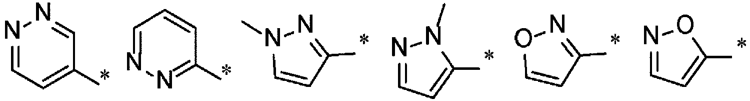 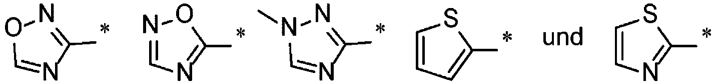
wobei 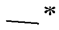 eine direkte Bindung zu NH anzeigt;
W1 und W2 Substituenten von Ring A sind, ausgewählt aus der Gruppe bestehend aus Wasserstoff, Halogen, (C₁-C₄)-Alkyl, (C₁-C₄)-Halogenalkyl, (C₁-C₄)-Alkoxy, (C₁-C₄)-Halogenalkoxy, R1R2N-(C₁-C₄)-Alkyl und (C₃-C₆)-Cycloalkyl;
Z ausgewählt ist aus der Gruppe, bestehend aus (C₁-C₄)-Alkyl, (C₁-C₄)-Halogenalkyl, (C₁-C₄)-Alkoxy, (C₁-C₄)-Halogenalkoxy, (C₁-C₄)-Hydroxyalkyl, Hydroxy und (C₃-C₆)-Cycloalkyl;
L ausgewählt ist aus der Gruppe, bestehend aus 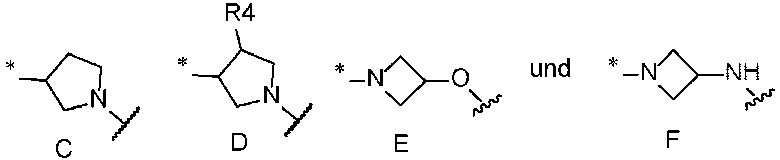
wobei 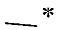 eine direkte Bindung an den Phenylring anzeigt und 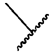 eine direkte Bindung an B anzeigt;
B ein mono- oder bicyclischer Heteroarylring ist;
Y1 und Y2 Substituenten von Ring B sind, die unabhängig voneinander ausgewählt sind aus der Gruppe, bestehend aus Wasserstoff, (C₁-C₄)-Alkyl, (C₁-C₄)-Halogenalkyl, (C₁-C₄)-Alkoxy, (C₁-C₄)-Alkoxy-(C₁-C₄)-Alkoxy, Hydroxy-(C₁-C₄)-Alkoxy, Hydroxy-(C₁-C₄)-Alkyl, (C₁-C₄)-Alkyl-Heterocycloalkyl-(C₀-C₄)-Alkoxy, (C₁-C₄)-Halogenalkoxy, Halogen, Cyano, Cyano-(C₁-C₄)-alkyl, Cyano-(C₁-C₄)-Alkyl-Heterocycloalkyl, CONR1R2, NHCOR1, NR1R2, R1R2N-(C₁-C₄)-Alkyl, Heterocycloalkyl, (C₁-C₄)-Alkyl-Heterocycloalkyl, Heterocycloalkyl-(C₁-C₄)-Alkyl, (C₁-C₄)-Alkyl-Heterocycloalkyl-Carbonyl, (C₀-C₄)-Alkyl-Heterocycloalkyl-Carbonyl, (C₁-C₄)-Alkyl-Phenyl und monocyclisches (C₁-C₄)-Alkyl-Heteroaryl;
R1 und R2 unabhängig ausgewählt sind aus der Gruppe, bestehend aus Wasserstoff, (C₁-C₄)-Alkyl, (C₁-C₄)-Hydroxyalkyl, (C₁-C₄)-Alkoxy(C₁-C₄)-Alkyl, (C₁-C₄)-Alkylamino-(C₁-C₄)-Alkyl, Di-(C₁-C₄)-Alkylamino-(C₁-C₄)-Alkyl, optional substituiertes (C₃-C₆)-Cycloalkyl, optional substituiertes Heterocycloalkyl und optional substituiertes Heterocycloalkyl-(C₁-C₄)-Alkoxy, wobei optionale Substituenten ein oder mehrere sind und ausgewählt sind aus (C₁-C₄)-Alkyl, (C₁-C₄)-Alkoxy, (C₁-C₄)-Halogenalkyl, Hydroxy und (C₁-C₄)-Halogenalkoxy;
R4 ausgewählt ist aus Hydroxy und Hydroxymethyl;
oder einem Stereoisomer, Tautomer, Solvat und pharmazeutisch verträglichem Salz davon.

2. Verbindung der Formel (I) nach Anspruch 1, wobei
L ausgewählt ist aus der Gruppe, bestehend aus 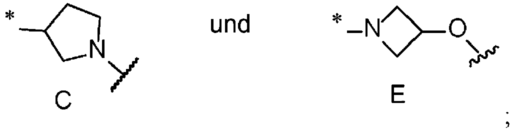
oder einem Stereoisomer, Tautomer, Solvat und pharmazeutisch verträglichem Salz davon.

3. Verbindung der Formel (I) nach Anspruch 1 oder 2, wobei
A ein Ring ist, der aus der Gruppe ausgewählt ist, bestehend aus: 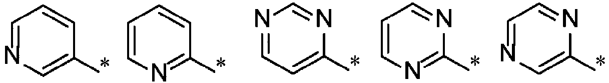 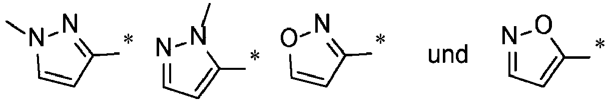
W1 und W2 sind unabhängig ausgewählt aus der Gruppe, bestehend aus H, F, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ und Cyclopropyl;
Z ist ausgewählt aus der Gruppe, bestehend aus CH₃, CF₂H, OCH₃, CH₂OH und Cyclopropyl;
B ist ausgewählt aus der Gruppe, bestehend aus Pyridinyl, Pyrimidinyl, Pyrazinyl, Pyrazolo[1,5-a]pyrazinyl, 1H-Pyrazolo[3,4-b]pyridinyl, Pyrazolo[1,5-a]pyridinyl, Pyrazolo[1,5-a]pyrimidinyl, Imidazo[1,2-b]pyridazinyl, Imidazo[1,2-a]pyrazinyl, Imidazo[1,2-a]pyridinyl, Thieno[3,2-d]pyrimidinyl, 1H-Pyrrolo[2,3-b]pyridinyl und Pyrazolyl;
Y1 ist ausgewählt aus der Gruppe, bestehend aus Wasserstoff, (C₁-C₄)-Halogenalkyl, (C₁-C₄)-Alkoxy, Cyano, CONR1R2, NHCOR1, NR1R2, (C₁-C₄)-Alkyl-Heterocycloalkyl-Carbonyl und monocyclischem (C₁-C₄)-Alkyl-Heteroaryl; und Y2 ist Wasserstoff;
R1 und R2 sind unabhängig voneinander ausgewählt aus der Gruppe, bestehend aus CH₂CH₂N(CH₃)₂, CH₂CH₂OCH₃, CH₃, CH₂CH₂OH, Oxetan-3-yl und 3-Hydroxycyclobutyl;
oder einem Stereoisomer, Tautomer, Solvat und pharmazeutisch verträglichem Salz davon.

4. Verbindung der Formel (I) nach Anspruch 3, wobei
Y1 ist ausgewählt aus der Gruppe, bestehend aus CONH₂, CF₂H, OCH₃, Cyano, NHCOCH₃, NH₂, 4-Methylpiperazin-1-carbonyl, 4-Methylpiperazin-1-yl und 1-Methyl-1H-pyrazol-4-yl; und Y2 ist Wasserstoff;
oder einem Stereoisomer, Tautomer, Solvat und pharmazeutisch verträglichem Salz davon.

5. Prozess nach einem der vorstehenden Ansprüche, wobei das Verfahren umfasst einen Schritt 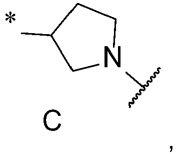 dargestellt durch Formel (la) 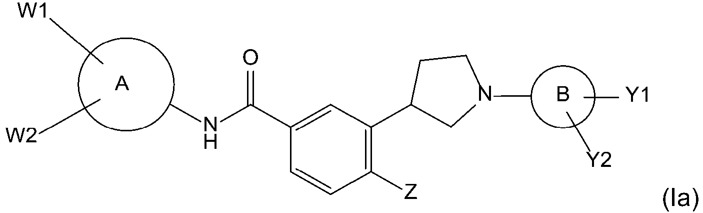 oder einem Stereoisomer, Tautomer, Solvat und pharmazeutisch verträglichem Salz davon.

6. Die Verbindung nach einem der vorangehenden Ansprüche, wobei L oder C C* ist. 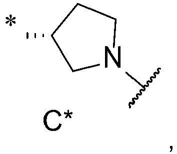 oder einem Stereoisomer, Tautomer, Solvat und pharmazeutisch verträglichem Salz davon.

7. Verbindung nach einem der Ansprüche 1 bis 4, wobei L ist 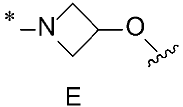 und die als Verbindung der Formel (Ib1) bezeichnet wird.

8. Verbindung der Formel (la) nach Anspruch 5, ausgewählt aus der Gruppe, bestehend aus:
(S)-5-(3-(2-Methyl-5-((5-(trifluormethyl)pyridin-3-yl)carbamoyl)phenyl)pyrrolidin-1-yl)nicotinamid (Beispiel 1);
(R)-5-(3-(2-Methyl-5-((5-(trifluormethyl)pyridin-3-yl)carbamoyl)phenyl)pyrrolidin-1-yl)nicotinamid (Beispiel 2);
(S)-5-(3-(5-((5-(tert-Butyl)-1-methyl-1H-pyrazol-3-yl)carbamoyl)-2-methylphenyl)pyrrolidin-1-yl)nicotinamid (Beispiel 3);
(S)-5-(3-(2-Methyl-5-((5-(1,1,1-trifluor-2-methylpropan-2-yl)isoxazol-3-yl)carbamoyl)phenyl)pyrrolidin-1-yl)nicotinamid (Beispiel 4);
(S)-5-(3-(5-((3-(tert-Butyl)-1-methyl-1H-pyrazol-5-yl)carbamoyl)-2-methylphenyl)pyrrolidin-1-yl)nicotinamid (Beispiel 5);
(S)-5-(3-(5-((3-(tert-Butyl)isoxazol-5-yl)carbamoyl)-2-methylphenyl)pyrrolidin-1-yl)nicotinamid (Beispiel 6);
(S)-N-(3-(tert-Butyl)isoxazol-5-yl)-3-(1-(5-cyanpyridin-3-yl)pyrrolidin-3-yl)-4-methylbenzamid (Beispiel 7);
(S)-3-(1-(5-Cyanpyridin-3-yl)pyrrolidin-3-yl)-4-methyl-N-(6-(trifluormethyl)pyrimidin-4-yl)benzamid (Beispiel 8);
(S)-5-(3-(2-Methyl-5-((4-(trifluormethoxy)pyridin-2-yl)carbamoyl)phenyl)pyrrolidin-1-yl)nicotinamid (Beispiel 9);
(S)-5-(3-(2-Methyl-5-((5-(trifluormethoxy)pyridin-2-yl)carbamoyl)phenyl)pyrrolidin-1-yl)nicotinamid (Beispiel 10);
(S)-5-(3-(5-((5-(tert-Butyl)isoxazol-3-yl)carbamoyl)-2-methylphenyl)pyrrolidin-1-yl)nicotinamid (Beispiel 11);
(S)-4-Methyl-3-(1-(pyrimidin-5-yl)pyrrolidin-3-yl)-N-(4-(trifluormethoxy)pyridin-2-yl)benzamid (Beispiel 12);
(S)-N-(5-(tert-Butyl)-1-methyl-1H-pyrazol-3-yl)-4-methyl-3-(1-(pyrimidin-5-yl)pyrrolidin-3-yl)benzamid (Beispiel 13);
(S)-4-Methyl-3-(1-(pyrimidin-5-yl)pyrrolidin-3-yl)-N-(5-(1,1,1-trifluor-2-methylpropan-2-yl)isoxazol-3-yl)benzamid (Beispiel 14);
(S)-4-Methyl-3-(1-(pyrimidin-5-yl)pyrrolidin-3-yl)-N-(4-(trifluormethyl)pyridin-2-yl)benzamid (Beispiel 15);
(S)-N-(5-(tert-Butyl)isoxazol-3-yl)-4-methyl-3-(1-(pyrimidin-5-yl)pyrrolidin-3-yl)benzamid (Beispiel 16);
(S)-N-(3-(tert-Butyl)-1-methyl-1H-pyrazol-5-yl)-4-methyl-3-(1-(pyrimidin-5-yl)pyrrolidin-3-yl)benzamid (Beispiel 17);
(S)-4-Methyl-3-(1-(pyrimidin-5-yl)pyrrolidin-3-yl)-N-(5-(trifluormethoxy)pyridin-3-yl)benzamid (Beispiel 18);
(S)-N-(3-(tert-Butyl)isoxazol-5-yl)-4-methyl-3-(1-(pyrimidin-5-yl)pyrrolidin-3-yl)benzamid (Beispiel 19);
(S)-4-Methyl-3-(1-(pyrimidin-5-yl)pyrrolidin-3-yl)-N-(5-(trifluormethoxy)pyridin-2-yl)benzamid (Beispiel 20);
(S)-N-(4-(Difluormethoxy)pyridin-2-yl)-4-methyl-3-(1-(pyrimidin-5-yl)pyrrolidin-3-yl)benzamid (Beispiel 21);
(S)-3-(1-(5-(Difluormethyl)pyridin-3-yl)pyrrolidin-3-yl)-4-methyl-N-(5-(trifluormethyl)pyridin-3-yl)benzamid (Beispiel 22);
(S)-N-(2-(Dimethylamino)ethyl)-5-(3-(2-methyl-5-((5-(trifluormethyl)pyridin-3-yl)carbamoyl)phenyl)pyrrolidin-1-yl)nicotinamid (Beispiel 23);
(S)-N-(2-Methoxyethyl)-5-(3-(2-methyl-5-((5-(trifluormethyl)pyridin-3-yl)carbamoyl)phenyl)pyrrolidin-1-yl)nicotinamid (Beispiel 24);
(S)-3-(1-(2-Methoxypyrimidin-5-yl)pyrrolidin-3-yl)-4-methyl-N-(5-(trifluormethyl)pyridin-3-yl)benzamid (Beispiel 25);
(S)-4-Methyl-3-(1-(pyrimidin-5-yl)pyrrolidin-3-yl)-N-(5-(trifluormethyl)pyridin-3-yl)benzamid (Beispiel 26);
(S)-3-(1-(5-Cyanpyridin-3-yl)pyrrolidin-3-yl)-4-methyl-N-(5-(trifluormethyl)pyridin-3-yl)benzamid (Beispiel 27);
(S)-3-(1-(5-Acetamidopyridin-3-yl)pyrrolidin-3-yl)-4-methyl-N-(5-(trifluormethyl)pyridin-3-yl)benzamid (Beispiel 28);
(S)-N-Methyl-5-(3-(2-methyl-5-((5-(trifluormethyl)pyridin-3-yl)carbamoyl)phenyl)pyrrolidin-1-yl)nicotinamid (Beispiel 29);
(S)-N-(2-Hydroxyethyl)-5-(3-(2-methyl-5-((5-(trifluormethyl)pyridin-3-yl)carbamoyl)phenyl)pyrrolidin-1-yl)nicotinamid (Beispiel 30);
(S)-3-(1-(3-Aminopyrazin-2-yl)pyrrolidin-3-yl)-4-methyl-N-(5-(trifluormethyl)pyridin-3-yl)benzamid (Beispiel 31);
(S)-4-Methyl-3-(1-(pyrazolo[1,5-a]pyrazin-3-yl)pyrrolidin-3-yl)-N-(5-(trifluormethyl)pyridin-3-yl)benzamid (Beispiel 32);
(S)-4-Methyl-3-(1-(pyrazin-2-yl)pyrrolidin-3-yl)-N-(5-(trifluormethyl)pyridin-3-yl)benzamid (Beispiel 33);
(S)-4-Methyl-3-(1-(pyrazolo[1,5-a]pyridin-3-yl)pyrrolidin-3-yl)-N-(5-(trifluormethyl)pyridin-3-yl)benzamid (Beispiel 34);
(S)-4-Methyl-3-(1-(pyrazolo[1,5-a]pyrimidin-3-yl)pyrrolidin-3-yl)-N-(5-(trifluormethyl)pyridin-3-yl)benzamid (Beispiel 35);
(S)-3-(1-(Imidazo[1,2-b]pyridazin-3-yl)pyrrolidin-3-yl)-4-methyl-N-(5-(trifluormethyl)pyridin-3-yl)benzamid (Beispiel 36);
(S)-3-(1-(Imidazo[1,2-a]pyrazin-3-yl)pyrrolidin-3-yl)-4-methyl-N-(5-(trifluormethyl)pyridin-3-yl)benzamid (Beispiel 37);
(S)-5-(3-(2-Methyl-5-((5-(trifluormethyl)pyridin-3-yl)carbamoyl)phenyl)pyrrolidin-1-yl)-N-(oxetan-3-yl)nicotinamid (Beispiel 38);
N-((1s,3R)-3-Hydroxycyclobutyl)-5-((S)-3-(2-methyl-5-((5-(trifluormethyl)pyridin-3-yl)carbamoyl)phenyl)pyrrolidin-1-yl)nicotinamid (Beispiel 39);
(S)-4-Methyl-3-(1-(5-(4-methylpiperazin-1-carbonyl)pyridin-3-yl)pyrrolidin-3-yl)-N-(5-(trifluormethyl)pyridin-3-yl)benzamid (Beispiel 40);
(S)-3-(3-(2-Methyl-5-((5-(trifluormethyl)pyridin-3-yl)carbamoyl)phenyl)pyrrolidin-1-yl)-N-(oxetan-3-yl)isonicotinamid (Beispiel 41);
(S)-3-(1-(2-Aminopyridin-3-yl)pyrrolidin-3-yl)-4-methyl-N-(5-(trifluormethyl)pyridin-3-yl)benzamid (Beispiel 42);
(S)-3-(1-(1H-Pyrazolo[3,4-b]pyridin-5-yl)pyrrolidin-3-yl)-4-methyl-N-(5-(trifluormethyl)pyridin-3-yl)benzamid (Beispiel 43);
(S)-6-(3-(5-((4-(Difluormethoxy)pyridin-2-yl)carbamoyl)-2-methylphenyl)pyrrolidin-1-yl)pyrazin-2-carboxamid (Beispiel 51);
(S)-4-(3-(5-((5-(tert-Butyl)-1-methyl-1H-pyrazol-3-yl)carbamoyl)-2-methylphenyl)pyrrolidin-1-yl)picolinamid (Beispiel 52);
(S)-4-(3-(5-((2-((Dimethylamino)methyl)-6-(trifluormethyl)pyridin-4-yl)carbamoyl)-2-methylphenyl)pyrrolidin-1-yl)picolinamid (Beispiel 53);
(S)-6-(3-(2-Methyl-5-((5-(trifluormethyl)pyridin-3-yl)carbamoyl)phenyl)pyrrolidin-1-yl)pyrimidin-4-carboxamid (Beispiel 54);
(S)-3-(1-(4-Cyanpyridin-3-yl)pyrrolidin-3-yl)-4-methyl-N-(5-(1,1,1-trifluor-2-methylpropan-2-yl)isoxazol-3-yl)benzamid (Beispiel 55);
(S)-3-(1-(4-Cyanpyridin-3-yl)pyrrolidin-3-yl)-N-(5-ethylisoxazol-3-yl)-4-methylbenzamid (Beispiel 56);
(S)-4-(3-(2-Methyl-5-((4-(trifluormethoxy)pyridin-2-yl)carbamoyl)phenyl)pyrrolidin-1-yl)picolinamid (Beispiel 57);
(*S*)-4-Methyl-3-(1-(pyrazin-2-yl)pyrrolidin-3-yl)-N-(5-(1,1,1-trifluor-2-methylpropan-2-yl)isoxazol-3-yl)benzamid (Beispiel 58);
(*S*)-*N*-(3-Cyclopropyl-1-methyl-1*H*-pyrazol-5-yl)-4-methyl-3-(1-(5-(1-methyl-1*H*-pyrazol-4-yl)pyridin-3-yl)pyrrolidin-3-yl)benzamid (Beispiel 59);
(R)-4-Methyl-3-(1-(5-(1-methyl-1H-pyrazol-4-yl)pyridin-3-yl)pyrrolidin-3-yl)-N-(5-(1,1,1-trifluor-2-methylpropan-2-yl)isoxazol-3-yl)benzamid (Beispiel 60);
(S)-4-Methyl-3-(1-(6-(morpholin-4-carbonyl)thieno[3,2-d]pyrimidin-4-yl)pyrrolidin-3-yl)-N-(5-(trifluormethyl)pyridin-3-yl)benzamid (Beispiel 61);
(S)-4-Methyl-3-(1-(pyrazolo[1,5-a]pyrimidin-3-yl)pyrrolidin-3-yl)-N-(5-(trifluormethoxy)pyridin-3-yl)benzamid (Beispiel 62);
(S)-4-(3-(2-Methyl-5-((5-(trifluormethyl)pyridin-3-yl)carbamoyl)phenyl)pyrrolidin-1-yl)picolinamid (Beispiel 63);
4-(3-(2-Methoxy-5-((5-(trifluormethyl)pyridin-3-yl)carbamoyl)phenyl)pyrrolidin-1-yl)picolinamid (Beispiel 64);
(S)-1-Methyl-4-(3-(2-methyl-5-((5-(trifluormethyl)pyridin-3-yl)carbamoyl)phenyl)pyrrolidin-1-yl)-1H-pyrazol-5-carboxamid (Beispiel 65);
(S)-3-(1-(3-Carbamoylphenyl)pyrrolidin-3-yl)-4-methyl-N-(5-(trifluormethyl)pyridin-3-yl)benzamid (Beispiel 66);
(S)-N-(4-(Difluormethyl)pyridin-2-yl)-4-methyl-3-(1-(pyrimidin-5-yl)pyrrolidin-3-yl)benzamid (Beispiel 67);
(S)-4-Methyl-N-(1-methyl-3-(trifluormethyl)-1H-pyrazol-5-yl)-3-(1-(pyrimidin-5-yl)pyrrolidin-3-yl)benzamid (Beispiel 68);
(S)-N-(5-Ethylisoxazol-3-yl)-4-methyl-3-(1-(pyrimidin-5-yl)pyrrolidin-3-yl)benzamid (Beispiel 69);
(S)-N-(3-Cyclopropyl-1-methyl-1H-pyrazol-5-yl)-4-methyl-3-(1-(pyrimidin-5-yl)pyrrolidin-3-yl)benzamid (Beispiel 70);
(S)-3-(1-(Imidazo[1,2-a]pyridin-3-yl)pyrrolidin-3-yl)-4-methyl-N-(5-(trifluormethyl)pyridin-3-yl)benzamid (Beispiel 71);
(S)-4-Methyl-3-(1-(6-(4-methylpiperazin-1-yl)imidazo[1,2-a]pyridin-3-yl)pyrrolidin-3-yl)-N-(5-(trifluormethyl)pyridin-3-yl)benzamid (Beispiel 72);
(S)-3-(1-(6-(4-(Cyanmethyl)piperazin-1-yl)imidazo[1,2-a]pyridin-3-yl)pyrrolidin-3-yl)-4-methyl-N-(5-(trifluormethyl)pyridin-3-yl)benzamid (Beispiel 73);
(S)-4-Methyl-3-(1-(5-(2-(4-methylpiperazin-1-yl)ethoxy)pyridin-3-yl)pyrrolidin-3-yl)-N-(5-(trifluormethyl)pyridin-3-yl)benzamid (Beispiel 74);
(S)-3-(1-(4-Cyanpyridin-3-yl)pyrrolidin-3-yl)-4-methyl-N-(5-(trifluormethyl)pyridin-3-yl)benzamid (Beispiel 75);
(S)-3-(1-(4-Methoxypyridin-3-yl)pyrrolidin-3-yl)-4-methyl-N-(5-(trifluormethyl)pyridin-3-yl)benzamid (Beispiel 76);
(S)-4-Methyl-3-(1-(6-(morpholinomethyl)imidazo[1,2-a]pyridin-3-yl)pyrrolidin-3-yl)-N-(5-(trifluormethyl)pyridin-3-yl)benzamid (Beispiel 77);
(S)-3-(1-(6-Cyanimidazo[1,2-a]pyridin-3-yl)pyrrolidin-3-yl)-4-methyl-N-(5-(trifluormethyl)pyridin-3-yl)benzamid (Beispiel 78);
(S)-3-(1-(5-(2-Methoxyethoxy)pyridin-3-yl)pyrrolidin-3-yl)-4-methyl-N-(5-(trifluormethyl)pyridin-3-yl)benzamid (Beispiel 79);
(S)-4-Methyl-3-(1-(6-(4-methylpiperazin-1-yl)imidazo[1,2-b]pyridazin-3-yl)pyrrolidin-3-yl)-N-(5-(trifluormethyl)pyridin-3-yl)benzamid (Beispiel 80);
(S)-4-Methyl-3-(1-(5-((1-methylpiperidin-4-yl)oxy)pyridin-3-yl)pyrrolidin-3-yl)-N-(5-(trifluormethyl)pyridin-3-yl)benzamid (Beispiel 81);
(S)-4-Methyl-3-(1-(6-(4-methylpiperazin-1-yl)imidazo[1,2-a]pyrazin-3-yl)pyrrolidin-3-yl)-N-(5-(trifluormethyl)pyridin-3-yl)benzamid (Beispiel 82);
(S)-4-Methyl-3-(1-(5-(4-methylpiperazin-1-yl)pyrazolo[1,5-a]pyrimidin-3-yl)pyrrolidin-3-yl)-N-(5-(trifluormethyl)pyridin-3-yl)benzamid (Beispiel 83);
(S)-4-Methyl-3-(1-(6-morpholinoimidazo[1,2-b]pyridazin-3-yl)pyrrolidin-3-yl)-N-(5-(trifluormethyl)pyridin-3-yl)benzamid (Beispiel 84);
(S)-3-(1-(2-Acetamidopyridin-4-yl)pyrrolidin-3-yl)-4-methyl-N-(5-(trifluormethyl)pyridin-3-yl)benzamid (Beispiel 85);
(S)-4-Methyl-3-(1-(5-methylpyrazolo[1,5-a]pyrimidin-3-yl)pyrrolidin-3-yl)-N-(5-(trifluormethyl)pyridin-3-yl)benzamid (Beispiel 86);
(S)-N-Methyl-4-(3-(2-methyl-5-((5-(trifluormethyl)pyridin-3-yl)carbamoyl)phenyl)pyrrolidin-1-yl)picolinamid (Beispiel 87);
(S)-4-Methyl-3-(1-(1-methyl-1H-pyrazol-4-yl)pyrrolidin-3-yl)-N-(5-(trifluormethyl)pyridin-3-yl)benzamid (Beispiel 88);
(S)-4-Methyl-3-(1-(5-(morpholin-4-carbonyl)pyrazolo[1,5-a]pyridin-3-yl)pyrrolidin-3-yl)-N-(5-(trifluormethyl)pyridin-3-yl)benzamid (Beispiel 89);
4-Methyl-3-[(3R)-1-pyrazolo[1,5-a]pyrimidin-3-ylpyrrolidin-3-yl]-N-[5-(trifluormethyl)-3-pyridyl]benzamid (Beispiel 90);
N-[4-(Difluormethoxy)-2-pyridyl]-4-methyl-3-[(3S)-1-pyrazolo[1,5-a]pyrimidin-3-ylpyrrolidin-3-yl]benzamid (Beispiel 91);
N-[4-(Difluormethoxy)-2-pyridyl]-4-methyl-3-[(3S)-1-pyrazin-2-ylpyrrolidin-3-yl]benzamid (Beispiel 92);
3-[(3S)-1-(4-Cyano-3-pyridyl)pyrrolidin-3-yl]-N-[4-(difluormethoxy)-2-pyridyl]-4-methyl-benzamid (Beispiel 93);
(S)-3-(1-(6-cyanopyrazin-2-yl)pyrrolidin-3-yl)-N-(4-(difluoromethoxy) pyridin-2-yl)-4-methyl-benzamide (Example 94);
(S)-N-(5-Ethylisoxazol-3-yl)-4-methyl-3-(1-(pyrazolo[1,5-a]pyrimidin-3-yl)pyrrolidin-3-yl)benzamid (Beispiel 95);
(S)-N-(5-Ethylisoxazol-3-yl)-4-methyl-3-(1-(pyrazolo[1,5-a]pyrazin-3-yl)pyrrolidin-3-yl)benzamid (Beispiel 96);
(S)-3-(1-(6-Methoxypyrazolo[1,5-a]pyrimidin-3-yl)pyrrolidin-3-yl)-4-methyl-N-(5-(trifluormethyl)pyridin-3-yl)benzamid (Beispiel 97);
(S)-4-Methyl-3-(1-(pyrazolo[1,5-a]pyrazin-3-yl)pyrrolidin-3-yl)-N-(5-(trifluormethoxy)pyridin-3-yl)benzamid (Beispiel 98);
(S)-4-Methyl-3-(1-(2-(methylamino)pyrimidin-5-yl)pyrrolidin-3-yl)-N-(5-(trifluormethyl)pyridin-3-yl)benzamid (Beispiel 99);
(S)-3-(1-(1H-Pyrazol-4-yl)pyrrolidin-3-yl)-4-methyl-N-(5-(trifluormethyl)pyridin-3-yl)benzamid (Beispiel 100);
(S)-3-(1-(3-((Dimethylamino)methyl)-1H-pyrazolo[3,4-b]pyridin-5-yl)pyrrolidin-3-yl)-4-methyl-N-(5-(trifluormethyl)pyridin-3-yl)benzamid (Beispiel 101);
(S)-5-(3-(2-Methyl-5-((5-(trifluormethyl)pyridin-3-yl)carbamoyl)phenyl)pyrrolidin-1-yl)-N-(oxetan-3-yl)-1H-pyrrolo[2,3-b]pyridin-3-carboxamid (Beispiel 102);
(S)-3-(1-(5-(2-Hydroxyethoxy)pyridin-3-yl)pyrrolidin-3-yl)-4-methyl-N-(5-(trifluormethyl)pyridin-3-yl)benzamid (Beispiel 103);
3-(4-(Hydroxymethyl)-1-(pyrimidin-5-yl)pyrrolidin-3-yl)-4-methyl-N-(5-(trifluormethyl)pyridin-3-yl)benzamid (Beispiel 104);
3-(4-Hydroxy-1-(pyrimidin-5-yl)pyrrolidin-3-yl)-4-methyl-N-(5-(trifluormethyl)pyridin-3-yl)benzamid (Beispiel 105);
(S)-4-Methyl-3-(1-(thieno[2,3-d]pyrimidin-4-yl)pyrrolidin-3-yl)-N-(5-(trifluormethyl)pyridin-3-yl)benzamid (Beispiel 106);
(S)-4-Methyl-3-(1-(thieno[3,2-d]pyrimidin-4-yl)pyrrolidin-3-yl)-N-(5-(trifluormethyl)pyridin-3-yl)benzamid (Beispiel 107);
(S)-N-Methyl-4-(3-(2-methyl-5-((5-(trifluormethyl)pyridin-3-yl)carbamoyl)phenyl)pyrrolidin-1-yl)thieno[3,2-d]pyrimidin-6-carboxamid (Beispiel 108);
4-Methoxy-3-(1-(pyrimidin-5-yl)pyrrolidin-3-yl)-N-(5-(trifluormethyl)pyridin-3-yl)benzamid, 1^{tes} eluiertes Enantiomer (Beispiel 109);
4-Methoxy-3-(1-(pyrimidin-5-yl)pyrrolidin-3-yl)-N-(5-(trifluormethyl)pyridin-3-yl)benzamid, 2^{tes} eluiertes Enantiomer (Beispiel 110);
4-Methoxy-3-(1-(pyrimidin-5-yl)pyrrolidin-3-yl)-N-(5-(trifluormethyl)pyridin-3-yl)benzamid, razemische Mischung (Beispiel 111);
4-Methoxy-3-(1-(pyrazolo[1,5-a]pyrimidin-3-yl)pyrrolidin-3-yl)-N-(5-(trifluormethyl)pyridin-3-yl)benzamid (Beispiel 112);
4-(Difluormethoxy)-3-(1-(pyrimidin-5-yl)pyrrolidin-3-yl)-N-(5-(trifluormethyl)pyridin-3-yl)benzamid (Beispiel 113);
4-Methoxy-3-(1-(pyrazolo[1,5-a]pyrimidin-3-yl)pyrrolidin-3-yl)-N-(5-(trifluormethyl)pyridin-3-yl)benzamid, 1^{tes} eluiertes Enantiomer (Beispiel 114);
4-Methoxy-3-(1-(pyrazolo[1,5-a]pyrimidin-3-yl)pyrrolidin-3-yl)-N-(5-(trifluormethyl)pyridin-3-yl)benzamid, 2^{tes} eluiertes Enantiomer (Beispiel 115);
4-(Difluormethoxy)-3-(1-(pyrimidin-5-yl)pyrrolidin-3-yl)-N-(5-(trifluormethyl)pyridin-3-yl)benzamid, 1tes eluiertes Enantiomer (Beispiel 116);
4-(Difluormethoxy)-3-(1-(pyrimidin-5-yl)pyrrolidin-3-yl)-N-(5-(trifluormethyl)pyridin-3-yl)benzamid, 2^{tes} eluiertes Enantiomer (Beispiel 117);
4-(3-(2-(Difluormethoxy)-5-((5-(trifluormethyl)pyridin-3-yl)carbamoyl)phenyl)pyrrolidin-1-yl)picolinamid, 1^{tes} eluiertes Enantiomer (Beispiel 118);
4-(3-(2-(Difluormethoxy)-5-((5-(trifluormethyl)pyridin-3-yl)carbamoyl)phenyl)pyrrolidin-1-yl)picolinamid, 2^{tes} eluiertes Enantiomer (Beispiel 119);
4-(Difluormethyl)-3-(1-(pyrimidin-5-yl)pyrrolidin-3-yl)-N-(5-(trifluormethyl)pyridin-3-yl)benzamid, (Beispiel 120);
(1-(Pyrimidin-5-yl)pyrrolidin-3-yl)-N-(5-(trifluormethyl)pyridin-3-yl)benzamid, 2^{tes} eluiertes Enantiomer (Beispiel 121);
3-(4-Hydroxy-1-(pyrimidin-5-yl)pyrrolidin-3-yl)-4-methyl-N-(5-(trifluormethyl)pyridin-3-yl)benzamid, 2^{tes} eluiertes Enantiomer (Beispiel 122);
(S)-(3-(1-(6-(Hydroxymethyl)imidazo[1,2-a]pyridin-3-yl)pyrrolidin-3-yl)-4-methylphenyl)(5-(trifluormethyl)pyridin-3-yl)methanon (Beispiel 123);
N-(5-(tert-Butyl)-1-methyl-1H-pyrazol-3-yl)-4-(difluormethyl)-3-(1-(pyrimidin-5-yl)pyrrolidin-3-yl)benzamid (Beispiel 124);
(S)-3-(1-(6-Bromthieno[3,2-d]pyrimidin-4-yl)pyrrolidin-3-yl)-4-methyl-N-(5-(trifluormethyl)pyridin-3-yl)benzamid (Beispiel 125);
(S)-4-Methyl-3-(1-(6-(4-methylpiperazin-1-carbonyl)thieno[3,2-d]pyrimidin-4-yl)pyrrolidin-3-yl)-N-(5-(trifluormethyl)pyridin-3-yl)benzamid, (Beispiel 126);
4-(3-(2-Methoxy-5-((5-(trifluormethyl)pyridin-3-yl)carbamoyl)phenyl)pyrrolidin-1-yl)-N-methylpicolinamid, (Beispiel 127);
(S)-3-(1-(Imidazo[1,5-a]pyrazin-8-yl)pyrrolidin-3-yl)-4-methyl-N-(5-(trifluormethyl)pyridin-3-yl)benzamid (Beispiel 128);
(S)-3-(1-(1H-Pyrazolo[3,4-d]pyrimidin-4-yl)pyrrolidin-3-yl)-4-methyl-N-(5-(trifluormethyl)pyridin-3-yl)benzamid (Beispiel 129);
(S)-3-(1-(2-Acetamidopyrimidin-5-yl)pyrrolidin-3-yl)-4-methyl-N-(5-(trifluormethyl)pyridin-3-yl)benzamid (Beispiel 130); und
(S)-N-(5-(tert-Butyl)-1-methyl-1H-pyrazol-3-yl)-4-methyl-3-(1-(thieno[3,2-d]pyrimidin-4-yl)pyrrolidin-3-yl)benzamid (Beispiel 131).

9. Verbindung der Formel (Ib1) nach Anspruch 7, ausgewählt aus der Gruppe, bestehend aus:
3-(3-((6-Aminopyridin-3-yl)oxy)azetidin-1-yl)-4-methyl-N-(5-(trifluormethyl)pyridin-3-yl)benzamid (Beispiel 44);
3-(3-((6-Acetamidopyridin-3-yl)oxy)azetidin-1-yl)-4-methyl-N-(5-(trifluormethyl)pyridin-3-yl)benzamid (Beispiel 45);
3-(3-((6-(2-Methoxyacetamido)pyridin-3-yl)oxy)azetidin-1-yl)-4-methyl-N-(5-(trifluormethyl)pyridin-3-yl)benzamid (Beispiel 46);
3-(3-((5-Aminopyridin-3-yl)oxy)azetidin-1-yl)-4-methyl-N-(5-(trifluormethyl)pyridin-3-yl)benzamid (Beispiel 47);
5-((1-(2-Methyl-5-((5-(trifluormethyl)pyridin-3-yl)carbamoyl)phenyl)azetidin-3-yl)oxy)nicotinamid (Beispiel 132); und
3-(3-((5-Acetamidopyridin-3-yl)oxy)azetidin-1-yl)-4-methyl-N-(5-(trifluormethyl)pyridin-3-yl)benzamid (Beispiel 133).

10. Pharmazeutische Zusammensetzung, umfassend eine Verbindung der Formel (I) nach einem der Ansprüche 1 bis 9 unter Beigabe eines oder mehrerer pharmazeutisch annehmbarer Träger und/oder Hilfsstoffe.

11. Pharmazeutische Zusammensetzung nach Anspruch 10 zur Verabreichung durch Inhalation.

12. Pharmazeutische Zusammensetzung nach Anspruch 10 und 11 zur Verwendung als Arzneimittel.

13. Pharmazeutische Zusammensetzung zur Verwendung nach Anspruch 12 bei der Prävention und/oder Behandlung einer Erkrankung, einer Störung oder eines Zustands, die/der mit einer Dysregulation von DDR assoziiert ist.

14. Pharmazeutische Zusammensetzung zur Verwendung nach Anspruch 12 oder 13 bei der Prävention und/oder Behandlung von Fibrose und/oder Erkrankungen, Störungen oder Zuständen, die mit Fibrose einhergehen.

15. Pharmazeutische Zusammensetzung zur Verwendung nach Anspruch 14 bei der Prävention und/oder Behandlung von Fibrose, einschließlich pulmonaler Fibrose, idiopathischer pulmonaler Fibrose (IPF), hepatischer Fibrose, renaler Fibrose, okularer Fibrose, kardialer Fibrose, arterieller Fibrose und systemischer Sklerose.

16. Pharmazeutische Zusammensetzung zur Verwendung nach Anspruch 15 bei der Prävention und/oder Behandlung von idiopathischer pulmonaler Fibrose (IPF).

17. Zwischenverbindung, ausgewählt aus der Verbindung der Formel (IV) 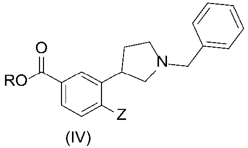 und der Verbindung der Formel (VI) 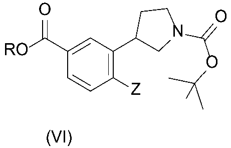 wobei R (C₁-C₄)-Alkyl ist und Z ausgewählt ist aus der Gruppe, bestehend aus (C₁-C₄)-Alkyl, (C₁-C₄)-Halogenalkyl, (C₁-C₄)-Alkoxy, (C₁-C₄)-Halogenalkoxy, (C₁-C₄)-Hydroxyalkyl und (C₃-C₆)-Cycloalkyl, und wobei die Verbindung der Formel (VI) in ihrer razemischen oder enantiomeren Form, (R) oder (S), vorliegt.

18. Verwendung der Zwischenverbindung der Formel (IV) und/oder der Zwischenverbindung der Formel (VI), wie in Anspruch 17 definiert, bei der Herstellung einer Verbindung der Formel (la), wie in Anspruch 5 definiert.

19. Prozess zur Herstellung einer Verbindung der Formel (la), wie in Anspruch 5 definiert, oder eines pharmazeutisch annehmbaren Salzes davon, umfassend den Schritt:
a) Umsetzen einer Verbindung der Formel (III) 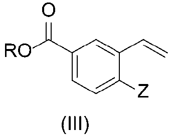 mit N-Benzyl-1-methoxy-N-((trimethylsilyl)methyl)methanamin 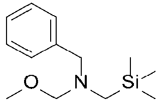 unter Säurekatalyse in Gegenwart eines Lösungsmittels wie zur Erhaltung einer Zwischenverbindung der Formel (IV) 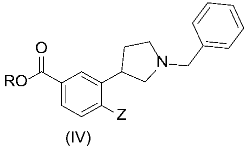 wobei R und Z wie in Anspruch 17 definiert sind.

20. Prozess nach Anspruch 19, ferner umfassend die Schritte:
b) Abspalten der Benzylgruppe der Zwischenverbindung der Formel (IV), um eine Zwischenverbindung der Formel (V) zu erhalten: 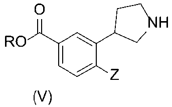 durch Reduktion unter Wasserstoffatmosphäre in Gegenwart eines Pd-Katalysators; und
c) Boc-Schützen des freien Stickstoffs der Zwischenverbindung der Formel (V), um eine Zwischenverbindung der Formel (VI) zu erhalten 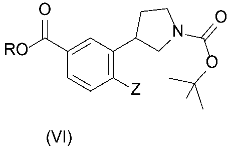 durch Umsetzung mit Di-*tert*-butyl-dicarbonat.

## Revendications

1. Composé de formule (I) 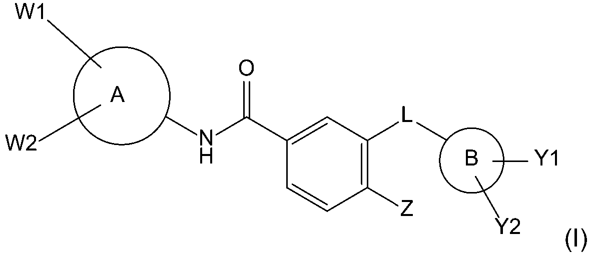 dans lequel
A est un cycle choisi dans le groupe constitué de : 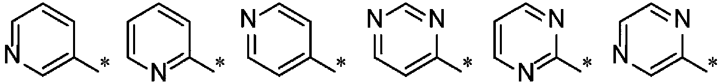 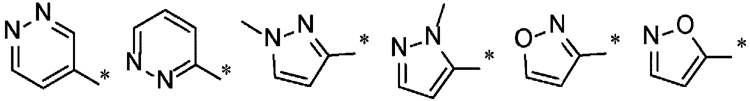 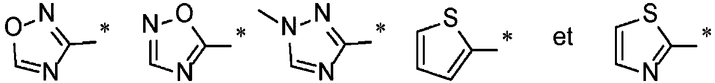
dans lequel 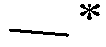 indique une liaison directe à NH ;
W1 et W2 sont des substituants du cycle A choisis dans le groupe constitué d'hydrogène, halogène, alkyle en (C₁-C₄), haloalkyle en (C₁-C₄), alcoxy en (C₁-C₄), haloalcoxy en (C₁-C₄), R1R2N-alkyle en (C₁-C₄) et cycloalkyle en (C₃-C₆) ;
Z est choisi dans le groupe constitué d'alkyle en (C₁-C₄), haloalkyle en (C₁-C₄), alcoxy en (C₁-C₄), haloalcoxy en (C₁-C₄), hydroxyalkyle en (C₁-C₄), hydroxy et cycloalkyle en (C₃-C₆) ;
L est choisi dans le groupe constitué de 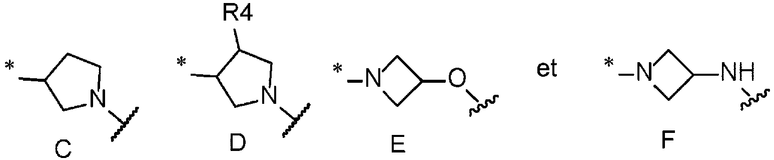
dans lequel 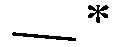 indique une liaison directe au cycle phényle et 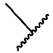 indique une liaison directe à B ;
B est un cycle hétéroaryle mono- ou bi-cyclique ;
Y1 et Y2 sont des substituants du cycle B indépendamment choisis dans le groupe constitué d'hydrogène, alkyle en (C₁-C₄), haloalkyle en (C₁-C₄), alcoxy en (C₁-C₄), alcoxy en (C₁-C₄)-alcoxy en (C₁-C₄), hydroxy-alcoxy en (C₁-C₄), hydroxy-alkyle en (C₁-C₄), alkyl en (C₁-C₄)-hétérocycloalkyl-alcoxy en (C₀-C₄), haloalcoxy en (C₁-C₄), halogène, cyano, cyano-alkyle en (C₁-C₄), cyano-alkyl en (C₁-C₄)-hétérocycloalkyle, CONR1R2, NHCOR1, NR1R2, R1R2N-alkyle en (C₁-C₄), hétérocycloalkyle, alkyl en (C₁-C₄)-hétérocycloalkyle, hétérocycloalkyl-alkyle en (C₁-C₄), alkyl en (C₁-C₄)-hétérocycloalkyl-carbonyle, alkyl en (C₀-C₄)-hétérocycloalkyl-carbonyle alkyl en (C₁-C₄)-phényle et alkyl en (C₁-C₄)-hétéroaryle monocyclique ;
R1 et R2 sont indépendamment choisis dans le groupe constitué d'hydrogène, alkyle en (C₁-C₄), hydroxyalkyle en (C₁-C₄), alcoxy en (C₁-C₄)-alkyle en (C₁-C₄), alkylamino en (C₁-C₄)-alkyle en (C₁-C₄), di-alkylamino en (C₁-C₄)-alkyle en (C₁-C₄), cycloalkyle en (C₃-C₆) facultativement substitué, hétérocycloalkyle facultativement substitué et hétérocycloalkyl-alcoxy en (C₁-C₄) facultativement substitué, dans lequel les substituants facultatifs sont un ou plusieurs et sont choisis parmi alkyle en (C₁-C₄), alcoxy en (C₁-C₄), haloalkyle en (C₁-C₄), hydroxy et haloalcoxy en (C₁-C₄) ;
R4 est choisi parmi hydroxy et hydroxyméthyle ;
ou stéréo-isomère, tautomère, solvate et sel pharmaceutiquement acceptable de celui-ci.

2. Composé de formule (I) selon la revendication 1, dans lequel
L est choisi dans le groupe constitué de 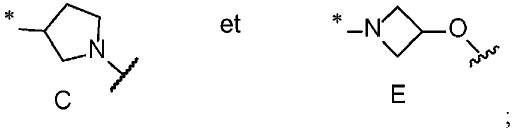
ou stéréo-isomère, tautomère, solvate et sel pharmaceutiquement acceptable de celui-ci.

3. Composé de formule (I) selon la revendication 1 ou 2, dans lequel
A est un cycle choisi dans le groupe constitué de : 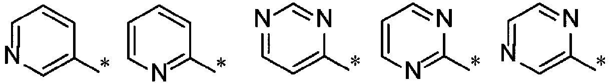 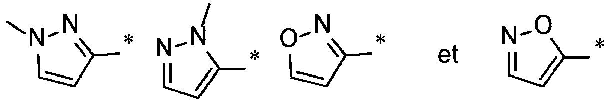
W1 et W2 sont indépendamment choisis dans le groupe constitué de H, F, CH₃, OCH₃, OCF₃, CF₃, C(CH₃)₃, CH₂CH₃, C(CH₃)₂CF₃, OCF₂H, CHF₂, CH₂CF₃, CH₂N(CH₃)₂ et cyclopropyle ;
Z est choisi dans le groupe constitué de CH₃, CF₂H, OCH₃, CH₂OH et cyclopropyle ;
B est choisi dans le groupe constitué de pyridinyle, pyrimidinyle, pyrazinyle, pyrazolo[1,5-a]pyrazinyle, 1H-pyrazolo[3,4-b]pyridinyle, pyrazolo[1,5-a]pyridinyle, pyrazolo[1,5-a]pyrimidinyle, imidazo[1,2-b]pyridazinyle, imidazo[1,2-a]pyrazinyle, imidazo[1,2-a]pyridinyle, thiéno[3,2-d]pyrimidinyle, 1H-pyrrolo[2,3-b]pyridinyle et pyrazolyle ;
Y1 est choisi dans le groupe constitué d'hydrogène, haloalkyle en (C₁-C₄), alcoxy en (C₁-C₄), cyano, CONR1R2, NHCOR1, NR1R2, alkyl en (C₁-C₄)-hétérocycloalkyl-carbonyle et alkyl en (C₁-C₄)-hétéroaryle monocyclique ; et Y2 est hydrogène ;
R1 et R2 sont indépendamment choisis dans le groupe constitué de CH₂CH₂N(CH₃)₂, CH₂CH₂OCH₃, CH₃, CH₂CH₂OH, oxétan-3-yle et 3-hydroxycyclobutyle ;
ou stéréo-isomère, tautomère, solvate et sel pharmaceutiquement acceptable de celui-ci.

4. Composé de formule (I) selon la revendication 3, dans lequel
Y1 est choisi dans le groupe constitué de CONH₂, CF₂H, OCH₃, cyano, NHCOCH₃, NH₂, 4-méthylpipérazine-1-carbonyle, 4-méthylpipérazin-1-yle et 1-méthyl-1H-pyrazol-4-yle ; et Y2 est hydrogène ;
ou stéréo-isomère, tautomère, solvate et sel pharmaceutiquement acceptable de celui-ci.

5. Composé selon l'une quelconque des revendications précédentes, dans lequel L est C 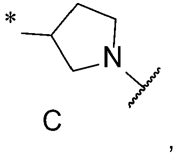 représenté par la formule (la) 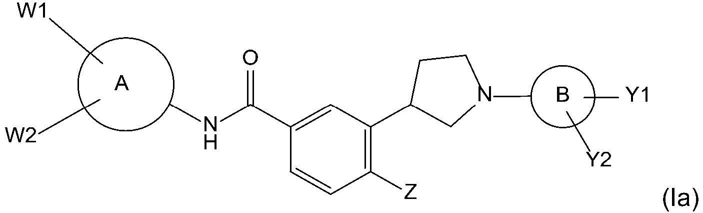 ou stéréo-isomère, tautomère, solvate et sel pharmaceutiquement acceptable de celui-ci.

6. Composé selon l'une quelconque des revendications précédentes, dans lequel L ou C est C* 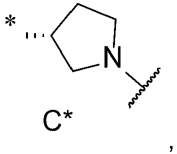 ou stéréo-isomère, tautomère, solvate et sel pharmaceutiquement acceptable de celui-ci.

7. Composé selon l'une quelconque des revendications 1 à 4, dans lequel L est 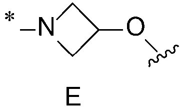 et qui est désigné en tant que composé de formule (lb1).

8. Composé de formule (la) selon la revendication 5 choisi dans le groupe constitué de :
(S)-5-(3-(2-méthyl-5-((5-(trifluorométhyl)pyridin-3-yl)carbamoyl)phényl)pyrrolidin-1-yl)nicotinamide (Exemple 1) ;
(R)-5-(3-(2-méthyl-5-((5-(trifluorométhyl)pyridin-3-yl)carbamoyl)phényl)pyrrolidin-1-yl)nicotinamide (Exemple 2) ;
(S)-5-(3-(5-((5-(tert-butyl)-1-méthyl-1H-pyrazol-3-yl)carbamoyl)-2-méthylphényl)pyrrolidin-1-yl)nicotinamide (Exemple 3) ;
(S)-5-(3-(2-méthyl-5-((5-(1,1,1-trifluoro-2-méthylpropan-2-yl)isoxazol-3-yl)carbamoyl)phényl)pyrrolidin-1-yl)nicotinamide (Exemple 4) ;
(S)-5-(3-(5-((3-(tert-butyl)-1-méthyl-1H-pyrazol-5-yl)carbamoyl)-2-méthylphényl)pyrrolidin-1-yl)nicotinamide (Exemple 5) ;
(S)-5-(3-(5-((3-(tert-butyl)isoxazol-5-yl)carbamoyl)-2-méthylphényl)pyrrolidin-1-yl)nicotinamide (Exemple 6) ;
(S)-N-(3-(tert-butyl)isoxazol-5-yl)-3-(1-(5-cyanopyridin-3-yl)pyrrolidin-3-yl)-4-méthylbenzamide (Exemple 7) ;
(S)-3-(1-(5-cyanopyridin-3-yl)pyrrolidin-3-yl)-4-méthyl-N-(6-(trifluorométhyl)pyrimidin-4-yl)benzamide (Exemple 8) ;
(S)-5-(3-(2-méthyl-5-((4-(trifluorométhoxy)pyridin-2-yl)carbamoyl)phényl)pyrrolidin-1-yl)nicotinamide (Exemple 9) ;
(S)-5-(3-(2-méthyl-5-((5-(trifluorométhoxy)pyridin-2-yl)carbamoyl)phényl)pyrrolidin-1-yl)nicotinamide (Exemple 10) ;
(S)-5-(3-(5-((5-(tert-butyl)isoxazol-3-yl)carbamoyl)-2-méthylphényl)pyrrolidin-1-yl)nicotinamide (Exemple 11) ;
(S)-4-méthyl-3-(1-(pyrimidin-5-yl)pyrrolidin-3-yl)-N-(4-(trifluorométhoxy)pyridin-2-yl)benzamide (Exemple 12) ;
(S)-N-(5-(tert-butyl)-1-méthyl-1H-pyrazol-3-yl)-4-méthyl-3-(1-(pyrimidin-5-yl)pyrrolidin-3-yl)benzamide (Exemple 13) ;
(S)-4-méthyl-3-(1-(pyrimidin-5-yl)pyrrolidin-3-yl)-N-(5-(1,1,1-trifluoro-2-méthylpropan-2-yl)isoxazol-3-yl)benzamide (Exemple 14) ;
(S)-4-méthyl-3-(1-(pyrimidin-5-yl)pyrrolidin-3-yl)-N-(4-(trifluorométhyl)pyridin-2-yl)benzamide (Exemple 15) ;
(S)-N-(5-(tert-butyl)isoxazol-3-yl)-4-méthyl-3-(1-(pyrimidin-5-yl)pyrrolidin-3-yl)benzamide (Exemple 16) ;
(S)-N-(3-(tert-butyl)-1-méthyl-1H-pyrazol-5-yl)-4-méthyl-3-(1-(pyrimidin-5-yl)pyrrolidin-3-yl)benzamide (Exemple 17) ;
(S)-4-méthyl-3-(1-(pyrimidin-5-yl)pyrrolidin-3-yl)-N-(5-(trifluorométhoxy)pyridin-3-yl)benzamide (Exemple 18) ;
(S)-N-(3-(tert-butyl)isoxazol-5-yl)-4-méthyl-3-(1-(pyrimidin-5-yl)pyrrolidin-3-yl)benzamide (Exemple 19) ;
(S)-4-méthyl-3-(1-(pyrimidin-5-yl)pyrrolidin-3-yl)-N-(5-(trifluorométhoxy)pyridin-2-yl)benzamide (Exemple 20) ;
(S)-N-(4-(difluorométhoxy)pyridin-2-yl)-4-méthyl-3-(1-(pyrimidin-5-yl)pyrrolidin-3-yl)benzamide (Exemple 21) ;
(S)-3-(1-(5-(difluorométhyl)pyridin-3-yl)pyrrolidin-3-yl)-4-méthyl-N-(5-(trifluorométhyl)pyridin-3-yl)benzamide (Exemple 22) ;
(S)-N-(2-(diméthylamino)éthyl)-5-(3-(2-méthyl-5-((5-(trifluorométhyl)pyridin-3-yl)carbamoyl)phényl)pyrrolidin-1-yl)nicotinamide (Exemple 23) ;
(S)-N-(2-méthoxyéthyl)-5-(3-(2-méthyl-5-((5-(trifluorométhyl)pyridin-3-yl)carbamoyl)phényl)pyrrolidin-1-yl)nicotinamide (Exemple 24) ;
(S)-3-(1-(2-méthoxypyrimidin-5-yl)pyrrolidin-3-yl)-4-méthyl-N-(5-(trifluorométhyl)pyridin-3-yl)benzamide (Exemple 25) ;
(S)-4-méthyl-3-(1-(pyrimidin-5-yl)pyrrolidin-3-yl)-N-(5-(trifluorométhyl)pyridin-3-yl)benzamide (Exemple 26) ;
(S)-3-(1-(5-cyanopyridin-3-yl)pyrrolidin-3-yl)-4-méthyl-N-(5-(trifluorométhyl)pyridin-3-yl)benzamide (Exemple 27) ;
(S)-3-(1-(5-acétamidopyridin-3-yl)pyrrolidin-3-yl)-4-méthyl-N-(5-(trifluorométhyl)pyridin-3-yl)benzamide (Exemple 28) ;
(S)-N-méthyl-5-(3-(2-méthyl-5-((5-(trifluorométhyl)pyridin-3-yl)carbamoyl)phényl)pyrrolidin-1-yl)nicotinamide (Exemple 29) ;
(S)-N-(2-hydroxyéthyl)-5-(3-(2-méthyl-5-((5-(trifluorométhyl)pyridin-3-yl)carbamoyl)phényl)pyrrolidin-1-yl)nicotinamide (Exemple 30) ;
(S)-3-(1-(3-aminopyrazin-2-yl)pyrrolidin-3-yl)-4-méthyl-N-(5-(trifluorométhyl)pyridin-3-yl)benzamide (Exemple 31) ;
(S)-4-méthyl-3-(1-(pyrazolo[1,5-a]pyrazin-3-yl)pyrrolidin-3-yl)-N-(5-(trifluorométhyl)pyridin-3-yl)benzamide (Exemple 32) ;
(S)-4-méthyl-3-(1-(pyrazin-2-yl)pyrrolidin-3-yl)-N-(5-(trifluorométhyl)pyridin-3-yl)benzamide (Exemple 33) ;
(S)-4-méthyl-3-(1-(pyrazolo[1,5-a]pyridin-3-yl)pyrrolidin-3-yl)-N-(5-(trifluorométhyl)pyridin-3-yl)benzamide (Exemple 34) ;
(S)-4-méthyl-3-(1-(pyrazolo[1,5-a]pyrimidin-3-yl)pyrrolidin-3-yl)-N-(5-(trifluorométhyl)pyridin-3-yl)benzamide (Exemple 35) ;
(S)-3-(1-(imidazo[1,2-b]pyridazin-3-yl)pyrrolidin-3-yl)-4-méthyl-N-(5-(trifluorométhyl)pyridin-3-yl)benzamide (Exemple 36) ;
(S)-3-(1-(imidazo[1,2-a]pyrazin-3-yl)pyrrolidin-3-yl)-4-méthyl-N-(5-(trifluorométhyl)pyridin-3-yl)benzamide (Exemple 37) ;
(S)-5-(3-(2-méthyl-5-((5-(trifluorométhyl)pyridin-3-yl)carbamoyl)phényl)pyrrolidin-1-yl)-N-(oxétan-3-yl)nicotinamide (Exemple 38) ;
N-((1s,3R)-3-hydroxycyclobutyl)-5-((S)-3-(2-méthyl-5-((5-(trifluorométhyl)pyridin-3-yl)carbamoyl)phényl)pyrrolidin-1-yl)nicotinamide (Exemple 39) ;
(S)-4-méthyl-3-(1-(5-(4-méthylpipérazine-1-carbonyl)pyridin-3-yl)pyrrolidin-3-yl)-N-(5-(trifluorométhyl)pyridin-3-yl)benzamide (Exemple 40) ;
(S)-3-(3-(2-méthyl-5-((5-(trifluorométhyl)pyridin-3-yl)carbamoyl)phényl)pyrrolidin-1-yl)-N-(oxétan-3-yl)isonicotinamide (Exemple 41) ;
(S)-3-(1-(2-aminopyridin-3-yl)pyrrolidin-3-yl)-4-méthyl-N-(5-(trifluorométhyl)pyridin-3-yl)benzamide (Exemple 42) ;
(S)-3-(1-(1H-pyrazolo[3,4-b]pyridin-5-yl)pyrrolidin-3-yl)-4-méthyl-N-(5-(trifluorométhyl)pyridin-3-yl)benzamide (Exemple 43) ;
(S)-6-(3-(5-((4-(difluorométhoxy)pyridin-2-yl)carbamoyl)-2-méthylphényl)pyrrolidin-1-yl)pyrazine-2-carboxamide (Exemple 51) ;
(S)-4-(3-(5-((5-(tert-butyl)-1-méthyl-1H-pyrazol-3-yl)carbamoyl)-2-méthylphényl)pyrrolidin-1-yl)picolinamide (Exemple 52) ;
(S)-4-(3-(5-((2-((diméthylamino)méthyl)-6-(trifluorométhyl)pyridin-4-yl)carbamoyl)-2-méthylphényl)pyrrolidin-1-yl)picolinamide (Exemple 53) ;
(S)-6-(3-(2-méthyl-5-((5-(trifluorométhyl)pyridin-3-yl)carbamoyl)phényl)pyrrolidin-1-yl)pyrimidine-4-carboxamide (Exemple 54) ;
(S)-3-(1-(4-cyanopyridin-3-yl)pyrrolidin-3-yl)-4-méthyl-N-(5-(1,1,1-trifluoro-2-méthylpropan-2-yl)isoxazol-3-yl)benzamide (Exemple 55) ;
(S)-3-(1-(4-cyanopyridin-3-yl)pyrrolidin-3-yl)-N-(5-éthylisoxazol-3-yl)-4-méthylbenzamide (Exemple 56) ;
(S)-4-(3-(2-méthyl-5-((4-(trifluorométhoxy)pyridin-2-yl)carbamoyl)phényl)pyrrolidin-1-yl)picolinamide (Exemple 57) ;
(*S*)-4-méthyl-3-(1-(pyrazin-2-yl)pyrrolidin-3-yl)-*N*-(5-(1,1,1-trifluoro-2-méthylpropan-2-yl)isoxazol-3-yl)benzamide (Exemple 58) ;
(*S*)-*N*-(3-cyclopropyl-1-méthyl-1*H*-pyrazol-5-yl)-4-méthyl-3-(1-(5-(1-méthyl-1*H*-pyrazol-4-yl)pyridin-3-yl)pyrrolidin-3-yl)benzamide (Exemple 59) ;
(R)-4-méthyl-3-(1-(5-(1-méthyl-1H-pyrazol-4-yl)pyridin-3-yl)pyrrolidin-3-yl)-N-(5-(1,1,1-trifluoro-2-méthylpropan-2-yl)isoxazol-3-yl)benzamide (Exemple 60) ;
(S)-4-méthyl-3-(1-(6-(morpholine-4-carbonyl)thiéno[3,2-d]pyrimidin-4-yl)pyrrolidin-3-yl)-N-(5-(trifluorométhyl)pyridin-3-yl)benzamide (Exemple 61) ;
(S)-4-méthyl-3-(1-(pyrazolo[1,5-a]pyrimidin-3-yl)pyrrolidin-3-yl)-N-(5-(trifluorométhoxy)pyridin-3-yl)benzamide (Exemple 62) ;
(S)-4-(3-(2-méthyl-5-((5-(trifluorométhyl)pyridin-3-yl)carbamoyl)phényl)pyrrolidin-1-yl)picolinamide (Exemple 63) ;
4-(3-(2-méthoxy-5-((5-(trifluorométhyl)pyridin-3-yl)carbamoyl)phényl)pyrrolidin-1-yl)picolinamide (Exemple 64) ;
(S)-1-méthyl-4-(3-(2-méthyl-5-((5-(trifluorométhyl)pyridin-3-yl)carbamoyl)phényl)pyrrolidin-1-yl)-1H-pyrazole-5-carboxamide (Exemple 65) ;
(S)-3-(1-(3-carbamoylphényl)pyrrolidin-3-yl)-4-méthyl-N-(5-(trifluorométhyl)pyridin-3-yl)benzamide (Exemple 66) ;
(S)-N-(4-(difluorométhyl)pyridin-2-yl)-4-méthyl-3-(1-(pyrimidin-5-yl)pyrrolidin-3-yl)benzamide (Exemple 67) ;
(S)-4-méthyl-N-(1-méthyl-3-(trifluorométhyl)-1H-pyrazol-5-yl)-3-(1-(pyrimidin-5-yl)pyrrolidin-3-yl)benzamide (Exemple 68) ;
(S)-N-(5-éthylisoxazol-3-yl)-4-méthyl-3-(1-(pyrimidin-5-yl)pyrrolidin-3-yl)benzamide (Exemple 69) ;
(S)-N-(3-cyclopropyl-1-méthyl-1H-pyrazol-5-yl)-4-méthyl-3-(1-(pyrimidin-5-yl)pyrrolidin-3-yl)benzamide (Exemple 70) ;
(S)-3-(1-(imidazo[1,2-a]pyridin-3-yl)pyrrolidin-3-yl)-4-méthyl-N-(5-(trifluorométhyl)pyridin-3-yl)benzamide (Exemple 71) ;
(S)-4-méthyl-3-(1-(6-(4-méthylpipérazin-1-yl)imidazo[1,2-a]pyridin-3-yl)pyrrolidin-3-yl)-N-(5-(trifluorométhyl)pyridin-3-yl)benzamide (Exemple 72) ;
(S)-3-(1-(6-(4-(cyanométhyl)pipérazin-1-yl)imidazo[1,2-a]pyridin-3-yl)pyrrolidin-3-yl)-4-méthyl-N-(5-(trifluorométhyl)pyridin-3-yl)benzamide (Exemple 73) ;
(S)-4-méthyl-3-(1-(5-(2-(4-méthylpipérazin-1-yl)éthoxy)pyridin-3-yl)pyrrolidin-3-yl)-N-(5-(trifluorométhyl)pyridin-3-yl)benzamide (Exemple 74) ;
(S)-3-(1-(4-cyanopyridin-3-yl)pyrrolidin-3-yl)-4-méthyl-N-(5-(trifluorométhyl)pyridin-3-yl)benzamide (Exemple 75) ;
(S)-3-(1-(4-méthoxypyridin-3-yl)pyrrolidin-3-yl)-4-méthyl-N-(5-(trifluorométhyl)pyridin-3-yl)benzamide (Exemple 76) ;
(S)-4-méthyl-3-(1-(6-(morpholinométhyl)imidazo[1,2-a]pyridin-3-yl)pyrrolidin-3-yl)-N-(5-(trifluorométhyl)pyridin-3-yl)benzamide (Exemple 77) ;
(S)-3-(1-(6-cyanoimidazo[1,2-a]pyridin-3-yl)pyrrolidin-3-yl)-4-méthyl-N-(5-(trifluorométhyl)pyridin-3-yl)benzamide (Exemple 78) ;
(S)-3-(1-(5-(2-méthoxyéthoxy)pyridin-3-yl)pyrrolidin-3-yl)-4-méthyl-N-(5-(trifluorométhyl)pyridin-3-yl)benzamide (Exemple 79) ;
(S)-4-méthyl-3-(1-(6-(4-méthylpipérazin-1-yl)imidazo[1,2-b]pyridazin-3-yl)pyrrolidin-3-yl)-N-(5-(trifluorométhyl)pyridin-3-yl)benzamide (Exemple 80) ;
(S)-4-méthyl-3-(1-(5-((1-méthylpipéridin-4-yl)oxy)pyridin-3-yl)pyrrolidin-3-yl)-N-(5-(trifluorométhyl)pyridin-3-yl)benzamide (Exemple 81) ;
(S)-4-méthyl-3-(1-(6-(4-méthylpipérazin-1-yl)imidazo[1,2-a]pyrazin-3-yl)pyrrolidin-3-yl)-N-(5-(trifluorométhyl)pyridin-3-yl)benzamide (Exemple 82) ;
(S)-4-méthyl-3-(1-(5-(4-méthylpipérazin-1-yl)pyrazolo[1,5-a]pyrimidin-3-yl)pyrrolidin-3-yl)-N-(5-(trifluorométhyl)pyridin-3-yl)benzamide (Exemple 83) ;
(S)-4-méthyl-3-(1-(6-morpholinoimidazo[1,2-b]pyridazin-3-yl)pyrrolidin-3-yl)-N-(5-(trifluorométhyl)pyridin-3-yl)benzamide (Exemple 84) ;
(S)-3-(1-(2-acétamidopyridin-4-yl)pyrrolidin-3-yl)-4-méthyl-N-(5-(trifluorométhyl)pyridin-3-yl)benzamide (Exemple 85) ;
(S)-4-méthyl-3-(1-(5-méthylpyrazolo[1,5-a]pyrimidin-3-yl)pyrrolidin-3-yl)-N-(5-(trifluorométhyl)pyridin-3-yl)benzamide (Exemple 86) ;
(S)-N-méthyl-4-(3-(2-méthyl-5-((5-(trifluorométhyl)pyridin-3-yl)carbamoyl)phényl)pyrrolidin-1-yl)picolinamide (Exemple 87) ;
(S)-4-méthyl-3-(1-(1-méthyl-1H-pyrazol-4-yl)pyrrolidin-3-yl)-N-(5-(trifluorométhyl)pyridin-3-yl)benzamide (Exemple 88) ;
(S)-4-méthyl-3-(1-(5-(morpholine-4-carbonyl)pyrazolo[1,5-a]pyridin-3-yl)pyrrolidin-3-yl)-N-(5-(trifluorométhyl)pyridin-3-yl)benzamide (Exemple 89) ;
4-méthyl-3-[(3R)-1-pyrazolo[1,5-a]pyrimidin-3-ylpyrrolidin-3-yl]-N-[5-(trifluorométhyl)-3-pyridyl]benzamide (Exemple 90) ;
N-[4-(difluorométhoxy)-2-pyridyl]-4-méthyl-3-[(3S)-1-pyrazolo[1,5-a]pyrimidin-3-ylpyrrolidin-3-yl]benzamide (Exemple 91) ;
N-[4-(difluorométhoxy)-2-pyridyl]-4-méthyl-3-[(3S)-1-pyrazin-2-ylpyrrolidin-3-yl]benzamide (Exemple 92) ;
3-[(3S)-1-(4-cyano-3-pyridyl)pyrrolidin-3-yl]-N-[4-(difluorométhoxy)-2-pyridyl]-4-méthyl-benzamide (Exemple 93) ;
(S)-3-(1-(6-cyanopyrazin-2-yl)pyrrolidin-3-yl)-N-(4-(difluorométhoxy)pyridin-2-yl)-4-méthyl-benzamide (Exemple 94) ;
(S)-N-(5-éthylisoxazol-3-yl)-4-méthyl-3-(1-(pyrazolo[1,5-a]pyrimidin-3-yl)pyrrolidin-3-yl)benzamide (Exemple 95) ;
(S)-N-(5-éthylisoxazol-3-yl)-4-méthyl-3-(1-(pyrazolo[1,5-a]pyrazin-3-yl)pyrrolidin-3-yl)benzamide (Exemple 96) ;
(S)-3-(1-(6-méthoxypyrazolo[1,5-a]pyrimidin-3-yl)pyrrolidin-3-yl)-4-méthyl-N-(5-(trifluorométhyl)pyridin-3-yl)benzamide (Exemple 97) ;
(S)-4-méthyl-3-(1-(pyrazolo[1,5-a]pyrazin-3-yl)pyrrolidin-3-yl)-N-(5-(trifluorométhoxy)pyridin-3-yl)benzamide (Exemple 98) ;
(S)-4-méthyl-3-(1-(2-(méthylamino)pyrimidin-5-yl)pyrrolidin-3-yl)-N-(5-(trifluorométhyl)pyridin-3-yl)benzamide (Exemple 99) ;
(S)-3-(1-(1H-pyrazol-4-yl)pyrrolidin-3-yl)-4-méthyl-N-(5-(trifluorométhyl)pyridin-3-yl)benzamide (Exemple 100) ;
(S)-3-(1-(3-((diméthylamino)méthyl)-1H-pyrazolo[3,4-b]pyridin-5-yl)pyrrolidin-3-yl)-4-méthyl-N-(5-(trifluorométhyl)pyridin-3-yl)benzamide (Exemple 101) ;
(S)-5-(3-(2-méthyl-5-((5-(trifluorométhyl)pyridin-3-yl)carbamoyl)phényl)pyrrolidin-1-yl)-N-(oxétan-3-yl)-1H-pyrrolo[2,3-b]pyridine-3-carboxamide (Exemple 102) ;
(S)-3-(1-(5-(2-hydroxyéthoxy)pyridin-3-yl)pyrrolidin-3-yl)-4-méthyl-N-(5-(trifluorométhyl)pyridin-3-yl)benzamide (Exemple 103) ;
3-(4-(hydroxyméthyl)-1-(pyrimidin-5-yl)pyrrolidin-3-yl)-4-méthyl-N-(5-(trifluorométhyl)pyridin-3-yl)benzamide (Exemple 104) ;
3-(4-hydroxy-1-(pyrimidin-5-yl)pyrrolidin-3-yl)-4-méthyl-N-(5-(trifluorométhyl)pyridin-3-yl)benzamide (Exemple 105) ;
(S)-4-méthyl-3-(1-(thiéno[2,3-d]pyrimidin-4-yl)pyrrolidin-3-yl)-N-(5-(trifluorométhyl)pyridin-3-yl)benzamide (Exemple 106) ;
(S)-4-méthyl-3-(1-(thiéno[3,2-d]pyrimidin-4-yl)pyrrolidin-3-yl)-N-(5-(trifluorométhyl)pyridin-3-yl)benzamide (Exemple 107) ;
(S)-N-méthyl-4-(3-(2-méthyl-5-((5-(trifluorométhyl)pyridin-3-yl)carbamoyl)phényl)pyrrolidin-1-yl)thiéno[3,2-d]pyrimidine-6-carboxamide (Exemple 108) ;
4-méthoxy-3-(1-(pyrimidin-5-yl)pyrrolidin-3-yl)-N-(5-(trifluorométhyl)pyridin-3-yl)benzamide, 1^{er} énantiomère élué (Exemple 109) ;
4-méthoxy-3-(1-(pyrimidin-5-yl)pyrrolidin-3-yl)-N-(5-(trifluorométhyl)pyridin-3-yl)benzamide, 2^{d} énantiomère élué (Exemple 110) ;
4-méthoxy-3-(1-(pyrimidin-5-yl)pyrrolidin-3-yl)-N-(5-(trifluorométhyl)pyridin-3-yl)benzamide, mélange racémique (Exemple 111) ;
4-méthoxy-3-(1-(pyrazolo[1,5-a]pyrimidin-3-yl)pyrrolidin-3-yl)-N-(5-(trifluorométhyl)pyridin-3-yl)benzamide (Exemple 112) ;
4-(difluorométhoxy)-3-(1-(pyrimidin-5-yl)pyrrolidin-3-yl)-N-(5-(trifluorométhyl)pyridin-3-yl)benzamide (Exemple 113) ;
4-méthoxy-3-(1-(pyrazolo[1,5-a]pyrimidin-3-yl)pyrrolidin-3-yl)-N-(5-(trifluorométhyl)pyridin-3-yl)benzamide, 1^{er} énantiomère élué (Exemple 114) ;
4-méthoxy-3-(1-(pyrazolo[1,5-a]pyrimidin-3-yl)pyrrolidin-3-yl)-N-(5-(trifluorométhyl)pyridin-3-yl)benzamide, 2^{d} énantiomère élué (Exemple 115) ;
4-(difluorométhoxy)-3-(1-(pyrimidin-5-yl)pyrrolidin-3-yl)-N-(5-(trifluorométhyl)pyridin-3-yl)benzamide, 1^{er} énantiomère élué (Exemple 116) ;
4-(difluorométhoxy)-3-(1-(pyrimidin-5-yl)pyrrolidin-3-yl)-N-(5-(trifluorométhyl)pyridin-3-yl)benzamide, 2^{d} énantiomère élué(Exemple 117) ;
4-(3-(2-(difluorométhoxy)-5-((5-(trifluorométhyl)pyridin-3-yl)carbamoyl)phényl)pyrrolidin-1-yl)picolinamide, 1^{er} énantiomère élué (Exemple 118) ;
4-(3-(2-(difluorométhoxy)-5-((5-(trifluorométhyl)pyridin-3-yl)carbamoyl)phényl)pyrrolidin-1-yl)picolinamide, 2^{d} énantiomère élué (Exemple 119) ;
4-(difluorométhyl)-3-(1-(pyrimidin-5-yl)pyrrolidin-3-yl)-N-(5-(trifluorométhyl)pyridin-3-yl)benzamide, (Exemple 120) ;
(1-(pyrimidin-5-yl)pyrrolidin-3-yl)-N-(5-(trifluorométhyl)pyridin-3-yl)benzamide, 2^{d} énantiomère élué (Exemple 121) ;
3-(4-hydroxy-1-(pyrimidin-5-yl)pyrrolidin-3-yl)-4-méthyl-N-(5-(trifluorométhyl)pyridin-3-yl)benzamide, 2^{d} énantiomère élué (Exemple 122) ;
(S)-(3-(1-(6-(hydroxyméthyl)imidazo[1,2-a]pyridin-3-yl)pyrrolidin-3-yl)-4-méthylphényl)(5-(trifluorométhyl)pyridin-3-yl)méthanone (Exemple 123) ;
N-(5-(tert-butyl)-1-méthyl-1H-pyrazol-3-yl)-4-(difluorométhyl)-3-(1-(pyrimidin-5-yl)pyrrolidin-3-yl)benzamide (Exemple 124) ;
(S)-3-(1-(6-bromothiéno[3,2-d]pyrimidin-4-yl)pyrrolidin-3-yl)-4-méthyl-N-(5-(trifluorométhyl)pyridin-3-yl)benzamide (Exemple 125) ;
(S)-4-méthyl-3-(1-(6-(4-méthylpipérazine-1-carbonyl)thiéno[3,2-d]pyrimidin-4-yl)pyrrolidin-3-yl)-N-(5-(trifluorométhyl)pyridin-3-yl)benzamide, (Exemple 126) ;
4-(3-(2-méthoxy-5-((5-(trifluorométhyl)pyridin-3-yl)carbamoyl)phényl)pyrrolidin-1-yl)-N-méthylpicolinamide, (Exemple 127) ;
(S)-3-(1-(imidazo[1,5-a]pyrazin-8-yl)pyrrolidin-3-yl)-4-méthyl-N-(5-(trifluorométhyl)pyridin-3-yl)benzamide (Exemple 128) ;
(S)-3-(1-(1H-pyrazolo [3,4-d]pyrimidin-4-yl)pyrrolidin-3-yl)-4-méthyl-N-(5-(trifluorométhyl)pyridin-3-yl)benzamide (Exemple 129) ;
(S)-3-(1-(2-acétamidopyrimidin-5-yl)pyrrolidin-3-yl)-4-méthyl-N-(5-(trifluorométhyl)pyridin-3-yl)benzamide (Exemple 130) ; et
(S)-N-(5-(tert-butyl)-1-méthyl-1H-pyrazol-3-yl)-4-méthyl-3-(1-(thiéno[3,2-d]pyrimidin-4-yl)pyrrolidin-3-yl)benzamide (Exemple 131).

9. Composé de formule (Ib1) selon la revendication 7, choisi dans le groupe constitué de :
3-(3-((6-aminopyridin-3-yl)oxy)azétidin-1-yl)-4-méthyl-N-(5-(trifluorométhyl)pyridin-3-yl)benzamide (Exemple 44) ;
3-(3-((6-acétamidopyridin-3-yl)oxy)azétidin-1-yl)-4-méthyl-N-(5-(trifluorométhyl)pyridin-3-yl)benzamide (Exemple 45) ;
3-(3-((6-(2-méthoxyacétamido)pyridin-3-yl)oxy)azétidin-1-yl)-4-méthyl-N-(5-(trifluorométhyl)pyridin-3-yl)benzamide (Exemple 46) ;
3-(3-((5-aminopyridin-3-yl)oxy)azétidin-1-yl)-4-méthyl-N-(5-(trifluorométhyl)pyridin-3-yl)benzamide (Exemple 47) ;
5-((1-(2-méthyl-5-((5-(trifluorométhyl)pyridin-3-yl)carbamoyl)phényl)azétidin-3-yl)oxy)nicotinamide (Exemple 132) ; et
3-(3-((5-acétamidopyridin-3-yl)oxy)azétidin-1-yl)-4-méthyl-N-(5-(trifluorométhyl)pyridin-3-yl)benzamide (Exemple 133).

10. Composition pharmaceutique comprenant un composé de formule (I) selon l'une quelconque des revendications 1 à 9, en mélange avec au moins un ou plusieurs véhicules et/ou excipients pharmaceutiquement acceptables.

11. Composition pharmaceutique selon la revendication 10 pour administration par inhalation.

12. Composition pharmaceutique selon la revendication 10 ou 11 pour utilisation en tant que médicament.

13. Composition pharmaceutique pour utilisation selon la revendication 12 dans la prévention et/ou le traitement d'une maladie, d'un trouble ou d'une affection, associé à une dysrégulation de DDR.

14. Composition pharmaceutique pour utilisation selon la revendication 12 ou 13 dans la prévention et/ou le traitement d'une fibrose et/ou de maladies, troubles ou affections qui impliquent une fibrose.

15. Composition pharmaceutique pour utilisation selon la revendication 14 dans la prévention et/ou le traitement d'une fibrose, y compris fibrose pulmonaire, fibrose pulmonaire idiopathique (FPI), fibrose hépatique, fibrose rénale, fibrose oculaire, fibrose cardiaque, fibrose artérielle et sclérose systémique.

16. Composition pharmaceutique pour utilisation selon la revendication 15 dans la prévention et/ou le traitement d'une fibrose pulmonaire idiopathique (FPI).

17. Composé intermédiaire choisi parmi le composé de formule (IV) 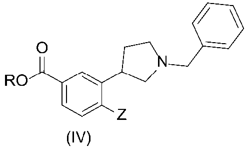 et le composé de formule (VI) 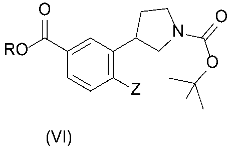 dans lequel R est alkyle en (C₁-C₄) et Z est choisi dans le groupe constitué d'alkyle en (C₁-C₄), haloalkyle en (C₁-C₄), alcoxy en (C₁-C₄), haloalcoxy en (C₁-C₄), hydroxyalkyle en (C₁-C₄) et cycloalkyle en (C₃-C₆), et dans lequel le composé de formule (VI) est dans sa forme racémique ou énantiomère, (R) ou (S).

18. Utilisation du composé intermédiaire de formule (IV) et/ou du composé intermédiaire de formule (VI), selon la revendication 17, dans la préparation d'un composé de formule (la) selon la revendication 5.

19. Procédé destiné à la préparation d'un composé de formule (la), selon la revendication 5, ou d'un sel pharmaceutiquement acceptable de celui-ci, comprenant l'étape consistant à :
a) faire réagir un composé de formule (III) 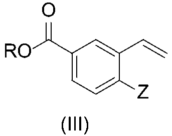 avec du N-benzyl-1-méthoxy-N-((triméthylsilyl)méthyl)méthanamine 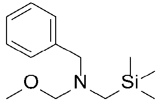 sous catalyse acide en présence d'un solvant de façon à obtenir un composé intermédiaire de formule (IV) 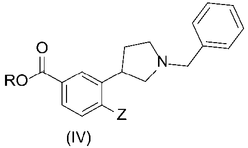 dans lequel R et Z sont tels que définis dans la revendication 17.

20. Procédé selon la revendication 19, comprenant en outre les étapes consistant à :
b) cliver le groupe benzyle du composé intermédiaire de formule (IV) de façon à obtenir un composé intermédiaire de formule (V) : 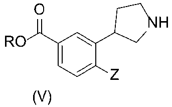 par réduction sous atmosphère d'hydrogène en présence d'un catalyseur au Pd ; et
c) protéger par Boc l'azote libre du composé intermédiaire de formule (V) de façon à obtenir un composé intermédiaire de formule (VI) 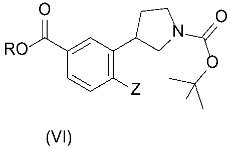 par réaction avec du di-*tert*-butyl-dicarbonate.
